(19) Europäisches Patentamt
European Patent Office
Office européen des brevets

(11) EP 1 354 954 A1

(12) EUROPEAN PATENT APPLICATION
published in accordance with Art. 158(3) EPC

(43) Date of publication:
**22.10.2003 Bulletin 2003/43**

(21) Application number: **01272513.1**

(22) Date of filing: **20.12.2001**

(51) Int Cl.[7]: **C12N 15/52**, C12P 7/04, C12N 1/19, C12N 1/21

(86) International application number:
**PCT/JP01/11213**

(87) International publication number:
**WO 02/053745 (11.07.2002 Gazette 2002/28)**

(84) Designated Contracting States:
**AT BE CH CY DE DK ES FI FR GB GR IE IT LI LU MC NL PT SE TR**

(30) Priority: **28.12.2000 JP 2000401701**

(71) Applicant: **Toyota Jidosha Kabushiki Kaisha**
**Toyota-Shi Aichi-ken 471-8561 (JP)**

(72) Inventors:
- **OHTO, Chikara,**
  **c/o Toyota Jidosha Kabushiki Kaisha**
  **Toyota-shi, Aichi 471-8571 (JP)**
- **OBATA, Shusei,**
  **c/o Toyota Jidosha Kabushiki Kaisha**
  **Toyota-shi, Aichi 471-8571 (JP)**

(74) Representative:
**Leson, Thomas Johannes Alois, Dipl.-Ing.**
**Patentanwälte**
**Tiedtke-Bühling-Kinne & Partner,**
**Bavariaring 4**
**80336 München (DE)**

# (54) PROCESS FOR PRODUCING PRENYL ALCOHOL

(57) The present invention provides a method of producing a prenyl alcohol, comprising creating a recombinant obtained by transferring into a host a recombinant DNA for expression or a DNA fragment for genomic integration each comprising:

(i) a hydroxymethylglutaryl-CoA reductase gene, an isopentenyl-diphosphate Δ-isomerase gene or a farnesyl-diphosphate synthase gene, or a mutant of any one of these genes,
(ii) a transcription promoter, and
(iii) a transcription terminator;

culturing the recombinant;
and recovering the prenyl alcohol from the resultant culture.

Fig.28

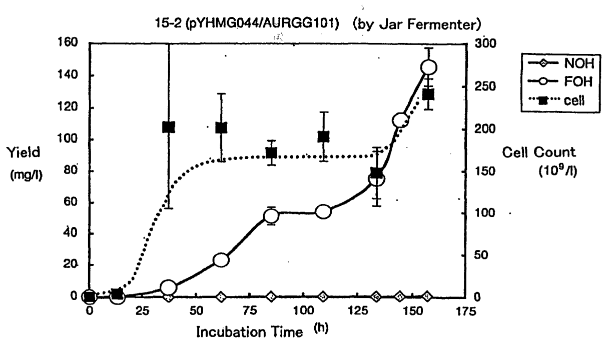


EP 1 354 954 A1

## Description

### TECHNICAL FIELD

The present invention relates to a method of producing prenyl alcohols.

### BACKGROUND ART

The biosynthesis of terpenoids (isoprenoids) begins with the synthesis of geranyl diphosphate (GPP; $C_{10}$), farnesyl diphosphate (FPP; $C_{15}$) and geranylgeranyl diphosphate (GGPP; $C_{20}$), which are straight chain prenyl diphosphates, through the sequential condensation reactions of isopentenyl diphosphate (IPP; $C_5$) with an allylic diphosphate substrate (Fig. 1). In Fig. 1, the abbreviations and words in boxes represent enzymes. Specifically, hmgR represents hydroxymethylglutaryl-CoA reductase; GGPS represents GGPP synthase; and FPS represents FPP synthase.

Among prenyl diphosphates, FPP is the most significant biosynthetic intermediate. It is a precursor for the synthesis of tremendous kinds of terpenoids, *e.g.* steroids including ergosterol (provitamin $D_2$), the side chains of quinone (vitamin K; VK), sesquiterpenes, squalene (SQ), the anchor molecules of farnesylated proteins, and natural rubber.

GGPP is also a key biosynthetic intermediate *in vivo,* and is essential for the biosynthesis of such compounds as retinol (vitamin A; VA), β-carotene (provitamin A), phylloquinone (vitamin $K_1$; $VK_1$), tocopherols (vitamin E; VE), the anchor molecules of geranylgeranylated proteins, the side chain of chlorophyll, gibberellins, and the ether lipid of Archaea.

Farnesol (FOH; $C_{15}$) and nerolidol (NOH; $C_{15}$), which are alcohol derivatives of FPP, and geranylgeraniol (GGOH; $C_{20}$), which is an alcohol derivative of GGPP, are known as fragrant substances in essential oils used as the ingredients of perfumes. FOH, NOH and GGOH are also important as the starting materials for the synthesis of various compounds (including the above-mentioned vitamins) useful as pharmacological agents (Fig. 1).

It is desired to establish a system in which a pure product of the so-called active-type prenyl alcohol, not a mixture containing isomers, can be produced in a large quantity.

Although it had been believed that all the biosynthesis of IPP is performed via the mevalonate pathway (a pathway in which IPP is synthesized from acetyl-CoA through mevalonate), M. Rohmer *et al.* elucidated a novel pathway for IPP synthesis using bacteria at the end of 1980's. This is called non-mevalonate pathway or DXP (1-deoxyxylulose 5-phosphate) pathway, in which IPP is synthesized from glyceraldehyde-3-phosphate and pyruvate through 1-deoxyxylulose 5-phosphate.

FOH and NOH are currently produced by chemical synthesis except for small amounts of them prepared from natural products such as essential oils. Chemically synthesized FOH and NOH generally have the same carbon skeletons, but they are obtained as mixtures of (E) type (*trans* type) and (*Z*) type (*cis* type) in double bond geometry. (*E, E*)-FOH or (*E*)-NOH, both of which are of *(all-E)* type, is the form synthesized in metabolic pathways in organisms and is industrially valuable. In order to obtain (*E, E*)-FOH or (*E*)-NOH in a pure form, refining by column chromatography, high precision distillation, *etc.* is necessary. However, it is difficult to carry out high precision distillation of FOH, a thermolabile allyl alcohol, or its isomer FOH. Also, the refining of these substances by column chromatography is not suitable in industrial practice since it requires large quantities of solvent and column packings as well as complicated operations of analyzing and recovering serially eluting fractions and removing the solvent; thus, this method is complicated and requires high cost. Under circumstances, it is desired to establish a method of biosynthesis of (*E, E*)-FOH (hereinafter, just referred to as "FOH") by controlling the production of (*E*)- and (*Z*)-geometrical isomers or by utilizing the repeat structure of reaction products. However, such a method has not been established yet. The substrates for FOH synthesis are provided via the mevalonate pathway in cells of, for example, *Saccharomyces cerevisiae,* a budding yeast. However, even when HMG-CoA reductase that is believed to be a key enzyme for FOH synthesis was used, it has only been discovered that the use of the reductase increases squalene synthesis ability (Japanese Unexamined Patent Publication No. 5-192184; Donald *et al.,* (1997) Appl. Environ. Microbiol. 63, 3341-3344). Further, even when a squalene synthase gene-deficient strain of a special budding yeast that had acquired sterol intake ability was cultured, accumulation of 1.3 mg of FOH per liter of culture broth was only revealed (Chambon *et al.*, (1990) Curr. Genet. 18, 41-46); no method of biosynthesis of (*E*)-NOH (hereinafter, just referred to as "NOH") has been known.

### DISCLOSURE OF THE INVENTION

It is an object of the invention to provide a method for producing a prenyl alcohol by culturing a recombinant prepared by transferring into a host cell a recombinant DNA for expression comprising an HMG-CoA reductase gene, an IPP Δ-isomerase gene or an FPP synthase gene, or a mutant of any one of these genes.

As a result of intensive and extensive researches toward solution of the above problems, the present inventors attempted to develop a prenyl alcohol production system by introducing into a host a gene of an enzyme involved in prenyl diphosphate synthesis. As the host, an unicellular eucaryote, in particular, yeast or procaryotes (such as bacterium, in particular, E. *coli)* that had been widely used in the fermentation industry from old times, that carries out the synthesis of prenyl diphosphate via the mevalonate pathway or DXP pathway; and that can be subjected to various genetic engineering techniques was used. In order to construct systems with which a gene of an enzyme involved in prenyl diphosphate synthesis (*e.g.*, HMG-CoA reductase gene) in yeast can be expressed artificially in a host cell, expression shuttle vectors were created which comprised a constitutive or inducible transcription promoter and various auxotrophic markers. Then, a gene of interest or a mutant thereof was inserted into these vectors, which were then introduced into various host cells. The inventors have succeeded in obtaining NOH or FOH from the culture of the resultant recombinant. Thus, the above-mentioned object has been achieved, and the present invention has been completed. When *E. coli* was used as a host, a gene of an enzyme involved in prenyl diphosphate synthesis (*e.g.*, FPP synthase gene or IPPΔ-isomerase gene) was introduced into the host cell using a conventional vector. Then, FOH was obtained from the culture of the resultant recombinant after dephosphorylation. Thus, the above-mentioned object has been achieved, and the present invention has been completed.

The present invention relates to a method of producing a prenyl alcohol(s), comprising creating a recombinant obtained by introducing into a host a recombinant DNA(s) for expression or a DNA fragment(s) for genomic integration each comprising:

(i) a hydroxymethylglutaryl-CoA reductase gene, an isopentenyl-diphosphate Δ-isomerase gene or a farnesyl-diphosphate synthase gene, or a mutant of any one of these genes,
(ii) a transcription promoter, and
(iii) a transcription terminator;

culturing the recombinant; and recovering the prenyl alcohol(s) from the resultant culture. Specific examples of the prenyl alcohol include $C_{15}$ prenyl alcohols such as FOH or NOH. Specific examples of the HMG-CoA reductase gene and mutant thereof include a gene encoding the amino acid sequence as shown in SEQ ID NO: 2, 4 or 6, or a deletion mutant thereof. For example, an HMG-CoA reductase gene comprising one nucleotide sequence selected from the group consisting of SEQ ID NOS: 1, 3, 5 and 7-16 may be given. Specific examples of the FPP synthase gene or mutant thereof include a gene encoding the amino acid sequence as shown in SEQ ID NO: 76, 78, 80, 82 or 84. For example, an FPP synthase gene comprising one nucleotide sequence selected from the group consisting of SEQ ID NOS: 75, 77, 79, 81 and 83 may be given. Specific examples of the IPPΔ-isomerase gene or mutant thereof include a gene encoding the amino acid sequence as shown in SEQ ID NO: 86. For example, an IPPΔ-isomerase gene comprising the nucleotide sequence as shown in SEQ ID NO: 85 may be given. As the transcription promoter, one selected from the group consisting *ofADH1* promoter, *TDH3 (GAP)* promoter, *PGK1* promoter, *TEF2* promoter, *GAL1* promoter and *tac* promoter may be used. Other transcription promoters may also be used which are functionally equivalent to these promoters in activity. As the transcription terminator, *ADH1* terminator or *CYC1* terminator may be used. Other transcription terminators may also be used which are functionally equivalent to these terminators in activity. As the host, yeast may be used, e.g. budding yeast such as *Saccharomyces cerevisia.* Specific examples of preferable *S. cerevisiae* strains include A451, YPH499, YPH500, W303-1A and W303-1B, or strains derived therefrom. Alternatively, a bacterium, e.g. *Escherichia coli* may be used. Specific examples of preferable *E. coli* strains include JM109 or strains derived therefrom.

According to the present invention, it is possible to produce a prenyl alcohol such as NOH or FOH at a concentration that cannot be achieved by merely culturing the untransformed host cell (at least 0.05 mg/L medium).

Further, the present invention relates to a recombinant obtained by transferring into a host a recombinant DNA for expression or a DNA fragment for genomic integration each comprising:

(i) a hydroxymethylglutaryl-CoA reductase gene, an isopentenyl-diphosphate Δ-isomerase gene or a farnesyl-diphosphate synthase gene, or a mutant of any one of these genes,
(ii) a transcription promoter, and
(iii) a transcription terminator,

the recombinant being capable of producing at least 0.05 mg/L of FOH or NOH. Specific examples of the host, the promoter and the terminator are the same as described above.

Hereinbelow, the present invention will be described in detail. The present specification encompasses the contents described in the specification and the drawings of Japanese Patent Application No. 2000-401701 based on which the present application claims priority.

The inventors have attempted to develop a system with which an active-type prenyl alcohol *(i.e.,* (*all-E*)-prenyl alcohol) can be produced in vivo, by using metabolic engineering techniques. Generally, FPP is synthesized by the catalytic action of farnesyl-diphosphate synthase (FPS) from IPP and DMAPP (3,3-dimethylallyl diphosphate) as substrates. Usually, this reaction does not proceed toward the synthesis of FOH, but proceeds toward the synthesis of squalene by squalene synthase, the synthesis of GGPP by geranygeranyl-diphosphate synthase, the synthesis of hexaprenyl diphosphate by hexaprenyl-diphosphate synthase, and so on (Fig. 1). In the present invention, transformant cells capable of producing not the usually expected squalene or major final products (sterols) but prenyl alcohols such as NOH and FOH not indicated in conventional metabolic pathway maps have been obtained by introducing into host cells an HMG-CoA reductase gene, FPP synthase gene or IPP Δ-isomerase gene that are believed to be involved in the activation of prenyl diphosphate synthesis via two different, independent pathways (the mevalonate pathway and DXP pathway) depending on organisms. Thus, biological, mass-production systems for prenyl alcohols have been developed. Furthermore, deletion mutants of HMG-CoA reductase gene with various patterns of deletions (Fig. 2) have been introduced into hosts in such a manner that the genes come under the control of a transcription promoter; or mutants of FPP synthase with amino acid substitutions have been introduced into hosts. Thus, biological, mass-production systems for the above-mentioned prenyl alcohols have been developed.

1. Preparation of Recombinant DNAs for Expression or DNA Fragments for Genomic Integration

In the present invention, the recombinant DNA for expression used in the transformation of hosts may be obtained by ligating or inserting a transcription promoter DNA and a transcription terminator DNA into a gene of interest to be expressed. Specifically, the gene to be expressed may be, for example, an HMG-CoA reductase genes *(e.g., HMG1*), *Escherichia coli* FPP synthase gene *ispA, Bacillus stearothermophilus* FPP synthase gene or IPPΔ-isomerase gene *idi* (ORF182) (hereinafter, referred to as an "HMG-CoA reductase gene or the like"). These genes can be isolated by cloning techniques using PCR or commercial kits.

It is also possible to prepare in advance a gene expression cassette comprising an HMG-CoA reductase gene or the like to which a transcription promoter and a transcription terminator have been ligated, and to incorporate the cassette into a vector. The ligation of the promoter and the terminator may be performed in any order. However, the promoter is ligated upstream of the HMG-CoA reductase gene or the like, and the terminator downstream of the gene. Alternatively, in the present invention, an HMG-CoA reductase gene or the like, a transcription promoter and a transcription terminator may be incorporated into an appropriate DNA, *e.g.* a vector, in succession. If the direction of transcription is properly considered, the incorporation may be performed in any order.

The DNA used for this purpose is not particularly limited as long as it may be retained in host cells hereditarily. Specific examples of DNA that may be used include plasmid DNA, bacteriophage, retrotransposon DNA and artificial chromosomal DNA (YAC: yeast artificial chromosome). With respect to recombinant DNA fragments for the gene expression by genomic integration, replication ability is not necessarily required in that DNA. The DNA fragments prepared by PCR or chemical synthesis may also be used.

Specific examples of useful plasmid DNA include YCp-type *E. coli*-yeast shuttle vectors such as pRS413, pRS414, pRS415, pRS416, YCp50, pAUR112 or pAUR123; YEp-type *E. coli*-yeast shuttle vectors such as pYES2 or YEp13; YIp-type *E. coli*-yeast shuttle vectors such as pRS403, pRS404, pRS405, pRS406, pAUR101 or pAUR135; E. *coli*-derived plasmids such as ColE plasmids (*e.g.*, pBR322, pBR325, pUC18, pUC19, pUC118, pUC119, pTV118N, pTV119N, pBluescript, pHSG298, pHSG396 or pTrc99A), p15A plasmids *(e.g.,* pACYC177 or pACYC184) and pSC101 plasmids *(e.g.,* pMW118, pMW119, pMW218 or pMW219); and *Bacillus subtilis-derived* plasmids (e.g., pUB110, pTP5). Specific examples of useful phage DNA include λ phage (Charon4A, Charon21A EMBL3, EMBL4, λgt10, λgt11, λZAP), ϕX174, M13mp18 and M13mp19. Specific examples of useful retrotransposon DNA include Ty factor. Specific examples of YAC vectors include pYACC2.

When recombinant DNAs are introduced into hosts, selection marker genes are used in many cases. However, the use of the marker genes are not necessarily required if there is an appropriate assay to select recombinants.

As the transcription promoter, a constitutive promoter or an inducible promoter may be used. The "constitutive promoter" means a transcription promoter of a gene involved in a major metabolic pathway. Such a promoter is believed to have transcription activity under any growth conditions. The "inducible promoter" means a promoter that has transcription activity only under specific growth conditions and whose activity is suppressed under other growth conditions.

Any transcription promoter may be used as long as it has activity in hosts such as yeast. For example, *GAL1* promoter, *GAL10* promoter, *TDH3* (GAP) promoter, *ADH1* promoter, *PGK1* promoter or *TEF2* promoter may be used to direct expression in yeast. To direct expression in *E. coli, trp* promoter, *lac* promoter, *trc* promoter or *tac* promoter may be used, for example.

The recombinant DNA may further comprise cis-elements such as an enhancer, a splicing signal, a poly A addition signal, selection markers, or the like, if desired. Specific examples of useful selection markers include marker genes such as *URA3, LEU2, TRP1* and *HIS3* that have non-auxotrophic phenotypes as indicators, and drug resistance genes such as $Amp^r$, $Tet^r$, $Cm^r$, $Km^r$ and *AUR1-C.*

A transcription terminator derived from any gene may be used as long as it has activity in hosts such as yeast.

For example, *ADH1* terminator or *CYC1* terminator may be used to direct the expression in yeast. To direct the expression in *E. coli, rrnB* terminator may be used, for example. It is also possible to incorporate an SD sequence (typically, 5'-AGGAGG-3') upstream of the initiation codon of the gene of a bacterium *(e.g., E. coli)* as a ribosome binding site for translation.

Expression vectors prepared in the present invention as recombinant DNAs for gene transfer may be designated and identified by indicating the name of the gene after the name of the plasmid used, unless otherwise noted. For example, when *HMG1* gene has been ligated to plasmid pRS434GAP having *TDH3* (GAP) promoter, the resultant plasmid is expressed as "pRS434GAP-HMG1". Except for special cases, this notational system applies to other expression vectors comprising other plasmids, promoters and genes.

In the present invention, an HMG-CoA reductase gene or the like may be a mutant in which a part of its regions (2217 nucleotides at the maximum) has been deleted, or a mutant that has deletion, substitution or addition of one or several to ten-odd nucleotides in the nucleotide sequence of a wild-type gene or a deletion mutant thereof With respect to amino acid sequences, an HMG-CoA reductase may be a deletion mutant in which 739 amino acids at the maximum have been deleted in the amino acid sequence of a wild-type HMG-CoA reductase (SEQ ID NO: 2), or it may be a mutant that has deletion, substitution or addition of one or several (*e.g.*, one to ten, preferably one to three) amino acids in the amino acid sequence of the wild-type enzyme or a deletion mutant thereof. Specifically, an HMG-CoA reductase gene may be a wild-type gene or a deletion mutant thereof as shown in Fig. 2B. Also, the amino acid sequence encoded by such a gene may have site-specific substitutiori(s) at one to ten sites as a result of nucleotide substitution (s), for example, as shown in Fig. 2A. An FPP synthase gene may also be a mutant that has deletion, substitution or addition of one or several to ten-odd nucleotides. Specifically, various mutant genes (SEQ ID NOS: 79, 81 and 83) each of which has substitution of five nucleotides in a wild-type FPP synthase gene (SEQ ID NO: 77) may be used. These mutant genes encode mutant enzymes in which the 79th amino acid residue Tyr of the wild-type FPP synthase (SEQ ID NO: 78) has been changed to Asp (SEQ ID NO: 80), Glu (SEQ ID NO: 82) or Met (SEQ ID NO: 84), respectively.

Substitution mutations of nucleotides that occur in DNA fragments obtained by amplifying wild-type DNA by PCR (polymerase chain reaction) using a DNA polymerase of low fidelity, such as Taq DNA polymerase, are called "PCR errors". In the present invention, for example, an HMG-CoA reductase gene in which encoded polypeptide has substitution mutations attributable to those nucleotide substitutions resulted from PCR errors when a wild-type HMG-CoA reductase gene (SEQ ID NO: 1) was used as a template may also be used. This HMG-CoA reductase gene is called *"HMG1'"*. An embodiment of nucleotide substitutions resulted from PCR errors when the wild-type HMG-CoA reductase gene (SEQ ID NO: 1) was used as a template is shown in Fig. 2A. *HMG1*' has the nucleotide sequence as shown in SEQ ID NO: 3, and the amino acid sequence encoded thereby is shown in SEQ ID NO: 4. In Fig. 2A, the mutations of nucleotides are expressed in the following order: the relevant nucleotide before substitution (in one letter abbreviation), the position of this nucleotide when the first nucleotide in the initiation codon of the HMG-CoA reductase gene is taken as position 1, and the nucleotide after substitution (in one letter abbreviation). The mutations of amino acids contained in the amino acid sequence of the PCR error-type HMG-CoA reductase are expressed in the following order: the relevant amino acid residue before substitution (in one letter abbreviation), the position of this amino acid in the HMG-CoA reductase, and the amino acid residue after substitution (in one letter abbreviation). Further, the PCR error-type nucleotide sequence described above may be corrected partially by techniques such as site-directed mutagenesis. Such a corrected HMG-CoA reductase gene may also be used in the invention. Further, those HMG-CoA reductase genes (including PCR error-type) may also be used in the invention that encode deletion mutants in which predicted transmembrane domains are deleted. For example, Fig. 2B shows examples of *HMG1Δ* genes that are deletion mutants of the PCR error-type HMG-CoA reductase gene *HMG1*' In Fig. 2B, the upper most row represents *HMG1'* gene without deletion. The portion indicated with thin solid line (—) is the deleted region. Table 1 below shows which region of *HMG1'* gene (SEQ ID NO: 3) has been deleted for each deletion mutant. Deletion mutants of *HMG1'* are expressed as *"HMG1Δxxy"* according to the deletion pattern, in which "xx" represents the deletion pattern and "y" a working number (any numerical figure). In Fig. 2B, "Δ026" is shown as one example of *HMG1*Δ02y. (Likewise, examples of other deletion patterns are also shown.)

Table 1. Embodiment of Deletions

| Designation of Deletion Mutant | Primer 1 | Primer 2 | Plasmid | Deletion of Predicted Transmembrane Domains | Deleted Region | Sequence after Deletion |
|---|---|---|---|---|---|---|
| HMG1 Δ 02y | HMG1(558-532) | HMG1(799-825) | pYHMG02X | #2-#3 | Nucleotide positions 559-798 | SEQ ID NO: 7 |
| HMG1 Δ 04y | HMG1(1191-1165) | HMG1(1267-1293) | pYHMG04X | #6 | Nucleotide positions 1192-1266 | SEQ ID NO: 8 |
| HMG1 Δ 05y | HMG1(1380-1354) | HMG1(1573-1599) | pYHMG05X | #7 | Nucleotide positions 1381-1572 | SEQ ID NO: 9 |
| HMG1 Δ 06y | HMG1(558-532) | HMG1(1267-1293) | pYHMG06X | #2-#6 | Nucleotide positions 559-1266 | SEQ ID NO: 10 |
| HMG1 Δ 07y | HMG1(558-532) | HMG1(1573-1599) | pYHMG07X | #2-#7 | Nucleotide positions 559-1572 | SEQ ID NO: 11 |
| HMG1 Δ 08y | HMG1(27-1) | HMG1(1573-1599) | pYHMG08X | #1-#7 | Nucleotide positions 27-1572 | SEQ ID NO: 12 |
| HMG1 Δ 10y | HMG1(27-1) | HMG1(1816-1842) | pYHMG10X | #1-#7(-605 aa) | Nucleotide positions 27-1815 | SEQ ID NO: 13 |
| HMG1 Δ 11y | HMG1(27-1) | HMG1(1891-1917) | pYHMG11X | #1-#7(-631 aa) | Nucleotide positions 27-1890 | SEQ ID NO: 14 |
| HMG1 Δ 12y | HMG1(27-1) | HMG1(1990-2016) | pYHMG12X | #1-#7(-663 aa) | Nucleotide positions 27-1989 | SEQ ID NO: 15 |
| HMG1 Δ 13y | HMG1(27-1) | HMG1(2218-2244) | pYHMG13X | #1-#7(-739 aa) | Nucleotide positions 27-2217 | SEQ ID NO: 16 |

| | Primer Sequence | |
|---|---|---|
| HMG1(27-1) | 5' TTT CAG TCC CTT GAA TAG CGG CGG CAT 3' | SEQ ID NO: 38 |
| HMG1(558-532) | 5' GTC TGC TTG GGT TAC ATT TTC TGA AAA 3' | SEQ ID NO: 39 |
| HMG1(799-825) | 5' CAC AAA ATC AAG ATT GCC CAG TAT GCC 3' | SEQ ID NO: 40 |
| HMG1(1191-1165) | 5' AGA AGA TAC GGA TTT CTT TTC TGC TTT 3' | SEQ ID NO: 41 |
| HMG1(1267-1293) | 5' AAC TTT GGT GCA AAT TGG GTC AAT GAT 3' | SEQ ID NO: 42 |
| HMG1(1380-1354) | 5' TTG CTC TTT AAA GTT TTC AGA GGC ATT 3' | SEQ ID NO: 43 |
| HMG1(1573-1599) | 5' CAT ACC AGT TAT ACT GCA GAC CAA TTG 3' | SEQ ID NO: 44 |
| HMG1(1816-1842) | 5' GCA TTA TTA AGT AGT GGA AAT ACA AAA 3' | SEQ ID NO: 45 |
| HMG1(1891-1917) | 5' CCT TTG TAC GCT TTG GAG AAA AAA TTA 3' | SEQ ID NO: 46 |
| HMG1(1990-2016) | 5' TCT GAT CGT TTA CCA TAT AAA AAT TAT 3' | SEQ ID NO: 47 |
| HMG1(2218-2244) | 5' AAG GAT GGT ATG ACA AGA GGC CCA GTA 3' | SEQ ID NO: 48 |

2. Preparation of Recombinants

The recombinant of the invention can be obtained by introducing into a host the recombinant DNA of the invention in such a manner that the HMG-CoA reductase gene or the like (including various mutants; the same applies to the rest of the present specification unless otherwise noted) can be expressed. The host used in the invention is not particularly limited. Any host may be used as long as it can produce a prenyl alcohol(s). Preferably, *E. coli* or yeast is used.

In the present invention, the recombinant DNA comprising a promoter, an HMG-CoA reductase gene or the like, and a terminator may be introduced into fungi including unicellular eucaryotes such as yeast; procaryotes such as *E. coli;* animal cells; plant cells; *etc.* to obtain recombinants.

Fungi useful in the invention include *Myxomycota*, *Phycomycetes, Ascomycota*, *Basidiomycota*, and *Fungi Imperfecti.* Among fungi, some unicellular eucaryotes are well known as yeast that is important in industrial applicability. For example, yeast belonging to *Ascomycota,* yeast belonging to *Basidiomycota,* or yeast belonging to *Fungi Imperfecti* may be enumerated. Specific examples of yeast include yeast belonging to Ascomycota, in particular, budding yeast such as *Saccharomyces cerevisiae* (known as Baker's yeast), *Candida utilis* or *Pichia pastris;* and fission yeast such as *Shizosaccharomyces pombe.* The yeast strain is not particularly limited as long as it can produce a prenyl alcohol (s). In the case of *S. cerevisiae,* specific examples of useful strains include A451, EUG8, EUG12, EUG27, YPH499, YPH500, W303-1A, W303-1B and AURGG101 strains as shown below. As a method for introducing the recombinant DNA into yeast, such method as electroporation, the spheroplast method, or the lithium acetate method may be employed.

A451 (ATCC200589; *MATa can1 leu2 trp1 ura3 aro7)*
YPH499 (ATCC76625; *MATa ura*3-52 *lys*2-801 *ade2*-101 *trp1*-Δ63 *his3*-Δ200 *leu2*-Δ1; Stratagene, La Jolla, CA)
YPH500 (ATCC76626; *MATa ura3*-52 *lys2*-801 *ade2*-101 *trp1*-Δ63 *his3*-Δ200 *leu2*-Δ1; Stratagene)
W303-1A (MATa leu2-3 leu*2*-112 *his3*-11 ade2-1 *ura3*-1 *trp1*-1 *can1*-100)
W303-1B *(MATa leu2*-3 *leu2*-112 *his3*-11 *ade2*-1 *ura3*-1 *trp1*-1 can1-100)
AURGG101 (A451, *aur1::AUR1-C)*
EUG8 (A451, *ERG9p:: URA3-GAL1p)*
EUG12 (YPH499, *ERG9p::URA3-GAL1p)*
EUG27 (YPH500, *ERG9p::URA3-GAL1P)*

As prokaryotes, archaea and bacteria may be enumerated. As archaea, methane producing microorganisms such as *Metanobacterium;* halophilic microorganisms such as Halobacterium, thermophilic acidophilic microorganisms such as *Sulfolobus,* may be enumerated. As bacteria, various Gram-negative or Gram-positive bacteria that are highly valuable in industrial or scientific applicability may be enumerated, e.g. *Escherichia* such as *E. coli*, *Bacillus* such as *B. subtilis* or *B. brevis, Pseudomonas* such as *P. putida*, *Agrobacterium* such as *A. tumefaciens* or *A. rhizogenes, Corynebacterium* such as *C.* glutamicum, *Lactobacillus* such as *L. plantarum,* and *Actinomycetes* such as *Actinomyces* or *Streptmyces.*

When a bacterium such as *E. coli* is used as a host, the recombinant DNA of the invention is preferably not only capable of autonomous replication in the host but also composed of a promoter, an SD sequence as a ribosome RNA binding site, and the gene of the invention. A transcription terminator may also be inserted appropriately. The recombinant DNA may also contain a gene that controls the promoter. Specific examples of *E. coli* strains include, but are not limited to, BL21, DH5a, HB101, JM101, MBV1184, TH2, XL1-Blue and Y-1088. As the transcription promoter, any promoter may be used as long as it can direct the expression of a gene in a host such as *E. coli.* For example, an *E. coli*- or phage-derived promoter such as *trp* promoter, *lac* promoter, PL promoter or $P_R$ promote may be used. An artificially altered promoter such as *tac* promoter may also be used. As a method for introducing the recombinant DNA into a bacterium, any method of DNA transfer into bacteria may be used. For example, a method using calcium ions, electroporation, or a method using a commercial kit may be employed.

Whether the gene of the invention has been transferred into the host cell or not can be confirmed by such methods as PCR or Southern blot hybridization. For example, DNA is prepared from the resultant recombinant, designed a primer(s) specific to the introduced DNA and subjected to PCR. Subsequently, the amplified product is subjected to agarose gel electrophoresis, polyacrylamide gel electrophoresis or capillary electrophoresis, followed by staining with ethidium bromide, SYBR Green solution or the like, or detection of DNA with a UV detector. Thus, by detecting the amplified product as a single band or peak, the introduced DNA can be confirmed. Alternatively, PCR may be performed using a primer(s) labeled with a fluorescent dye or the like to detect the amplified product.

3. Production of Prenyl Alcohols

In the present invention, a prenyl alcohol(s) can be obtained by culturing the above-described recombinant comprising a transferred HMG-CoA reductase gene or the like, and recovering the prenyl alcohol(s) from the resultant culture. The term "culture" used herein means any of the following materials: culture supernatant, cultured cells or microorganisms *per se,* or disrupted products from cultured cells or microorganisms. The recombinant of the invention is cultured by conventional methods used in the culture of hosts. As the prenyl alcohol, $C_{15}$ prenyl alcohols such as farnesol (FOH) or nerolidol (NOH) may be enumerated. These prenyl alcohols are accumulated in the culture independently or as a mixture.

As a medium to culture the recombinant obtained from a microorganism host, either a natural or synthetic medium may be used as long as it contains carbon sources, nitrogen sources and inorganic salts assimilable by the microorganism and is capable of effective cultivation of the recombinant. As carbon sources, carbohydrates such as glucose, galactose, fructose, sucrose, raffinose, starch; organic acids such as acetic acid, propionic acid; and alcohols such as ethanol and propanol may be used. As nitrogen sources, ammonia; ammonium salts of inorganic or organic acids such as ammonium chloride, ammonium sulfate, ammonium acetate, ammonium phosphate; other nitrogen-containing compounds; Peptone; meat extract; corn steep liquor, various amino acids, etc. may be used. As inorganic substances, potassium dihydrogen phosphate, dipotassium hydrogen phosphate, magnesium phosphate, magnesium sulfate, sodium chloride, iron(II) sulfate, manganese sulfate, copper sulfate, calcium carbonate and the like may be used. Usually, the recombinant is subjected to shaking culture or aeration agitation culture under aerobic conditions at 26 to 36 °C. Preferably, when the host is *S. cerevisiae,* the recombinant is cultured at 30°C for 2 to 7 days. When the host is *E. coli,* the recombinant is cultured at 37°C for 12 to 18 hours. The adjustment of pH is carried out using an inorganic or organic acid, an alkali solution or the like. During the cultivation, antibiotics such as ampicillin, chloramphenicol or aureobasidin A may be added to the medium if necessary.

When a recombinant incorporating an expression vector containing an inducible transcription promoter is cultured, an inducer may be added to the medium if necessary. For example, when *GAL1* promoter was used, galactose may be used as a carbon source. When a microorganism (e.g., *E. coli)* transformed with an expression vector containing a promoter that is inducible by isopropyl-β-D-thiogalactopyranoside (IPTG) is cultured, IPTG may be added to the medium.

When cultured under the above-described conditions, the recombinant of the invention can produce prenyl alcohol(s) at high yield(s). In particular, when the host is AURGG101 and the vector is pYHMG044, the recombinant can produce 32 mg or more of prenyl alcohols per liter of the medium. It can produce even 150 mg/L or more depending on the culture conditions.

In the present invention, it is possible to increase the production efficiency of prenyl alcohols by adding to the above-described medium such substances as terpenoids, oils, or surfactants. Specific examples of these additives include the following.

Terpenoids: squalene, tocopherol, IPP, DMAPP
Oils: soybean oil, fish oil, almond oil, olive oil
Surfactants: Tergitol, Triton X-305, Span 85, Adekanol LG109 (Asahi Denka), Adekanol LG294 (Asahi Denka), Adekanol LG295S (Asahi Denka), Adekanol LG297 (Asahi Denka), Adekanol B-3009A (Asahi Denka), Adekapluronic L-61 (Asahi Denka).

The concentrations of oils are 0.01% or more, preferably 1-3%. The concentrations of surfactants are 0.005-1%, preferably 0.05-0.5%. The concentrations of terpenoids are 0.01% or more, preferably 1-3%.

After the cultivation, the prenyl alcohol of interest is recovered by disrupting the microorganisms or cells by, *e.g.*, homogenizing, when the alcohol(s) is produced within the microorganisms or cells. Alternatively, the alcohol(s) may be extracted directly using organic solvents without disrupting the cells. When the prenyl alcohol(s) of the invention is produced outside the microorganisms or cells, the culture broth is used as it is or subjected to centrifugation or the like to remove the microorganisms or cells. Thereafter, the prenyl alcohol(s) of interest is extracted from the culture by, *e.g.,* extraction with an organic solvent. If necessary, the alcohol(s) may be further isolated and purified by various types of chromatography or the like.

In the present invention, preferable combinations of host strains and vectors as recombinant DNAs, as well as relationships between these combinations and yields of prenyl alcohols are as illustrated in Table 2 below.

Table 2

| Promoter | Gene | Host | FOH Yield (mg/L) | NOH Yield (mg/L) |
|---|---|---|---|---|
| GAP | *HMG1* | *S. cerevisiae* A451 | 0.05, 0.65-11.2, 4.9-11.2 | 0.05, 0.05-0.16, 0.16 |
| GAP | *HMG1* | *S. cerevisiae* EUG8(from A451) | 0.2, 0.20-1.8, 1.8 | –, –, – |
| ADH,GAP,PGK,TEF | *HMG1* | *S. cerevisiae* YPH499 | 0.05, 0.05-0.11, 0.11 | –, –, – |
| GAP | *HMG1* | *S. cerevisiae* EUG12(from YPH499) | 5.9, 5.9-18.3, 18.3 | 0.13, 0.13-0.30, 0.30 |
| PGK,TEF | *HMG1* | *S. cerevisiae* YPH500 | –, –, – | –, –, – |
| GAP | *HMG1* | *S. cerevisiae* EUG27(from YPH500) | 3.2, 3.2-13.6, 13.6 | 0.05, 0.05-0.22, 0.22 |
| GAP | *HMG1* | *S. cerevisiae* W303-1A | –, –, – | –, –, – |
| GAP | *HMG1* | *S. cerevisiae* W303-1B | –, –, – | –, –, – |
| GAL | *HMG1'* | *S. cerevisiae* A451 | –, –, – | 0.05, –, – |
| GAL | *HMG1'* | *S. cerevisiae* AURGG101(from A451) | 0.05, 0.29-8.2, 8.2 | 0.05, 0.095-2.7, 2.7 |
| GAL | *HMG1'* | *S. cerevisiae* YPH499 | 0.05, 0.05-0.057, 0.057 | –, –, – |
| GAL | *HMG1'* | *S. cerevisiae* YPH500 | –, –, – | –, –, – |
| GAL | *HMG1'* | *S. cerevisiae* W303-1A | –, –, – | 0.05, 0.10-0.15, 0.15 |
| GAL | *HMG1'* | *S. cerevisiae* W303-1B | –, –, – | 0.05, 0.091-0.14, 0.14 |
| GAL | *HMG04y* | *S. cerevisiae* A451 | 0.05, 0.22-0.51, 0.51 | 0.05, 0.05-0.058, 0.058 |
| GAL | *HMG04y* | *S. cerevisiae* AURGG101(from A451) | 0.05, 0.05-158, 53-158 | 0.05, 0.05-23, 2.4-23 |
| GAL | *HMG04y* | *S. cerevisiae* YPH499 | –, –, – | –, –, – |
| GAL | *HMG04y* | *S. cerevisiae* YPH500 | –, –, – | –, –, – |
| GAL | *HMG04y* | *S. cerevisiae* W303-1A | –, –, – | –, –, – |
| GAL | *HMG04y* | *S. cerevisiae* W303-1B | –, –, – | –, –, – |
| GAL | *HMGxxy* | *S. cerevisiae* A451 | 0.05, 0.05-0.21, 0.21 | 0.05, 0.05-0.12, 0.12 |
| GAL | *HMGxxy* | *S. cerevisiae* AURGG101(from A451) | 0.05, 0.05-0.13, 0.13 | 0.05, 0.05-0.11, 0.11 |
| GAL | *HMGxxy* | *S. cerevisiae* YPH499 | –, –, – | –, –, – |
| GAL | *HMGxxy* | *S. cerevisiae* YPH500 | –, –, – | –, –, – |
| GAL | *HMGxxy* | *S. cerevisiae* W303-1A | –, –, – | –, –, – |
| GAL | *HMGxxy* | *S. cerevisiae* W303-1B | –, –, – | –, –, – |
| GAP&GAL | *HMG&HMG04* | *S. cerevisiae* AURGG101(from A451) | 22, 22-66, 66 | 12, 12-28, 28 |
| | *ispA* | *E. coli* JM109 | 11, 11-93, 73-93 | –, –, – |
| | *fps* | *E. coli* JM109 | 12, –, – | –, –, – |
| | *ispA & idi* | *E. coli* JM109 | 0.15, 0.15-0.16, – | –, –, – |

In the columns of FOH yield and NOH yield, lower limit, preferable range and more preferable range are shown in this order from the left side.
Mark " – " means no data.

From Table 2, the following yields can be presented, for example.

(1) When a DNA comprising *HMG1* or a mutant thereof (e.g., *HMG1 ') or* a deletion mutant of this mutant (*HMG*xxy) ligated downstream of a constitutive promoter had been introduced into *S. cerevisiae* cells, the cells produced FOH at least at 0.05 mg/L, preferably at 0.05-18.3 mg/L, and produced NOH at least at 0.05 mg/L, preferably at 0.05-0.3 mg/L.

(2) When a DNA comprising *HMG1* or a mutant thereof (e.g., *HMG1')* or a deletion mutant of this mutant (*HMG*xxy) ligated downstream of an inducible promoter had been introduced into *S. cerevisiae* cells, the cells produced FOH at least at 0.05 mg/L, preferably at 0.05-158 mg/L, more preferably at 53-158 mg/L, and produced NOH at least at 0.05 mg/L, preferably at 0.05-23 mg/L, more preferably at 2.4-23 mg/L.

(3) When two DNAs comprising *HMG1* ligated downstream of a constitutive promoter and *HMG*04y (a deletion mutant of *HMG1'*) ligated downstream of an inducible promoter, respectively, had been introduced into S. *cerevisiae* cells, the cells produced FOH at least at 22 mg/L, preferably at 22-66 mg/L, and produced NOH at least at 12 mg/L, preferably at 12-28 mg/L.

(4) When a DNA comprising *HMG1* or a mutant thereof (e.g., *HMG1'*) or a deletion mutant of this mutant (*HMG*xxy) had been introduced into *S. cerevisiae* A451 cells or A451-derived cells, the cells produced FOH at least at 0.05 mg/L, preferably at 0.05-158 mg/L, more preferably at 53-158 mg/L, and produced NOH at least at 0.05 mg/L, preferably at 0.05-23 mg/L, more preferably at 2.4-23 mg/L.

(5) When a DNA comprising *HMG1* or a mutant thereof (e.g., *HMG1')* or a deletion mutant of this mutant (*HMG*xxy) had been introduced into *S. cerevisiae* YPH499 cells or YPH499-derived cells, the cells produced FOH at least at 0.05 mg/L, preferably at 0.05-18.3 mg/L, more preferably at 5.9-18.3 mg/L, and produced NOH at least at 0.05 mg/L, preferably at 0.13-0.30 mg/L.

(6) When a DNA comprising *HMG1* or a mutant thereof (e.g., *HMG1')* or a deletion mutant of this mutant (*HMG*xxy) had been introduced into *S. cerevisiae* YPH500 cells or YPH500-derived cells, the cells produced FOH at least at 3.2 mg/L, preferably at 3.2-13.6 mg/L, and produced NOH at least at 0.05 mg/L, preferably at 0.05-0.22 mg/L.

(7) When a DNA comprising *HMG1* or a mutant thereof *(e.g., HMG1')* had been introduced into *S. cerevisiae* cells, the cells produced FOH at least at 0.05 mg/L, preferably at 0.05-18.3 mg/L, and produced NOH at least at 0.05 mg/L, preferably at 0.05-2.7 mg/L.

(8) When a DNA comprising *HMG*xxy (a deletion mutant of *HMG1')* had been introduced into *S. cerevisiae* cells, the cells produced FOH at least at 0.05 mg/L, preferably at 0.05-158 mg/L, more preferably at 53-158 mg/L, and produced NOH at least at 0.05 mg/L, preferably at 0.05-23 mg/L, more preferably at 2.4-23 mg/L.

(9) A plasmid comprising a substitution mutant of *E. coli* FPP synthase gene *ispA* was introduced into *E. coli.* When the resultant cells were cultured in a liquid medium containing IPP and DMAPP and then treated with phosphatase, the cells produced FOH at least at 11 mg/L, preferably at 11-90 mg/L, more preferably at 64-90 mg/L.

(10) When *ispA* and *idi* had been introduced into *E. coli,* the cells produced FOH at least at 0.15 mg/L, preferably at 0.15-0.16 mg/L, as a result of phosphatase treatment even without the addition of IPP and DMAPP.

BRIEF DESCRIPTION OF THE DRAWINGS

Fig. 1 is a diagram showing a metabolic pathway in which mevalonate pathway-related enzymes are involved.
Fig. 2A is a diagram showing construction of deletion mutants of *HMG1* gene.
Fig. 2B shows patterns of substitution mutations.
Fig. 3 is a diagram showing plasmid pRS414.
Fig. 4 is a diagram showing plasmid pYES2.
Fig. 5 is a diagram showing sequences *for ADH1* promoter and terminator.
Fig. 6A is a diagram showing plasmid pRS414PTadh.
Fig. 6B is a diagram showing plasmid pRS414TPadh.

Fig. 7A-1 is a diagram showing plasmid pRS434ADH.
Fig. 7A-2 is a diagram showing plasmid pRS434GAP.
Fig. 7B-1 is a diagram showing plasmid pRS434PGK.
Fig. 7B-2 is a diagram showing plasmid pRS434TEF.
Fig. 7C-1 is a diagram showing plasmid pRS436ADH.
Fig. 7C-2 is a diagram showing plasmid pRS436GAP.
Fig. 7D-1 is a diagram showing plasmid pRS436PGK.
Fig. 7D-2 is a diagram showing plasmid pRS436TEF.
Fig. 7E-1 is a diagram showing plasmid pRS444ADH.
Fig. 7E-2 is a diagram showing plasmid pRS444GAP.
Fig. 7F-1 is a diagrams showing plasmid pRS444PGK.
Fig. 7F-2 is a diagram showing plasmid pRS444TEF.
Fig. 7G-1 is a diagram showing plasmid pRS446ADH.
Fig. 7G-2 is a diagram showing plasmid pRS446GAP.
Fig. 7H-1 is a diagram showing plasmid pRS446PGK.
Fig. 7H-2 is a diagram showing plasmid pRS446TEF.
Fig. 8 is a physiological map of plasmid pALHMG106.
Fig. 9 presents photographs showing the results of Southern blotting.
Fig. 10 presents photographs showing the results of PCR mapping.
Fig. 11 presents photographs showing the results of Northern blotting.
Fig. 12A presents graphs showing the specific activity of each prenyl-diphosphate synthase in a crude enzyme solution.
Fig. 12B presents graphs showing the specific activity of each prenyl-diphosphate synthase in a crude enzyme solution.
Fig. 13 is a graph showing prenyl alcohol yields when pRS434GAP-HMG1 or pRS444GAP-HMG1 has been transferred into A451 strain.
Fig. 14 is a graph showing prenyl alcohol yields when pRS434GAP-HMG1 or pRS444GAP-HMG1has been transferred into A451 strain.
Fig. 15 presents graphs showing prenyl alcohol yields when pRS434GAP-HMG1 or pRS444GAP-HMG1has been transferred into A451 strain.
Fig. 16 presents graphs showing prenyl alcohol yields when pRS414PTadh-HMG1, pRS414TPadh-HMG1, pRS434GAP-HMG1, pRS444GAP-HMG1, pRS434PGK-HMG1, pRS444PGK-HMG1, pRS434TEF-HMG1 or pRS444TEF-HMG1 has been transferred into YPH499 strain.
Fig. 17 presents graphs showing prenyl alcohol yields when pRS434GAP-HMG1 or pRS444GAP-HMGI has been transferred into EUG8 strain.
Fig. 18 presents graphs showing prenyl alcohol yields when pRS434GAP-HMG1 or pRS444GAP-HMG1 has been transferred into EUG12 strain.
Fig. 19 presents graphs showing prenyl alcohol yields when pRS434GAP-HMG1 or pRS444GAP-HMG1 has been transferred into EUG27 strain.
Fig. 20A presents graphs showing prenyl alcohol yields when pYES-HMG1 or pYHMG044 has been transferred into A451 strain.
Fig. 20B presents graphs showing prenyl alcohol yields when pYES-HMG1 or pYHMG044 has been transferred into AURGG101 strain.
Fig. 21 presents graphs showing prenyl alcohol yields when pYES-HMG1 has been transferred into W303-1A or W303-1B.
Fig. 22 presents graphs showing prenyl alcohol yields when pYHMG026, pYHMG044, pYHMG056, pYHMG062, pYHMG076, pYHMG081, pYHMG100, pYHMG112 or pYHMG122 has been transferred into A451 strain.
Fig. 23 presents graphs showing prenyl alcohol yields when pYHMG026, pYHMG044, pYHMG056, pYHMG062, pYHMG076, pYHMG081, pYHMG100, pYHMG112 or pYHMG122 has been transferred into AURGG101 strain.
Fig. 24 presents graphs showing prenyl alcohol yields when pYHMG026, pYHMG044, pYHMG056, pYHMG062, pYHMG076, pYHMG081, pYHMG100, pYHMG112 or pYHMG122 has been transferred into AURGG101 strain (the graphs in Fig. 23 are enlarged).
Fig. 25 is a graph showing prenyl alcohol yields when pRS434GAP-HMG1 or pRS444GAP-HMG1 has been introduced into AURGG101 strain together with pYHMG044.
Fig. 26 is a graph showing prenyl alcohol yields when a mutant *ispA* gene-transferred *E. coli* was cultured in a liquid medium containing IPP and DMAPP.
Fig. 27 is a graph showing prenyl alcohol yields when a mutant *ispA* gene-transferred *E. coli* was cultured in a liquid medium without IPP and DMAPP.

Fig. 28 is a graph showing prenyl alcohol yields and cell counts when a recombinant 15-2 clone (pYHMG044/AURGG101) was cultured in a jar fermenter.

BEST MODE FOR CARRYING OUT THE INVENTION

Hereinbelow, the present invention will be described more specifically with reference to the following Examples. However, the technical scope of the present invention is not limited to these Examples.

[EXAMPLE 1 ] Construction of Expression Vectors

Vectors were constructed using *E. coli* SURE2 supercompetent cells purchased from Stratagene (La Jolla, CA) as a host. For the preparation of genomic DNA from *S. cerevisiae* and for testing the introduction of resultant vectors, YPH499 strain (Stratagene) was used.

(1) *E. coli-S. cerevisiae* Shuttle Vectors

Plasmids pRS404 and pRS414 (Fig. 3) were purchased from Stratagene. Plasmid pAUR123 was purchased from Takara, and plasmid pYES2 (Fig. 4) was purchased from Invitrogen (Carlsbad, CA).

(2) Genomic DNA

Dr. GenTLE$^{TM}$, a genomic DNA preparation kit for yeast, was purchased from Takara. Genomic DNA was prepared from *S. cerevisiae* YPH499 according to the protocol attached to the kit.

(3) Insertion *ofADH1p-ADH1*t Fragment into pRS414

Plasmid pRS414 (Fig. 3) was digested with *NaeI* and *Pvu*II to obtain a 4.1 kbp fragment without fl *ori* and *LacZ* moieties. This fragment was purified by agarose gel electrophoresis. Plasmid pAUR123 was digested with *BamHI* and blunt-ended with Klenow enzyme. Then, a 1.0 kbp fragment containing *ADH1* transcription promoter (*ADH*1p) and *ADH1* transcription terminator (*ADH1*t) (Fig. 5; SEQ ID NO: 17) was purified by agarose gel electrophoresis. The 4.1 kbp fragment from pRS414 still retained the replication origins for *E. coli* and yeast, a transformation marker *Amp*$^r$ for *E. coli,* and an auxotrophic marker *TRP1* for yeast. On the other hand, the 1.0 kbp fragment from pAUR123 contained *ADH1*p, *ADH1*t, and a cloning site flanked by them. These two fragments were ligated to each other with a DNA ligation kit (Takara) and transformed into SURE2 cells.

Plasmid DNA was prepared from the resultant recombinant. Mapping of the DNA with *SalI* and *ScaI* revealed that the *ADH1*p-*ADH*t fragment has been inserted into pRS414 in opposite directions to thereby yield two plasmids pRS414PTadh and pRS414TPadh (Fig. 6).

(4) Insertion of *CYC1*t Fragment into pRS Vectors

*CYC1*t *(CYC1* transcription terminator) fragment was prepared by PCR. The following oligo-DNAs, XhoI-Tcyc1FW and ApaI-Tcyc1RV, were used as PCR primers. As a template. DYES2 was used.

XhoI-Tcyc1FW : 5'- TGC ATC TCG AGG GCC GCA TCA TGT AAT TAG -3' (SEQ ID NO: 18)

ApaI-Tcyc1RV : 5'- CAT TAG GGC CCG GCC GCA AAT TAA AGC CTT CG -3' (SEQ ID NO: 19)

Briefly, 50 µl of a reaction solution containing 0.1 µg of pYES2, 50 pmol of each primer DNA, 1x *Pfu* buffer containing $MgSO_4$ (Promega, Madison, WI), 10 nmol dNTPs, 1.5 units of *Pfu* DNA polymerase (Promega) and 1 µl of Perfect Match polymerase enhancer (Stratagene) was prepared. The reaction conditions were as follows: first denaturation at 95°C for 2min; 30 cycles of denaturation at 95°C for 45 sec, annealing at 60°C for 30 sec, and extension at 72°C for 1 min; and final extension at 72°C for 5 min. After completion of the reaction, the solution was stored at 4°C. The amplified DNA was digested with XhoI and *ApaI,* and the resultant 260 bp DNA fragment was purified by agarose gel electrophoresis to obtain CYC1t-XA.

CYC1t-XA was inserted into the *XhoI-Apa*I site of pRS404 and pRS406 to thereby obtain pRS404Tcyc and pRS406Tcyc, respectively.

(5) Preparation of Transcription Promoters

DNA fragments comprising transcription promoters were prepared by PCR using pAUR123 or yeast genomic DNA as a template. The DNA primers used are as follows.

SacI-Padh1FW: 5'-GAT CGA GCT CCT CCC TAA CAT GTA GGT GGC GG-3' (SEQ ID NO: 20)

SacII-Padh1RV: 5'-CCC GCC GCG GAG TTG ATT GTA TGC TTG GTA TAG C-3' (SEQ ID NO: 21)

SacI-Ptdh3FW: 5'-CAC GGA GCT CCA GTT CGA GTT TAT CAT TAT CAA-3' (SEQ ID NO: 22)

SacII-Ptdh3RV: 5'-CTC TCC GCG GTT TGT TTG TTT ATG TGT GTT TAT TC -3' (SEQ ID NO: 23)

SacI-Ppgk1FW: 5'-TAG GGA GCT CCA AGA ATT ACT CGT GAG TAA GG-3' (SEQ ID NO: 24)

SacII-Ppgk1RV: 5'-ATA ACC GCG GTG TTT TAT ATT TGT TGT AAA AAG TAG-3' (SEQ ID NO: 25)

SacI-Ptef2FW: 5'-CCG CGA GCT CTT ACC CAT AAG GTT GTT TGT GAC G-3 (SEQ ID NO: 26)

SacII-Ptef2RV: 5'-CTT TCC GCG GGT TTA GTT AAT TAT AGT TCG TTG ACC-3' (SEQ ID NO: 27)

For the amplification of *ADH1* transcription promoter (*ADH1*p), SacI-Padh1FW and SacII-PadhlRV were used as PCR primers and pAUR123 as a template. For the amplification of *TDH3* (GAP) transcription promoter (*TDH3*p (GAPp)), SacI-Ptdh3FW and SacII-Ptdh3RV were used as PCR primers; for the amplification of *PGK1* transcription promoter (*PGK1*p), SacI-Ppgk1FW and SacII-Ppgk1RV were used as PCR primers; and for the amplification of *TEF2* transcription promoter (*TEF2*p), SacI-Ptef2FW and SacII-Ptef2RV were used as PCR primers. For these promoters, yeast genomic DNA was used as a template. As a reaction solution, a 100 µl solution containing 0.1 µg of pAUR123 or 0.46 µg of yeast genomic DNA, 100 pmol of each primer DNA, 1 x ExTaq buffer (Takara), 20 nmol dNTPs, 0.5 U of ExTaq DNA polymerase (Takara) and 1 µl of Perfect Match polymerase enhancer was prepared. The reaction conditions were as follows: first denaturation at 95°C for 2min; 30 cycles of denaturation at 95°C for 45 sec, annealing at 60°C for 1 min, and extension at 72°C for 2 min; and final extension at 72°C for 4 min. After completion of the reaction, the solution was stored at 4°C. The amplified 4 types of DNAs were digested with *SacI* and *SacII,* and the resultant 620 bp, 680 bp, 710 bp and 400 bp DNA fragments were purified separately by agarose gel electrophoresis to thereby obtain *ADH1*p, *TDH3*p, *PGK1*p and *TEF2*p, respectively.

(6) Preparation of 2μ DNA Replication Origin Site

pYES2, which is a YEp vector, was digested with *Ssp*I and *Nhe*I. The resultant 1.5 kbp fragment containing 2μ DNA replication origin (2μ *ori*) was purified by agarose gel electrophoresis and then blunt-ended. This DNA fragment was designated 2μOriSN.

(7) Preparation of YEp Type Expression Vectors

2μOriSN was inserted into the *Nae*I site of pRS404Tcyc and pRS406Tcyc pretreated with BAP (bacterial alkaline phosphatase: Takara). The resultant plasmids were transformed into *E. coli* SURE2, and then plasmid DNA was prepared. The plasmid DNA was digested with *Dra*III; and *EcoR*I, *Hpa*I or *Pst*I; and *Pvu*II, followed by agarose gel electrophoresis to examine the insertion and the direction of 2μ *ori.* The resultant pRS404Tcyc and pRS406Tcyc into which 2μ. *ori* had been inserted in the same direction as in pYES2 were designated pRS434Tcyc2μOri and pRS436Tcyc2μOri, respectively. The resultant pRS404Tcyc and pRS406Tcyc into which 2μ *ori* had been inserted in the opposite direction to that in pYES2 were designated pRS444Tcyc2μOri and pRS446Tcyc2μOri, respectively.

A transcription promoter-containing fragment, i.e., *ADH1*p, *TDH3*p (*GAP*p), *PGK1*p or *TEF2*p, was inserted into the *Sac*I-*Sac*II site of the above-described four plasmids pRS434Tcyc2μOri, pRS436Tcyc2μOri, pRS444Tcyc2μOri and pRS446Tcyc2μOri to clone the DNA. As a result, the following plasmids were obtained: (i) pRS434ADH, pRS434GAP, pRS434PGK and pRS434TEF from pRS434Tcyc2μOri; (ii) pRS436ADH, pRS436GAP, pRS436PGK and pRS436TEF from pRS436Tcyc2μOri; (iii) pRS444ADH, pRS444GAP, pRS444PGK and pRS444TEF from pRS444Tcyc2μOri; (iv) pRS446ADH, pRS446GAP, pRS446PGK and pRS446TEF from pRS446Tcyc2μOri (Figs. 7A-7H).

The expression vectors prepared in the present invention are summarized in Table 3 below.

Table 3

| Vector | Type | Marker and Direction* | | Promoter, Terminator and Direction* | | | ori and Direction* | |
|---|---|---|---|---|---|---|---|---|
| pRS414PTadh | YCp | TRP1 | + | ADH1 | ADH1 | + | ARS4 & CEN6 | + |
| pRS414TPadh | YCp | TRP1 | + | ADH1 | ADH1 | - | ARS4 & CEN6 | + |
| pRS434ADH | YEp | TRP1 | + | ADH1 | CYC1 | - | 2μ | + |
| pRS434GAP | YEp | TRP1 | + | TDH3 | CYC1 | - | 2μ | + |
| pRS434PGK | YEp | TRP1 | + | PGK1 | CYC1 | - | 2μ | + |
| pRS434TEF | YEp | TRP1 | + | TEF2 | CYC1 | - | 2μ | + |
| pRS436ADH | YEp | URA3 | + | ADH1 | CYC1 | - | 2μ | + |
| pRS436GAP | YEp | URA3 | + | TDH3 | CYC1 | - | 2μ | + |
| pRS436PGK | YEp | URA3 | + | PGK1 | CYC1 | - | 2μ | + |
| pRS436TEF | YEp | URA3 | + | TEF2 | CYC1 | - | 2μ | + |
| pRS444ADH | YEp | TRP1 | + | ADH1 | CYC1 | - | 2μ | - |
| pRS444GAP | YEp | TRP1 | + | TDH3 | CYC1 | - | 2μ | - |
| pRS444PGK | YEp | TRP1 | + | PGK1 | CYC1 | - | 2μ | - |
| pRS444TEF | YEp | TRP1 | + | TEF2 | CYC1 | - | 2μ | - |
| pRS446ADH | YEp | URA3 | + | ADH1 | CYC1 | - | 2μ | - |
| pRS446GAP | YEp | URA3 | + | TDH3 | CYC1 | - | 2μ | - |
| pRS446PGK | YEp | URA3 | + | PGK1 | CYC1 | - | 2μ | - |

* The + and - marks appearing after marker and gene expression transcription unit indicate downstream direction and upstream direction, respectively. The + mark appearing after *ori* indicates that *ori* is inserted in the same direction as in pRS (for YCp vectors) or pYES (for YEp vectors); the - mark indicates that ori is inserted in the direction opposite to that in pRS (for YCp vectors) or pYES (for YEp vectors).

Table 3 (continued)

| Vector | Type | Marker and Direction* | | Promoter, Terminator and Direction* | | | ori and Direction* | |
|---|---|---|---|---|---|---|---|---|
| pRS446TEF | YEp | URA3 | + | TEF2 | CYC1 | - | 2μ | - |

* The + and - marks appearing after marker and gene expression transcription unit indicate downstream direction and upstream direction, respectively. The + mark appearing after *ori* indicates that *ori* is inserted in the same direction as in pRS (for YCp vectors) or pYES (for YEp vectors); the - mark indicates that ori is inserted in the direction opposite to that in pRS (for YCp vectors) or pYES (for YEp vectors).

(8) Introduction of YEp Type Expression Vectors into Yeast

In order to examine whether the DNA replication region of the prepared YEp type expression vectors functions or not, about 40 ng of each YEp type expression vector was introduced into YPH499 strain using Frozen-EZ Yeast Transformation II (Zymo Research, Orange, CA). (The procedures followed the protocol attached to the kit.) Then, colonies growing on SD-W (DOB+CMS (-Trp); BIO101, Vista, CA) agar plate at 30°C were examined. The results are shown in Table 4 below.

Table 4

| | | ADH | GAP | PGK | TEF |
|---|---|---|---|---|---|
| pRS | 434 | >1000 | >1000 | >1000 | >1000 |
| | 436 | 500 | >1000 | >1000 | 300 |
| | 444 | >1000 | >1000 | >1000 | >1000 |
| | 446 | 250 | >1000 | >1000 | 100 |

The results shown in Table 4 revealed that each of the YEp type vectors prepared in the invention functions normally as a vector.

[EXAMPLE 2] Cloning of Genes

(I) Cloning of HMG-CoA Reductase Gene (*HMG1*' Gene) by PCR

The cloning of *S. cerevisiae HMG1'* gene was carried out as described below.

Based on information on *S. cerevisiae-derived HMG1* gene (Accession No. M22002) (M.E. Basson, *et al.,* Mol. Cell. Biol. 8, 3797-3808 (1988): SEQ ID NO: 1) registered in the GenBank, a pair of primers were designed which are specific to those nucleotide sequences corresponding to an N-terminal and a C-terminal region of the protein encoded by this gene. Using these primers and a yeast cDNA library (Clontech; No. CL7220-1 derived from *S. cerevisiae* DBY746) as a template, PCR was carried out.

N-terminal primer (Primer 1): 5'-ATG CCG CCG CTA TTC AAG GGA CT-3' (SEQ ID NO: 28)

C-terminal primer (Primer 2): 5'-TTA GGA TTT AAT GCA GGT GAC GG-3' (SEQ ID NO: 29)

The PCR was carried out in the reaction solution as described below under the following conditions: 30 cycles of denaturation at 94°C for 45 sec, annealing at 55°C for 1 min and extension at 72°C for 2 min.

| | |
|---|---|
| 10 x ExTaq buffer (Takara) | 5 μl |
| 2.5 mM dNTP mix | 4 μl |
| 5 U/μl ExTaq (Takara) | 1 μl |
| 10 pmol Primer 1 | |

(continued)

| | |
|---|---|
| 10 pmol Primer 2<br>0.5 ng cDNA | |

To give a 50 µl solution in total

Agarose gel electrophoresis performed after the PCR confirmed a fragment at the expected location (3.2 kbp). This 3.2 kbp DNA fragment was cloned into pT7Blue T vector (Novagen, Madison, WI) capable of TA cloning, to thereby obtain pT7HMG1. The nucleotide sequence of the thus cloned HMG-CoA reductase gene was determined. As a result, the nucleotide sequence as shown in SEQ ID NO: 3 and the amino acid sequence as shown in SEQ ID NO: 4 were obtained. The thus determined nucleotide sequence was partially different from the corresponding nucleotide sequence registered in the GenBank (http://www.ncbi.nlm.nih.gov/Genbank/index.html) (Fig. 2A). This gene that comprises PCR errors and encodes the amino acid sequence of a mutant HMG-CoA reductase (SEQ ID NO: 4) is designated *HMG1'*.

(2) Correction of PCR Errors in *HMG1 '*

An *HMG1'* fragment was subcloned from plasmid pT7HMG1 comprising *HMG1'* encoding a mutant HMG-CoA reductase. Then, the amino acid substitutions resulted from the PCR errors occurred in the coding region of the wild-type HMG-CoA reductase gene were corrected by site-directed mutagenesis to thereby prepare pALHMG106. The details of this preparation are as described below.

Plasmid pT7HMG1 was used as cloned *HMG1'*. As a vector for introducing site-directed mutations, pALTER-1 (Promega) was used.

Site-directed mutagenesis was carried out according to the procedures described in "Protocols and Application Guide, 3rd edition, 1996 Promega, ISBN 1-882274-57-1" published by Promega. As oligos for introducing mutations, the following three oligos were synthesized chemically.

HMG1(190-216) 5'-CCAAATAAAGACTCCAACACTCTATTT-3' (SEQ ID NO: 30)

HMG1 (1807-1833) 5'-GAATTAGAAGCATTATTAAGTAGTGGA-3' (SEQ ID NO: 31)

HMG1 (2713-2739) 5'-GGATTTAACGCACATGCAGCTAATTTA-3' (SEQ ID NO: 32)

First, pT7HMG1 was digested with *Sma*I, *ApaL*I and *Sal*I, and a 3.2 kbp *HMG1'* fragment was prepared by agarose gel electrophoresis. This fragment was inserted into the *Sma*I-*Sal*I site of pALTER-1 to prepare pALHMG1. After denaturation of this plasmid with alkali, the above-described oligos for introducing mutations, Amp repair oligo (Promega) as repair oligos, and Tet knockout oligo (Promega) as knockout oligos were annealed thereto. The resultant plasmid was introduced into *E. coli* ES1301 (Promega). Transformants that retained plasmids into which site-directed mutations had been introduced were selected and cultured with 125 µg/ml ampicillin to prepare plasmid DNA. The nucleotide sequence of the resultant plasmid DNA was examined with primers having the sequences as shown below. As a result, all the sequences corresponding to HMG1 (190-216), HMG1 (1807-1833) and HMG1 (2713-2739) were corrected so that they had the sequences of these oligonucleotides (SEQ ID NO: 5). The amino acid sequence encoded by the corrected nucleotide sequence (SEQ ID NO: 6) was consistent with the amino acid sequence encoded by the wild-type *HMG1* (SEQ ID NO: 2); the corrected sequence retained only silent mutations. Since this PCR error-corrected *HMG1* encodes a polypeptide having the same amino acid sequence as that of the wild-type enzyme though it has a partially different nucleotide sequence, this gene is also designated *HMG1* and used herein without distinction between this and the wild-type gene *HMG1.*

HMG1 (558-532) 5'-GTCTGCTTGGGTTACATTTTCTGAAAA-3' (SEQ ID NO: 33)

HMG1 (1573-1599) 5'-CATACCAGTTATACTGCAGACCAATTG-3' (SEQ ID NO: 34)

HMG1 (2458-2484) 5'-GAATACTCATTAAAGCAAATGGTAGAA-3' (SEQ ID NO: 35)

The plasmid carrying the thus corrected *HMG1* sequence was designated pALHMG106 (Fig. 8).

(3) Cloning of Geranylgeranyl Diphosphate Synthase Gene *BTS1*

*S.* cerevisiae *BTS1* gene (also called GGPP synthase gene) was cloned as described below.

Based on information on *S. cerevisiae-derived* GGPP synthase gene registered in the GenBank (Accession No. U31632) (Y. Jiang, *et al.*, J. Biol. Chem. 270 (37), 21793-21799 (1995)), a pair of primers described below matching an N-terminal and a C-terminal region of the enzyme were designed. Using these primers and a yeast cDNA library (CL7220-1) as a template, PCR was carried out.

N-terminal primer: 5'-ATG GAG GCC AAG ATA GAT GAG CT-3' (SEQ ID NO: 36)

C-terminal primer: 5'-TCA CAA TTC GGA TAA GTG GTC TA-3' (SEQ ID NO: 37)

The PCR was performed in a reaction solution having a composition similar to that of the reaction solution described in (1) above under the following conditions: 30 cycles of denaturation at 94°C for 45 sec, annealing at 55°C for 1 min and extension at 72°C for 2 min.

Agarose gel electrophoresis performed after the PCR confirmed a fragment having the proper mobility (corresponding to approx. 1.0 kbp). This *BTS1* gene was cloned into pT7Blue T vector capable of TA cloning, followed by sequencing of the entire region of this *BTS1* gene. The results revealed that the nucleotide sequence of this gene was completely identical with the nucleotide sequence registered in the GenBank. Thus, it was confirmed that this gene is the *S. cerevisiae-derived* GGPP synthase gene.

The pT7Blue T vector was digested with *Bam*HI and *Sail*I to cut out the *BTS1* gene, which was then introduced into the *Bam*HI-*Xho*I site of pYES2 (Invitrogen). The recombinant plasmid obtained was designated pYESGGPS.

(4) Cloning *of Escherichia* coli-derived FPP Synthase Gene *ispA*

*E. coli* genomic DNA was prepared from *E. coli* JM109 (Takara) by the following procedures. JM109 cells were cultured in 1.5 ml of 2xYT medium and harvested by centrifugation. To these cells, 567 μl of TE (pH 8.0), 3 μl of 20 mg/ml proteinase K (Boehringer Mannheim, Mannheim, Germany) and 30 μl of 10% SDS were added. The resultant mixture was left at 37°C for 1 hr, and then 100 μl of 5M NaC1 was added thereto and mixed. Eighty μl of CTAB/NaCl solution (10% CTAB, 0.7 M NaCl) was added thereto, and the resultant mixture was heated at 65°C for 10 min. This mixture was then treated with 700 μl of chloroform/isoamyl alcohol (24:1) extraction, and a further extraction was carried out with 600 μl of phenol/chloroform/isoamyl alcohol (25:24:1) to the obtained aqueous layer,. which was then centrifuged. The precipitate was washed with 70% ethanol, dried, and then dissolved in 100 μl of TE (pH 8.0) to thereby obtain an *E. coli* genomic DNA solution. The DNA was measured and quantitatively determined at $OD_{260}$. Then, TE was added to the solution to give a DNA concentration of 1 μg/μl.

Using the thus obtained *E. coli* genomic DNA as a template and the following synthetic oligo-DNA primers, *E. coli*-derived FPP synthase gene *ispA* was cloned by PCR.

ISPA1: 5'-TGA GGC ATG CAA TTT CCG CAG CAA CTC G-3' (SEQ ID NO: 68)

ISPA2: 5'-TC AGA ATT CAT CAG GGG CCT ATT AAT AC-3' (SEQ ID NO: 69)

PCR was carried out in a 100 µl reaction solution containing 1x ExTaq buffer, 0.5 mM dNTP, 100 pmol of ISPA1, 100 pmol of ISPA2, 0.2 µg of *E. coli* genomic DNA and 5 units of ExTaq under the following conditions: 30 cycles of denaturation at 94°Cfor 1 min, annealing at 55°C for 1 min and extension at 72°C for 1.5 min. The PCR product was digested with *Eco*RI and *Sph*I. Then, the resultant 1.0 kbp fragment was purified by agarose gel electrophoresis and inserted into the *Eco*RI-*Sph*I site of pALTER-Ex2 (Promega), which was then introduced into E. *coli* JM109 for the cloning of the gene. Restriction enzyme mapping using *Eco*RI, *Sph*I, *Nde*I, *Sma*I, and *Bam*HI revealed that *ispA* gene (SEQ ID NO: 77) had been introduced correctly into earned three plasmids, *i.e.*, pALispA4, pALispA16 and pALispA18.

(5) Preparation of Mutant FPP Synthase Genes

Using plasmid pALispA16, the codon encoding the amino acid residue Tyr at position 79 of the polypeptide encoded by *E. coli ispA* was modified by substitution according to the procedures described in "Protocols and Applications Guide, the 3rd edition, 1996, Promega, ISBN 1-882274-57-1" published by Promega. The following oligos for introducing mutations (also called "mutant oligos") were prepared by chemical synthesis.

ISPA-D: 5'-ATC ATG AAT TAA TGA GTC AGC GTG GAT GCA TTC AAC GGC GGC AGC-3' (SEQ ID NO: 70)

ISPA-E: 5'-ATC ATG AAT TAA TGA TTC AGC GTG GAT GCA TTC AAC GGC GGC AGC-3' (SEQ ID NO: 71)

ISPA-M: 5'-ATC ATG AAT TAA TGA CAT AGC GTG GAT GCA TTC AAC GGC GGC AGC-3' (SEQ ID NO: 72)

The above-described mutant oligo ISPA-M was designed so that the nucleotides from position 16 to position 18 (the three nucleotides underlined) encode Met, which nucleotides correspond to the codon for the 79th amino acid residue Tyr in the wild-type gene. Similarly, mutant oligos ISPA-D and ISPA-E were designed so that the corresponding codons encode Asp and Glu, respectively. In these mutant oligos, the nucleotides from position 26 to position 31 (the six nucleotides underlined) were designed so that *Eco*T22I (*NsiI*) site is newly formed by the substitution mutation. Thus, it is so arranged that these mutant genes can be easily distinguished from the wild-type gene by restriction enzyme mapping. The mutant oligos were treated with T4 polynucleotide kinase (Promega) in advance to phosphorylate their 5' end and purified by gel filtration with Nick Column (Pharmacia Biotech, Uppsala, Sweden) before use. For the introduction of mutations, Cm repair oligo (Promega) as the repair oligo, and Tet knockout oligo (Promega) as the knockout oligo were also used. Cm repair oligo, Tet knockout oligo and the mutant oligos were annealed to alkali-denatured pALispA16, which was then transformed into *E. coli* ES1301 mutS (Promega). Plasmid DNA was prepared from *E. coli* colonies growing in the presence of 20 µg/ml chloramphenicol (Cm), and transformed into *E. coli* JM109. Plasmid DNA was prepared from *E. coli* colonies growing on agar plates containing 20 µg/ml Cm. Plasmids containing substitution-mutated *ispA* genes (designated *ispA*m genes) that were prepared using pALispA4 as a template and ISPA-D, ISPA-E and ISPA-M as mutant oligos were designated p4D, p4E and p4M, respectively. Those plasmids prepared similarly using pALispA16 as a template were designated p16D, p16E and p16M, respectively. Those plasmids prepared similarly using pALispA18 as a template were designated p18D, p18E and p18M, respectively.

(6) Cloning of IPPΔ-Isomerase Gene *idi*

*E. coli* IPPΔ-isomerase gene was formerly called as ORF182 (according to NCBI BLAST search; GenBank Accession No. AE000372), but Hahn *et al.* ((1999) J. Bacteriol., 181: 4499-4504) designated this gene *idi.* As plasmids in which *idi* (SEQ ID NO: 85; encoding the amino acid sequence as shown in SEQ ID NO: 86) is cloned, p3-47-11 and p3-47-13 described in Hemmi *et al.,* (1998) J. Biochem., 123: 1088-1096 were used in the invention.

(7) Cloning of *Bacillus stearothermophilus* FPP Synthase Gene

Plasmid pFE15 described in Japanese Unexamined Patent Publication No. 5-219961 was digested with *Not*I and *Sma*I. The resultant 2.9 kbp *Bacillus stearothermophilus* FPP synthase gene (hereinafter, referred to as *"fps")* (SEQ ID NO: 75; encoding the amino acid sequence as shown in SEQ ID NO: 76) fragment containing a transcription unit was purified and inserted into the *Sca*I site of pACYC177 (Nippon Gene) to obtain plasmid pFE15NS2.9-1.

[EXAMPLE 3] Insertion of Genes into Expression Vectors

(1) Subcloning into pRS Expression Vectors

*HMG1* gene was introduced into individual pRS vectors (Figs. 6 and 7) prepared in the present invention which are *E. coli-S. cerevisiae* YEp shuttle vectors containing a constitutive transcription promoter.

pALHMG106 (Fig. 8) containing the PCR error-corrected HMG-CoA reductase gene was digested with *Sma*I and *Sal*I. The resultant 3.2 kbp *HMG1* fragment was purified by agarose gel electrophoresis and inserted into the *Sma*I-*Sail*I site of pRS434GAP, pRS444GAP, pRS434TEF, pRS444TEF, pRS434PGK and pRS444PGK. Those plasmids into which the gene had been subcloned were examined for their physical maps by restriction enzyme mapping with *Xho*I, *Spe*I, *Nae*I and *Sph*I*,* and by confirmation of the nucleotide sequences: of the border regions of the inserted 3.2 kbp *HMG1* fragment. Then, those plasmids created exactly as planned were selected and designated pRS434GAP-HMG1, pRS444GAP-HMG1, pRS434TEF-HMG1, pRS444TEF-HMG1, pRS434PGK-HMG1 and pRS444PGK-HMG1.

(2) Preparation of pRS414PTadh-HMG1 and pRS414TPadh-HMG1

Vectors pRS414PTadh and pRS414TPadh (Fig. 6) containing a constitutive transcription promoter *ADH1p* were digested with *Sma*I and *Sal*I, followed by the same operations as described in (1) above. As a result, plasmids pRS414PTadh-HMG1 and pRS414TPadh-HMG1 each containing *HMG1* gene inserted thereinto were created.

(3) Preparation of *HMG1'* Expression Plasmid pYES-HMG1

pT7HMG1 prepared in (1) in Example 2 was digested with *Bam*HI, *Sal*I and *Sca*I to cut out the *HMG1'* gene encoding the mutant HMG-CoA reductase resulted from PCR errors. Then, this gene was inserted into the *Bam*HI-*Xho*I site of pYES2 (Invitrogen, Carlsbad, CA). The resultant recombinant vector was designated pYES-HMG1. As a result of determination of the nucleotide sequence within this vector, it was confirmed that the sequence is identical with the nucleotide sequence as shown in SEQ ID NO: 3. The above plasmid pYES2 is a shuttle vector for expression in yeast that has yeast 2μm DNA ori as a replication origin and *GAL1* transcription promoter inducible by galactose (Fig. 4).

(4) Preparation of Deletion Mutant *HMG*1' Expression Plasmid pYHMGxxy

In order to prepare vectors for expressing deletion mutants of HMG-CoA reductase gene having deletion of a nucleotide sequence encoding a region upstream of a domain that is believed to be the catalytic domain of HMG-CoA reductase, a fragment lacking a part of the *HMG1'* coding region together with the vector moiety was prepared by PCR using pYES-HMG1 created in (3) above as a template. The resultant fragment was blunt-ended with Klenow enzyme and then circularized again by self-ligation, followed by transformation into E. *coli* JM109. Then, plasmid DNA was prepared from the transformant. The sequences of the synthetic DNAs used as primers and their combinations are shown in Table 1.

For each of the plasmid DNA obtained, it was confirmed with 373A DNA sequencer (Perkin Elmer, Foster City, CA) that there was no shift in the reading frame of amino acids upstream and downstream of *HMG1,* and that there was no amino acid substitution resulting from PCR errors around the junction site. As a result, the following plasmids were obtained which have no amino acid substitution resulting from PCR errors around the junction site and in which a deletion could be made successively without any shift in the reading frame. Deletion mutants of *HMG1* gene are expressed as, *e.g.,* "Δ02y" according to the deletion pattern (where y represents a working number that may be any figure), and pYES2 vectors comprising Δ02y are expressed as, e.g., pYHMG026. (This is applicable to other deletion mutants.)

*HMG1*Δ02y. SEQ ID NO: 7

*HMG1*Δ04y: SEQ ID NO: 8
*HMG1*Δ05y: SEQ ID NO: 9
*HMG1*Δ06y: SEQ ID NO: 10
*HMG1*Δ07y: SEQ ID NO: 11
*HMG1*Δ08y: SEQ ID NO: 12
*HMG1*Δ10y: SEQ ID NO: 13
*HMG1*Δ11y: SEQ ID NO: 14
*HMG1*Δ12y. SEQ ID NO: 15
*HMG1*Δ13y: SEQ ID NO: 16
Vectors: YHMG026, pYHMG027, pYHMG044, pYHMG045, pYHMG062, pYHMG063, PYHMG065, pYHMG076, pYHMG081, pYHMG083, pYHMG085, pYHMG094, pYHMG100, pYHMG106, pYHMG107, pYHMG108, pYHMG109, pYHMG112, pYHMG122, pYHMG123, pYHMG125 and pYHMG133

[EXAMPLE 4] Preparation of AURGG 101

A 1.9 kbp *Sal*I fragment having a primary structure *of GAL1* transcription promoter-*BTS1-CYC1* transcription terminator (*G4L1P-BTS1-CYC1*t) was prepared by PCR using pYESGGPS described in (3) in Example 2 as a template and the following primers PYES2 (1-27) and PYES2 (861-835).

PYES2 (1-27): 5'-GGC CGC AAA TTA AAG CCT TCG AGC GTC-3' (SEQ ID NO: 73)

PYES2 (861-835): 5'-ACG GAT TAG AAG CCG CCG AGC GGG TGA-3' (SEQ ID NO: 74)

This fragment was inserted into the SalI site of pAUR101 (Takara) to obtain pAURGG115. It was confirmed by DNA sequencing that the *BTS1* gene in pAURGG115 had no PCR error.
pAURGG115 was linearized with *Eco*065I and introduced into A451 strain by the lithium acetate method. Then, colonies growing on YPD agar plates (1% yeast extract, 2% peptone, 2% dextrose, 2% agar) containing 1μg/ml aureobasidin at 30°C were selected as transformants. The resultant transformants were cultured again on aureobasidin selection plates to select a single colony.
As a result, two clones AURGG101 and AURGG102 were obtained as recombinants from A451 strain. In the present invention, AURGG101 was used as one of A451-derived host clones.
As revealed by Southern blot hybridization (Fig. 9) and PCR mapping (Fig. 10), *BTS1* is integrated in the genome in AURGG102 but not integrated therein in AURGG101. In AURGG101, it was found that *AUR1* has been merely replaced with *AUR1-C* (a marker gene). Since *AUR1* is not directly involved in the synthesis of prenyl alcohol or prenyl diphosphate, it is possible to use AURGG101 as one example of A451-derived host clones.
Details of the Southern blot hybridization, Northern blot hybridization and PCR mapping are provided in Example 6 described later.

[EXAMPLE 5] Preparation of EUG Strains

A gene map around squalene synthase gene *ERG9* was obtained from a yeast genome database. Based on this map, PCR primer DNAs for amplifying DNA fragments for replacing *ERG9* transcription promoter (*ERG9*p) were designed (Fig. 2). On the other hand, a 1.8 kbp DNA fragment comprising a transformant selection marker gene *URA3* and a transcription promoter *GAL1*p was prepared by PCR amplification using, as a template, pYES2Δ obtained by digesting pYES2 with *Nae*I and *Nhe*I, blunt-ending with Klenow enzyme and deleting 2μ *ori* by self-ligation.
The primers used in the PCR are as follows.

E-MCSf: 5'- GCC GTT GAC AGA GGG TCC GAG CTC GGT ACC AAG-3' (SEQ ID NO: 49)

E-URA3r: 5'- CAT ACT GAC CCA TTG TCA ATG GGT AAT AAC TGA T-3' (SEQ ID NO: 50)

In the above primers, an *Eam*1105I recognition site (the underlined portion) is added so that T/A ligation can be conducted by using (i) a 0.7 kbp DNA fragment comprising a downstream portion of the open reading frame YHR189W in the genome of *S. cerevisiae* and (ii) a 0.9 kbp DNA fragment comprising an upstream portion of *ERG9*. The YHR189W fragment was prepared by PCR using the following primers YHR189Wf and YHR189Wr, and YPH499 genomic DNA as a template. The *ERG9* fragment was prepared by PCR using the following primers ERG9f and ERG9r, and YPH499 genomic DNA as a template. YPH499 genomic DNA was prepared with Dr. GenTLE™.

YHR189Wf: 5'-TGT CCG GTA AAT GGA GAC-3' (SEQ ID NO: 51)

YHR189Wr: 5'-TGT TCT CGC TGC TCG TTT-3' (SEQ ID NO: 52)

ERG9f: 5'-ATG GGA AAG CTA TTA CAA T-3' (SEQ ID NO: 53)

ERG9r: 5'-CAA GGT TGC AAT GGC CAT-3' (SEQ ID NO: 54)

The 1.8 kbp DNA fragment was digested with *Eam*111105I and then ligated to the 0.7 kbp DNA fragment. With the resultant fragment as a template, 2nd PCR was carried out using the above-described primers YHR189Wf and E-MCSf. The amplified 2.5 kbp DNA fragment was digested with *Eam*1105I and then ligated to the 0.9 kbp fragment. With the resultant fragment as a template, 3rd PCR was carried out using the following primers YHR189W-3f and ERG9-2r. As a result, a 3.4 kbp DNA fragment was amplified. This was used as a DNA fragment for transformation.

YHR189W-3f: 5'-CAA TGT AGG GCT ATA TAT G-3' (SEQ ID NO: 55)

ERG9-2r: 5'-AAC TTG GGG AAT GGC ACA-3' (SEQ ID NO: 56)

A vector was introduced into yeast strains using Frozen EZ Yeast Transformation II kit purchased from Zymo Research (Orange, CA). The resultant recombinants were cultured on an agar medium called SGR-U medium that had been obtained by adding CSM (-URA) (purchased from BIO 101, Vista, CA) and adenine sulfate (final concentration 40 mg/L) to SGR medium (a variation of SD medium in which the glucose component is replaced with galactose and raffinose), at 30°C. Colonies grown on the medium were spread on the same medium again, cultured and then subjected to single colony isolation.

The resultant recombinants were designated EUG (*ERG9*p::*URA3-GAL1*p) strain. Of these, clones derived from A451 strain were designated EUG1 through EUG10; clones derived from YPH499 strain were designated EUG11 through EUG20; and clones derived from YPH500 strain were designated EUG21through EUG30.

They were cultured on SD medium to select those clones that exhibit growth exhibition as a result of the inhibition of ERG9 expression due to glucose repression. As a result, EUG8 from A451, EUG12 from YPH499 and EUG27 from YPH500 were obtained.

Genomic DNA was prepared from EUG8, EUG12 and EUG27, separately, using Dr. GenTLE™. The results of PCR using the genomic DNA as a template confirmed that the 1.8 kbp PCR fragment containing *URA3* and *GAL1*p is integrated into the genome of each strain upstream of the *ERG9* coding region.

[EXAMPLE 6] Analysis of Genes and Enzyme Activity

In this Example, the expression of genes in various recombinant yeasts prepared in the invention (for the preparation thereof, see Examples 7 and 8 describing the production of prenyl alcohols) was analyzed by determining the enzyme activity of prenyl-diphosphate synthase and by various techniques including Northern blot hybridization, Southern blot hybridization and PCR mapping. Of the prepared recombinants, the host strain and the recombinants listed below were used in this Example. The introduction of individual vectors into the host was carried out according to the lithium acetate method described in Current Protocols in Molecular Biology, John Wiley & Sons, Inc., pp. 13.7.1-13.7.2 or by a method using Frozen EZ Yeast Transformation II kit (Zymo Research, Orange, CA) according to the protocol attached to the kit. Clone 1-2 was obtained by introducing pYES-HMG1 into A451; clone 3-2 was obtained by introducing pYHMG044 A451; clone 13-2 was obtained by introducing pYES-HMG1 into AURGG101; and clone 15-2 was obtained by introducing pYHMG044 into AURGG101.

No.1 host strain: A451
No.2 *GAL1*p-*BTS1* (YIp): AURGG101 (A451, *aur1::AUR1-C)*
No.3 *GAL1*p-*BTS1* (YIp): AURGG102 (A451, *aur1....BTS1-AUR1-C)*
No.4 *GAL1*p-*HMG1* (YEp): 1-2 (pYES-HMG1/A451)
No.5 *GAL1*p-*HMG1*Δ (YEp): 3-2 (pYHMG044/A451)
No.6 *GAL1*p-*HMG1* (YEp): 13-2 (pYES-HMG1/AURGG101)
No.7 *GAL1*p-*HMG1*Δ (YEp): 15-2 (pYHMG044/AURGG101)

Clones No. 1 to No. 7 were precultured separately at 26°C. One milliliter of the preculture was washed with physiological saline, added to 100 ml of a culture broth and cultured in a 300 ml Erlenmeyer flask at 26°C with reciprocal shaking at 120 times/mitt. SD medium or SG medium (in which the glucose component of SD medium is replaced with galactose). was used for the cultivation. Recombinants retaining *URA3* marker were cultured in SD-U [CSM (-URA)-added SD medium] or SG-U [CSM (-URA)-added SG medium]. AURGG clones were cultured in the presence of aureobasidin at 1 μg/L.

Cell growth was determined at $OD_{600}$. Cultivation was stopped when the value at $OD_{600}$ reached about 3-4 (23-52 hours). The culture was cooled in ice and then subjected to the preparation of DNA, RNA and crude enzyme solution, as described below.

(1) Southern Blotting

Yeast DNA was prepared using the yeast DNA preparation kit Dr. GenTLE™ according to the protocol attached to the kit.

The DNA thus prepared from yeast was digested with *NdeI* and *SluI,* followed by 0.8% agarose gel electrophoresis (3 μg/lane). As molecular weight markers, 0.5 μg each of I kb ladder and λ/*Hind*III (both from Promega, Madison, WI) were used. After the electrophoresis, the DNA was denatured with alkali, neutralized and then transferred onto Hybond N nylon membrane (Amersham, Buckinghamshire, England) by capillary blotting with 20 x SSC according to conventional methods. The resultant membrane was subjected to UV irradiation with a UV cross-linker (Stratagene) under conditions of optimal cross-linking, to thereby fix the DNA on the membrane.

(2) Northern Blotting

RNA was prepared according to the method described in Current Protocols in Molecular Biology, John Wiley & Sons, Inc., pp. 13.12.2-13.12.3 with partial modification. The modification was that once prepared RNA samples were further treated with DNase I.

After separation of RNA by formaldehyde-denatured agarose gel electrophoresis, the RNA was transferred onto Hybond N nylon membrane by capillary blotting with 20 x SSC according to conventional methods. Five micrograms of total RNA was electrophoresed per lane. As a molecular marker, 20 ng of DIG-RNA Marker I was used. The resultant membrane was subjected to UV irradiation with a UV cross-linker (Stratagene) under conditions of optimal cross-linking, to thereby fix the RNA on the membrane.

(3) PCR Mapping

In order to examine how a fragment from pAURGG115 (a YIp vector prepared in Example 4) is integrated into the genome, PCR was carried out using 0.3-0.6 μg of the yeast DNA prepared above as a template and a combination of synthetic oligonucleotide primers AUR-FWc and AUR-RVc, or AUR-SAL1 and AUR-SAL2. PCR conditions were as follows: 30 cycles of denaturation at 94°C for 30 sec, annealing at 55°C for 1 min and extension at 72°C for 3 min.

AUR-FWc: 5'-TCT CGA AAA AGG GTT TGC CAT-3' (SEQ ID NO: 57)

AUR-RVc: 5'-TCA CTA GGT GTA AAG AGG GCT-3' (SEQ ID NO: 58)

AUR-SAL1: 5'-TGT TGA AGC TTG CAT GCC TGC-3' (SEQ ID NO: 59)

AUR-SAL2: 5'-TTG TAA AAC GAC GGC CAG TGA-3' (SEQ ID NO: 60)

(4) Preparation of DIG-Labeled Probe DNAs

As hybridization probes, Probes I, II, III and V were prepared (Table 5).

Table 5.

| Hybridization Probes | | | | |
|---|---|---|---|---|
| Probe No. | Gene | Template | Primer 1 | Primer 2 |
| I | ERG20 | pT7ERG20 | SCFPS1 | SCFPS2 |
| II | BTS1 | pYES2-GGPS6 | BTS1 | (1008-982) |
| III V | HMG1 *AUR*01 | PYHMG1 pAUR123 | HMG1 AUR-RV | HMG1 AUR-FW |

Probe I:

Using the following synthetic oligonucleotides SCEPS1 and SCEPS2 as primers, a PCR fragment was obtained from an *S. cerevisiae* cDNA library (Clontech, Palo Alto, CA) and cloned into pT7blue T vector. Thus, pT7ERG20 was prepared.

SCFPS1: 5'-ATG GCT TCA GAA AAA GAA ATT AG-3' (SEQ ID NO: 61)

SCFPS2: 5'-CTA TTT GCT TCT CTT GTA AAC TT-3' (SEQ ID NO: 62)

Using pT7ERG20 as a template and SCEPS1 and SCEPS2 as primers, a DIG (digoxigenin)-labeld probe DNA was synthesized with PCR DIG Probe Synthesis Kit (Roche Diagnostics, Mannheim Germany). Experimental conditions were in accordance with the manufacturer's protocol attached to the kit.

PCR conditions were as follows: 30 cycles of denaturation at 94°C for 30 sec, annealing at 58°C for 1 min and extension at 72°C for 3 min. The resultant DIG-labeled probe DNA was subjected to agarose gel electrophoresis to examine the state of synthesis.

Probe II:

A DIG-labeled probe DNA was synthesized in the same manner as used for Probe I, using the following synthetic oligonucleotides as primers and pYESGGPS (see (3) in Example 2) as a template.

BTS1 (1-21): 5'-ATG GAG GCC AAG ATA GAT GAG-3' (SEQ ID NO: 63)

BTS1 (1008-988): 5'-TCA CAA TTC GGA TAA GTG GTC-3' (SEQ ID NO: 64)

Probe III:

A DIG-labeled probe DNA was synthesized in the same manner as used for Probe I, using the following synthetic oligonucleotides as primers and pYES-HMG1 (see (3) in Example 3) as a template.

HMG1 (1267-1293): 5'-AAC TTT GGT GCA AAT TGG GTC AAT GAT-3' (SEQ ID NO: 42)

HMG1 (2766-2740): 5'-TCC TAA TGC CAA GAA AAC AGC TGT CAC-3' (SEQ ID NO: 65)

Probe V:

A DIG-labeled probe DNA was synthesized in the same manner as used for Probe I, using the following synthetic oligonucleotides as primers and pAUR123 (Takara) as a template.

AUR-FW: 5'-ATG GCA AAC CCT TTT TCG AGA-3' (SEQ ID NO: 66)

AUR-RV: 5'-AGC CCT CTT TAC ACC TAG TGA-3' (SEQ ID NO: 67)

(5) Hybridization and Detection of Probes

Southern blot hybridization was carried out at a probe concentration of 20 ng/ml at 42°C for 24 hr using DIG Easy Hyb (Roche). Northern blot hybridization was carried out at a probe concentration of 100 ng/ml at 50°C for 24 hr using DIG Easy Hyb. Prior to each hybridization, prehybridization was carried out for 24 hr in DIG Easy Hyb solution at the same temperature used for the hybridization. After the hybridization, the membrane was washed 3 times with 2x SSC, 0.1% SDS at 65°C for 10 min each, and then 2 times with 0.2x SSC, 0.1% SDS at 65°C for 15-20 min each. Thereafter, the DIG-labeled probe in the membrane was allowed to generate chemiluminescence by using DIG Luminescent Detection Kit (Roche), followed by exposure of the blot to X-ray film for visualization.

(6) Determination of Enzyme Activity

Cells were harvested from each culture broth by centrifugation and disrupted at 4°C with glass beads in the same manner as in the preparation of RNA. Then, cells were suspended in sterilized water. The suspension was centrifuged at 12,000 r.p.m. for 10 min with a refrigerated microcentrifuge, and the resultant supernatant was recovered as a crude enzyme fraction. The protein concentration in the crude enzyme fraction was determined by Bio-Rad Protein Assay (Bio-Rad, Hercules, CA) using BSA as a standard protein. Ten µg of the crude enzyme fraction was reacted in 200 µl of the following reaction cocktail at 37°C for 40 min.

| |
|---|
| 0.125 mM [$^{14}C$] IPP (185 GBq/mol) |
| 0.125 mM geranyl diphosphate (Sigma Chemical, St. Louis, MO) |
| 100 mM Tris HCl (pH 7.0) |
| 10 mM NaF, 5 mM $MgCl_2$ |
| 5 mM 2-mercaptoethanol |
| 0.05% Triton X-100 |
| 0.005% BSA |

After the reaction, extended prenyl diphosphate was extracted with water-saturated butanol. An aliquot of the prenyl diphosphate was subjected to determination of radioactivity with a liquid scintillation counter. The remaining sample was dephosphorylated with potato acid phosphatase, spotted onto thin layer chromatography plate [plate: LKC18 (Whatman, Clifton, NJ], and then the plate was developed [developer solvent: $H_2O$/acetone = 1:19]. The autoradiogram was visualized with Bio Image Analyzer BAS2000 (Fuji Film) and the relative radioactivities were determined, according to the method of Koyama *et al.* (Koyama T., Fujii, H. and Ogura, K., 1985, Meth. Enzymol. 110: 153-155).

(7) Results and Observations

(7-1) Southern Blot Hybridization and PCR Mapping

The results of southern blot hybridization are shown in Fig. 9. The results of PCR mapping in the vicinity of *AUR1* are shown in Fig. 10. In Figs. 9 and 10, lanes I to 7 correspond to the numbers of clones (No. 1 to No. 7) used in (6). "N" represents DNA digested with *Nde*I; and "S" represents DNA digested with *Stu*I. DNAs used in individual lanes were prepared from the following strains.

Lane 1: A451; Lane 2: AURGG101; Lane 3: AURGG102; Lane 4: pYES-HMG1/A451; Lane 5: pYHMG044/A451; Lane 6: pYES-HMG1/AURGG101; Lane 7: pYHMG044/AURGG101

It was found that *ERG20* (FPP synthase gene) is contained in the same manner in all of the clones tested and that there is no change in the vicinity of *ERG20* in the genome of each clone (Fig. 9).

When *BTS*1 (GGPP synthase gene) and *AUR1* were used as probes, it was found that *BTS1* is integrated into the region of *AUR1* in AURGG102, but the bands appearing in AURGG101 are the same as those appearing in the host strain A451. In AuRGG101, only *AUR1* gene is replaced with pAUR101-derived *AUR1-C* gene; it was found that the *GAL1-BTS1* fragment is not integrated into the genome of this clone. Duplication of *AUR*1 locus resulting from genomic integration was detected by PCR. As expected, a band was not detected in AURGG101 but detected only in AURGG102 (Fig. 10).

In Fig. 9, when *HMG1* was used as a probe, a plasmid-derived band appeared in NdeI-digested DNAs (lanes 4-7). In *Stu*I-digested DNAs, it is expected that a 8.2 kbp band derived from the plasmid (overlapping a 8.3 kbp band derived from the genome) should appear as in clone 1-2 (No. 4). However, a band shift was observed in clone 13-2 (No. 6) and clone 15-2 (No. 7) as a result of recombination between the vicinity of *HMG1* in the genome and the plasmid introduced.

From the results of Southern blot hybridization and PCR mapping, the genotypes of the clones used this time can be summarized as shown in Table 6 below. In this Table, "AUR" means a medium to which aureobasidin has been added. "Medium 1" means a medium for preculture, and "Medium 2" means a medium for subsequent culture.

Table 6

| Clone No. | Designation | Integrated Gene | Gene in Plasmid | Medium 1 | Medium 2 |
|---|---|---|---|---|---|
| 1 | A451 | - | - | SD | SG |
| 2 | AURGG101 | - | - | SD-AUR | SG-AUR |

Table 6 (continued)

| Clone No. | Designation | Integrated Gene | Gene in Plasmid | Medium 1 | Medium 2 |
|---|---|---|---|---|---|
| 3 | AURGG102 | *BTS1* | - | SD-AUR | SG-AUR |
| 4 | 1-2 | - | *HMG1* | SD-U | SG-U |
| 5 | 3-2 | - | *HMG1Δ 044* | SD-U | SG-U |
| 6 | 13-2 | - | *HMG1* | SD-U-AUR | SG-U-AUR |
| 7 | 15-2 | - | *HMG1Δ 044* | SD-U-AUR | SG-U-AUR |

(7-2) Northern Blot Hybridization

The results of Northern blot hybridization are shown in Fig. 11. Probes I, II and III as shown in Table 5 were used as probe.

In Fig. 11, the clones used in lanes 1 to 7 are the same as used in Fig. 9. Mark "-" indicates transcripts in SD medium, and mark "+" indicates transcripts in SG medium.

*ERG20* transcript showed a tendency to decrease in clone 13-2 (No. 6) and clone 15-2 (No. 7) when *GAL1*p transcriptional induction was applied by SG medium.

When the transcription of genes under the control of *GAL1* transcription promoter was induced by SG medium, the induction of *BTS1* transcript increased only in a clone in which *GAL1*p-BTS1 fragment has been integrated into the genome, i.e., AURGG102 (No. 3).

However, when compared with *HMG1* transcript, it is seen that the degree of transcription induction of BTS1 is lower. When transcription was induced by SG medium, *HMG1* transcript increased remarkably in clones No.4 to No. 7 in which *GAL1*p-*HMG1* fragment has been transferred by a plasmid.

(7-3) Prenyl-diphosphate synthase Activity

The activity of prenyl-diphosphate synthase in the crude enzyme fraction was determined using geranyl diphosphate (GPP) and [$^{14}$C]-labeled IPP as allylic diphosphate substrates.

The prenyl diphosphates synthesized with GPP and [$^{14}$C] IPP as substrates were dephosphorylated and analized by TLC. Then, the ratioactivity of each spot on the plate was examined. As a result, FPP synthase activity was high, and next to that, HexPP (hexaprenyl diphosphate) synthase activity was detected that was by far higher than GGPP synthase activity. Then, relative amounts of reaction products were calculated from autoradiogram, followed by calculation of specific activity per gross protein. The results are shown in Fig. 12. In Fig. 12A, the upper panel shows FPP synthase (FPS) activity, and the lower panel shows GGPP synthase (GGPS) activity. In Fig. 12B, the upper panel shows HexPP synthase (HexPS) activity, and the lower panel shows PTase (total prenyl-diphosphate synthase) activity. Gray columns show the results in SD medium, and white columns show the results in SG medium. A large part of the total prenyl-diphosphate synthase activity is FPP synthase activity. An increase in this activity caused by SG medium was observed. In particular, total prenyl-diphosphate synthase activity remarkably increased in clone 13-2 (No. 6) and clone 15-2 (No. 7) that produce FOH in a large quantity (see Example 9). As a whole, when GPP is used as an allylic substrate, GGPP synthase activity is about 1/20000 of FPP synthase activity and about 1/300 of HexPP synthase activity. HexPP synthase activity decreased in SG medium.

[EXAMPLE 7] Cultivation of Recombinants and Production of Prenyl Alcohols

(1) Production of Prenyl Alcohols When *HMG1* Gene with a Constitutive Promoter Was Introduced into A451 (such a recombinant is expressed as "Constitutive Promoter; *HMG1 ;* A451"; this way of expression applies to the remaining part of the specification). For industrial application of FOH high yielding recombinants, the use of a constitutive promoter is advantageous since it allows the use of cheap, conventional media. Then, HMG1 gene was expressed under the control of a constitutive promoter using as a host *S. Cerevisiae* A451 (ATCC200589) that was recognized in preliminary experiments to have potentiality to produce FOH.

*HMG1* gene (PCR error-corrected gene) was introduced into vector pRS434GAP or pRS444GAP each containing a constitutive promoter GAPp (=*TDH3*p) to thereby prepare pRS434GAP-HMG1 and pRS444GAP-HMG1, respectively. These plasmids were introduced into A451 to obtain recombinants, which were designated pRS434GAP-HMG1/A451 and pRS444GAP-HMG1/A451.

Ten colonies were selected randomly from each of the yeast recombinants into which HMG-CoA reductase gene had been introduced. These colonies were inoculated into SD-W medium [obtained by adding CSM (-TRP) to SD] that is an SD selection medium for a marker gene *TRP1,* and precultured therein. Then, 250 µl of the preculture (when a yeast recombinant with a constitutive promoter was precultured, this amount was added not only in this experiment but in other experiments described later) was added to 2.5 ml of YM medium and cultured at 26°C for 4 days with rotary shaking at 130 r.p.m.

After completion of the cultivation, 2.5 ml of methanol was added to the culture broth and mixed. Then, about 5 ml of pentane was added thereto and agitated vigorously. The resultant mixture was left stationary. The pentane layer was transferred into a new glass tube, followed by evaporating the pentane in a draft to thereby concentrate solute components. Then, the resultant solution was subjected to gas chromatography/mass spectrography (GC/MS) to identify prenyl alcohols and quantitatively determine them with undecanol as an internal standard. At that time, in order to examine the degree of cell growth, 50 μl of the culture broth was diluted 30-fold with water, followed by determination of absorbance at 600 nm.

GC/MS was carried out with HP6890/5973 GC/MS system (Hewlett-Packard, Wilmington, DE). The column used was HP-SMS (0.25 mm x 30 m; film thickness 0.25 μm). Analytical conditions were as described below. The same conditions were used for all the GC/MS analyses in this specification.

Inlet temperature: 250°C
Detector temperature: 260°C

[MS zone temperatures]

MS Quad:150°C
MS Source: 230°C
Mass scan range: 35-200

[Injection parameters]

Automated injection mode
Sample volume: 2μl
Methanol washing: 3 times; hexane washing: twice
Split ratio: 1/20
Carrier gas: helium 1.0 ml/min
Solvent retardation: 2 min

[Oven heating conditions]

115°C far 90 sec
Heating up to 250°C at 70°C /min and retaining for 2 min
Heating up to 300°C at 70°C /min and retaining for 7 min

After Time 0

Internal standard: 0.01μl of 1-undecanol in ethanol
Reliable standards: (*E*)-Nerolidol (Eisai)
(*all-E*)-Farnesol (Sigma)
(*all-E*)-Geranylgeraniol (Eisai)
Squalene (Tokyo Kasei Kogyo)

The results of determination of prenyl alcohol yields are shown in Figs. 13-15. Fig. 14 shows a result selecting 10 colonies from clone No. 3 of pRS434 shown in Fig. 13. Fig. 15 shows a summary of data shown in Fig. 13. An FOH yield of 4.9 mg/L was recognized in colony No. 10 (pRS434) in Fig. 14. In Fig. 15, "434" and "444" represent the results when pRS434GAP and pRS444GAP vectors were used, respectively. The column at the utmost right represents the results when the host (A451) before gene transfer was cultured.

These results revealed that, when A451 was used as a host, the productivity of both NOH and FOH increased in pRS434GAP-HMG/A451. FOH could be produced at 3.8 mg/L on the average, with 11.2 mg/L at the highest, by merely activating the transcription of *HMG1* gene (Fig. 13). In pRS444GAP-HMG1/A451, the yield of NOH was 0.16 mg/L at the highest; this clone was found to be effective mainly in the production of FOH.

It is believed that A451 is different from conventionally used recombinant DNA host strains (such as YPH499) in the balance between squalene synthase activity and mevalonate pathway activity, and that farnesyl diphosphate (FPP), an intermediate metabolite, is accumulated when multiple copies *of HMG1* gene are present or the transcription of this gene is activated; as a result, FOH (a dephosphorylated product of FPP) is produced. Alternatively, it is believed that the ability to produce FOH was rendered to A451 as a result of mutation of *CAN1* or *ARO7* seen in the genotype of A451. This means that any strain having a balance similar to that of A451 between squalene synthase activity and mevalonate pathway activity, or any strain having mutation in *CAN1* and/or *ARO7* can be expected to produce FOH upon introduction of *HMG1.* With respect to FOH production, a tendency was observed that the use of pRS434GAP vector exhibits better productivity than pRS444GAP vector.

(2) Constitutive Promoter; *HMG1;* YPH499

The plasmids listed below that had been obtained by inserting *HMG1* gene (PCR error-corrected gene) into vector pRS414PTadh, pRS414TPadh, pRS434GAP, pRS444GAP, pRS434PGK, pRS444PGK, pRS434TEF or pRS444TEF comprising a constitutive promoter *ADH1*p, *GAP*p (=*TDH3*p), *PGK1*p or *TEF2*p, were introduced into YPH499.

pRS414PTadh-HMG1
pRS414TPadh-HMG1
pRS434GAP-HMG1
pRS444GAP-HMG1
pRS434PGK-HMG1
pRS444PGK-HMG1
pRS434TEF-HMG1
pRS444TEF-HMG1

The resultant recombinants were cultured in YM medium supplemented with adenine sulfate at 40µg/ml (the same medium was also used for other recombinants when YPH499 was used as a host). Culture conditions were the same as in (1) above. After completion of the cultivation, the pentane extract fraction from the culture broth was subjected to GC/MS analyses. The yields of prenyl alcohols (NOH and FOH) were determined.

The results are shown in Fig. 16. In Fig. 16, "414PT", "414TP", "434" and "444" represent the results when pRS414PTadh, pRS414TPadh, pRS434xxx and pRS444xxx (where xxx indicates the alphabetical part of the name of the gene used in the promoter) vectors were used, respectively. The right utmost column represents the results when the host (YPH499) before gene transfer was cultured. As shown in Fig. 16, the yield of FOH is improved in every recombinant, and an increase in NOH productivity is observed in pRS434GAP-HMG1-, pRS444GAP-HMG1-, pRS434TEF-HMG1-, pRS444TEF-HMG1-, pRS434PGK-HMG1- or pRS444PGK-HMG1-introduced YPH499 clone.

(3) Constitutive Promoter; *HMG1;* EUG

A451-, YPH499- or YPH500-derived EUG clones that exhibit Glc growth inhibition and have integrated the DNA of interest into the genome completely were selected (i.e., EUG8, EUG12 and EUG27). Then, plasmid pRS434GAP-HMG1 or pRS444GAP-HMG1 obtained by inserting *HMG1* gene (PCR error-corrected gene) into vector pRS434GAP or pRS444GAP comprising a constitutive promoter *GAP*p (=TDH3p) was introduced into EUG8 (NOH yield: 0.021 mg/L; FOH yield: 0.20 mg/L), EUG12 (NOH yield: 0.13 mg/L; FOH yield: 5.9 mg/L) and EUG27 (NOH yield: 0.038 mg/L; FOH yield: 3.2 mg/L). The yields of prenyl alcohols in the resultant recombinants were determined.

The results are shown in Fig. 17 (EUG8), Fig. 18 (EUG12) and Fig. 19 (EUG27).

EUG clones produce FOH when cultured in YM medium containing glucose (Glc) as the carbon source. The introduction of *HMG1* gene improved the productivity of FOH. A451-derived EUG8 is different from YPH499-derived EUG12 and YPH500-derived EUG27 in production profile. It is believed that clones derived from YPH strains are more suitable for production.

These results revealed that it is possible to improve the productivity of prenyl alcohols in A451-derived clones, YPH499-derived clones and YPH500-derived clones by introducing *HMG1* thereinto.

(4) Inducible promoter; *HMG1*; A451 or AURGG101

Plasmid pYES2-HMG obtained by inserting *HMG1'* (a PCR error mutant of *HMG1)* into vector pYES2 comprising an inducible promoter *GAL1*p was introduced into A451 and AURGG101 (A451, *aur1::AUR1-C)* prepared in Example 4.

Each of the resultant recombinants was precultured. Then, 25 µl of the preculture (when a yeast recombinant with an inducible promoter was precultured, this amount was added not only in this experiment but in other experiments described later) was added to 2.5 ml of SG medium and cultured at 26°C for 4 days with rotary shaking at 130 r.p.m. Prior to the addition to SG medium, cells were washed with physiological saline so that no glucose component was brought into SG medium. After completion of the cultivation in SG medium, the yields of prenyl alcohols (NOH and FOH) were determined.

As a result, pYES-HMG1/AURGG101 clones produced NOH at 1.43 mg/L on the average and FOH at 4.31 mg/L on the average. Thus, prenyl alcohol high yielding clones were obtained even in those recombinants in which pYES-HMG1 comprising *HMG1'* (a mutant *MMG1)* has been transferred (Fig. 20). Fig. 20A shows the results when A451 was used. Fig. 20B shows the results when AURGG101 was used. pYES is a vector that was used for the gene transfer.

When AURGG101 derived from A451 was used as a host and *GAL1*p as a promoter, clones were obtained that highly produced FOH in particular.

(5) Inducible Promoter; *HMG1;* W303-1A or W303-1B

Plasmid pYES2-HMG obtained by inserting *HMG1* into vector pYES2 comprising an inducible promoter *GAL1*p was introduced into W303-1A and W303-1B. The resultant recombinants were cultured in SG medium. Thereafter, the yields of prenyl alcohols (NOH and FOH) were determined (Fig. 21).

When *HMG1* was introduced (the column at the left in each graph), the yields of both products increased. W303 clones were characterized by their effectiveness in the production of NOH.

[EXAMPLE 8] Production of Prenyl Alcohols by High Expression of Deletion Mutant Type HMG-CoA Reductase Gene

In Example 7, prenyl alcohol-producing recombinant yeasts were developed using a full-length HMG-CoA reductase gene or a mutant thereof. In this Example, prenyl alcohol-producing recombinant yeasts were developed using a deletion mutant of HMG-CoA reductase gene, and alcohol production was carried out.

(1) Inducible Promoter; *HMG1Δ;* A451

The following plasmids (described in (4) in Example 3) obtained by inserting a deletion mutant of *HMG1'* gene into a vector pYES2 comprising an inducible promoter *GAL1*p were introduced separately into A451.

pYHMG026
pYHMG044
pYHMG056
pYHMG062
pYHMG076
pYHMG081
pYHMG100
pYHMG112
pYHMG122

After completion of cultivation in SG medium, the yields of prenyl alcohols (NOH and FOH) were determined (Fig. 22).

When a deletion mutant type *HMG1* gene was expressed with an inducible promoter, FOH high yielding clones were obtained. For FOH production, *HMG1Δ044* and *HMG1Δ*122 were effective (FOH yield was 0.0 mg/L on the average in *HMG1*/A451 clones).

(2) Inducible promoter; *HMG1Δ;* AURGG101

The following plasmids (described in (4) in Example 3) obtained by inserting a deletion mutant of *HMG1'* gene into a vector pYES2 comprising an inducible promoter *GAL1p* were introduced separately into AURGG101.

pYHMG026
pYHMG044
pYHMG056
pYHMG062
pYHMG076
pYHMG081
pYHMG100
pYHMG112
pYHMG122
pYHMG133

After completion of cultivation in SG medium, the yields of prenyl alcohols (NOH and FOH) were determined (Figs. 22 and 23). In Fig. 23, the right utmost columns represent the yields of host AURGG101 before gene transfer. Fig. 24 shows enlarged graphs of Fig. 23.

In particular, when *HMG1Δ044* was expressed with an inducible promoter, a prenyl alcohol high yielding clone (clone 15-2) was obtained. NOH yield and FOH yield in this recombinant were 12 mg/L and 31.7 mg/L on the average, respectively (Fig. 23). The maximum yields were 23 mg/L and 53 mg/L, respectively. In those recombinants integrating *HMG1Δ* other than *HMG1Δ*044, clones were obtained that produce NOH and FOH at about 0.05-0.06 mg/L (Fig. 24). The recombinant integrating *HMG1Δ*062 produced NOH at 0.11 mg/L and FOH at 0.13 mg/L at the maximum.

(3) Constitutive Promoter; HMG1, Inducible promoter; *HMG1Δ;* AURGG101

pRS434GAP-HMG1 or pRS444GAP-HMG1 prepared in (2) in Example 7 was introduced into clone 15-2 prepared in (2) above in this Example. After completion of cultivation in SG medium, the yields of prenyl alcohols (NOH and FOH) were determined (Fig 25).

As a result, a clone was obtained that produced FOH at 66 mg/L at the maximum, improving the FOH yield of 53 mg/L of clone 15-2.

[EXAMPLE 9] Production of Prenyl Alcohols by *Escherichia coli*

(1) The plasmids obtained in (4), (5) and (7) in Example 2 were introduced separately into E. *coli* JM109. To a 50 ml medium containing 2x YT and 1 mM IPTG in a 300 ml flask, 0.5 ml of a preculture was added. Antibiotics (ampicillin and chloramphenicol), if necessary, 5 mM (about 0.12% (w/v)) IPP and 5 mM DMAPP were added thereto, and the cells were cultured at 37°C for 16 hr under shaking.

After completion of the cultivation at 37°C for 16 hr, potato acid phosphatase was added to the culture supernatant and the cell pellet disrupted by sonication, followed by extraction of prenyl alcohols with pentane as an organic solvent. Then, the prenyl alcohols were identified and quantitatively determined by GC/MS as described in (1) in Example 7.

As a result, FOH yield in the presence of IPP and DMAPP was 86.4 mg/L when wild type *ispA* was introduced (pALispA in Fig. 29) and 12.0 mg/L when wild type *ƒps* was introduced (pFE15NS2.9-1 in Fig. 26). Even when a mutant *ispA* was introduced, JM109 retaining p18M or p18E produced FOH at 11.1 mg/L and 16.3 mg/L, respectively; JM109 retaining p4D produced FOH at 72.7 mg/L; and in JM109 retaining p16D, FOH yield reached 93.3 mg/L (Fig. 26).

(2) In order to ascertain whether or not prenyl alcohol production can be carried out without the addition of IPP and DMAPP, plasmids pALispA4 and p3-47-11 or plasmids pALispA4 and p3-47-13 obtained in (4) and (6) in Example 2 were introduced into *E. coli* JM109. To a medium containing 50 ml of 2x YT per 300 ml flask and 1 mM IPTG, 0.5 ml of a preculture was added. Antibiotics (ampicillin and chloramphenicol) were added thereto, if necessary. Then, the cells were cultured at 37°C for 16 hr under shaking. The results revealed that JM109 retaining pALispA4 and p3-47-11 has FOH production ability of 0.15 mg/L and that JM109 retaining pALispA4 and p3-47-13 has FOH production ability of 0.16 mg/L (Fig. 27).

Thus, it was found that *E. coli* retining plasmid p3-47-11 or p3-47-13 containing *idi* and plasmid pALispA4 containing *ispA, i.e., E. coli* incorporating *idi* and *ispA* has ability to produce FOH at 0.15-0.16 mg/L even without the addition of IPP and DMAPP.

[EXAMPLE 10] Mass Production of FOH

1. Culture Conditions

One platinum loopful of the recombinant yeast clone 15-2 (AURGG101 retaining pYHMG044) described in (2) in Example 8 was inoculated from slants into CSM-URA medium (BIO 101 Inc.) and DOB medium (BIO 101 Inc.) (200 ml in a 500 ml Erlenmeyer flask with baffle plates) and cultured at 30°C for 2 days under shaking at 130 r.p.m. Then, in order to remove the glucose contained in the culture broth completely, centrifugation (at 1500 g, for 5 min, at 4°C) and washing with sterilized physiological saline were repeated 3 times. Subsequently, 50 ml of the culture was inoculated into a fermenter (1%) and cultured under the conditions described below.

Fermenter medium:

5% galactose
Amino acid-containing YNB (Difco)
1% soybean oil (Nacalai Tesque)
0.1% Adekanol LG109 (Asahi Denka)

Operational conditions:

Cultivation apparatus: MSJ-U 10 L Cultivation Apparatus (B. E. Marubishi)
Medium volume: 5 L
Cultivation temperature: 26°C
Aeration rate: 1 vvm
Agitation: 300 rpm
pH: controlled proportionally using 4 N sodium hydroxide solution and 2N hydrochloric acid solution, and with the following parameters:

| | |
|---|---|
| Proportional Band | 1.00 |
| Non Sensitive Band | 0.15 |
| Control Period | 16 Sec |
| Full Stroke | 1 Sec |
| Minimum Stroke | 0 Sec |

2. Cell Counting

One hundred microliters of the culture broth was diluted 1- to 20-fold with physiological saline. Then, cells were counted with a hematometer (Hayashi Rikagaku). The number of cells in 0.06 $mm^2$ (corresponding to 9 minimum grids) was counted, followed by calculation of the average of 4 counts. Then, using the formula below, cell count per liter of the culture broth was calculated.

$$\text{Cell count } (1\text{x}10^9/\text{L broth}) = 0.444 \text{ x (cell count in } 0.06 \text{ mm}^2) \text{ x dilution rate}$$

3. Quantitative Determination of FOH

FOH was identified and quantitatively determined in the same manner as in Example 8.

4. Results

The results are shown in Fig. 28. As seen from Fig. 28, it was demonstrated that a recombinant yeast obtained by introducing *HMG1*Δ044 (a deletion mutant of the mutant type HMG-CoA reductase gene HMG1') into A451-derived AURGG101 can produce 146 mg of FOH per liter of the culture broth on the average and 158 mg/L at the maximum.
All publications, patents and patent applications cited herein are incorporated herein by reference in their entirety.

INDUSTRIAL APPLICABILITY

According to the present invention, a method of producing prenyl alcohols is provided. According to the present invention, biologically active prenyl alcohols can be obtained in large quantities. From these prenyl alcohols, isoprenoids/terpenoids with various physiological activities can be synthesized. The active prenyl alcohols provided in the invention may also be used as materials to find out those substances having a novel physiological activity.

SEQUENCE LISTING FREE TEXT

SEQ ID NOS: 18-74: synthetic DNA

## Claims

1. A method of producing a prenyl alcohol, comprising creating a recombinant obtained by transferring into a host a recombinant DNA for expression or a DNA fragment for genomic integration each comprising:

   (i) a hydroxymethylglutaryl-CoA reductase gene, an isopentenyl-diphosphate Δ-isomerase gene or a famesyl-diphosphate synthase gene, or a mutant of any one of said genes,
   (ii) a transcription promoter, and
   (iii) a transcription terminator;

   culturing said recombinant;
   and recovering the prenyl alcohol from the resultant culture.

2. The method according to claim 1, wherein the prenyl alcohol is a $C_{15}$ prenyl alcohol.

3. The method according to claim 2, wherein the $C_{15}$ prenyl alcohol is farnesol or nerolidol.

4. The method according to claim 3, wherein the concentration of farnesol or nerolidol in the resultant culture is at least 0.05 mg/L.

5. The method according to any one of claims 1 to 4, wherein the hydroxymethylglutaryl-CoA reductase gene or mutant thereof comprises one nucleotide sequence selected from the group consisting of SEQ ID NOS: 1, 3, 5 and 7-16.

6. The method according to any one of claims 1 to 4, wherein the farnesyl-diphosphate synthase gene or mutant thereof comprises one nucleotide sequence selected from the group consisting of SEQ ID NOS: 75, 77, 79, 81 and 83.

7. The method according to any one of claims 1 to 4, wherein the isopentenyl-diphosphate Δ-isomerase gene or mutant thereof comprises the nucleotide sequence as shown in SEQ ID NO: 85.

8. The method according to any one of claims 1 to 7, wherein the transcription promoter is one selected from the group consisting of *ADH1* promoter, *TDH3* (GAP) promoter, *PGK1* promoter, *TEF2* promoter, *GAL1* promoter and *tac* promoter.

9. The method according to any one of claims 1 to 7, wherein the transcription terminator is *ADH1* terminator or *CYC1* terminator.

10. The method according to any one of claims 1 to 9, wherein the host is yeast or *Escherichia coli.*

**11.** The method according to claim 10, wherein the yeast is *Saccharomyces cerevisiae.*

**12.** The method according to claim 11, wherein the *Saccharomyces cerevisiae* is A451 strain, YPH499 strain, YPH500 strain, W303-1A strain or W303-1B strain, or a strain derived from any one of said strains.

**13.** A recombinant obtained by transferring into a host a recombinant DNA for expression or a DNA fragment for genomic integration each comprising:

(i) a hydroxymethylglutaryl-CoA reductase gene, an isopentenyl-diphosphate Δ-isomerase gene or a farnesyl-diphosphate synthase gene, or a mutant of any one of said genes,
(ii) a transcription promoter, and
(iii) a transcription terminator,

said recombinant being capable of producing at least 0.05 mg/L of farnesol or nerolidol.

**14.** The recombinant according to claim 13, wherein the hydroxymethylglutaryl-CoA reductase gene or mutant thereof comprises one nucleotide sequence selected from the group consisting of SEQ ID NOS: 1, 3, 5 and 7-16.

**15.** The recombinant according to claim 13, wherein the farnesyl-diphosphate synthase gene or mutant thereof comprises one nucleotide sequence selected from the group consisting of SEQ ID NOS: 75, 77, 79, 81 and 83.

**16.** The recombinant according to claim 13, wherein the isopentenyl-diphosphate Δ-isomerase gene or mutant thereof comprises the nucleotide sequence as shown in SEQ ID NO: 85.

**17.** The recombinant according to any one of claims 13 to 16, wherein the transcription promoter is one selected from the group consisting of *ADH1* promoter, *TDH3* (GAP) promoter, *PGK1* promoter, *TEF2* promoter, *GAL1* promoter and *tac* promoter.

**18.** The recombinant according to any one of claims 13 to 16, wherein the transcription terminator is *ADH1* terminator or *CYC1* terminator.

**19.** The recombinant according to any one of claims 13 to 18, wherein the host is yeast or *Escherichia coli.*

**20.** The recombinant according to claim 19, wherein the yeast is *Saccharomyces cerevisiae.*

**21.** The recombinant according to claim 20, wherein the *Saccharomyces cerevisiae* is A451 strain, YPH499 strain, YPH500 strain, W303-1A strain or W303-1B strain, or a strain derived from any one of said strains.

SEQUENCE LISTING

<110> TOYOTA JIDOSHA KABUSHIKI KAISHA

<120> A method of producing prenylalcohol

<130> PH-1412PCT

<140>

<141>

<150> JP2000-401701

<151> 2000-12-28

<160> 86

<170> PatentIn Ver. 2.0

<210> 1

<211> 3165

<212> DNA

<213> Saccharomyces cerevisiae

<220>

<221> CDS

<222> (1)..(3162)

<400> 1

atg ccg ccg cta ttc aag gga ctg aaa cag atg gca aag cca att gcc 48
Met Pro Pro Leu Phe Lys Gly Leu Lys Gln Met Ala Lys Pro Ile Ala
1 5 10 15

tat gtt tca aga ttt tcg gcg aaa cga cca att cat ata ata ctt ttt 96
Tyr Val Ser Arg Phe Ser Ala Lys Arg Pro Ile His Ile Ile Leu Phe
20 25 30

tct cta atc ata tcc gca ttc gct tat cta tcc gtc att cag tat tac 144
Ser Leu Ile Ile Ser Ala Phe Ala Tyr Leu Ser Val Ile Gln Tyr Tyr
35 40 45

ttc aat ggt tgg caa cta gat tca aat agt gtt ttt gaa act gct cca 192
Phe Asn Gly Trp Gln Leu Asp Ser Asn Ser Val Phe Glu Thr Ala Pro
50 55 60

aat aaa gac tcc aac act cta ttt caa gaa tgt tcc cat tac tac aga 240
Asn Lys Asp Ser Asn Thr Leu Phe Gln Glu Cys Ser His Tyr Tyr Arg
65 70 75 80

gat tcc tct cta gat ggt tgg gta tca atc acc gcg cat gaa gct agt 288
Asp Ser Ser Leu Asp Gly Trp Val Ser Ile Thr Ala His Glu Ala Ser
85 90 95

gag tta cca gcc cca cac cat tac tat cta tta aac ctg aac ttc aat 336
Glu Leu Pro Ala Pro His His Tyr Tyr Leu Leu Asn Leu Asn Phe Asn
100 105 110

agt cct aat gaa act gac tcc att cca gaa cta gct aac acg gtt ttt 384
Ser Pro Asn Glu Thr Asp Ser Ile Pro Glu Leu Ala Asn Thr Val Phe
115 120 125

gag aaa gat aat aca aaa tat att ctg caa gaa gat ctc agt gtt tcc 432
Glu Lys Asp Asn Thr Lys Tyr Ile Leu Gln Glu Asp Leu Ser Val Ser
130 135 140

aaa gaa att tct tct act gat gga acg aaa tgg agg tta aga agt gac 480
Lys Glu Ile Ser Ser Thr Asp Gly Thr Lys Trp Arg Leu Arg Ser Asp
145 150 155 160

aga aaa agt ctt ttc gac gta aag acg tta gca tat tct ctc tac gat 528
Arg Lys Ser Leu Phe Asp Val Lys Thr Leu Ala Tyr Ser Leu Tyr Asp
165 170 175

gta ttt tca gaa aat gta acc caa gca gac ccg ttt gac gtc ctt att 576
Val Phe Ser Glu Asn Val Thr Gln Ala Asp Pro Phe Asp Val Leu Ile
180 185 190

atg gtt act gcc tac cta atg atg ttc tac acc ata ttc ggc ctc ttc 624
Met Val Thr Ala Tyr Leu Met Met Phe Tyr Thr Ile Phe Gly Leu Phe
195 200 205

aat gac atg agg aag acc ggg tca aat ttt tgg ttg agc gcc tct aca 672
Asn Asp Met Arg Lys Thr Gly Ser Asn Phe Trp Leu Ser Ala Ser Thr
210 215 220

gtg gtc aat tct gca tca tca ctt ttc tta gca ttg tat gtc acc caa 720

Val Val Asn Ser Ala Ser Ser Leu Phe Leu Ala Leu Tyr Val Thr Gln
225 230 235 240

tgt att cta ggc aaa gaa gtt tcc gca tta act ctt ttt gaa ggt ttg 768
Cys Ile Leu Gly Lys Glu Val Ser Ala Leu Thr Leu Phe Glu Gly Leu
245 250 255

cct ttc att gta gtt gtt gtt ggt ttc aag cac aaa atc aag att gcc 816
Pro Phe Ile Val Val Val Val Gly Phe Lys His Lys Ile Lys Ile Ala
260 265 270

cag tat gcc ctg gag aaa ttt gaa aga gtc ggt tta tct aaa agg att 864
Gln Tyr Ala Leu Glu Lys Phe Glu Arg Val Gly Leu Ser Lys Arg Ile
275 280 285

act acc gat gaa atc gtt ttt gaa tcc gtg agc gaa gag ggt ggt cgt 912
Thr Thr Asp Glu Ile Val Phe Glu Ser Val Ser Glu Glu Gly Gly Arg
290 295 300

ttg att caa gac cat ttg ctt tgt att ttt gcc ttt atc gga tgc tct 960
Leu Ile Gln Asp His Leu Leu Cys Ile Phe Ala Phe Ile Gly Cys Ser
305 310 315 320

atg tat gct cac caa ttg aag act ttg aca aac ttc tgc ata tta tca 1008
Met Tyr Ala His Gln Leu Lys Thr Leu Thr Asn Phe Cys Ile Leu Ser
325 330 335

gca ttt atc cta att ttt gaa ttg att tta act cct aca ttt tat tct 1056
Ala Phe Ile Leu Ile Phe Glu Leu Ile Leu Thr Pro Thr Phe Tyr Ser

340 345 350

gct atc tta gcg ctt aga ctg gaa atg aat gtt atc cac aga tct act 1104
Ala Ile Leu Ala Leu Arg Leu Glu Met Asn Val Ile His Arg Ser Thr
355 360 365

att atc aag caa aca tta gaa gaa gac ggt gtt gtt cca tct aca gca 1152
Ile Ile Lys Gln Thr Leu Glu Glu Asp Gly Val Val Pro Ser Thr Ala
370 375 380

aga atc att tct aaa gca gaa aag aaa tcc gta tct tct ttc tta aat 1200
Arg Ile Ile Ser Lys Ala Glu Lys Lys Ser Val Ser Ser Phe Leu Asn
385 390 395 400

ctc agt gtg gtt gtc att atc atg aaa ctc tct gtc ata ctg ttg ttt 1248
Leu Ser Val Val Val Ile Ile Met Lys Leu Ser Val Ile Leu Leu Phe
405 410 415

gtc ttc atc aac ttt tat aac ttt ggt gca aat tgg gtc aat gat gcc 1296
Val Phe Ile Asn Phe Tyr Asn Phe Gly Ala Asn Trp Val Asn Asp Ala
420 425 430

ttc aat tca ttg tac ttc gat aag gaa cgt gtt tct cta cca gat ttt 1344
Phe Asn Ser Leu Tyr Phe Asp Lys Glu Arg Val Ser Leu Pro Asp Phe
435 440 445

att acc tcg aat gcc tct gaa aac ttt aaa gag caa gct att gtt agt 1392
Ile Thr Ser Asn Ala Ser Glu Asn Phe Lys Glu Gln Ala Ile Val Ser
450 455 460

```
gtc acc cca tta tta tat tac aaa ccc att aag tcc tac caa cgc att    1440
Val Thr Pro Leu Leu Tyr Tyr Lys Pro Ile Lys Ser Tyr Gln Arg Ile
465                 470                 475                 480

gag gat atg gtt ctt cta ttg ctt cgt aat gtc agt gtt gcc att cgt    1488
Glu Asp Met Val Leu Leu Leu Leu Arg Asn Val Ser Val Ala Ile Arg
                485                 490                 495

gat agg ttc gtc agt aaa tta gtt ctt tcc gcc tta gta tgc agt gct    1536
Asp Arg Phe Val Ser Lys Leu Val Leu Ser Ala Leu Val Cys Ser Ala
            500                 505                 510

gtc atc aat gtg tat tta ttg aat gct gct aga att cat acc agt tat    1584
Val Ile Asn Val Tyr Leu Leu Asn Ala Ala Arg Ile His Thr Ser Tyr
        515                 520                 525

act gca gac caa ttg gtg aaa act gaa gtc acc aag aag tct ttt act    1632
Thr Ala Asp Gln Leu Val Lys Thr Glu Val Thr Lys Lys Ser Phe Thr
    530                 535                 540

gct cct gta caa aag gct tct aca cca gtt tta acc aat aaa aca gtc    1680
Ala Pro Val Gln Lys Ala Ser Thr Pro Val Leu Thr Asn Lys Thr Val
545                 550                 555                 560

att tct gga tcg aaa gtc aaa agt tta tca tct gcg caa tcg agc tca    1728
Ile Ser Gly Ser Lys Val Lys Ser Leu Ser Ser Ala Gln Ser Ser Ser
                565                 570                 575
```

tca gga cct tca tca tct agt gag gaa gat gat tcc cgc gat att gaa 1776
Ser Gly Pro Ser Ser Ser Ser Glu Glu Asp Asp Ser Arg Asp Ile Glu
580 585 590

agc ttg gat aag aaa ata cgt cct tta gaa gaa tta gaa gca tta tta 1824
Ser Leu Asp Lys Lys Ile Arg Pro Leu Glu Glu Leu Glu Ala Leu Leu
595 600 605

agt agt gga aat aca aaa caa ttg aag aac aaa gag gtc gct gcc ttg 1872
Ser Ser Gly Asn Thr Lys Gln Leu Lys Asn Lys Glu Val Ala Ala Leu
610 615 620

gtt att cac ggt aag tta cct ttg tac gct ttg gag aaa aaa tta ggt 1920
Val Ile His Gly Lys Leu Pro Leu Tyr Ala Leu Glu Lys Lys Leu Gly
625 630 635 640

gat act acg aga gcg gtt gcg gta cgt agg aag gct ctt tca att ttg 1968
Asp Thr Thr Arg Ala Val Ala Val Arg Arg Lys Ala Leu Ser Ile Leu
645 650 655

gca gaa gct cct gta tta gca tct gat cgt tta cca tat aaa aat tat 2016
Ala Glu Ala Pro Val Leu Ala Ser Asp Arg Leu Pro Tyr Lys Asn Tyr
660 665 670

gac tac gac cgc gta ttt ggc gct tgt tgt gaa aat gtt ata ggt tac 2064
Asp Tyr Asp Arg Val Phe Gly Ala Cys Cys Glu Asn Val Ile Gly Tyr
675 680 685

atg cct ttg ccc gtt ggt gtt ata ggc ccc ttg gtt atc gat ggt aca 2112

Met Pro Leu Pro Val Gly Val Ile Gly Pro Leu Val Ile Asp Gly Thr
690 695 700

tct tat cat ata cca atg gca act aca gag ggt tgt ttg gta gct tct 2160
Ser Tyr His Ile Pro Met Ala Thr Thr Glu Gly Cys Leu Val Ala Ser
705 710 715 720

gcc atg cgt ggc tgt aag gca atc aat gct ggc ggt ggt gca aca act 2208
Ala Met Arg Gly Cys Lys Ala Ile Asn Ala Gly Gly Gly Ala Thr Thr
725 730 735

gtt tta act aag gat ggt atg aca aga ggc cca gta gtc cgt ttc cca 2256
Val Leu Thr Lys Asp Gly Met Thr Arg Gly Pro Val Val Arg Phe Pro
740 745 750

act ttg aaa aga tct ggt gcc tgt aag ata tgg tta gac tca gaa gag 2304
Thr Leu Lys Arg Ser Gly Ala Cys Lys Ile Trp Leu Asp Ser Glu Glu
755 760 765

gga caa aac gca att aaa aaa gct ttt aac tct aca tca aga ttt gca 2352
Gly Gln Asn Ala Ile Lys Lys Ala Phe Asn Ser Thr Ser Arg Phe Ala
770 775 780

cgt ctg caa cat att caa act tgt cta gca gga gat tta ctc ttc atg 2400
Arg Leu Gln His Ile Gln Thr Cys Leu Ala Gly Asp Leu Leu Phe Met
785 790 795 800

aga ttt aga aca act act ggt gac gca atg ggt atg aat atg att tct 2448
Arg Phe Arg Thr Thr Thr Gly Asp Ala Met Gly Met Asn Met Ile Ser

805 810 815

aaa ggt gtc gaa tac tca tta aag caa atg gta gaa gag tat ggc tgg 2496
Lys Gly Val Glu Tyr Ser Leu Lys Gln Met Val Glu Glu Tyr Gly Trp
820 825 830

gaa gat atg gag gtt gtc tcc gtt tct ggt aac tac tgt acc gac aaa 2544
Glu Asp Met Glu Val Val Ser Val Ser Gly Asn Tyr Cys Thr Asp Lys
835 840 845

aaa cca gct gcc atc aac tgg atc gaa ggt cgt ggt aag agt gtc gtc 2592
Lys Pro Ala Ala Ile Asn Trp Ile Glu Gly Arg Gly Lys Ser Val Val
850 855 860

gca gaa gct act att cct ggt gat gtt gtc aga aaa gtg tta aaa agt 2640
Ala Glu Ala Thr Ile Pro Gly Asp Val Val Arg Lys Val Leu Lys Ser
865 870 875 880

gat gtt tcc gca ttg gtt gag ttg aac att gct aag aat ttg gtt gga 2688
Asp Val Ser Ala Leu Val Glu Leu Asn Ile Ala Lys Asn Leu Val Gly
885 890 895

tct gca atg gct ggg tct gtt ggt gga ttt aac gca cat gca gct aat 2736
Ser Ala Met Ala Gly Ser Val Gly Gly Phe Asn Ala His Ala Ala Asn
900 905 910

tta gtg aca gct gtt ttc ttg gca tta gga caa gat cct gca caa aat 2784
Leu Val Thr Ala Val Phe Leu Ala Leu Gly Gln Asp Pro Ala Gln Asn
915 920 925

gtt gaa agt tcc aac tgt ata aca ttg atg aaa gaa gtg gac ggt gat 2832
Val Glu Ser Ser Asn Cys Ile Thr Leu Met Lys Glu Val Asp Gly Asp
930 935 940

ttg aga att tcc gta tcc atg cca tcc atc gaa gta ggt acc atc ggt 2880
Leu Arg Ile Ser Val Ser Met Pro Ser Ile Glu Val Gly Thr Ile Gly
945 950 955 960

ggt ggt act gtt cta gaa cca caa ggt gcc atg ttg gac tta tta ggt 2928
Gly Gly Thr Val Leu Glu Pro Gln Gly Ala Met Leu Asp Leu Leu Gly
965 970 975

gta aga ggc ccg cat gct acc gct cct ggt acc aac gca cgt caa tta 2976
Val Arg Gly Pro His Ala Thr Ala Pro Gly Thr Asn Ala Arg Gln Leu
980 985 990

gca aga ata gtt gcc tgt gcc gtc ttg gca ggt gaa tta tcc tta tgt 3024
Ala Arg Ile Val Ala Cys Ala Val Leu Ala Gly Glu Leu Ser Leu Cys
995 1000 1005

gct gcc cta gca gcc ggc cat ttg gtt caa agt cat atg acc cac aac 3072
Ala Ala Leu Ala Ala Gly His Leu Val Gln Ser His Met Thr His Asn
1010 1015 1020

agg aaa cct gct gaa cca aca aaa cct aac aat ttg gac gcc act gat 3120
Arg Lys Pro Ala Glu Pro Thr Lys Pro Asn Asn Leu Asp Ala Thr Asp
1025 1030 1035 1040

ata aat cgt ttg aaa gat ggg tcc gtc acc tgc att aaa tcc taa 3165
Ile Asn Arg Leu Lys Asp Gly Ser Val Thr Cys Ile Lys Ser
1045 1050

<210> 2
<211> 1054
<212> PRT
<213> Saccharomyces cerevisiae

<400> 2
Met Pro Pro Leu Phe Lys Gly Leu Lys Gln Met Ala Lys Pro Ile Ala
1 5 10 15

Tyr Val Ser Arg Phe Ser Ala Lys Arg Pro Ile His Ile Ile Leu Phe
20 25 30

Ser Leu Ile Ile Ser Ala Phe Ala Tyr Leu Ser Val Ile Gln Tyr Tyr
35 40 45

Phe Asn Gly Trp Gln Leu Asp Ser Asn Ser Val Phe Glu Thr Ala Pro
50 55 60

Asn Lys Asp Ser Asn Thr Leu Phe Gln Glu Cys Ser His Tyr Tyr Arg
65 70 75 80

Asp Ser Ser Leu Asp Gly Trp Val Ser Ile Thr Ala His Glu Ala Ser
85 90 95

Glu Leu Pro Ala Pro His His Tyr Tyr Leu Leu Asn Leu Asn Phe Asn
100 105 110

Ser Pro Asn Glu Thr Asp Ser Ile Pro Glu Leu Ala Asn Thr Val Phe
115 120 125

Glu Lys Asp Asn Thr Lys Tyr Ile Leu Gln Glu Asp Leu Ser Val Ser
130 135 140

Lys Glu Ile Ser Ser Thr Asp Gly Thr Lys Trp Arg Leu Arg Ser Asp
145 150 155 160

Arg Lys Ser Leu Phe Asp Val Lys Thr Leu Ala Tyr Ser Leu Tyr Asp
165 170 175

Val Phe Ser Glu Asn Val Thr Gln Ala Asp Pro Phe Asp Val Leu Ile
180 185 190

Met Val Thr Ala Tyr Leu Met Met Phe Tyr Thr Ile Phe Gly Leu Phe
195 200 205

Asn Asp Met Arg Lys Thr Gly Ser Asn Phe Trp Leu Ser Ala Ser Thr
210 215 220

Val Val Asn Ser Ala Ser Ser Leu Phe Leu Ala Leu Tyr Val Thr Gln
225 230 235 240

Cys Ile Leu Gly Lys Glu Val Ser Ala Leu Thr Leu Phe Glu Gly Leu
245 250 255

Pro Phe Ile Val Val Val Val Gly Phe Lys His Lys Ile Lys Ile Ala
260 265 270

Gln Tyr Ala Leu Glu Lys Phe Glu Arg Val Gly Leu Ser Lys Arg Ile
275 280 285

Thr Thr Asp Glu Ile Val Phe Glu Ser Val Ser Glu Glu Gly Gly Arg
290 295 300

Leu Ile Gln Asp His Leu Leu Cys Ile Phe Ala Phe Ile Gly Cys Ser
305 310 315 320

Met Tyr Ala His Gln Leu Lys Thr Leu Thr Asn Phe Cys Ile Leu Ser
325 330 335

Ala Phe Ile Leu Ile Phe Glu Leu Ile Leu Thr Pro Thr Phe Tyr Ser
340 345 350

Ala Ile Leu Ala Leu Arg Leu Glu Met Asn Val Ile His Arg Ser Thr
355 360 365

Ile Ile Lys Gln Thr Leu Glu Glu Asp Gly Val Val Pro Ser Thr Ala
370 375 380

Arg Ile Ile Ser Lys Ala Glu Lys Lys Ser Val Ser Ser Phe Leu Asn
385 390 395 400

Leu Ser Val Val Val Ile Ile Met Lys Leu Ser Val Ile Leu Leu Phe

405 410 415

Val Phe Ile Asn Phe Tyr Asn Phe Gly Ala Asn Trp Val Asn Asp Ala

420 425 430

Phe Asn Ser Leu Tyr Phe Asp Lys Glu Arg Val Ser Leu Pro Asp Phe

435 440 445

Ile Thr Ser Asn Ala Ser Glu Asn Phe Lys Glu Gln Ala Ile Val Ser

450 455 460

Val Thr Pro Leu Leu Tyr Tyr Lys Pro Ile Lys Ser Tyr Gln Arg Ile

465 470 475 480

Glu Asp Met Val Leu Leu Leu Leu Arg Asn Val Ser Val Ala Ile Arg

485 490 495

Asp Arg Phe Val Ser Lys Leu Val Leu Ser Ala Leu Val Cys Ser Ala

500 505 510

Val Ile Asn Val Tyr Leu Leu Asn Ala Ala Arg Ile His Thr Ser Tyr

515 520 525

Thr Ala Asp Gln Leu Val Lys Thr Glu Val Thr Lys Lys Ser Phe Thr

530 535 540

Ala Pro Val Gln Lys Ala Ser Thr Pro Val Leu Thr Asn Lys Thr Val

545 550 555 560

Ile Ser Gly Ser Lys Val Lys Ser Leu Ser Ser Ala Gln Ser Ser Ser
565 570 575

Ser Gly Pro Ser Ser Ser Ser Glu Glu Asp Asp Ser Arg Asp Ile Glu
580 585 590

Ser Leu Asp Lys Lys Ile Arg Pro Leu Glu Glu Leu Glu Ala Leu Leu
595 600 605

Ser Ser Gly Asn Thr Lys Gln Leu Lys Asn Lys Glu Val Ala Ala Leu
610 615 620

Val Ile His Gly Lys Leu Pro Leu Tyr Ala Leu Glu Lys Lys Leu Gly
625 630 635 640

Asp Thr Thr Arg Ala Val Ala Val Arg Arg Lys Ala Leu Ser Ile Leu
645 650 655

Ala Glu Ala Pro Val Leu Ala Ser Asp Arg Leu Pro Tyr Lys Asn Tyr
660 665 670

Asp Tyr Asp Arg Val Phe Gly Ala Cys Cys Glu Asn Val Ile Gly Tyr
675 680 685

Met Pro Leu Pro Val Gly Val Ile Gly Pro Leu Val Ile Asp Gly Thr
690 695 700

Ser Tyr His Ile Pro Met Ala Thr Thr Glu Gly Cys Leu Val Ala Ser
705 710 715 720

Ala Met Arg Gly Cys Lys Ala Ile Asn Ala Gly Gly Gly Ala Thr Thr
725 730 735

Val Leu Thr Lys Asp Gly Met Thr Arg Gly Pro Val Val Arg Phe Pro
740 745 750

Thr Leu Lys Arg Ser Gly Ala Cys Lys Ile Trp Leu Asp Ser Glu Glu
755 760 765

Gly Gln Asn Ala Ile Lys Lys Ala Phe Asn Ser Thr Ser Arg Phe Ala
770 775 780

Arg Leu Gln His Ile Gln Thr Cys Leu Ala Gly Asp Leu Leu Phe Met
785 790 795 800

Arg Phe Arg Thr Thr Thr Gly Asp Ala Met Gly Met Asn Met Ile Ser
805 810 815

Lys Gly Val Glu Tyr Ser Leu Lys Gln Met Val Glu Glu Tyr Gly Trp
820 825 830

Glu Asp Met Glu Val Val Ser Val Ser Gly Asn Tyr Cys Thr Asp Lys
835 840 845

Lys Pro Ala Ala Ile Asn Trp Ile Glu Gly Arg Gly Lys Ser Val Val
850 855 860

Ala Glu Ala Thr Ile Pro Gly Asp Val Val Arg Lys Val Leu Lys Ser

865 870 875 880

Asp Val Ser Ala Leu Val Glu Leu Asn Ile Ala Lys Asn Leu Val Gly
885 890 895

Ser Ala Met Ala Gly Ser Val Gly Gly Phe Asn Ala His Ala Ala Asn
900 905 910

Leu Val Thr Ala Val Phe Leu Ala Leu Gly Gln Asp Pro Ala Gln Asn
915 920 925

Val Glu Ser Ser Asn Cys Ile Thr Leu Met Lys Glu Val Asp Gly Asp
930 935 940

Leu Arg Ile Ser Val Ser Met Pro Ser Ile Glu Val Gly Thr Ile Gly
945 950 955 960

Gly Gly Thr Val Leu Glu Pro Gln Gly Ala Met Leu Asp Leu Leu Gly
965 970 975

Val Arg Gly Pro His Ala Thr Ala Pro Gly Thr Asn Ala Arg Gln Leu
980 985 990

Ala Arg Ile Val Ala Cys Ala Val Leu Ala Gly Glu Leu Ser Leu Cys
995 1000 1005

Ala Ala Leu Ala Ala Gly His Leu Val Gln Ser His Met Thr His Asn
1010 1015 1020

Arg Lys Pro Ala Glu Pro Thr Lys Pro Asn Asn Leu Asp Ala Thr Asp
1025 1030 1035 1040

Ile Asn Arg Leu Lys Asp Gly Ser Val Thr Cys Ile Lys Ser
1045 1050

<210> 3
<211> 3165
<212> DNA
<213> Saccharomyces cerevisiae

<220>
<221> CDS
<222> (1)..(3162)

<400> 3
atg ccg ccg cta ttc aag gga ctg aaa cag atg gca aag cca att gcc 48
Met Pro Pro Leu Phe Lys Gly Leu Lys Gln Met Ala Lys Pro Ile Ala
1 5 10 15

tat gtt tca aga ttt tcg gcg aaa cga cca att cat ata ata ctt ttt 96
Tyr Val Ser Arg Phe Ser Ala Lys Arg Pro Ile His Ile Ile Leu Phe
20 25 30

tct cta atc ata tcc gca ttc gct tat cta tcc gtc att cag tat tac 144
Ser Leu Ile Ile Ser Ala Phe Ala Tyr Leu Ser Val Ile Gln Tyr Tyr
35 40 45

ttc aat ggt tgg caa cta gat tca aat agt gtt ttt gaa act gct cca 192
Phe Asn Gly Trp Gln Leu Asp Ser Asn Ser Val Phe Glu Thr Ala Pro
50 55 60

aat aaa gac ttc aac act cta ttt caa gaa tgt tcc cat tac tac aga 240
Asn Lys Asp Phe Asn Thr Leu Phe Gln Glu Cys Ser His Tyr Tyr Arg
65 70 75 80

gat tcc tct cta gat ggt tgg gta tca atc acc gcg cat gaa gct agt 288
Asp Ser Ser Leu Asp Gly Trp Val Ser Ile Thr Ala His Glu Ala Ser
85 90 95

gag tta cca gcc cca cac cat tac tat cta tta aac ctg aac ttc aat 336
Glu Leu Pro Ala Pro His His Tyr Tyr Leu Leu Asn Leu Asn Phe Asn
100 105 110

agt cct aat gaa act gac tcc att cca gaa cta gct aac acg gtt ttt 384
Ser Pro Asn Glu Thr Asp Ser Ile Pro Glu Leu Ala Asn Thr Val Phe
115 120 125

gag aaa gat aat aca aaa tat att ctg caa gaa gat ctc agc gtt tcc 432
Glu Lys Asp Asn Thr Lys Tyr Ile Leu Gln Glu Asp Leu Ser Val Ser
130 135 140

aaa gaa att tct tct act gat gga acg aaa tgg agg tta aga agt gac 480
Lys Glu Ile Ser Ser Thr Asp Gly Thr Lys Trp Arg Leu Arg Ser Asp
145 150 155 160

aga aaa agt ctt ttc gac gta aag acg tta gca tat tct ctc tac gat 528

Arg Lys Ser Leu Phe Asp Val Lys Thr Leu Ala Tyr Ser Leu Tyr Asp
165 170 175

gta ttt tca gaa aat gta acc caa gca gac ccg ttt gac gtc ctt att 576
Val Phe Ser Glu Asn Val Thr Gln Ala Asp Pro Phe Asp Val Leu Ile
180 185 190

atg gtt act gcc tac cta atg atg ttc tac acc ata ttc ggc ctc ttc 624
Met Val Thr Ala Tyr Leu Met Met Phe Tyr Thr Ile Phe Gly Leu Phe
195 200 205

aat gac atg agg aag acc ggg tca aat ttt tgg ttg agc gcc tct aca 672
Asn Asp Met Arg Lys Thr Gly Ser Asn Phe Trp Leu Ser Ala Ser Thr
210 215 220

gtg gtc aat tct gca tca tca ctt ttc tta gca ttg tat gtc acc caa 720
Val Val Asn Ser Ala Ser Ser Leu Phe Leu Ala Leu Tyr Val Thr Gln
225 230 235 240

tgt att cta ggc aaa gaa gtt tcc gca tta act ctt ttt gaa ggt ttg 768
Cys Ile Leu Gly Lys Glu Val Ser Ala Leu Thr Leu Phe Glu Gly Leu
245 250 255

cct ttc att gta gtt gtt gtt ggt ttc aag cac aaa atc aag att gcc 816
Pro Phe Ile Val Val Val Val Gly Phe Lys His Lys Ile Lys Ile Ala
260 265 270

cag tat gcc ctg gag aaa ttt gaa aga gtc ggt tta tct aaa agg att 864
Gln Tyr Ala Leu Glu Lys Phe Glu Arg Val Gly Leu Ser Lys Arg Ile

275 280 285

act acc gat gaa atc gtt ttt gaa tcc gtg agc gaa gag ggt ggt cgt 912
Thr Thr Asp Glu Ile Val Phe Glu Ser Val Ser Glu Glu Gly Gly Arg
290 295 300

ttg att caa gac cat ttg ctt tgt att ttt gcc ttt atc gga tgc tct 960
Leu Ile Gln Asp His Leu Leu Cys Ile Phe Ala Phe Ile Gly Cys Ser
305 310 315 320

atg tat gct cac caa ttg aag act ttg aca aac ttc tgc ata tta tca 1008
Met Tyr Ala His Gln Leu Lys Thr Leu Thr Asn Phe Cys Ile Leu Ser
325 330 335

gca ttt atc cta att ttc gaa ttg att tta act cct aca ttt tat tct 1056
Ala Phe Ile Leu Ile Phe Glu Leu Ile Leu Thr Pro Thr Phe Tyr Ser
340 345 350

gct atc tta gcg ctt aga ctg gaa atg aat gtt atc cac aga tct act 1104
Ala Ile Leu Ala Leu Arg Leu Glu Met Asn Val Ile His Arg Ser Thr
355 360 365

att atc aag caa aca tta gaa gaa gac ggt gtt gtt cca tct aca gca 1152
Ile Ile Lys Gln Thr Leu Glu Glu Asp Gly Val Val Pro Ser Thr Ala
370 375 380

aga atc att tct aag gca gaa aag aaa tcc gta tct tct ttc tta aat 1200
Arg Ile Ile Ser Lys Ala Glu Lys Lys Ser Val Ser Ser Phe Leu Asn
385 390 395 400

ctc agt gtg gtt gtc att atc atg aaa ctc tct gtc ata ctg ttg ttc 1248
Leu Ser Val Val Val Ile Ile Met Lys Leu Ser Val Ile Leu Leu Phe
405 410 415

gtc ttc atc aac ttt tat aac ttt ggt gca aat tgg gtc aat gat gcc 1296
Val Phe Ile Asn Phe Tyr Asn Phe Gly Ala Asn Trp Val Asn Asp Ala
420 425 430

ttc aat tca ttg tac ttc gat aag gaa cgt gtt tct cta cca gat ttt 1344
Phe Asn Ser Leu Tyr Phe Asp Lys Glu Arg Val Ser Leu Pro Asp Phe
435 440 445

att acc tcg aat gcc tct gaa aac ttt aaa gag caa gct att gtt agt 1392
Ile Thr Ser Asn Ala Ser Glu Asn Phe Lys Glu Gln Ala Ile Val Ser
450 455 460

gtc acc cca tta tta tat tac aaa ccc att aag tcc tac caa cgc att 1440
Val Thr Pro Leu Leu Tyr Tyr Lys Pro Ile Lys Ser Tyr Gln Arg Ile
465 470 475 480

gag gat atg gtt ctt cta ttg ctt cgt aat gtc agt gtt gcc att cgt 1488
Glu Asp Met Val Leu Leu Leu Leu Arg Asn Val Ser Val Ala Ile Arg
485 490 495

gat agg ttc gtc agt aaa tta gtt ctt tcc gcc tta gta tgc agt gct 1536
Asp Arg Phe Val Ser Lys Leu Val Leu Ser Ala Leu Val Cys Ser Ala
500 505 510

gtc atc aat gtg tat tta tta aat gct gct aga att cat acc agt tat 1584
Val Ile Asn Val Tyr Leu Leu Asn Ala Ala Arg Ile His Thr Ser Tyr
515 520 525

act gca gac caa ttg gtg aag act gaa gtc acc aag aag tct ttt act 1632
Thr Ala Asp Gln Leu Val Lys Thr Glu Val Thr Lys Lys Ser Phe Thr
530 535 540

gct cct gta caa aag gct tct aca cca gtt tta acc aat aaa aca gtc 1680
Ala Pro Val Gln Lys Ala Ser Thr Pro Val Leu Thr Asn Lys Thr Val
545 550 555 560

att tct gga tcg aaa gtc aaa agt tta tca tct gcg caa tcg agc tca 1728
Ile Ser Gly Ser Lys Val Lys Ser Leu Ser Ser Ala Gln Ser Ser Ser
565 570 575

tca gga cct tca tca tct agt gag gaa gat gat tcc cgc gat att gaa 1776
Ser Gly Pro Ser Ser Ser Ser Glu Glu Asp Asp Ser Arg Asp Ile Glu
580 585 590

agc ttg gat aag aaa ata cgt cct tta gaa gaa tta gaa gca tca tta 1824
Ser Leu Asp Lys Lys Ile Arg Pro Leu Glu Glu Leu Glu Ala Ser Leu
595 600 605

agt agt gga aat aca aaa caa ttg aag aac aaa gag gtc gct gcc ttg 1872
Ser Ser Gly Asn Thr Lys Gln Leu Lys Asn Lys Glu Val Ala Ala Leu
610 615 620

gtt att cac ggt aag tta cct ttg tac gct ttg gag aaa aaa tta ggt 1920

Val Ile His Gly Lys Leu Pro Leu Tyr Ala Leu Glu Lys Lys Leu Gly
625 630 635 640

gat act acg aga gcg gtt gcg gta cgt agg aag gct ctt tca att ttg 1968
Asp Thr Thr Arg Ala Val Ala Val Arg Arg Lys Ala Leu Ser Ile Leu
645 650 655

gca gaa gct cct gta tta gca tct gat cgt tta cca tat aaa aat tat 2016
Ala Glu Ala Pro Val Leu Ala Ser Asp Arg Leu Pro Tyr Lys Asn Tyr
660 665 670

gac tac gac cgc gta ttt ggc gct tgt tgt gaa aat gtt ata ggt tac 2064
Asp Tyr Asp Arg Val Phe Gly Ala Cys Cys Glu Asn Val Ile Gly Tyr
675 680 685

atg cct ttg ccc gtt ggt gtt ata ggc ccc ttg gtt atc gat ggt aca 2112
Met Pro Leu Pro Val Gly Val Ile Gly Pro Leu Val Ile Asp Gly Thr
690 695 700

tct tat cat ata cca atg gca act aca gag ggt tgt ttg gta gct tct 2160
Ser Tyr His Ile Pro Met Ala Thr Thr Glu Gly Cys Leu Val Ala Ser
705 710 715 720

gcc atg cgt ggc tgt aag gca atc aat gct ggc ggt ggt gca aca act 2208
Ala Met Arg Gly Cys Lys Ala Ile Asn Ala Gly Gly Gly Ala Thr Thr
725 730 735

gtt tta act aag gat ggt atg aca aga ggc cca gta gtc cgt ttc cca 2256
Val Leu Thr Lys Asp Gly Met Thr Arg Gly Pro Val Val Arg Phe Pro

740 745 750

act ttg aaa aga tct ggt gcc tgt aag ata tgg tta gac tca gaa gag 2304
Thr Leu Lys Arg Ser Gly Ala Cys Lys Ile Trp Leu Asp Ser Glu Glu
755 760 765

gga caa aac gca att aaa aaa gct ttt aac tct aca tca aga ttt gca 2352
Gly Gln Asn Ala Ile Lys Lys Ala Phe Asn Ser Thr Ser Arg Phe Ala
770 775 780

cgt ctg caa cat att caa act tgt cta gca gga gat tta ctc ttc atg 2400
Arg Leu Gln His Ile Gln Thr Cys Leu Ala Gly Asp Leu Leu Phe Met
785 790 795 800

aga ttt aga aca act act ggt gac gca atg ggt atg aat atg att tct 2448
Arg Phe Arg Thr Thr Thr Gly Asp Ala Met Gly Met Asn Met Ile Ser
805 810 815

aag ggt gtc gaa tac tca tta aag caa atg gta gaa gag tat ggc tgg 2496
Lys Gly Val Glu Tyr Ser Leu Lys Gln Met Val Glu Glu Tyr Gly Trp
820 825 830

gaa gat atg gag gtt gtc tcc gtt tct ggt aac tac tgt acc gac aaa 2544
Glu Asp Met Glu Val Val Ser Val Ser Gly Asn Tyr Cys Thr Asp Lys
835 840 845

aaa cca gct gcc atc aac tgg atc gaa ggt cgt ggt aag agt gtc gtc 2592
Lys Pro Ala Ala Ile Asn Trp Ile Glu Gly Arg Gly Lys Ser Val Val
850 855 860

gca gaa gct act att cct ggt gat gtt gtc aga aaa gtg tta aaa agt 2640
Ala Glu Ala Thr Ile Pro Gly Asp Val Val Arg Lys Val Leu Lys Ser
865 870 875 880

gat gtt tcc gca ttg gtt gag ttg aac att gct aag aat ttg gtt gga 2688
Asp Val Ser Ala Leu Val Glu Leu Asn Ile Ala Lys Asn Leu Val Gly
885 890 895

tct gca atg gct ggg tct gtt ggt gga ttt aac gca cgt gca gct aat 2736
Ser Ala Met Ala Gly Ser Val Gly Gly Phe Asn Ala Arg Ala Ala Asn
900 905 910

tta gtg aca gct gtt ttc ttg gca tta gga caa gat cct gca caa aat 2784
Leu Val Thr Ala Val Phe Leu Ala Leu Gly Gln Asp Pro Ala Gln Asn
915 920 925

gtc gaa agt tcc aac tgt ata aca ttg atg aaa gaa gtg gac ggt gat 2832
Val Glu Ser Ser Asn Cys Ile Thr Leu Met Lys Glu Val Asp Gly Asp
930 935 940

ttg aga att tcc gta tcc atg cca tcc atc gaa gta ggt acc atc ggt 2880
Leu Arg Ile Ser Val Ser Met Pro Ser Ile Glu Val Gly Thr Ile Gly
945 950 955 960

ggt ggt act gtt cta gaa cca caa ggt gcc atg ttg gac tta tta ggt 2928
Gly Gly Thr Val Leu Glu Pro Gln Gly Ala Met Leu Asp Leu Leu Gly
965 970 975

gta aga ggc cca cat gct acc gct cct ggt acc aac gca cgt caa tta 2976
Val Arg Gly Pro His Ala Thr Ala Pro Gly Thr Asn Ala Arg Gln Leu
980 985 990

gca aga ata gtt gcc tgt gcc gtc ttg gca ggt gaa tta tcc tta tgt 3024
Ala Arg Ile Val Ala Cys Ala Val Leu Ala Gly Glu Leu Ser Leu Cys
995 1000 1005

gct gcc cta gca gcc ggc cat ttg gtt caa agt cat atg acc cac aac 3072
Ala Ala Leu Ala Ala Gly His Leu Val Gln Ser His Met Thr His Asn
1010 1015 1020

agg aaa cct gct gaa cca aca aaa cct aac aat ttg gac gcc act gat 3120
Arg Lys Pro Ala Glu Pro Thr Lys Pro Asn Asn Leu Asp Ala Thr Asp
1025 1030 1035 1040

ata aat cgt ttg aaa gat ggg tcc gtc acc tgc att aaa tcc taa 3165
Ile Asn Arg Leu Lys Asp Gly Ser Val Thr Cys Ile Lys Ser
1045 1050

<210> 4
<211> 1054
<212> PRT
<213> Saccharomyces cerevisiae

<400> 4
Met Pro Pro Leu Phe Lys Gly Leu Lys Gln Met Ala Lys Pro Ile Ala
1 5 10 15

Tyr Val Ser Arg Phe Ser Ala Lys Arg Pro Ile His Ile Ile Leu Phe
20 25 30

Ser Leu Ile Ile Ser Ala Phe Ala Tyr Leu Ser Val Ile Gln Tyr Tyr
35 40 45

Phe Asn Gly Trp Gln Leu Asp Ser Asn Ser Val Phe Glu Thr Ala Pro
50 55 60

Asn Lys Asp Phe Asn Thr Leu Phe Gln Glu Cys Ser His Tyr Tyr Arg
65 70 75 80

Asp Ser Ser Leu Asp Gly Trp Val Ser Ile Thr Ala His Glu Ala Ser
85 90 95

Glu Leu Pro Ala Pro His His Tyr Tyr Leu Leu Asn Leu Asn Phe Asn
100 105 110

Ser Pro Asn Glu Thr Asp Ser Ile Pro Glu Leu Ala Asn Thr Val Phe
115 120 125

Glu Lys Asp Asn Thr Lys Tyr Ile Leu Gln Glu Asp Leu Ser Val Ser
130 135 140

Lys Glu Ile Ser Ser Thr Asp Gly Thr Lys Trp Arg Leu Arg Ser Asp
145 150 155 160

Arg Lys Ser Leu Phe Asp Val Lys Thr Leu Ala Tyr Ser Leu Tyr Asp

165 170 175

Val Phe Ser Glu Asn Val Thr Gln Ala Asp Pro Phe Asp Val Leu Ile

180 185 190

Met Val Thr Ala Tyr Leu Met Met Phe Tyr Thr Ile Phe Gly Leu Phe

195 200 205

Asn Asp Met Arg Lys Thr Gly Ser Asn Phe Trp Leu Ser Ala Ser Thr

210 215 220

Val Val Asn Ser Ala Ser Ser Leu Phe Leu Ala Leu Tyr Val Thr Gln

225 230 235 240

Cys Ile Leu Gly Lys Glu Val Ser Ala Leu Thr Leu Phe Glu Gly Leu

245 250 255

Pro Phe Ile Val Val Val Val Gly Phe Lys His Lys Ile Lys Ile Ala

260 265 270

Gln Tyr Ala Leu Glu Lys Phe Glu Arg Val Gly Leu Ser Lys Arg Ile

275 280 285

Thr Thr Asp Glu Ile Val Phe Glu Ser Val Ser Glu Glu Gly Gly Arg

290 295 300

Leu Ile Gln Asp His Leu Leu Cys Ile Phe Ala Phe Ile Gly Cys Ser

305 310 315 320

Met Tyr Ala His Gln Leu Lys Thr Leu Thr Asn Phe Cys Ile Leu Ser
325 330 335

Ala Phe Ile Leu Ile Phe Glu Leu Ile Leu Thr Pro Thr Phe Tyr Ser
340 345 350

Ala Ile Leu Ala Leu Arg Leu Glu Met Asn Val Ile His Arg Ser Thr
355 360 365

Ile Ile Lys Gln Thr Leu Glu Glu Asp Gly Val Val Pro Ser Thr Ala
370 375 380

Arg Ile Ile Ser Lys Ala Glu Lys Lys Ser Val Ser Ser Phe Leu Asn
385 390 395 400

Leu Ser Val Val Val Ile Ile Met Lys Leu Ser Val Ile Leu Leu Phe
405 410 415

Val Phe Ile Asn Phe Tyr Asn Phe Gly Ala Asn Trp Val Asn Asp Ala
420 425 430

Phe Asn Ser Leu Tyr Phe Asp Lys Glu Arg Val Ser Leu Pro Asp Phe
435 440 445

Ile Thr Ser Asn Ala Ser Glu Asn Phe Lys Glu Gln Ala Ile Val Ser
450 455 460

Val Thr Pro Leu Leu Tyr Tyr Lys Pro Ile Lys Ser Tyr Gln Arg Ile
465 470 475 480

Glu Asp Met Val Leu Leu Leu Leu Arg Asn Val Ser Val Ala Ile Arg
485 490 495

Asp Arg Phe Val Ser Lys Leu Val Leu Ser Ala Leu Val Cys Ser Ala
500 505 510

Val Ile Asn Val Tyr Leu Leu Asn Ala Ala Arg Ile His Thr Ser Tyr
515 520 525

Thr Ala Asp Gln Leu Val Lys Thr Glu Val Thr Lys Lys Ser Phe Thr
530 535 540

Ala Pro Val Gln Lys Ala Ser Thr Pro Val Leu Thr Asn Lys Thr Val
545 550 555 560

Ile Ser Gly Ser Lys Val Lys Ser Leu Ser Ser Ala Gln Ser Ser Ser
565 570 575

Ser Gly Pro Ser Ser Ser Ser Glu Glu Asp Asp Ser Arg Asp Ile Glu
580 585 590

Ser Leu Asp Lys Lys Ile Arg Pro Leu Glu Glu Leu Glu Ala Ser Leu
595 600 605

Ser Ser Gly Asn Thr Lys Gln Leu Lys Asn Lys Glu Val Ala Ala Leu
610 615 620

Val Ile His Gly Lys Leu Pro Leu Tyr Ala Leu Glu Lys Lys Leu Gly

625 630 635 640

Asp Thr Thr Arg Ala Val Ala Val Arg Arg Lys Ala Leu Ser Ile Leu
645 650 655

Ala Glu Ala Pro Val Leu Ala Ser Asp Arg Leu Pro Tyr Lys Asn Tyr
660 665 670

Asp Tyr Asp Arg Val Phe Gly Ala Cys Cys Glu Asn Val Ile Gly Tyr
675 680 685

Met Pro Leu Pro Val Gly Val Ile Gly Pro Leu Val Ile Asp Gly Thr
690 695 700

Ser Tyr His Ile Pro Met Ala Thr Thr Glu Gly Cys Leu Val Ala Ser
705 710 715 720

Ala Met Arg Gly Cys Lys Ala Ile Asn Ala Gly Gly Gly Ala Thr Thr
725 730 735

Val Leu Thr Lys Asp Gly Met Thr Arg Gly Pro Val Val Arg Phe Pro
740 745 750

Thr Leu Lys Arg Ser Gly Ala Cys Lys Ile Trp Leu Asp Ser Glu Glu
755 760 765

Gly Gln Asn Ala Ile Lys Lys Ala Phe Asn Ser Thr Ser Arg Phe Ala
770 775 780

Arg Leu Gln His Ile Gln Thr Cys Leu Ala Gly Asp Leu Leu Phe Met
785 790 795 800

Arg Phe Arg Thr Thr Thr Gly Asp Ala Met Gly Met Asn Met Ile Ser
805 810 815

Lys Gly Val Glu Tyr Ser Leu Lys Gln Met Val Glu Glu Tyr Gly Trp
820 825 830

Glu Asp Met Glu Val Val Ser Val Ser Gly Asn Tyr Cys Thr Asp Lys
835 840 845

Lys Pro Ala Ala Ile Asn Trp Ile Glu Gly Arg Gly Lys Ser Val Val
850 855 860

Ala Glu Ala Thr Ile Pro Gly Asp Val Val Arg Lys Val Leu Lys Ser
865 870 875 880

Asp Val Ser Ala Leu Val Glu Leu Asn Ile Ala Lys Asn Leu Val Gly
885 890 895

Ser Ala Met Ala Gly Ser Val Gly Gly Phe Asn Ala Arg Ala Ala Asn
900 905 910

Leu Val Thr Ala Val Phe Leu Ala Leu Gly Gln Asp Pro Ala Gln Asn
915 920 925

Val Glu Ser Ser Asn Cys Ile Thr Leu Met Lys Glu Val Asp Gly Asp
930 935 940

Leu Arg Ile Ser Val Ser Met Pro Ser Ile Glu Val Gly Thr Ile Gly
945 950 955 960

Gly Gly Thr Val Leu Glu Pro Gln Gly Ala Met Leu Asp Leu Leu Gly
965 970 975

Val Arg Gly Pro His Ala Thr Ala Pro Gly Thr Asn Ala Arg Gln Leu
980 985 990

Ala Arg Ile Val Ala Cys Ala Val Leu Ala Gly Glu Leu Ser Leu Cys
995 1000 1005

Ala Ala Leu Ala Ala Gly His Leu Val Gln Ser His Met Thr His Asn
1010 1015 1020

Arg Lys Pro Ala Glu Pro Thr Lys Pro Asn Asn Leu Asp Ala Thr Asp
1025 1030 1035 1040

Ile Asn Arg Leu Lys Asp Gly Ser Val Thr Cys Ile Lys Ser
1045 1050

<210> 5
<211> 3165
<212> DNA
<213> Saccharomyces cerevisiae

<220>

<221> CDS

<222> (1)..(3162)

<400> 5

atg ccg ccg cta ttc aag gga ctg aaa cag atg gca aag cca att gcc 48
Met Pro Pro Leu Phe Lys Gly Leu Lys Gln Met Ala Lys Pro Ile Ala
1 5 10 15

tat gtt tca aga ttt tcg gcg aaa cga cca att cat ata ata ctt ttt 96
Tyr Val Ser Arg Phe Ser Ala Lys Arg Pro Ile His Ile Ile Leu Phe
20 25 30

tct cta atc ata tcc gca ttc gct tat cta tcc gtc att cag tat tac 144
Ser Leu Ile Ile Ser Ala Phe Ala Tyr Leu Ser Val Ile Gln Tyr Tyr
35 40 45

ttc aat ggt tgg caa cta gat tca aat agt gtt ttt gaa act gct cca 192
Phe Asn Gly Trp Gln Leu Asp Ser Asn Ser Val Phe Glu Thr Ala Pro
50 55 60

aat aaa gac tcc aac act cta ttt caa gaa tgt tcc cat tac tac aga 240
Asn Lys Asp Ser Asn Thr Leu Phe Gln Glu Cys Ser His Tyr Tyr Arg
65 70 75 80

gat tcc tct cta gat ggt tgg gta tca atc acc gcg cat gaa gct agt 288
Asp Ser Ser Leu Asp Gly Trp Val Ser Ile Thr Ala His Glu Ala Ser
85 90 95

gag tta cca gcc cca cac cat tac tat cta tta aac ctg aac ttc aat 336

Glu Leu Pro Ala Pro His His Tyr Tyr Leu Leu Asn Leu Asn Phe Asn
100 105 110

agt cct aat gaa act gac tcc att cca gaa cta gct aac acg gtt ttt 384
Ser Pro Asn Glu Thr Asp Ser Ile Pro Glu Leu Ala Asn Thr Val Phe
115 120 125

gag aaa gat aat aca aaa tat att ctg caa gaa gat ctc agc gtt tcc 432
Glu Lys Asp Asn Thr Lys Tyr Ile Leu Gln Glu Asp Leu Ser Val Ser
130 135 140

aaa gaa att tct tct act gat gga acg aaa tgg agg tta aga agt gac 480
Lys Glu Ile Ser Ser Thr Asp Gly Thr Lys Trp Arg Leu Arg Ser Asp
145 150 155 160

aga aaa agt ctt ttc gac gta aag acg tta gca tat tct ctc tac gat 528
Arg Lys Ser Leu Phe Asp Val Lys Thr Leu Ala Tyr Ser Leu Tyr Asp
165 170 175

gta ttt tca gaa aat gta acc caa gca gac ccg ttt gac gtc ctt att 576
Val Phe Ser Glu Asn Val Thr Gln Ala Asp Pro Phe Asp Val Leu Ile
180 185 190

atg gtt act gcc tac cta atg atg ttc tac acc ata ttc ggc ctc ttc 624
Met Val Thr Ala Tyr Leu Met Met Phe Tyr Thr Ile Phe Gly Leu Phe
195 200 205

aat gac atg agg aag acc ggg tca aat ttt tgg ttg agc gcc tct aca 672
Asn Asp Met Arg Lys Thr Gly Ser Asn Phe Trp Leu Ser Ala Ser Thr

210 215 220

gtg gtc aat tct gca tca tca ctt ttc tta gca ttg tat gtc acc caa 720
Val Val Asn Ser Ala Ser Ser Leu Phe Leu Ala Leu Tyr Val Thr Gln
225 230 235 240

tgt att cta ggc aaa gaa gtt tcc gca tta act ctt ttt gaa ggt ttg 768
Cys Ile Leu Gly Lys Glu Val Ser Ala Leu Thr Leu Phe Glu Gly Leu
245 250 255

cct ttc att gta gtt gtt gtt ggt ttc aag cac aaa atc aag att gcc 816
Pro Phe Ile Val Val Val Val Gly Phe Lys His Lys Ile Lys Ile Ala
260 265 270

cag tat gcc ctg gag aaa ttt gaa aga gtc ggt tta tct aaa agg att 864
Gln Tyr Ala Leu Glu Lys Phe Glu Arg Val Gly Leu Ser Lys Arg Ile
275 280 285

act acc gat gaa atc gtt ttt gaa tcc gtg agc gaa gag ggt ggt cgt 912
Thr Thr Asp Glu Ile Val Phe Glu Ser Val Ser Glu Glu Gly Gly Arg
290 295 300

ttg att caa gac cat ttg ctt tgt att ttt gcc ttt atc gga tgc tct 960
Leu Ile Gln Asp His Leu Leu Cys Ile Phe Ala Phe Ile Gly Cys Ser
305 310 315 320

atg tat gct cac caa ttg aag act ttg aca aac ttc tgc ata tta tca 1008
Met Tyr Ala His Gln Leu Lys Thr Leu Thr Asn Phe Cys Ile Leu Ser
325 330 335

gca ttt atc cta att ttc gaa ttg att tta act cct aca ttt tat tct 1056
Ala Phe Ile Leu Ile Phe Glu Leu Ile Leu Thr Pro Thr Phe Tyr Ser
340 345 350

gct atc tta gcg ctt aga ctg gaa atg aat gtt atc cac aga tct act 1104
Ala Ile Leu Ala Leu Arg Leu Glu Met Asn Val Ile His Arg Ser Thr
355 360 365

att atc aag caa aca tta gaa gaa gac ggt gtt gtt cca tct aca gca 1152
Ile Ile Lys Gln Thr Leu Glu Glu Asp Gly Val Val Pro Ser Thr Ala
370 375 380

aga atc att tct aag gca gaa aag aaa tcc gta tct tct ttc tta aat 1200
Arg Ile Ile Ser Lys Ala Glu Lys Lys Ser Val Ser Ser Phe Leu Asn
385 390 395 400

ctc agt gtg gtt gtc att atc atg aaa ctc tct gtc ata ctg ttg ttc 1248
Leu Ser Val Val Val Ile Ile Met Lys Leu Ser Val Ile Leu Leu Phe
405 410 415

gtc ttc atc aac ttt tat aac ttt ggt gca aat tgg gtc aat gat gcc 1296
Val Phe Ile Asn Phe Tyr Asn Phe Gly Ala Asn Trp Val Asn Asp Ala
420 425 430

ttc aat tca ttg tac ttc gat aag gaa cgt gtt tct cta cca gat ttt 1344
Phe Asn Ser Leu Tyr Phe Asp Lys Glu Arg Val Ser Leu Pro Asp Phe
435 440 445

att acc tcg aat gcc tct gaa aac ttt aaa gag caa gct att gtt agt 1392
Ile Thr Ser Asn Ala Ser Glu Asn Phe Lys Glu Gln Ala Ile Val Ser
450 455 460

gtc acc cca tta tta tat tac aaa ccc att aag tcc tac caa cgc att 1440
Val Thr Pro Leu Leu Tyr Tyr Lys Pro Ile Lys Ser Tyr Gln Arg Ile
465 470 475 480

gag gat atg gtt ctt cta ttg ctt cgt aat gtc agt gtt gcc att cgt 1488
Glu Asp Met Val Leu Leu Leu Leu Arg Asn Val Ser Val Ala Ile Arg
485 490 495

gat agg ttc gtc agt aaa tta gtt ctt tcc gcc tta gta tgc agt gct 1536
Asp Arg Phe Val Ser Lys Leu Val Leu Ser Ala Leu Val Cys Ser Ala
500 505 510

gtc atc aat gtg tat tta tta aat gct gct aga att cat acc agt tat 1584
Val Ile Asn Val Tyr Leu Leu Asn Ala Ala Arg Ile His Thr Ser Tyr
515 520 525

act gca gac caa ttg gtg aag act gaa gtc acc aag aag tct ttt act 1632
Thr Ala Asp Gln Leu Val Lys Thr Glu Val Thr Lys Lys Ser Phe Thr
530 535 540

gct cct gta caa aag gct tct aca cca gtt tta acc aat aaa aca gtc 1680
Ala Pro Val Gln Lys Ala Ser Thr Pro Val Leu Thr Asn Lys Thr Val
545 550 555 560

att tct gga tcg aaa gtc aaa agt tta tca tct gcg caa tcg agc tca 1728

Ile Ser Gly Ser Lys Val Lys Ser Leu Ser Ser Ala Gln Ser Ser Ser
565 570 575

tca gga cct tca tca tct agt gag gaa gat gat tcc cgc gat att gaa 1776
Ser Gly Pro Ser Ser Ser Ser Glu Glu Asp Asp Ser Arg Asp Ile Glu
580 585 590

agc ttg gat aag aaa ata cgt cct tta gaa gaa tta gaa gca tta tta 1824
Ser Leu Asp Lys Lys Ile Arg Pro Leu Glu Glu Leu Glu Ala Leu Leu
595 600 605

agt agt gga aat aca aaa caa ttg aag aac aaa gag gtc gct gcc ttg 1872
Ser Ser Gly Asn Thr Lys Gln Leu Lys Asn Lys Glu Val Ala Ala Leu
610 615 620

gtt att cac ggt aag tta cct ttg tac gct ttg gag aaa aaa tta ggt 1920
Val Ile His Gly Lys Leu Pro Leu Tyr Ala Leu Glu Lys Lys Leu Gly
625 630 635 640

gat act acg aga gcg gtt gcg gta cgt agg aag gct ctt tca att ttg 1968
Asp Thr Thr Arg Ala Val Ala Val Arg Arg Lys Ala Leu Ser Ile Leu
645 650 655

gca gaa gct cct gta tta gca tct gat cgt tta cca tat aaa aat tat 2016
Ala Glu Ala Pro Val Leu Ala Ser Asp Arg Leu Pro Tyr Lys Asn Tyr
660 665 670

gac tac gac cgc gta ttt ggc gct tgt tgt gaa aat gtt ata ggt tac 2064
Asp Tyr Asp Arg Val Phe Gly Ala Cys Cys Glu Asn Val Ile Gly Tyr

675 680 685

atg cct ttg ccc gtt ggt gtt ata ggc ccc ttg gtt atc gat ggt aca 2112
Met Pro Leu Pro Val Gly Val Ile Gly Pro Leu Val Ile Asp Gly Thr
690 695 700

tct tat cat ata cca atg gca act aca gag ggt tgt ttg gta gct tct 2160
Ser Tyr His Ile Pro Met Ala Thr Thr Glu Gly Cys Leu Val Ala Ser
705 710 715 720

gcc atg cgt ggc tgt aag gca atc aat gct ggc ggt ggt gca aca act 2208
Ala Met Arg Gly Cys Lys Ala Ile Asn Ala Gly Gly Gly Ala Thr Thr
725 730 735

gtt tta act aag gat ggt atg aca aga ggc cca gta gtc cgt ttc cca 2256
Val Leu Thr Lys Asp Gly Met Thr Arg Gly Pro Val Val Arg Phe Pro
740 745 750

act ttg aaa aga tct ggt gcc tgt aag ata tgg tta gac tca gaa gag 2304
Thr Leu Lys Arg Ser Gly Ala Cys Lys Ile Trp Leu Asp Ser Glu Glu
755 760 765

gga caa aac gca att aaa aaa gct ttt aac tct aca tca aga ttt gca 2352
Gly Gln Asn Ala Ile Lys Lys Ala Phe Asn Ser Thr Ser Arg Phe Ala
770 775 780

cgt ctg caa cat att caa act tgt cta gca gga gat tta ctc ttc atg 2400
Arg Leu Gln His Ile Gln Thr Cys Leu Ala Gly Asp Leu Leu Phe Met
785 790 795 800

aga ttt aga aca act act ggt gac gca atg ggt atg aat atg att tct 2448
Arg Phe Arg Thr Thr Thr Gly Asp Ala Met Gly Met Asn Met Ile Ser
805 810 815

aag ggt gtc gaa tac tca tta aag caa atg gta gaa gag tat ggc tgg 2496
Lys Gly Val Glu Tyr Ser Leu Lys Gln Met Val Glu Glu Tyr Gly Trp
820 825 830

gaa gat atg gag gtt gtc tcc gtt tct ggt aac tac tgt acc gac aaa 2544
Glu Asp Met Glu Val Val Ser Val Ser Gly Asn Tyr Cys Thr Asp Lys
835 840 845

aaa cca gct gcc atc aac tgg atc gaa ggt cgt ggt aag agt gtc gtc 2592
Lys Pro Ala Ala Ile Asn Trp Ile Glu Gly Arg Gly Lys Ser Val Val
850 855 860

gca gaa gct act att cct ggt gat gtt gtc aga aaa gtg tta aaa agt 2640
Ala Glu Ala Thr Ile Pro Gly Asp Val Val Arg Lys Val Leu Lys Ser
865 870 875 880

gat gtt tcc gca ttg gtt gag ttg aac att gct aag aat ttg gtt gga 2688
Asp Val Ser Ala Leu Val Glu Leu Asn Ile Ala Lys Asn Leu Val Gly
885 890 895

tct gca atg gct ggg tct gtt ggt gga ttt aac gca cat gca gct aat 2736
Ser Ala Met Ala Gly Ser Val Gly Gly Phe Asn Ala His Ala Ala Asn
900 905 910

tta gtg aca gct gtt ttc ttg gca tta gga caa gat cct gca caa aat 2784
Leu Val Thr Ala Val Phe Leu Ala Leu Gly Gln Asp Pro Ala Gln Asn
915 920 925

gtc gaa agt tcc aac tgt ata aca ttg atg aaa gaa gtg gac ggt gat 2832
Val Glu Ser Ser Asn Cys Ile Thr Leu Met Lys Glu Val Asp Gly Asp
930 935 940

ttg aga att tcc gta tcc atg cca tcc atc gaa gta ggt acc atc ggt 2880
Leu Arg Ile Ser Val Ser Met Pro Ser Ile Glu Val Gly Thr Ile Gly
945 950 955 960

ggt ggt act gtt cta gaa cca caa ggt gcc atg ttg gac tta tta ggt 2928
Gly Gly Thr Val Leu Glu Pro Gln Gly Ala Met Leu Asp Leu Leu Gly
965 970 975

gta aga ggc cca cat gct acc gct cct ggt acc aac gca cgt caa tta 2976
Val Arg Gly Pro His Ala Thr Ala Pro Gly Thr Asn Ala Arg Gln Leu
980 985 990

gca aga ata gtt gcc tgt gcc gtc ttg gca ggt gaa tta tcc tta tgt 3024
Ala Arg Ile Val Ala Cys Ala Val Leu Ala Gly Glu Leu Ser Leu Cys
995 1000 1005

gct gcc cta gca gcc ggc cat ttg gtt caa agt cat atg acc cac aac 3072
Ala Ala Leu Ala Ala Gly His Leu Val Gln Ser His Met Thr His Asn
1010 1015 1020

agg aaa cct gct gaa cca aca aaa cct aac aat ttg gac gcc act gat 3120

Arg Lys Pro Ala Glu Pro Thr Lys Pro Asn Asn Leu Asp Ala Thr Asp
1025 1030 1035 1040

ata aat cgt ttg aaa gat ggg tcc gtc acc tgc att aaa tcc taa 3165
Ile Asn Arg Leu Lys Asp Gly Ser Val Thr Cys Ile Lys Ser
1045 1050

<210> 6
<211> 1054
<212> PRT
<213> Saccharomyces cerevisiae

<400> 6
Met Pro Pro Leu Phe Lys Gly Leu Lys Gln Met Ala Lys Pro Ile Ala
1 5 10 15

Tyr Val Ser Arg Phe Ser Ala Lys Arg Pro Ile His Ile Ile Leu Phe
20 25 30

Ser Leu Ile Ile Ser Ala Phe Ala Tyr Leu Ser Val Ile Gln Tyr Tyr
35 40 45

Phe Asn Gly Trp Gln Leu Asp Ser Asn Ser Val Phe Glu Thr Ala Pro
50 55 60

Asn Lys Asp Ser Asn Thr Leu Phe Gln Glu Cys Ser His Tyr Tyr Arg
65 70 75 80

Asp Ser Ser Leu Asp Gly Trp Val Ser Ile Thr Ala His Glu Ala Ser
85 90 95

Glu Leu Pro Ala Pro His His Tyr Tyr Leu Leu Asn Leu Asn Phe Asn
100 105 110

Ser Pro Asn Glu Thr Asp Ser Ile Pro Glu Leu Ala Asn Thr Val Phe
115 120 125

Glu Lys Asp Asn Thr Lys Tyr Ile Leu Gln Glu Asp Leu Ser Val Ser
130 135 140

Lys Glu Ile Ser Ser Thr Asp Gly Thr Lys Trp Arg Leu Arg Ser Asp
145 150 155 160

Arg Lys Ser Leu Phe Asp Val Lys Thr Leu Ala Tyr Ser Leu Tyr Asp
165 170 175

Val Phe Ser Glu Asn Val Thr Gln Ala Asp Pro Phe Asp Val Leu Ile
180 185 190

Met Val Thr Ala Tyr Leu Met Met Phe Tyr Thr Ile Phe Gly Leu Phe
195 200 205

Asn Asp Met Arg Lys Thr Gly Ser Asn Phe Trp Leu Ser Ala Ser Thr
210 215 220

Val Val Asn Ser Ala Ser Ser Leu Phe Leu Ala Leu Tyr Val Thr Gln
225 230 235 240

Cys Ile Leu Gly Lys Glu Val Ser Ala Leu Thr Leu Phe Glu Gly Leu
245 250 255

Pro Phe Ile Val Val Val Val Gly Phe Lys His Lys Ile Lys Ile Ala
260 265 270

Gln Tyr Ala Leu Glu Lys Phe Glu Arg Val Gly Leu Ser Lys Arg Ile
275 280 285

Thr Thr Asp Glu Ile Val Phe Glu Ser Val Ser Glu Glu Gly Gly Arg
290 295 300

Leu Ile Gln Asp His Leu Leu Cys Ile Phe Ala Phe Ile Gly Cys Ser
305 310 315 320

Met Tyr Ala His Gln Leu Lys Thr Leu Thr Asn Phe Cys Ile Leu Ser
325 330 335

Ala Phe Ile Leu Ile Phe Glu Leu Ile Leu Thr Pro Thr Phe Tyr Ser
340 345 350

Ala Ile Leu Ala Leu Arg Leu Glu Met Asn Val Ile His Arg Ser Thr
355 360 365

Ile Ile Lys Gln Thr Leu Glu Glu Asp Gly Val Val Pro Ser Thr Ala
370 375 380

Arg Ile Ile Ser Lys Ala Glu Lys Lys Ser Val Ser Ser Phe Leu Asn

385 390 395 400

Leu Ser Val Val Val Ile Ile Met Lys Leu Ser Val Ile Leu Leu Phe

405 410 415

Val Phe Ile Asn Phe Tyr Asn Phe Gly Ala Asn Trp Val Asn Asp Ala

420 425 430

Phe Asn Ser Leu Tyr Phe Asp Lys Glu Arg Val Ser Leu Pro Asp Phe

435 440 445

Ile Thr Ser Asn Ala Ser Glu Asn Phe Lys Glu Gln Ala Ile Val Ser

450 455 460

Val Thr Pro Leu Leu Tyr Tyr Lys Pro Ile Lys Ser Tyr Gln Arg Ile

465 470 475 480

Glu Asp Met Val Leu Leu Leu Leu Arg Asn Val Ser Val Ala Ile Arg

485 490 495

Asp Arg Phe Val Ser Lys Leu Val Leu Ser Ala Leu Val Cys Ser Ala

500 505 510

Val Ile Asn Val Tyr Leu Leu Asn Ala Ala Arg Ile His Thr Ser Tyr

515 520 525

Thr Ala Asp Gln Leu Val Lys Thr Glu Val Thr Lys Lys Ser Phe Thr

530 535 540

Ala Pro Val Gln Lys Ala Ser Thr Pro Val Leu Thr Asn Lys Thr Val
545 550 555 560

Ile Ser Gly Ser Lys Val Lys Ser Leu Ser Ser Ala Gln Ser Ser Ser
565 570 575

Ser Gly Pro Ser Ser Ser Ser Glu Glu Asp Asp Ser Arg Asp Ile Glu
580 585 590

Ser Leu Asp Lys Lys Ile Arg Pro Leu Glu Glu Leu Glu Ala Leu Leu
595 600 605

Ser Ser Gly Asn Thr Lys Gln Leu Lys Asn Lys Glu Val Ala Ala Leu
610 615 620

Val Ile His Gly Lys Leu Pro Leu Tyr Ala Leu Glu Lys Lys Leu Gly
625 630 635 640

Asp Thr Thr Arg Ala Val Ala Val Arg Arg Lys Ala Leu Ser Ile Leu
645 650 655

Ala Glu Ala Pro Val Leu Ala Ser Asp Arg Leu Pro Tyr Lys Asn Tyr
660 665 670

Asp Tyr Asp Arg Val Phe Gly Ala Cys Cys Glu Asn Val Ile Gly Tyr
675 680 685

Met Pro Leu Pro Val Gly Val Ile Gly Pro Leu Val Ile Asp Gly Thr
690 695 700

Ser Tyr His Ile Pro Met Ala Thr Thr Glu Gly Cys Leu Val Ala Ser
705 710 715 720

Ala Met Arg Gly Cys Lys Ala Ile Asn Ala Gly Gly Gly Ala Thr Thr
725 730 735

Val Leu Thr Lys Asp Gly Met Thr Arg Gly Pro Val Val Arg Phe Pro
740 745 750

Thr Leu Lys Arg Ser Gly Ala Cys Lys Ile Trp Leu Asp Ser Glu Glu
755 760 765

Gly Gln Asn Ala Ile Lys Lys Ala Phe Asn Ser Thr Ser Arg Phe Ala
770 775 780

Arg Leu Gln His Ile Gln Thr Cys Leu Ala Gly Asp Leu Leu Phe Met
785 790 795 800

Arg Phe Arg Thr Thr Thr Gly Asp Ala Met Gly Met Asn Met Ile Ser
805 810 815

Lys Gly Val Glu Tyr Ser Leu Lys Gln Met Val Glu Glu Tyr Gly Trp
820 825 830

Glu Asp Met Glu Val Val Ser Val Ser Gly Asn Tyr Cys Thr Asp Lys
835 840 845

Lys Pro Ala Ala Ile Asn Trp Ile Glu Gly Arg Gly Lys Ser Val Val

850 855 860

Ala Glu Ala Thr Ile Pro Gly Asp Val Val Arg Lys Val Leu Lys Ser
865 870 875 880

Asp Val Ser Ala Leu Val Glu Leu Asn Ile Ala Lys Asn Leu Val Gly
885 890 895

Ser Ala Met Ala Gly Ser Val Gly Gly Phe Asn Ala His Ala Ala Asn
900 905 910

Leu Val Thr Ala Val Phe Leu Ala Leu Gly Gln Asp Pro Ala Gln Asn
915 920 925

Val Glu Ser Ser Asn Cys Ile Thr Leu Met Lys Glu Val Asp Gly Asp
930 935 940

Leu Arg Ile Ser Val Ser Met Pro Ser Ile Glu Val Gly Thr Ile Gly
945 950 955 960

Gly Gly Thr Val Leu Glu Pro Gln Gly Ala Met Leu Asp Leu Leu Gly
965 970 975

Val Arg Gly Pro His Ala Thr Ala Pro Gly Thr Asn Ala Arg Gln Leu
980 985 990

Ala Arg Ile Val Ala Cys Ala Val Leu Ala Gly Glu Leu Ser Leu Cys
995 1000 1005

Ala Ala Leu Ala Ala Gly His Leu Val Gln Ser His Met Thr His Asn
1010 1015 1020

Arg Lys Pro Ala Glu Pro Thr Lys Pro Asn Asn Leu Asp Ala Thr Asp
1025 1030 1035 1040

Ile Asn Arg Leu Lys Asp Gly Ser Val Thr Cys Ile Lys Ser
1045 1050

<210> 7
<211> 2925
<212> DNA
<213> Saccharomyces cerevisiae

<400> 7
atgccgccgc tattcaaggg actgaaacag atggcaaagc caattgccta tgtttcaaga 60

ttttcggcga aacgaccaat tcatataata ctttttctc taatcatatc cgcattcgct 120

tatctatccg tcattcagta ttacttcaat ggttggcaac tagattcaaa tagtgttttt 180

gaaactgctc caaataaaga cttcaacact ctatttcaag aatgttccca ttactacaga 240

gattcctctc tagatggttg ggtatcaatc accgcgcatg aagctagtga gttaccagcc 300

ccacaccatt actatctatt aaacctgaac ttcaatagtc ctaatgaaac tgactccatt 360

ccagaactag ctaacacggt tttgagaaa gataatacaa aatatattct gcaagaagat 420

ctcagcgttt ccaaagaaat ttcttctact gatggaacga aatggaggtt aagaagtgac 480

agaaaaagtc ttttcgacgt aaagacgtta gcatattctc tctacgatgt attttcagaa 540

aatgtaaccc aagcagacca caaaatcaag attgcccagt atgccctgga gaaatttgaa 600

agagtcggtt tatctaaaag gattactacc gatgaaatcg tttttgaatc cgtgagcgaa 660

gagggtggtc gtttgattca agaccatttg ctttgtattt ttgcctttat cggatgctct 720

atgtatgctc accaattgaa gactttgaca aacttctgca tattatcagc atttatccta 780

attttcgaat tgattttaac tcctacattt tattctgcta tcttagcgct tagactggaa 840

atgaatgtta tccacagatc tactattatc aagcaaacat tagaagaaga cggtgttgtt 900

ccatctacag caagaatcat ttctaaggca gaaaagaaat ccgtatcttc tttcttaaat 960

ctcagtgtgg ttgtcattat catgaaactc tctgtcatac tgttgttcgt cttcatcaac 1020

ttttataact ttggtgcaaa ttgggtcaat gatgccttca attcattgta cttcgataag 1080

gaacgtgttt ctctaccaga ttttattacc tcgaatgcct ctgaaaactt taaagagcaa 1140

gctattgtta gtgtcacccc attattatat tacaaaccca ttaagtccta ccaacgcatt 1200

gaggatatgg ttcttctatt gcttcgtaat gtcagtgttg ccattcgtga taggttcgtc 1260

agtaaattag ttctttccgc cttagtatgc agtgctgtca tcaatgtgta tttattaaat 1320

gctgctagaa ttcataccag ttatactgca gaccaattgg tgaagactga agtcaccaag 1380

aagtctttta ctgctcctgt acaaaaggct tctacaccag ttttaaccaa taaaacagtc 1440

atttctggat cgaaagtcaa aagtttatca tctgcgcaat cgagctcatc aggaccttca 1500

tcatctagtg aggaagatga ttcccgcgat attgaaagct tggataagaa aatacgtcct 1560

ttagaagaat tagaagcatc attaagtagt ggaaatacaa aacaattgaa gaacaaagag 1620

gtcgctgcct tggttattca cggtaagtta cctttgtacg ctttggagaa aaaattaggt 1680

gatactacga gagcggttgc ggtacgtagg aaggctcttt caattttggc agaagctcct 1740

gtattagcat ctgatcgttt accatataaa aattatgact acgaccgcgt atttggcgct 1800

tgttgtgaaa atgttatagg ttacatgcct ttgcccgttg gtgttatagg ccccttggtt 1860

atcgatggta catcttatca tataccaatg gcaactacag agggttgttt ggtagcttct 1920

gccatgcgtg gctgtaaggc aatcaatgct ggcggtggtg caacaactgt tttaactaag 1980

gatggtatga caagaggccc agtagtccgt ttcccaactt tgaaaagatc tggtgcctgt 2040

aagatatggt tagactcaga agagggacaa aacgcaatta aaaaagcttt taactctaca 2100

tcaagatttg cacgtctgca acatattcaa acttgtctag caggagattt actcttcatg 2160

agatttagaa caactactgg tgacgcaatg ggtatgaata tgatttctaa gggtgtcgaa 2220

tactcattaa agcaaatggt agaagagtat ggctgggaag atatggaggt tgtctccgtt 2280

tctggtaact actgtaccga caaaaaacca gctgccatca actggatcga aggtcgtggt 2340

aagagtgtcg tcgcagaagc tactattcct ggtgatgttg tcagaaaagt gttaaaaagt 2400

gatgtttccg cattggttga gttgaacatt gctaagaatt tggttggatc tgcaatggct 2460

gggtctgttg gtggatttaa cgcacgtgca gctaatttag tgacagctgt tttcttggca 2520

ttaggacaag atcctgcaca aaatgtcgaa agttccaact gtataacatt gatgaaagaa 2580

gtggacggtg atttgagaat ttccgtatcc atgccatcca tcgaagtagg taccatcggt 2640

ggtggtactg ttctagaacc acaaggtgcc atgttggact tattaggtgt aagaggccca 2700

catgctaccg ctcctggtac caacgcacgt caattagcaa gaatagttgc ctgtgccgtc 2760

ttggcaggtg aattatcctt atgtgctgcc ctagcagccg gccatttggt tcaaagtcat 2820

atgacccaca acaggaaacc tgctgaacca acaaaaccta acaatttgga cgccactgat 2880

ataaatcgtt tgaaagatgg gtccgtcacc tgcattaaat cctaa 2925

<210> 8

<211> 3090

<212> DNA

<213> Saccharomyces cerevisiae

<400> 8

atgccgccgc tattcaaggg actgaaacag atggcaaagc caattgccta tgtttcaaga 60

ttttcggcga aacgaccaat tcatataata ctttttctc taatcatatc cgcattcgct 120

tatctatccg tcattcagta ttacttcaat ggttggcaac tagattcaaa tagtgttttt 180

gaaactgctc caaataaaga cttcaacact ctatttcaag aatgttccca ttactacaga 240

gattcctctc tagatggttg ggtatcaatc accgcgcatg aagctagtga gttaccagcc 300

ccacaccatt actatctatt aaacctgaac ttcaatagtc ctaatgaaac tgactccatt 360

ccagaactag ctaacacggt tttgagaaa gataatacaa aatatattct gcaagaagat 420

ctcagcgttt ccaaagaaat ttcttctact gatggaacga aatggaggtt aagaagtgac 480

agaaaaagtc ttttcgacgt aaagacgtta gcatattctc tctacgatgt attttcagaa 540

aatgtaaccc aagcagaccc gtttgacgtc cttattatgg ttactgccta cctaatgatg 600

ttctacacca tattcggcct cttcaatgac atgaggaaga ccgggtcaaa tttttggttg 660

agcgcctcta cagtggtcaa ttctgcatca tcacttttct tagcattgta tgtcacccaa 720

tgtattctag gcaaagaagt ttccgcatta actctttttg aaggtttgcc tttcattgta 780

gttgttgttg gtttcaagca caaaatcaag attgcccagt atgccctgga gaaatttgaa 840

agagtcggtt tatctaaaag gattactacc gatgaaatcg tttttgaatc cgtgagcgaa 900

gagggtggtc gtttgattca agaccatttg ctttgtattt ttgcctttat cggatgctct 960

atgtatgctc accaattgaa gactttgaca aacttctgca tattatcagc atttatccta 1020

attttcgaat tgattttaac tcctacattt tattctgcta tcttagcgct tagactggaa 1080

atgaatgtta tccacagatc tactattatc aagcaaacat tagaagaaga cggtgttgtt 1140

ccatctacag caagaatcat ttctaaggca gaaaagaaat ccgtatcttc taactttggt 1200

gcaaattggg tcaatgatgc cttcaattca ttgtacttcg ataaggaacg tgtttctcta 1260

ccagatttta ttacctcgaa tgcctctgaa aactttaaag agcaagctat tgttagtgtc 1320

accccattat tatattacaa acccattaag tcctaccaac gcattgagga tatggttctt 1380

ctattgcttc gtaatgtcag tgttgccatt cgtgataggt tcgtcagtaa attagttctt 1440

tccgccttag tatgcagtgc tgtcatcaat gtgtatttat taaatgctgc tagaattcat 1500

accagttata ctgcagacca attggtgaag actgaagtca ccaagaagtc ttttactgct 1560

cctgtacaaa aggcttctac accagtttta accaataaaa cagtcatttc tggatcgaaa 1620

gtcaaaagtt tatcatctgc gcaatcgagc tcatcaggac cttcatcatc tagtgaggaa 1680

gatgattccc gcgatattga aagcttggat aagaaaatac gtcctttaga agaattagaa 1740

gcatcattaa gtagtggaaa tacaaaacaa ttgaagaaca aagaggtcgc tgccttggtt 1800

attcacggta agttaccttt gtacgctttg gagaaaaaat taggtgatac tacgagagcg 1860

gttgcggtac gtaggaaggc tctttcaatt ttggcagaag ctcctgtatt agcatctgat 1920

cgtttaccat ataaaaatta tgactacgac cgcgtatttg gcgcttgttg tgaaaatgtt 1980

ataggttaca tgcctttgcc cgttggtgtt ataggcccct tggttatcga tggtacatct 2040

tatcatatac caatggcaac tacagagggt tgtttggtag cttctgccat gcgtggctgt 2100

aaggcaatca atgctggcgg tggtgcaaca actgttttaa ctaaggatgg tatgacaaga 2160

ggcccagtag tccgtttccc aactttgaaa agatctggtg cctgtaagat atggttagac 2220

tcagaagagg gacaaaacgc aattaaaaaa gcttttaact ctacatcaag atttgcacgt 2280

ctgcaacata ttcaaacttg tctagcagga gatttactct tcatgagatt tagaacaact 2340

actggtgacg caatgggtat gaatatgatt tctaagggtg tcgaatactc attaaagcaa 2400

atggtagaag agtatggctg ggaagatatg gaggttgtct ccgtttctgg taactactgt 2460

accgacaaaa aaccagctgc catcaactgg atcgaaggtc gtggtaagag tgtcgtcgca 2520

gaagctacta ttcctggtga tgttgtcaga aaagtgttaa aaagtgatgt ttccgcattg 2580

gttgagttga acattgctaa gaatttggtt ggatctgcaa tggctgggtc tgttggtgga 2640

tttaacgcac gtgcagctaa tttagtgaca gctgttttct tggcattagg acaagatcct 2700

gcacaaaatg tcgaaagttc caactgtata acattgatga aagaagtgga cggtgatttg 2760

agaatttccg tatccatgcc atccatcgaa gtaggtacca tcggtggtgg tactgttcta 2820

gaaccacaag gtgccatgtt ggacttatta ggtgtaagag gcccacatgc taccgctcct 2880

ggtaccaacg cacgtcaatt agcaagaata gttgcctgtg ccgtcttggc aggtgaatta 2940

tccttatgtg ctgccctagc agccggccat ttggttcaaa gtcatatgac ccacaacagg 3000

aaacctgctg aaccaacaaa acctaacaat ttggacgcca ctgatataaa tcgtttgaaa 3060

gatgggtccg tcacctgcat taaatcctaa 3090

<210> 9

<211> 2973

<212> DNA

<213> Saccharomyces cerevisiae

<400> 9

atgccgccgc tattcaaggg actgaaacag atggcaaagc caattgccta tgtttcaaga 60

ttttcggcga aacgaccaat tcatataata ctttttctc taatcatatc cgcattcgct 120

tatctatccg tcattcagta ttacttcaat ggttggcaac tagattcaaa tagtgttttt 180

gaaactgctc caaataaaga cttcaacact ctatttcaag aatgttccca ttactacaga 240

gattcctctc tagatggttg ggtatcaatc accgcgcatg aagctagtga gttaccagcc 300

ccacaccatt actatctatt aaacctgaac ttcaatagtc ctaatgaaac tgactccatt 360

ccagaactag ctaacacggt tttgagaaa gataatacaa aatatattct gcaagaagat 420

ctcagcgttt ccaaagaaat ttcttctact gatggaacga aatggaggtt aagaagtgac 480

agaaaaaagtc ttttcgacgt aaagacgtta gcatattctc tctacgatgt attttcagaa 540

aatgtaaccc aagcagaccc gtttgacgtc cttattatgg ttactgccta cctaatgatg 600

ttctacacca tattcggcct cttcaatgac atgaggaaga ccgggtcaaa tttttggttg 660

agcgcctcta cagtggtcaa ttctgcatca tcacttttct tagcattgta tgtcacccaa 720

tgtattctag gcaaagaagt ttccgcatta actcttttg aaggtttgcc tttcattgta 780

gttgttgttg gtttcaagca caaaatcaag attgcccagt atgccctgga gaaatttgaa 840

agagtcggtt tatctaaaag gattactacc gatgaaatcg tttttgaatc cgtgagcgaa 900

gagggtggtc gtttgattca agaccatttg ctttgtattt ttgcctttat cggatgctct 960

atgtatgctc accaattgaa gactttgaca aacttctgca tattatcagc atttatccta 1020

attttcgaat tgattttaac tcctacattt tattctgcta tcttagcgct tagactggaa 1080

atgaatgtta tccacagatc tactattatc aagcaaacat tagaagaaga cggtgttgtt 1140

ccatctacag caagaatcat ttctaaggca gaaaagaaat ccgtatcttc tttcttaaat 1200

ctcagtgtgg ttgtcattat catgaaactc tctgtcatac tgttgttcgt cttcatcaac 1260

ttttataact ttggtgcaaa ttgggtcaat gatgccttca attcattgta cttcgataag 1320

gaacgtgttt ctctaccaga ttttattacc tcgaatgcct ctgaaaactt taaagagcaa 1380

cataccagtt atactgcaga ccaattggtg aagactgaag tcaccaagaa gtcttttact 1440

gctcctgtac aaaaggcttc tacaccagtt ttaaccaata aaacagtcat ttctggatcg 1500

aaagtcaaaa gtttatcatc tgcgcaatcg agctcatcag gaccttcatc atctagtgag 1560

gaagatgatt cccgcgatat tgaaagcttg gataagaaaa tacgtccttt agaagaatta 1620

gaagcatcat taagtagtgg aaatacaaaa caattgaaga acaaagaggt cgctgccttg 1680

gttattcacg gtaagttacc tttgtacgct ttggagaaaa aattaggtga tactacgaga 1740

gcggttgcgg tacgtaggaa ggctctttca attttggcag aagctcctgt attagcatct 1800

gatcgtttac catataaaaa ttatgactac gaccgcgtat ttggcgcttg ttgtgaaaat 1860

gttataggtt acatgccttt gcccgttggt gttataggcc ccttggttat cgatggtaca 1920

tcttatcata taccaatggc aactacagag ggttgtttgg tagcttctgc catgcgtggc 1980

tgtaaggcaa tcaatgctgg cggtggtgca acaactgttt taactaagga tggtatgaca 2040

agaggcccag tagtccgttt cccaactttg aaaagatctg gtgcctgtaa gatatggtta 2100

gactcagaag agggacaaaa cgcaattaaa aaagctttta actctacatc aagatttgca 2160

cgtctgcaac atattcaaac ttgtctagca ggagatttac tcttcatgag atttagaaca 2220

actactggtg acgcaatggg tatgaatatg atttctaagg gtgtcgaata ctcattaaag 2280

caaatggtag aagagtatgg ctgggaagat atggaggttg tctccgtttc tggtaactac 2340

tgtaccgaca aaaaaccagc tgccatcaac tggatcgaag gtcgtggtaa gagtgtcgtc 2400

gcagaagcta ctattcctgg tgatgttgtc agaaaagtgt taaaaagtga tgtttccgca 2460

ttggttgagt tgaacattgc taagaatttg gttggatctg caatggctgg gtctgttggt 2520

ggatttaacg cacgtgcagc taatttagtg acagctgttt tcttggcatt aggacaagat 2580

cctgcacaaa atgtcgaaag ttccaactgt ataacattga tgaaagaagt ggacggtgat 2640

ttgagaattt ccgtatccat gccatccatc gaagtaggta ccatcggtgg tggtactgtt 2700

ctagaaccac aaggtgccat gttggactta ttaggtgtaa gaggcccaca tgctaccgct 2760

cctggtacca acgcacgtca attagcaaga atagttgcct gtgccgtctt ggcaggtgaa 2820

ttatccttat gtgctgccct agcagccggc catttggttc aaagtcatat gacccacaac 2880

aggaaacctg ctgaaccaac aaaacctaac aatttggacg ccactgatat aaatcgtttg 2940

aaagatgggt ccgtcacctg cattaaatcc taa 2973

<210> 10

<211> 2457

<212> DNA

<213> Saccharomyces cerevisiae

<400> 10

atgccgccgc tattcaaggg actgaaacag atggcaaagc caattgccta tgtttcaaga 60

ttttcggcga aacgaccaat tcatataata ctttttctc taatcatatc cgcattcgct 120

tatctatccg tcattcagta ttacttcaat ggttggcaac tagattcaaa tagtgttttt 180

gaaactgctc caaataaaga cttcaacact ctatttcaag aatgttccca ttactacaga 240

gattcctctc tagatggttg ggtatcaatc accgcgcatg aagctagtga gttaccagcc 300

ccacaccatt actatctatt aaacctgaac ttcaatagtc ctaatgaaac tgactccatt 360

ccagaactag ctaacacggt ttttgagaaa gataatacaa aatatattct gcaagaagat 420

ctcagcgttt ccaaagaaat ttcttctact gatggaacga aatggaggtt aagaagtgac 480

agaaaaagtc ttttcgacgt aaagacgtta gcatattctc tctacgatgt attttcagaa 540

aatgtaaccc aagcagacaa ctttggtgca aattgggtca atgatgcctt caattcattg 600

tacttcgata aggaacgtgt ttctctacca gattttatta cctcgaatgc ctctgaaaac 660

tttaaagagc aagctattgt tagtgtcacc ccattattat attacaaacc cattaagtcc 720

taccaacgca ttgaggatat ggttcttcta ttgcttcgta atgtcagtgt tgccattcgt 780

gataggttcg tcagtaaatt agttctttcc gccttagtat gcagtgctgt catcaatgtg 840

tatttattaa atgctgctag aattcatacc agttatactg cagaccaatt ggtgaagact 900

gaagtcacca agaagtcttt tactgctcct gtacaaaagg cttctacacc agttttaacc 960

aataaaacag tcatttctgg atcgaaagtc aaaagtttat catctgcgca atcgagctca 1020

tcaggacctt catcatctag tgaggaagat gattcccgcg atattgaaag cttggataag 1080

aaaatacgtc ctttagaaga attagaagca tcattaagta gtggaaatac aaaacaattg 1140

aagaacaaag aggtcgctgc cttggttatt cacggtaagt tacctttgta cgctttggag 1200

aaaaaattag gtgatactac gagagcggtt gcggtacgta ggaaggctct ttcaattttg 1260

gcagaagctc ctgtattagc atctgatcgt ttaccatata aaaattatga ctacgaccgc 1320

gtatttggcg cttgttgtga aaatgttata ggttacatgc ctttgccccgt tggtgttata 1380

ggccccttgg ttatcgatgg tacatcttat catataccaa tggcaactac agagggttgt 1440

ttggtagctt ctgccatgcg tggctgtaag gcaatcaatg ctggcggtgg tgcaacaact 1500

gttttaacta aggatggtat gacaagaggc ccagtagtcc gtttcccaac tttgaaaaga 1560

tctggtgcct gtaagatatg gttagactca gaagagggac aaaacgcaat taaaaaagct 1620

tttaactcta catcaagatt tgcacgtctg caacatattc aaacttgtct agcaggagat 1680

ttactcttca tgagatttag aacaactact ggtgacgcaa tgggtatgaa tatgatttct 1740

aagggtgtcg aatactcatt aaagcaaatg gtagaagagt atggctggga agatatggag 1800

gttgtctccg tttctggtaa ctactgtacc gacaaaaaac cagctgccat caactggatc 1860

gaaggtcgtg gtaagagtgt cgtcgcagaa gctactattc ctggtgatgt tgtcagaaaa 1920

gtgttaaaaa gtgatgtttc cgcattggtt gagttgaaca ttgctaagaa tttggttgga 1980

tctgcaatgg ctgggtctgt tggtggattt aacgcacgtg cagctaattt agtgacagct 2040

gttttcttgg cattaggaca agatcctgca caaaatgtcg aaagttccaa ctgtataaca 2100

ttgatgaaag aagtggacgg tgatttgaga atttccgtat ccatgccatc catcgaagta 2160

ggtaccatcg gtggtggtac tgttctagaa ccacaaggtg ccatgttgga cttattaggt 2220

gtaagaggcc cacatgctac cgctcctggt accaacgcac gtcaattagc aagaatagtt 2280

gcctgtgccg tcttggcagg tgaattatcc ttatgtgctg ccctagcagc cggccatttg 2340

gttcaaagtc atatgaccca caacaggaaa cctgctgaac caacaaaacc taacaatttg 2400

gacgccactg atataaatcg tttgaaagat gggtccgtca cctgcattaa atcctaa 2457

<210> 11

<211> 2151

<212> DNA

<213> Saccharomyces cerevisiae

<400> 11

atgccgccgc tattcaaggg actgaaacag atggcaaagc caattgccta tgtttcaaga 60

ttttcggcga aacgaccaat tcatataata ctttttctc taatcatatc cgcattcgct 120

tatctatccg tcattcagta ttacttcaat ggttggcaac tagattcaaa tagtgttttt 180

gaaactgctc caaataaaga cttcaacact ctatttcaag aatgttccca ttactacaga 240

gattcctctc tagatggttg ggtatcaatc accgcgcatg aagctagtga gttaccagcc 300

ccacaccatt actatctatt aaacctgaac ttcaatagtc ctaatgaaac tgactccatt 360

ccagaactag ctaacacggt ttttgagaaa gataatacaa aatatattct gcaagaagat 420

ctcagcgttt ccaaagaaat ttcttctact gatggaacga aatggaggtt aagaagtgac 480

agaaaaagtc ttttcgacgt aaagacgtta gcatattctc tctacgatgt attttcagaa 540

aatgtaaccc aagcagacca taccagttat actgcagacc aattggtgaa gactgaagtc 600

accaagaagt cttttactgc tcctgtacaa aaggcttcta caccagtttt aaccaataaa 660

acagtcattt ctggatcgaa agtcaaaagt ttatcatctg cgcaatcgag ctcatcagga 720

ccttcatcat ctagtgagga agatgattcc cgcgatattg aaagcttgga taagaaaata 780

cgtcctttag aagaattaga agcatcatta agtagtggaa atacaaaaca attgaagaac 840

aaagaggtcg ctgccttggt tattcacggt aagttacctt tgtacgcttt ggagaaaaaa 900

ttaggtgata ctacgagagc ggttgcggta cgtaggaagg ctctttcaat tttggcagaa 960

gctcctgtat tagcatctga tcgtttacca tataaaaatt atgactacga ccgcgtattt 1020

ggcgcttgtt gtgaaaatgt tataggttac atgcctttgc ccgttggtgt tataggcccc 1080

ttggttatcg atggtacatc ttatcatata ccaatggcaa ctacagaggg ttgtttggta 1140

gcttctgcca tgcgtggctg taaggcaatc aatgctggcg gtggtgcaac aactgtttta 1200

actaaggatg gtatgacaag aggcccagta gtccgtttcc caactttgaa aagatctggt 1260

gcctgtaaga tatggttaga ctcagaagag ggacaaaacg caattaaaaa agcttttaac 1320

tctacatcaa gatttgcacg tctgcaacat attcaaactt gtctagcagg agatttactc 1380

ttcatgagat ttagaacaac tactggtgac gcaatgggta tgaatatgat ttctaagggt 1440

gtcgaatact cattaaagca aatggtagaa gagtatggct gggaagatat ggaggttgtc 1500

tccgtttctg gtaactactg taccgacaaa aaaccagctg ccatcaactg gatcgaaggt 1560

cgtggtaaga gtgtcgtcgc agaagctact attcctggtg atgttgtcag aaaagtgtta 1620

aaaagtgatg tttccgcatt ggttgagttg aacattgcta agaatttggt tggatctgca 1680

atggctgggt ctgttggtgg atttaacgca cgtgcagcta atttagtgac agctgttttc 1740

ttggcattag gacaagatcc tgcacaaaat gtcgaaagtt ccaactgtat aacattgatg 1800

aaagaagtgg acggtgattt gagaatttcc gtatccatgc catccatcga agtaggtacc 1860

atcggtggtg gtactgttct agaaccacaa ggtgccatgt tggacttatt aggtgtaaga 1920

ggcccacatg ctaccgctcc tggtaccaac gcacgtcaat tagcaagaat agttgcctgt 1980

gccgtcttgg caggtgaatt atccttatgt gctgccctag cagccggcca tttggttcaa 2040

agtcatatga cccacaacag gaaacctgct gaaccaacaa aacctaacaa tttggacgcc 2100

actgatataa atcgtttgaa agatgggtcc gtcacctgca ttaaatccta a 2151

<210> 12
<211> 1620
<212> DNA
<213> Saccharomyces cerevisiae

<400> 12
atgccgccgc tattcaaggg actgaaacat accagttata ctgcagacca attggtgaag 60

actgaagtca ccaagaagtc ttttactgct cctgtacaaa aggcttctac accagtttta 120

accaataaaa cagtcatttc tggatcgaaa gtcaaaagtt tatcatctgc gcaatcgagc 180

tcatcaggac cttcatcatc tagtgaggaa gatgattccc gcgatattga aagcttggat 240

aagaaaatac gtcctttaga agaattagaa gcatcattaa gtagtggaaa tacaaaacaa 300

ttgaagaaca aagaggtcgc tgccttggtt attcacggta agttaccttt gtacgctttg 360

gagaaaaaat taggtgatac tacgagagcg gttgcggtac gtaggaaggc tctttcaatt 420

ttggcagaag ctcctgtatt agcatctgat cgtttaccat ataaaaatta tgactacgac 480

cgcgtatttg gcgcttgttg tgaaaatgtt ataggttaca tgcctttgcc cgttggtgtt 540

ataggcccct tggttatcga tggtacatct tatcatatac caatggcaac tacagagggt 600

tgtttggtag cttctgccat gcgtggctgt aaggcaatca atgctggcgg tggtgcaaca 660

actgttttaa ctaaggatgg tatgacaaga ggcccagtag tccgtttccc aactttgaaa 720

agatctggtg cctgtaagat atggttagac tcagaagagg gacaaaacgc aattaaaaaa 780

gcttttaact ctacatcaag atttgcacgt ctgcaacata ttcaaacttg tctagcagga 840

gatttactct tcatgagatt tagaacaact actggtgacg caatgggtat gaatatgatt 900

tctaagggtg tcgaatactc attaaagcaa atggtagaag agtatggctg ggaagatatg 960

gaggttgtct ccgtttctgg taactactgt accgacaaaa aaccagctgc catcaactgg 1020

atcgaaggtc gtggtaagag tgtcgtcgca gaagctacta ttcctggtga tgttgtcaga 1080

aaagtgttaa aaagtgatgt ttccgcattg gttgagttga acattgctaa gaatttggtt 1140

ggatctgcaa tggctgggtc tgttggtgga tttaacgcac gtgcagctaa tttagtgaca 1200

gctgttttct tggcattagg acaagatcct gcacaaaatg tcgaaagttc caactgtata 1260

acattgatga aagaagtgga cggtgatttg agaatttccg tatccatgcc atccatcgaa 1320

gtaggtacca tcggtggtgg tactgttcta gaaccacaag gtgccatgtt ggacttatta 1380

ggtgtaagag gcccacatgc taccgctcct ggtaccaacg cacgtcaatt agcaagaata 1440

gttgcctgtg ccgtcttggc aggtgaatta tccttatgtg ctgccctagc agccggccat 1500

ttggttcaaa gtcatatgac ccacaacagg aaacctgctg aaccaacaaa acctaacaat 1560

ttggacgcca ctgatataaa tcgtttgaaa gatgggtccg tcacctgcat taaatcctaa 1620

<210> 13

<211> 1377

<212> DNA

<213> Saccharomyces cerevisiae

<400> 13

atgccgccgc tattcaaggg actgaaagca tcattaagta gtggaaatac aaaacaattg 60

aagaacaaag aggtcgctgc cttggttatt cacggtaagt tacctttgta cgctttggag 120

aaaaaattag gtgatactac gagagcggtt gcggtacgta ggaaggctct ttcaattttg 180

gcagaagctc ctgtattagc atctgatcgt ttaccatata aaaattatga ctacgaccgc 240

gtatttggcg cttgttgtga aaatgttata ggttacatgc ctttgcccgt tggtgttata 300

ggcccccttgg ttatcgatgg tacatcttat catataccaa tggcaactac agagggttgt 360

ttggtagctt ctgccatgcg tggctgtaag gcaatcaatg ctggcggtgg tgcaacaact 420

gttttaacta aggatggtat gacaagaggc ccagtagtcc gtttcccaac tttgaaaaga 480

tctggtgcct gtaagatatg gttagactca gaagagggac aaaacgcaat taaaaaagct 540

tttaactcta catcaagatt tgcacgtctg caacatattc aaacttgtct agcaggagat 600

ttactcttca tgagatttag aacaactact ggtgacgcaa tgggtatgaa tatgatttct 660

aagggtgtcg aatactcatt aaagcaaatg gtagaagagt atggctggga agatatggag 720

gttgtctccg tttctggtaa ctactgtacc gacaaaaaac cagctgccat caactggatc 780

gaaggtcgtg gtaagagtgt cgtcgcagaa gctactattc ctggtgatgt tgtcagaaaa 840

gtgttaaaaa gtgatgtttc cgcattggtt gagttgaaca ttgctaagaa tttggttgga 900

tctgcaatgg ctgggtctgt tggtggattt aacgcacgtg cagctaattt agtgacagct 960

gttttcttgg cattaggaca agatcctgca caaaatgtcg aaagttccaa ctgtataaca 1020

ttgatgaaag aagtggacgg tgatttgaga atttccgtat ccatgccatc catcgaagta 1080

ggtaccatcg gtggtggtac tgttctagaa ccacaaggtg ccatgttgga cttattaggt 1140

gtaagaggcc cacatgctac cgctcctggt accaacgcac gtcaattagc aagaatagtt 1200

gcctgtgccg tcttggcagg tgaattatcc ttatgtgctg ccctagcagc cggccatttg 1260

gttcaaagtc atatgaccca caacaggaaa cctgctgaac caacaaaacc taacaatttg 1320

gacgccactg atataaatcg tttgaaagat gggtccgtca cctgcattaa atcctaa 1377

<210> 14

<211> 1302

<212> DNA

<213> Saccharomyces cerevisiae

<400> 14

atgccgccgc tattcaaggg actgaaacct ttgtacgctt tggagaaaaa attaggtgat 60

actacgagag cggttgcggt acgtaggaag gctctttcaa ttttggcaga agctcctgta 120

ttagcatctg atcgtttacc atataaaaat tatgactacg accgcgtatt tggcgcttgt 180

tgtgaaaatg ttataggtta catgcctttg cccgttggtg ttataggccc cttggttatc 240

gatggtacat cttatcatat accaatggca actacagagg gttgtttggt agcttctgcc 300

atgcgtggct gtaaggcaat caatgctggc ggtggtgcaa caactgtttt aactaaggat 360

ggtatgacaa gaggcccagt agtccgtttc ccaactttga aaagatctgg tgcctgtaag 420

atatggttag actcagaaga gggacaaaac gcaattaaaa aagcttttaa ctctacatca 480

agatttgcac gtctgcaaca tattcaaact tgtctagcag gagatttact cttcatgaga 540

tttagaacaa ctactggtga cgcaatgggt atgaatatga tttctaaggg tgtcgaatac 600

tcattaaagc aaatggtaga agagtatggc tgggaagata tggaggttgt ctccgtttct 660

ggtaactact gtaccgacaa aaaaccagct gccatcaact ggatcgaagg tcgtggtaag 720

agtgtcgtcg cagaagctac tattcctggt gatgttgtca gaaaagtgtt aaaaagtgat 780

gtttccgcat tggttgagtt gaacattgct aagaatttgg ttggatctgc aatggctggg 840

tctgttggtg gatttaacgc acgtgcagct aatttagtga cagctgtttt cttggcatta 900

ggacaagatc ctgcacaaaa tgtcgaaagt tccaactgta taacattgat gaaagaagtg 960

gacggtgatt tgagaatttc cgtatccatg ccatccatcg aagtaggtac catcggtggt 1020

ggtactgttc tagaaccaca aggtgccatg ttggacttat taggtgtaag aggcccacat 1080

gctaccgctc ctggtaccaa cgcacgtcaa ttagcaagaa tagttgcctg tgccgtcttg 1140

gcaggtgaat tatccttatg tgctgcccta gcagccggcc atttggttca aagtcatatg 1200

acccacaaca ggaaacctgc tgaaccaaca aaacctaaca atttggacgc cactgatata 1260

aatcgtttga aagatgggtc cgtcacctgc attaaatcct aa 1302

<210> 15

<211> 1203

<212> DNA

<213> Saccharomyces cerevisiae

<400> 15

atgccgccgc tattcaaggg actgaaatct gatcgtttac catataaaaa ttatgactac 60

gaccgcgtat ttggcgcttg ttgtgaaaat gttataggtt acatgccttt gcccgttggt 120

gttataggcc ccttggttat cgatggtaca tcttatcata taccaatggc aactacagag 180

ggttgtttgg tagcttctgc catgcgtggc tgtaaggcaa tcaatgctgg cggtggtgca 240

acaactgttt taactaagga tggtatgaca agaggcccag tagtccgttt cccaactttg 300

aaaagatctg gtgcctgtaa gatatggtta gactcagaag agggacaaaa cgcaattaaa 360

aaagctttta actctacatc aagatttgca cgtctgcaac atattcaaac ttgtctagca 420

ggagatttac tcttcatgag atttagaaca actactggtg acgcaatggg tatgaatatg 480

atttctaagg gtgtcgaata ctcattaaag caaatggtag aagagtatgg ctgggaagat 540

atggaggttg tctccgtttc tggtaactac tgtaccgaca aaaaaccagc tgccatcaac 600

tggatcgaag gtcgtggtaa gagtgtcgtc gcagaagcta ctattcctgg tgatgttgtc 660

agaaaagtgt taaaaagtga tgtttccgca ttggttgagt tgaacattgc taagaatttg 720

gttggatctg caatggctgg gtctgttggt ggatttaacg cacgtgcagc taatttagtg 780

acagctgttt tcttggcatt aggacaagat cctgcacaaa atgtcgaaag ttccaactgt 840

ataacattga tgaaagaagt ggacggtgat ttgagaattt ccgtatccat gccatccatc 900

gaagtaggta ccatcggtgg tggtactgtt ctagaaccac aaggtgccat gttggactta 960

ttaggtgtaa gaggcccaca tgctaccgct cctggtacca acgcacgtca attagcaaga 1020

atagttgcct gtgccgtctt ggcaggtgaa ttatccttat gtgctgccct agcagccggc 1080

catttggttc aaagtcatat gacccacaac aggaaacctg ctgaaccaac aaaacctaac 1140

aatttggacg ccactgatat aaatcgtttg aaagatgggt ccgtcacctg cattaaatcc 1200

taa 1203

<210> 16

<211> 975

<212> DNA

<213> Saccharomyces cerevisiae

<400> 16

atgccgccgc tattcaaggg actgaaaaag gatggtatga caagaggccc agtagtccgt 60

ttcccaactt tgaaaagatc tggtgcctgt aagatatggt tagactcaga agagggacaa 120

```
aacgcaatta aaaaagcttt taactctaca tcaagatttg cacgtctgca acatattcaa 180

acttgtctag caggagattt actcttcatg agatttagaa caactactgg tgacgcaatg 240

ggtatgaata tgatttctaa gggtgtcgaa tactcattaa agcaaatggt agaagagtat 300

ggctgggaag atatggaggt tgtctccgtt tctggtaact actgtaccga caaaaaacca 360

gctgccatca actggatcga aggtcgtggt aagagtgtcg tcgcagaagc tactattcct 420

ggtgatgttg tcagaaaagt gttaaaaagt gatgtttccg cattggttga gttgaacatt 480

gctaagaatt tggttggatc tgcaatggct gggtctgttg gtggatttaa cgcacgtgca 540

gctaatttag tgacagctgt tttcttggca ttaggacaag atcctgcaca aaatgtcgaa 600

agttccaact gtataacatt gatgaaagaa gtggacggtg atttgagaat ttccgtatcc 660

atgccatcca tcgaagtagg taccatcggt ggtggtactg ttctagaacc acaaggtgcc 720

atgttggact tattaggtgt aagaggccca catgctaccg ctcctggtac caacgcacgt 780

caattagcaa gaatagttgc ctgtgccgtc ttggcaggtg aattatcctt atgtgctgcc 840

ctagcagccg gccatttggt tcaaagtcat atgacccaca acaggaaacc tgctgaacca 900

acaaaaccta acaatttgga cgccactgat ataaatcgtt tgaaagatgg gtccgtcacc 960

tgcattaaat cctaa                                                  975
```

<210> 17

<211> 992

<212> DNA

<213> Saccharomyces cerevisiae

<400> 17

ggatcctcta gctccctaac atgtaggtgg cggaggggag atatacaata gaacagatac 60

cagacaagac ataatgggct aaacaagact acaccaatta cactgcctca ttgatggtgg 120

tacataacga actaatactg tagccctaga cttgatagcc atcatcatat cgaagtttca 180

ctaccctttt tccatttgcc atctattgaa gtaataatag gcgcatgcaa cttcttttct 240

tttttttttct tttctctctc ccccgttgtt gtctcaccat atccgcaatg acaaaaaaat 300

gatggaagac actaaaggaa aaaattaacg acaaagacag caccaacaga tgtcgttgtt 360

ccagagctga tgaggggtat ctcgaagcac acgaaacttt ttccttcctt cattcacgca 420

cactactctc taatgagcaa cggtatacgg ccttccttcc agttacttga atttgaaata 480

aaaaaagttt gctgtcttgc tatcaagtat aaatagacct gcaattatta atcttttgtt 540

tcctcgtcat tgttctcgtt ccctttcttc cttgtttctt tttctgcaca atatttcaag 600

ctataccaag catacaatca actggtaccc gggtcgactc gagctctaga ggttaactaa 660

gcgaatttct tatgatttat gatttttatt attaaataag ttataaaaaa aataagtgta 720

tacaaatttt aaagtgactc ttaggtttta aaacgaaaat tcttattctt gagtaactct 780

ttcctgtagg tcaggttgct ttctcaggta tagcatgagg tcgctcttat tgaccacatc 840

tctaccggca tgccgagcaa atgcctgcaa atcgctcccc atttcaccca attgtagata 900

tgctaactcc agcaatgagt tgatgaatct cggtgtgtat tttatgtcct cagaggacaa 960

cacctgttgt aatcgttctt ccacacggat cc 992

<210> 18
<211> 30
<212> DNA
<213> Artificial Sequence

<220>
<223> Description of Artificial Sequence: synthetic DNA

<400> 18
tgcatctcga gggccgcatc atgtaattag 30

<210> 19
<211> 32
<212> DNA

<213> Artificial Sequence

<220>

<223> Description of Artificial Sequence: synthetic DNA

<400> 19

cattagggcc cggccgcaaa ttaaagcctt cg 32

<210> 20

<211> 32

<212> DNA

<213> Artificial Sequence

<220>

<223> Description of Artificial Sequence: synthetic DNA

<400> 20

gatcgagctc ctccctaaca tgtaggtggc gg 32

<210> 21

<211> 34

<212> DNA

<213> Artificial Sequence

<220>

<223> Description of Artificial Sequence: synthetic DNA

<400> 21

cccgccgcgg agttgattgt atgcttggta tagc 34

<210> 22

<211> 33

<212> DNA

<213> Artificial Sequence

<220>

<223> Description of Artificial Sequence: synthetic DNA

<400> 22

cacggagctc cagttcgagt ttatcattat caa 33

<210> 23

<211> 35

<212> DNA

<213> Artificial Sequence

<220>

<223> Description of Artificial Sequence: synthetic DNA

<400> 23

ctctccgcgg tttgtttgtt tatgtgtgtt tattc 35

<210> 24

<211> 32

<212> DNA

<213> Artificial Sequence

<220>

<223> Description of Artificial Sequence: synthetic DNA

<400> 24

tagggagctc caagaattac tcgtgagtaa gg 32

<210> 25

<211> 36

<212> DNA

<213> Artificial Sequence

<220>

<223> Description of Artificial Sequence: synthetic DNA

<400> 25

ataaccgcgg tgttttatat ttgttgtaaa aagtag 36

<210> 26

<211> 34

<212> DNA

<213> Artificial Sequence

<220>

<223> Description of Artificial Sequence: synthetic DNA

<400> 26
ccgcgagctc ttacccataa ggttgtttgt gacg 34

<210> 27
<211> 36
<212> DNA
<213> Artificial Sequence

<220>
<223> Description of Artificial Sequence: synthetic DNA

<400> 27
ctttccgcgg gtttagttaa ttatagttcg ttgacc 36

<210> 28
<211> 23
<212> DNA
<213> Artificial Sequence

<220>
<223> Description of Artificial Sequence: synthetic DNA

<400> 28
atgccgccgc tattcaaggg act 23

<210> 29
<211> 23
<212> DNA
<213> Artificial Sequence

<220>
<223> Description of Artificial Sequence: synthetic DNA

<400> 29
ttaggattta atgcaggtga cgg 23

<210> 30
<211> 27
<212> DNA
<213> Artificial Sequence

<220>
<223> Description of Artificial Sequence: synthetic DNA

<400> 30
ccaaataaag actccaacac tctattt 27

<210> 31
<211> 27
<212> DNA
<213> Artificial Sequence

<220>

<223> Description of Artificial Sequence: synthetic DNA

<400> 31

gaattagaag cattattaag tagtgga 27

<210> 32

<211> 27

<212> DNA

<213> Artificial Sequence

<220>

<223> Description of Artificial Sequence: synthetic DNA

<400> 32

ggatttaacg cacatgcagc taattta 27

<210> 33

<211> 27

<212> DNA

<213> Artificial Sequence

<220>

<223> Description of Artificial Sequence: synthetic DNA

<400> 33

gtctgcttgg gttacatttt ctgaaaa 27

<210> 34

<211> 27

<212> DNA

<213> Artificial Sequence

<220>

<223> Description of Artificial Sequence: synthetic DNA

<400> 34

cataccagtt atactgcaga ccaattg 27

<210> 35

<211> 27

<212> DNA

<213> Artificial Sequence

<220>

<223> Description of Artificial Sequence: synthetic DNA

<400> 35

gaatactcat taaagcaaat ggtagaa 27

<210> 36

<211> 23

<212> DNA

<213> Artificial Sequence

<220>

<223> Description of Artificial Sequence: synthetic DNA

<400> 36

atggaggcca agatagatga gct 23

<210> 37

<211> 23

<212> DNA

<213> Artificial Sequence

<220>

<223> Description of Artificial Sequence: synthetic DNA

<400> 37

tcacaattcg gataagtggt cta 23

<210> 38

<211> 27

<212> DNA

<213> Artificial Sequence

<220>

<223> Description of Artificial Sequence: synthetic DNA

<400> 38
tttcagtccc ttgaatagcg gcggcat 27

<210> 39
<211> 27
<212> DNA
<213> Artificial Sequence

<220>
<223> Description of Artificial Sequence: synthetic DNA

<400> 39
gtctgcttgg gttacatttt ctgaaaa 27

<210> 40
<211> 27
<212> DNA
<213> Artificial Sequence

<220>
<223> Description of Artificial Sequence: synthetic DNA

<400> 40
cacaaaatca agattgccca gtatgcc 27

<210> 41

<211> 27

<212> DNA

<213> Artificial Sequence

<220>

<223> Description of Artificial Sequence: synthetic DNA

<400> 41

agaagatacg gatttctttt ctgcttt 27

<210> 42

<211> 27

<212> DNA

<213> Artificial Sequence

<220>

<223> Description of Artificial Sequence: synthetic DNA

<400> 42

aactttggtg caaattgggt caatgat 27

<210> 43

<211> 27

<212> DNA

<213> Artificial Sequence

<220>

<223> Description of Artificial Sequence: synthetic DNA

<400> 43

ttgctcttta aagttttcag aggcatt 27

<210> 44

<211> 27

<212> DNA

<213> Artificial Sequence

<220>

<223> Description of Artificial Sequence: synthetic DNA

<400> 44

cataccagtt atactgcaga ccaattg 27

<210> 45

<211> 27

<212> DNA

<213> Artificial Sequence

<220>

<223> Description of Artificial Sequence: synthetic DNA

<400> 45

gcattattaa gtagtggaaa tacaaaa 27

<210> 46
<211> 27
<212> DNA
<213> Artificial Sequence

<220>
<223> Description of Artificial Sequence: synthetic DNA

<400> 46
cctttgtacg ctttggagaa aaaatta 27

<210> 47
<211> 27
<212> DNA
<213> Artificial Sequence

<220>
<223> Description of Artificial Sequence: synthetic DNA

<400> 47
tctgatcgtt taccatataa aaattat 27

<210> 48
<211> 27
<212> DNA

<213> Artificial Sequence

<220>

<223> Description of Artificial Sequence: synthetic DNA

<400> 48

aaggatggta tgacaagagg cccagta 27

<210> 49

<211> 33

<212> DNA

<213> Artificial Sequence

<220>

<223> Description of Artificial Sequence: synthetic DNA

<400> 49

gccgttgaca gagggtccga gctcggtacc aag 33

<210> 50

<211> 34

<212> DNA

<213> Artificial Sequence

<220>

<223> Description of Artificial Sequence: synthetic DNA

<400> 50
catactgacc cattgtcaat gggtaataac tgat 34

<210> 51
<211> 18
<212> DNA
<213> Artificial Sequence

<220>
<223> Description of Artificial Sequence: synthetic DNA

<400> 51
tgtccggtaa atggagac 18

<210> 52
<211> 18
<212> DNA
<213> Artificial Sequence

<220>
<223> Description of Artificial Sequence: synthetic DNA

<400> 52
tgttctcgct gctcgttt 18

<210> 53

<211> 19

<212> DNA

<213> Artificial Sequence

<220>

<223> Description of Artificial Sequence: synthetic DNA

<400> 53

atgggaaagc tattacaat 19

<210> 54

<211> 18

<212> DNA

<213> Artificial Sequence

<220>

<223> Description of Artificial Sequence: synthetic DNA

<400> 54

caaggttgca atggccat 18

<210> 55

<211> 19

<212> DNA

<213> Artificial Sequence

<220>

<223> Description of Artificial Sequence: synthetic DNA

<400> 55
caatgtaggg ctatatatg 19

<210> 56
<211> 18
<212> DNA
<213> Artificial Sequence

<220>
<223> Description of Artificial Sequence: synthetic DNA

<400> 56
aacttgggga atggcaca 18

<210> 57
<211> 21
<212> DNA
<213> Artificial Sequence

<220>
<223> Description of Artificial Sequence: synthetic DNA

<400> 57
tctcgaaaaa gggtttgcca t 21

<210> 58
<211> 21
<212> DNA
<213> Artificial Sequence

<220>
<223> Description of Artificial Sequence: synthetic DNA

<400> 58
tcactaggtg taaagagggc t 21

<210> 59
<211> 21
<212> DNA
<213> Artificial Sequence

<220>
<223> Description of Artificial Sequence: synthetic DNA

<400> 59
tgttgaagct tgcatgcctg c 21

<210> 60
<211> 21
<212> DNA
<213> Artificial Sequence

<220>

<223> Description of Artificial Sequence: synthetic DNA

<400> 60

ttgtaaaacg acggccagtg a 21

<210> 61

<211> 23

<212> DNA

<213> Artificial Sequence

<220>

<223> Description of Artificial Sequence: synthetic DNA

<400> 61

atggcttcag aaaaagaaat tag 23

<210> 62

<211> 23

<212> DNA

<213> Artificial Sequence

<220>

<223> Description of Artificial Sequence: synthetic DNA

<400> 62

ctatttgctt ctcttgtaaa ctt 23

<210> 63
<211> 21
<212> DNA
<213> Artificial Sequence

<220>
<223> Description of Artificial Sequence: synthetic DNA

<400> 63
atggaggcca agatagatga g 21

<210> 64
<211> 21
<212> DNA
<213> Artificial Sequence

<220>
<223> Description of Artificial Sequence: synthetic DNA

<400> 64
tcacaattcg gataagtggt c 21

<210> 65
<211> 27

<212> DNA

<213> Artificial Sequence

<220>

<223> Description of Artificial Sequence: synthetic DNA

<400> 65

tcctaatgcc aagaaaacag ctgtcac 27

<210> 66

<211> 21

<212> DNA

<213> Artificial Sequence

<220>

<223> Description of Artificial Sequence: synthetic DNA

<400> 66

atggcaaacc ctttttcgag a 21

<210> 67

<211> 21

<212> DNA

<213> Artificial Sequence

<220>

<223> Description of Artificial Sequence: synthetic DNA

<400> 67
agccctcttt acacctagtg a 21

<210> 68
<211> 28
<212> DNA
<213> Artificial Sequence

<220>
<223> Description of Artificial Sequence:synthetic DNA

<400> 68
tgaggcatgc aatttccgca gcaactcg 28

<210> 69
<211> 28
<212> DNA
<213> Artificial Sequence

<220>
<223> Description of Artificial Sequence:synthetic DNA

<400> 69
tcagaattca tcaggggcct attaatac 28

<210> 70

<211> 45

<212> DNA

<213> Artificial Sequence

<220>

<223> Description of Artificial Sequence:synthetic DNA

<400> 70

atcatgaatt aatgagtcag cgtggatgca ttcaacggcg gcagc 45

<210> 71

<211> 45

<212> DNA

<213> Artificial Sequence

<220>

<223> Description of Artificial Sequence:synthetic DNA

<400> 71

atcatgaatt aatgattcag cgtggatgca ttcaacggcg gcagc 45

<210> 72

<211> 45

<212> DNA

<213> Artificial Sequence

<220>

<223> Description of Artificial Sequence:synthetic DNA

<400> 72

atcatgaatt aatgacatag cgtggatgca ttcaacggcg gcagc 45

<210> 73

<211> 27

<212> DNA

<213> Artificial Sequence

<220>

<223> Description of Artificial Sequence:synthetic DNA

<400> 73

ggccgcaaat taaagccttc gagcgtc 27

<210> 74

<211> 27

<212> DNA

<213> Artificial Sequence

<220>

<223> Description of Artificial Sequence:synthetic DNA

<400> 74

acggattaga agccgccgag cgggtga 27

<210> 75

<211> 894

<212> DNA

<213> Bacillus stearothermophilus

<220>

<221> CDS

<222> (1)..(891)

<400> 75

gtg gcg cag ctt tca gtt gaa cag ttt ctc aac gag caa aaa cag gcg 48
Val Ala Gln Leu Ser Val Glu Gln Phe Leu Asn Glu Gln Lys Gln Ala
1 5 10 15

gtg gaa aca gcg ctc tcc cgt tat ata gag cgc tta gaa ggg ccg gcg 96
Val Glu Thr Ala Leu Ser Arg Tyr Ile Glu Arg Leu Glu Gly Pro Ala
20 25 30

aag ctg aaa aag gcg atg gcg tac tca ttg gag gcc ggc ggc aaa cga 144
Lys Leu Lys Lys Ala Met Ala Tyr Ser Leu Glu Ala Gly Gly Lys Arg
35 40 45

atc cgt ccg ttg ctg ctt ctg tcc acc gtt cgg gcg ctc ggc aaa gac 192
Ile Arg Pro Leu Leu Leu Leu Ser Thr Val Arg Ala Leu Gly Lys Asp
50 55 60

ccg gcg gtc gga ttg ccc gtc gcc tgc gcg att gaa atg atc cat acg 240

Pro Ala Val Gly Leu Pro Val Ala Cys Ala Ile Glu Met Ile His Thr
65 70 75 80

tac tct ttg atc cat gat gat ttg ccg agc atg gac aac gat gat ttg 288
Tyr Ser Leu Ile His Asp Asp Leu Pro Ser Met Asp Asn Asp Asp Leu
85 90 95

cgg cgc ggc aag ccg acg aac cat aaa gtg ttc ggc gag gcg atg gcc 336
Arg Arg Gly Lys Pro Thr Asn His Lys Val Phe Gly Glu Ala Met Ala
100 105 110

atc ttg gcg ggg gac ggg ttg ttg acg tac gcg ttt caa ttg atc acc 384
Ile Leu Ala Gly Asp Gly Leu Leu Thr Tyr Ala Phe Gln Leu Ile Thr
115 120 125

gaa atc gac gat gag cgc atc cct cct tcc gtc cgg ctt cgg ctc atc 432
Glu Ile Asp Asp Glu Arg Ile Pro Pro Ser Val Arg Leu Arg Leu Ile
130 135 140

gaa cgg ctg gcg aaa gcg gcc ggt ccg gaa ggg atg gtc gcc ggt cag 480
Glu Arg Leu Ala Lys Ala Ala Gly Pro Glu Gly Met Val Ala Gly Gln
145 150 155 160

gca gcc gat atg gaa gga gag ggg aaa acg ctg acg ctt tcg gag ctc 528
Ala Ala Asp Met Glu Gly Glu Gly Lys Thr Leu Thr Leu Ser Glu Leu
165 170 175

gaa tac att cat cgg cat aaa acc ggg aaa atg ctg caa tac agc gtg 576
Glu Tyr Ile His Arg His Lys Thr Gly Lys Met Leu Gln Tyr Ser Val

180 185 190

cac gcc ggc gcc ttg atc ggc ggc gct gat gcc cgg caa acg cgg gag 624
His Ala Gly Ala Leu Ile Gly Gly Ala Asp Ala Arg Gln Thr Arg Glu
195 200 205

ctt gac gaa ttc gcc gcc cat cta ggc ctt gcc ttt caa att cgc gat 672
Leu Asp Glu Phe Ala Ala His Leu Gly Leu Ala Phe Gln Ile Arg Asp
210 215 220

gat att ctc gat att gaa ggg gca gaa gaa aaa atc ggc aag ccg gtc 720
Asp Ile Leu Asp Ile Glu Gly Ala Glu Glu Lys Ile Gly Lys Pro Val
225 230 235 240

ggc agc gac caa agc aac aac aaa gcg acg tat cca gcg ttg ctg tcg 768
Gly Ser Asp Gln Ser Asn Asn Lys Ala Thr Tyr Pro Ala Leu Leu Ser
245 250 255

ctt gcc ggc gcg aag gaa aag ttg gcg ttc cat atc gag gcg gcg cag 816
Leu Ala Gly Ala Lys Glu Lys Leu Ala Phe His Ile Glu Ala Ala Gln
260 265 270

cgc cat tta cgg aac gcc gac gtt gac ggc gcc gcg ctc gcc tat att 864
Arg His Leu Arg Asn Ala Asp Val Asp Gly Ala Ala Leu Ala Tyr Ile
275 280 285

tgc gaa ctg gtc gcc gcc cgc gac cat taa 894
Cys Glu Leu Val Ala Ala Arg Asp His
290 295

<210> 76

<211> 297

<212> PRT

<213> Bacillus stearothermophilus

<400> 76

Val Ala Gln Leu Ser Val Glu Gln Phe Leu Asn Glu Gln Lys Gln Ala
1 5 10 15

Val Glu Thr Ala Leu Ser Arg Tyr Ile Glu Arg Leu Glu Gly Pro Ala
20 25 30

Lys Leu Lys Lys Ala Met Ala Tyr Ser Leu Glu Ala Gly Gly Lys Arg
35 40 45

Ile Arg Pro Leu Leu Leu Leu Ser Thr Val Arg Ala Leu Gly Lys Asp
50 55 60

Pro Ala Val Gly Leu Pro Val Ala Cys Ala Ile Glu Met Ile His Thr
65 70 75 80

Tyr Ser Leu Ile His Asp Asp Leu Pro Ser Met Asp Asn Asp Asp Leu
85 90 95

Arg Arg Gly Lys Pro Thr Asn His Lys Val Phe Gly Glu Ala Met Ala
100 105 110

Ile Leu Ala Gly Asp Gly Leu Leu Thr Tyr Ala Phe Gln Leu Ile Thr
115 120 125

Glu Ile Asp Asp Glu Arg Ile Pro Pro Ser Val Arg Leu Arg Leu Ile
130 135 140

Glu Arg Leu Ala Lys Ala Ala Gly Pro Glu Gly Met Val Ala Gly Gln
145 150 155 160

Ala Ala Asp Met Glu Gly Glu Gly Lys Thr Leu Thr Leu Ser Glu Leu
165 170 175

Glu Tyr Ile His Arg His Lys Thr Gly Lys Met Leu Gln Tyr Ser Val
180 185 190

His Ala Gly Ala Leu Ile Gly Gly Ala Asp Ala Arg Gln Thr Arg Glu
195 200 205

Leu Asp Glu Phe Ala Ala His Leu Gly Leu Ala Phe Gln Ile Arg Asp
210 215 220

Asp Ile Leu Asp Ile Glu Gly Ala Glu Glu Lys Ile Gly Lys Pro Val
225 230 235 240

Gly Ser Asp Gln Ser Asn Asn Lys Ala Thr Tyr Pro Ala Leu Leu Ser
245 250 255

Leu Ala Gly Ala Lys Glu Lys Leu Ala Phe His Ile Glu Ala Ala Gln
260 265 270

Arg His Leu Arg Asn Ala Asp Val Asp Gly Ala Ala Leu Ala Tyr Ile
275 280 285

Cys Glu Leu Val Ala Ala Arg Asp His
290 295

<210> 77
<211> 900
<212> DNA
<213> Escherichia coli

<220>
<221> CDS
<222> (1)..(897)

<400> 77
atg gac ttt ccg cag caa ctc gaa gcc tgc gtt aag cag gcc aac cag 48
Met Asp Phe Pro Gln Gln Leu Glu Ala Cys Val Lys Gln Ala Asn Gln
1 5 10 15

gcg ctg agc cgt ttt atc gcc cca ctg ccc ttt cag aac act ccc gtg 96
Ala Leu Ser Arg Phe Ile Ala Pro Leu Pro Phe Gln Asn Thr Pro Val
20 25 30

gtc gaa acc atg cag tat ggc gca tta tta ggt ggt aag cgc ctg cga 144
Val Glu Thr Met Gln Tyr Gly Ala Leu Leu Gly Gly Lys Arg Leu Arg
35 40 45

```
cct ttc ctg gtt tat gcc acc ggt cat atg ttc ggc gtt agc aca aac    192
Pro Phe Leu Val Tyr Ala Thr Gly His Met Phe Gly Val Ser Thr Asn
     50                  55                  60

acg ctg gac gca ccc gct gcc gcc gtt gag tgt atc cac gct tac tca    240
Thr Leu Asp Ala Pro Ala Ala Ala Val Glu Cys Ile His Ala Tyr Ser
 65                  70                  75                  80

tta att cat gat gat tta ccg gca atg gat gat gac gat ctg cgt cgc    288
Leu Ile His Asp Asp Leu Pro Ala Met Asp Asp Asp Asp Leu Arg Arg
                 85                  90                  95

ggt ttg cca acc tgc cat gtg aag ttt ggc gaa gca aac gcg att ctc    336
Gly Leu Pro Thr Cys His Val Lys Phe Gly Glu Ala Asn Ala Ile Leu
            100                 105                 110

gct ggc gac gct tta caa acg ctg gcg ttc tcg att tta agc gat gcc    384
Ala Gly Asp Ala Leu Gln Thr Leu Ala Phe Ser Ile Leu Ser Asp Ala
        115                 120                 125

gat atg ccg gaa gtg tcg gac cgc gac aga att tcg atg att tct gaa    432
Asp Met Pro Glu Val Ser Asp Arg Asp Arg Ile Ser Met Ile Ser Glu
    130                 135                 140

ctg gcg agc gcc agt ggt att gcc gga atg tgc ggt ggt cag gca tta    480
Leu Ala Ser Ala Ser Gly Ile Ala Gly Met Cys Gly Gly Gln Ala Leu
145                 150                 155                 160
```

gat tta gac gcg gaa ggc aaa cac gta cct ctg gac gcg ctt gag cgt 528
Asp Leu Asp Ala Glu Gly Lys His Val Pro Leu Asp Ala Leu Glu Arg
165 170 175

att cat cgt cat aaa acc ggc gca ttg att cgc gcc gcc gtt cgc ctt 576
Ile His Arg His Lys Thr Gly Ala Leu Ile Arg Ala Ala Val Arg Leu
180 185 190

ggt gca tta agc gcc gga gat aaa gga cgt cgt gct ctg ccg gta ctc 624
Gly Ala Leu Ser Ala Gly Asp Lys Gly Arg Arg Ala Leu Pro Val Leu
195 200 205

gac aag tat gca gag agc atc ggc ctt gcc ttc cag gtt cag gat gac 672
Asp Lys Tyr Ala Glu Ser Ile Gly Leu Ala Phe Gln Val Gln Asp Asp
210 215 220

atc ctg gat gtg gtg gga gat act gca acg ttg gga aaa cgc cag ggt 720
Ile Leu Asp Val Val Gly Asp Thr Ala Thr Leu Gly Lys Arg Gln Gly
225 230 235 240

gcc gac cag caa ctt ggt aaa agt acc tac cct gca ctt ctg ggt ctt 768
Ala Asp Gln Gln Leu Gly Lys Ser Thr Tyr Pro Ala Leu Leu Gly Leu
245 250 255

gag caa gcc cgg aag aaa gcc cgg gat ctg atc gac gat gcc cgt cag 816
Glu Gln Ala Arg Lys Lys Ala Arg Asp Leu Ile Asp Asp Ala Arg Gln
260 265 270

tcg ctg aaa caa ctg gct gaa cag tca ctc gat acc tcg gca ctg gaa 864

Ser Leu Lys Gln Leu Ala Glu Gln Ser Leu Asp Thr Ser Ala Leu Glu
275 280 285

gcg cta gcg gac tac atc atc cag cgt aat aaa taa 900
Ala Leu Ala Asp Tyr Ile Ile Gln Arg Asn Lys
290 295

<210> 78
<211> 299
<212> PRT
<213> Escherichia coli

<400> 78
Met Asp Phe Pro Gln Gln Leu Glu Ala Cys Val Lys Gln Ala Asn Gln
1 5 10 15

Ala Leu Ser Arg Phe Ile Ala Pro Leu Pro Phe Gln Asn Thr Pro Val
20 25 30

Val Glu Thr Met Gln Tyr Gly Ala Leu Leu Gly Gly Lys Arg Leu Arg
35 40 45

Pro Phe Leu Val Tyr Ala Thr Gly His Met Phe Gly Val Ser Thr Asn
50 55 60

Thr Leu Asp Ala Pro Ala Ala Ala Val Glu Cys Ile His Ala Tyr Ser
65 70 75 80

Leu Ile His Asp Asp Leu Pro Ala Met Asp Asp Asp Asp Leu Arg Arg
85 90 95

Gly Leu Pro Thr Cys His Val Lys Phe Gly Glu Ala Asn Ala Ile Leu
100 105 110

Ala Gly Asp Ala Leu Gln Thr Leu Ala Phe Ser Ile Leu Ser Asp Ala
115 120 125

Asp Met Pro Glu Val Ser Asp Arg Asp Arg Ile Ser Met Ile Ser Glu
130 135 140

Leu Ala Ser Ala Ser Gly Ile Ala Gly Met Cys Gly Gly Gln Ala Leu
145 150 155 160

Asp Leu Asp Ala Glu Gly Lys His Val Pro Leu Asp Ala Leu Glu Arg
165 170 175

Ile His Arg His Lys Thr Gly Ala Leu Ile Arg Ala Ala Val Arg Leu
180 185 190

Gly Ala Leu Ser Ala Gly Asp Lys Gly Arg Arg Ala Leu Pro Val Leu
195 200 205

Asp Lys Tyr Ala Glu Ser Ile Gly Leu Ala Phe Gln Val Gln Asp Asp
210 215 220

Ile Leu Asp Val Val Gly Asp Thr Ala Thr Leu Gly Lys Arg Gln Gly
225 230 235 240

Ala Asp Gln Gln Leu Gly Lys Ser Thr Tyr Pro Ala Leu Leu Gly Leu
245 250 255

Glu Gln Ala Arg Lys Lys Ala Arg Asp Leu Ile Asp Asp Ala Arg Gln
260 265 270

Ser Leu Lys Gln Leu Ala Glu Gln Ser Leu Asp Thr Ser Ala Leu Glu
275 280 285

Ala Leu Ala Asp Tyr Ile Ile Gln Arg Asn Lys
290 295

<210> 79
<211> 900
<212> DNA
<213> Escherichia coli

<220>
<221> CDS
<222> (1)..(897)

<400> 79
atg gac ttt ccg cag caa ctc gaa gcc tgc gtt aag cag gcc aac cag 48
Met Asp Phe Pro Gln Gln Leu Glu Ala Cys Val Lys Gln Ala Asn Gln
1 5 10 15

gcg ctg agc cgt ttt atc gcc cca ctg ccc ttt cag aac act ccc gtg 96

Ala Leu Ser Arg Phe Ile Ala Pro Leu Pro Phe Gln Asn Thr Pro Val
20 25 30

gtc gaa acc atg cag tat ggc gca tta tta ggt ggt aag cgc ctg cga 144
Val Glu Thr Met Gln Tyr Gly Ala Leu Leu Gly Gly Lys Arg Leu Arg
35 40 45

cct ttc ctg gtt tat gcc acc ggt cat atg ttc ggc gtt agc aca aac 192
Pro Phe Leu Val Tyr Ala Thr Gly His Met Phe Gly Val Ser Thr Asn
50 55 60

acg ctg gac gca ccc gct gcc gcc gtt gaa tgc atc cac gct gac tca 240
Thr Leu Asp Ala Pro Ala Ala Ala Val Glu Cys Ile His Ala Asp Ser
65 70 75 80

tta att cat gat gat tta ccg gca atg gat gat gac gat ctg cgt cgc 288
Leu Ile His Asp Asp Leu Pro Ala Met Asp Asp Asp Asp Leu Arg Arg
85 90 95

ggt ttg cca acc tgc cat gtg aag ttt ggc gaa gca aac gcg att ctc 336
Gly Leu Pro Thr Cys His Val Lys Phe Gly Glu Ala Asn Ala Ile Leu
100 105 110

gct ggc gac gct tta caa acg ctg gcg ttc tcg att tta agc gat gcc 384
Ala Gly Asp Ala Leu Gln Thr Leu Ala Phe Ser Ile Leu Ser Asp Ala
115 120 125

gat atg ccg gaa gtg tcg gac cgc gac aga att tcg atg att tct gaa 432
Asp Met Pro Glu Val Ser Asp Arg Asp Arg Ile Ser Met Ile Ser Glu

130 135 140

ctg gcg agc gcc agt ggt att gcc gga atg tgc ggt ggt cag gca tta 480
Leu Ala Ser Ala Ser Gly Ile Ala Gly Met Cys Gly Gly Gln Ala Leu
145 150 155 160

gat tta gac gcg gaa ggc aaa cac gta cct ctg gac gcg ctt gag cgt 528
Asp Leu Asp Ala Glu Gly Lys His Val Pro Leu Asp Ala Leu Glu Arg
165 170 175

att cat cgt cat aaa acc ggc gca ttg att cgc gcc gcc gtt cgc ctt 576
Ile His Arg His Lys Thr Gly Ala Leu Ile Arg Ala Ala Val Arg Leu
180 185 190

ggt gca tta agc gcc gga gat aaa gga cgt cgt gct ctg ccg gta ctc 624
Gly Ala Leu Ser Ala Gly Asp Lys Gly Arg Arg Ala Leu Pro Val Leu
195 200 205

gac aag tat gca gag agc atc ggc ctt gcc ttc cag gtt cag gat gac 672
Asp Lys Tyr Ala Glu Ser Ile Gly Leu Ala Phe Gln Val Gln Asp Asp
210 215 220

atc ctg gat gtg gtg gga gat act gca acg ttg gga aaa cgc cag ggt 720
Ile Leu Asp Val Val Gly Asp Thr Ala Thr Leu Gly Lys Arg Gln Gly
225 230 235 240

gcc gac cag caa ctt ggt aaa agt acc tac cct gca ctt ctg ggt ctt 768
Ala Asp Gln Gln Leu Gly Lys Ser Thr Tyr Pro Ala Leu Leu Gly Leu
245 250 255

gag caa gcc cgg aag aaa gcc cgg gat ctg atc gac gat gcc cgt cag 816
Glu Gln Ala Arg Lys Lys Ala Arg Asp Leu Ile Asp Asp Ala Arg Gln
260 265 270

tcg ctg aaa caa ctg gct gaa cag tca ctc gat acc tcg gca ctg gaa 864
Ser Leu Lys Gln Leu Ala Glu Gln Ser Leu Asp Thr Ser Ala Leu Glu
275 280 285

gcg cta gcg gac tac atc atc cag cgt aat aaa taa 900
Ala Leu Ala Asp Tyr Ile Ile Gln Arg Asn Lys
290 295

<210> 80
<211> 299
<212> PRT
<213> Escherichia coli

<400> 80
Met Asp Phe Pro Gln Gln Leu Glu Ala Cys Val Lys Gln Ala Asn Gln
1 5 10 15

Ala Leu Ser Arg Phe Ile Ala Pro Leu Pro Phe Gln Asn Thr Pro Val
20 25 30

Val Glu Thr Met Gln Tyr Gly Ala Leu Leu Gly Gly Lys Arg Leu Arg
35 40 45

Pro Phe Leu Val Tyr Ala Thr Gly His Met Phe Gly Val Ser Thr Asn
50 55 60

Thr Leu Asp Ala Pro Ala Ala Ala Val Glu Cys Ile His Ala Asp Ser
65 70 75 80

Leu Ile His Asp Asp Leu Pro Ala Met Asp Asp Asp Asp Leu Arg Arg
85 90 95

Gly Leu Pro Thr Cys His Val Lys Phe Gly Glu Ala Asn Ala Ile Leu
100 105 110

Ala Gly Asp Ala Leu Gln Thr Leu Ala Phe Ser Ile Leu Ser Asp Ala
115 120 125

Asp Met Pro Glu Val Ser Asp Arg Asp Arg Ile Ser Met Ile Ser Glu
130 135 140

Leu Ala Ser Ala Ser Gly Ile Ala Gly Met Cys Gly Gly Gln Ala Leu
145 150 155 160

Asp Leu Asp Ala Glu Gly Lys His Val Pro Leu Asp Ala Leu Glu Arg
165 170 175

Ile His Arg His Lys Thr Gly Ala Leu Ile Arg Ala Ala Val Arg Leu
180 185 190

Gly Ala Leu Ser Ala Gly Asp Lys Gly Arg Arg Ala Leu Pro Val Leu
195 200 205

Asp Lys Tyr Ala Glu Ser Ile Gly Leu Ala Phe Gln Val Gln Asp Asp
    210                 215                 220

Ile Leu Asp Val Val Gly Asp Thr Ala Thr Leu Gly Lys Arg Gln Gly
225                 230                 235                 240

Ala Asp Gln Gln Leu Gly Lys Ser Thr Tyr Pro Ala Leu Leu Gly Leu
                245                 250                 255

Glu Gln Ala Arg Lys Lys Ala Arg Asp Leu Ile Asp Asp Ala Arg Gln
            260                 265                 270

Ser Leu Lys Gln Leu Ala Glu Gln Ser Leu Asp Thr Ser Ala Leu Glu
        275                 280                 285

Ala Leu Ala Asp Tyr Ile Ile Gln Arg Asn Lys
    290                 295

<210> 81
<211> 900
<212> DNA
<213> Escherichia coli

<220>
<221> CDS
<222> (1)..(897)

<400> 81

```
atg gac ttt ccg cag caa ctc gaa gcc tgc gtt aag cag gcc aac cag      48
Met Asp Phe Pro Gln Gln Leu Glu Ala Cys Val Lys Gln Ala Asn Gln
  1               5                  10                  15

gcg ctg agc cgt ttt atc gcc cca ctg ccc ttt cag aac act ccc gtg      96
Ala Leu Ser Arg Phe Ile Ala Pro Leu Pro Phe Gln Asn Thr Pro Val
             20                  25                  30

gtc gaa acc atg cag tat ggc gca tta tta ggt ggt aag cgc ctg cga     144
Val Glu Thr Met Gln Tyr Gly Ala Leu Leu Gly Gly Lys Arg Leu Arg
         35                  40                  45

cct ttc ctg gtt tat gcc acc ggt cat atg ttc ggc gtt agc aca aac     192
Pro Phe Leu Val Tyr Ala Thr Gly His Met Phe Gly Val Ser Thr Asn
     50                  55                  60

acg ctg gac gca ccc gct gcc gcc gtt gaa tgc atc cac gct gaa tca     240
Thr Leu Asp Ala Pro Ala Ala Ala Val Glu Cys Ile His Ala Glu Ser
 65                  70                  75                  80

tta att cat gat gat tta ccg gca atg gat gat gac gat ctg cgt cgc     288
Leu Ile His Asp Asp Leu Pro Ala Met Asp Asp Asp Asp Leu Arg Arg
                 85                  90                  95

ggt ttg cca acc tgc cat gtg aag ttt ggc gaa gca aac gcg att ctc     336
Gly Leu Pro Thr Cys His Val Lys Phe Gly Glu Ala Asn Ala Ile Leu
            100                 105                 110
```

gct ggc gac gct tta caa acg ctg gcg ttc tcg att tta agc gat gcc 384
Ala Gly Asp Ala Leu Gln Thr Leu Ala Phe Ser Ile Leu Ser Asp Ala
115 120 125

gat atg ccg gaa gtg tcg gac cgc gac aga att tcg atg att tct gaa 432
Asp Met Pro Glu Val Ser Asp Arg Asp Arg Ile Ser Met Ile Ser Glu
130 135 140

ctg gcg agc gcc agt ggt att gcc gga atg tgc ggt ggt cag gca tta 480
Leu Ala Ser Ala Ser Gly Ile Ala Gly Met Cys Gly Gly Gln Ala Leu
145 150 155 160

gat tta gac gcg gaa ggc aaa cac gta cct ctg gac gcg ctt gag cgt 528
Asp Leu Asp Ala Glu Gly Lys His Val Pro Leu Asp Ala Leu Glu Arg
165 170 175

att cat cgt cat aaa acc ggc gca ttg att cgc gcc gcc gtt cgc ctt 576
Ile His Arg His Lys Thr Gly Ala Leu Ile Arg Ala Ala Val Arg Leu
180 185 190

ggt gca tta agc gcc gga gat aaa gga cgt cgt gct ctg ccg gta ctc 624
Gly Ala Leu Ser Ala Gly Asp Lys Gly Arg Arg Ala Leu Pro Val Leu
195 200 205

gac aag tat gca gag agc atc ggc ctt gcc ttc cag gtt cag gat gac 672
Asp Lys Tyr Ala Glu Ser Ile Gly Leu Ala Phe Gln Val Gln Asp Asp
210 215 220

atc ctg gat gtg gtg gga gat act gca acg ttg gga aaa cgc cag ggt 720

Ile Leu Asp Val Val Gly Asp Thr Ala Thr Leu Gly Lys Arg Gln Gly
225 230 235 240

gcc gac cag caa ctt ggt aaa agt acc tac cct gca ctt ctg ggt ctt 768
Ala Asp Gln Gln Leu Gly Lys Ser Thr Tyr Pro Ala Leu Leu Gly Leu
245 250 255

gag caa gcc cgg aag aaa gcc cgg gat ctg atc gac gat gcc cgt cag 816
Glu Gln Ala Arg Lys Lys Ala Arg Asp Leu Ile Asp Asp Ala Arg Gln
260 265 270

tcg ctg aaa caa ctg gct gaa cag tca ctc gat acc tcg gca ctg gaa 864
Ser Leu Lys Gln Leu Ala Glu Gln Ser Leu Asp Thr Ser Ala Leu Glu
275 280 285

gcg cta gcg gac tac atc atc cag cgt aat aaa taa 900
Ala Leu Ala Asp Tyr Ile Ile Gln Arg Asn Lys
290 295

<210> 82
<211> 299
<212> PRT
<213> Escherichia coli

<400> 82
Met Asp Phe Pro Gln Gln Leu Glu Ala Cys Val Lys Gln Ala Asn Gln
1 5 10 15

Ala Leu Ser Arg Phe Ile Ala Pro Leu Pro Phe Gln Asn Thr Pro Val
20 25 30

Val Glu Thr Met Gln Tyr Gly Ala Leu Leu Gly Gly Lys Arg Leu Arg
35 40 45

Pro Phe Leu Val Tyr Ala Thr Gly His Met Phe Gly Val Ser Thr Asn
50 55 60

Thr Leu Asp Ala Pro Ala Ala Ala Val Glu Cys Ile His Ala Glu Ser
65 70 75 80

Leu Ile His Asp Asp Leu Pro Ala Met Asp Asp Asp Asp Leu Arg Arg
85 90 95

Gly Leu Pro Thr Cys His Val Lys Phe Gly Glu Ala Asn Ala Ile Leu
100 105 110

Ala Gly Asp Ala Leu Gln Thr Leu Ala Phe Ser Ile Leu Ser Asp Ala
115 120 125

Asp Met Pro Glu Val Ser Asp Arg Asp Arg Ile Ser Met Ile Ser Glu
130 135 140

Leu Ala Ser Ala Ser Gly Ile Ala Gly Met Cys Gly Gly Gln Ala Leu
145 150 155 160

Asp Leu Asp Ala Glu Gly Lys His Val Pro Leu Asp Ala Leu Glu Arg
165 170 175

Ile His Arg His Lys Thr Gly Ala Leu Ile Arg Ala Ala Val Arg Leu
180 185 190

Gly Ala Leu Ser Ala Gly Asp Lys Gly Arg Arg Ala Leu Pro Val Leu
195 200 205

Asp Lys Tyr Ala Glu Ser Ile Gly Leu Ala Phe Gln Val Gln Asp Asp
210 215 220

Ile Leu Asp Val Val Gly Asp Thr Ala Thr Leu Gly Lys Arg Gln Gly
225 230 235 240

Ala Asp Gln Gln Leu Gly Lys Ser Thr Tyr Pro Ala Leu Leu Gly Leu
245 250 255

Glu Gln Ala Arg Lys Lys Ala Arg Asp Leu Ile Asp Asp Ala Arg Gln
260 265 270

Ser Leu Lys Gln Leu Ala Glu Gln Ser Leu Asp Thr Ser Ala Leu Glu
275 280 285

Ala Leu Ala Asp Tyr Ile Ile Gln Arg Asn Lys
290 295

<210> 83
<211> 900
<212> DNA

<213> Escherichia coli

<220>
<221> CDS
<222> (1)..(897)

<400> 83

atg gac ttt ccg cag caa ctc gaa gcc tgc gtt aag cag gcc aac cag 48
Met Asp Phe Pro Gln Gln Leu Glu Ala Cys Val Lys Gln Ala Asn Gln
1 5 10 15

gcg ctg agc cgt ttt atc gcc cca ctg ccc ttt cag aac act ccc gtg 96
Ala Leu Ser Arg Phe Ile Ala Pro Leu Pro Phe Gln Asn Thr Pro Val
20 25 30

gtc gaa acc atg cag tat ggc gca tta tta ggt ggt aag cgc ctg cga 144
Val Glu Thr Met Gln Tyr Gly Ala Leu Leu Gly Gly Lys Arg Leu Arg
35 40 45

cct ttc ctg gtt tat gcc acc ggt cat atg ttc ggc gtt agc aca aac 192
Pro Phe Leu Val Tyr Ala Thr Gly His Met Phe Gly Val Ser Thr Asn
50 55 60

acg ctg gac gca ccc gct gcc gcc gtt gaa tgc atc cac gct atg tca 240
Thr Leu Asp Ala Pro Ala Ala Ala Val Glu Cys Ile His Ala Met Ser
65 70 75 80

tta att cat gat gat tta ccg gca atg gat gat gac gat ctg cgt cgc 288
Leu Ile His Asp Asp Leu Pro Ala Met Asp Asp Asp Asp Leu Arg Arg

85 90 95

ggt ttg cca acc tgc cat gtg aag ttt ggc gaa gca aac gcg att ctc 336
Gly Leu Pro Thr Cys His Val Lys Phe Gly Glu Ala Asn Ala Ile Leu
100 105 110

gct ggc gac gct tta caa acg ctg gcg ttc tcg att tta agc gat gcc 384
Ala Gly Asp Ala Leu Gln Thr Leu Ala Phe Ser Ile Leu Ser Asp Ala
115 120 125

gat atg ccg gaa gtg tcg gac cgc gac aga att tcg atg att tct gaa 432
Asp Met Pro Glu Val Ser Asp Arg Asp Arg Ile Ser Met Ile Ser Glu
130 135 140

ctg gcg agc gcc agt ggt att gcc gga atg tgc ggt ggt cag gca tta 480
Leu Ala Ser Ala Ser Gly Ile Ala Gly Met Cys Gly Gly Gln Ala Leu
145 150 155 160

gat tta gac gcg gaa ggc aaa cac gta cct ctg gac gcg ctt gag cgt 528
Asp Leu Asp Ala Glu Gly Lys His Val Pro Leu Asp Ala Leu Glu Arg
165 170 175

att cat cgt cat aaa acc ggc gca ttg att cgc gcc gcc gtt cgc ctt 576
Ile His Arg His Lys Thr Gly Ala Leu Ile Arg Ala Ala Val Arg Leu
180 185 190

ggt gca tta agc gcc gga gat aaa gga cgt cgt gct ctg ccg gta ctc 624
Gly Ala Leu Ser Ala Gly Asp Lys Gly Arg Arg Ala Leu Pro Val Leu
195 200 205

gac aag tat gca gag agc atc ggc ctt gcc ttc cag gtt cag gat gac 672
Asp Lys Tyr Ala Glu Ser Ile Gly Leu Ala Phe Gln Val Gln Asp Asp
210 215 220

atc ctg gat gtg gtg gga gat act gca acg ttg gga aaa cgc cag ggt 720
Ile Leu Asp Val Val Gly Asp Thr Ala Thr Leu Gly Lys Arg Gln Gly
225 230 235 240

gcc gac cag caa ctt ggt aaa agt acc tac cct gca ctt ctg ggt ctt 768
Ala Asp Gln Gln Leu Gly Lys Ser Thr Tyr Pro Ala Leu Leu Gly Leu
245 250 255

gag caa gcc cgg aag aaa gcc cgg gat ctg atc gac gat gcc cgt cag 816
Glu Gln Ala Arg Lys Lys Ala Arg Asp Leu Ile Asp Asp Ala Arg Gln
260 265 270

tcg ctg aaa caa ctg gct gaa cag tca ctc gat acc tcg gca ctg gaa 864
Ser Leu Lys Gln Leu Ala Glu Gln Ser Leu Asp Thr Ser Ala Leu Glu
275 280 285

gcg cta gcg gac tac atc atc cag cgt aat aaa taa 900
Ala Leu Ala Asp Tyr Ile Ile Gln Arg Asn Lys
290 295

<210> 84
<211> 299
<212> PRT

<213> Escherichia coli

<400> 84

Met Asp Phe Pro Gln Gln Leu Glu Ala Cys Val Lys Gln Ala Asn Gln
1 5 10 15

Ala Leu Ser Arg Phe Ile Ala Pro Leu Pro Phe Gln Asn Thr Pro Val
20 25 30

Val Glu Thr Met Gln Tyr Gly Ala Leu Leu Gly Gly Lys Arg Leu Arg
35 40 45

Pro Phe Leu Val Tyr Ala Thr Gly His Met Phe Gly Val Ser Thr Asn
50 55 60

Thr Leu Asp Ala Pro Ala Ala Ala Val Glu Cys Ile His Ala Met Ser
65 70 75 80

Leu Ile His Asp Asp Leu Pro Ala Met Asp Asp Asp Asp Leu Arg Arg
85 90 95

Gly Leu Pro Thr Cys His Val Lys Phe Gly Glu Ala Asn Ala Ile Leu
100 105 110

Ala Gly Asp Ala Leu Gln Thr Leu Ala Phe Ser Ile Leu Ser Asp Ala
115 120 125

Asp Met Pro Glu Val Ser Asp Arg Asp Arg Ile Ser Met Ile Ser Glu
130 135 140

Leu Ala Ser Ala Ser Gly Ile Ala Gly Met Cys Gly Gly Gln Ala Leu
145 150 155 160

Asp Leu Asp Ala Glu Gly Lys His Val Pro Leu Asp Ala Leu Glu Arg
165 170 175

Ile His Arg His Lys Thr Gly Ala Leu Ile Arg Ala Ala Val Arg Leu
180 185 190

Gly Ala Leu Ser Ala Gly Asp Lys Gly Arg Arg Ala Leu Pro Val Leu
195 200 205

Asp Lys Tyr Ala Glu Ser Ile Gly Leu Ala Phe Gln Val Gln Asp Asp
210 215 220

Ile Leu Asp Val Val Gly Asp Thr Ala Thr Leu Gly Lys Arg Gln Gly
225 230 235 240

Ala Asp Gln Gln Leu Gly Lys Ser Thr Tyr Pro Ala Leu Leu Gly Leu
245 250 255

Glu Gln Ala Arg Lys Lys Ala Arg Asp Leu Ile Asp Asp Ala Arg Gln
260 265 270

Ser Leu Lys Gln Leu Ala Glu Gln Ser Leu Asp Thr Ser Ala Leu Glu
275 280 285

Ala Leu Ala Asp Tyr Ile Ile Gln Arg Asn Lys

290 295

<210> 85

<211> 549

<212> DNA

<213> Escherichia coli

<220>

<221> CDS

<222> (1)..(546)

<400> 85

atg caa acg gaa cac gtc att tta ttg aat gca cag gga gtt ccc acg 48
Met Gln Thr Glu His Val Ile Leu Leu Asn Ala Gln Gly Val Pro Thr
1 5 10 15

ggt acg ctg gaa aag tat gcc gca cac acg gca gac acc cgc tta cat 96
Gly Thr Leu Glu Lys Tyr Ala Ala His Thr Ala Asp Thr Arg Leu His
20 25 30

ctc gcg ttc tcc agt tgg ctg ttt aat gcc aaa gga caa tta tta gtt 144
Leu Ala Phe Ser Ser Trp Leu Phe Asn Ala Lys Gly Gln Leu Leu Val
35 40 45

acc cgc cgc gca ctg agc aaa aaa gca tgg cct ggc gtg tgg act aac 192
Thr Arg Arg Ala Leu Ser Lys Lys Ala Trp Pro Gly Val Trp Thr Asn
50 55 60

tcg gtt tgt ggg cac cca caa ctg gga gaa agc aac gaa gac gca gtg 240
Ser Val Cys Gly His Pro Gln Leu Gly Glu Ser Asn Glu Asp Ala Val
65 70 75 80

atc cgc cgt tgc cgt tat gag ctt ggc gtg gaa att acg cct cct gaa 288
Ile Arg Arg Cys Arg Tyr Glu Leu Gly Val Glu Ile Thr Pro Pro Glu
85 90 95

tct atc tat cct gac ttt cgc tac cgc gcc acc gat ccg agt ggc att 336
Ser Ile Tyr Pro Asp Phe Arg Tyr Arg Ala Thr Asp Pro Ser Gly Ile
100 105 110

gtg gaa aat gaa gtg tgt ccg gta ttt gcc gca cgc acc act agt gcg 384
Val Glu Asn Glu Val Cys Pro Val Phe Ala Ala Arg Thr Thr Ser Ala
115 120 125

tta cag atc aat gat gat gaa gtg atg gat tat caa tgg tgt gat tta 432
Leu Gln Ile Asn Asp Asp Glu Val Met Asp Tyr Gln Trp Cys Asp Leu
130 135 140

gca gat gta tta cac ggt att gat gcc acg ccg tgg gcg ttc agt ccg 480
Ala Asp Val Leu His Gly Ile Asp Ala Thr Pro Trp Ala Phe Ser Pro
145 150 155 160

tgg atg gtg atg cag gcg aca aat cgc gaa gcc aga aaa cga tta tct 528
Trp Met Val Met Gln Ala Thr Asn Arg Glu Ala Arg Lys Arg Leu Ser
165 170 175

gca ttt acc cag ctt aaa taa 549

Ala Phe Thr Gln Leu Lys
180

<210> 86
<211> 182
<212> PRT
<213> Escherichia coli

<400> 86
Met Gln Thr Glu His Val Ile Leu Leu Asn Ala Gln Gly Val Pro Thr
1 5 10 15

Gly Thr Leu Glu Lys Tyr Ala Ala His Thr Ala Asp Thr Arg Leu His
20 25 30

Leu Ala Phe Ser Ser Trp Leu Phe Asn Ala Lys Gly Gln Leu Leu Val
35 40 45

Thr Arg Arg Ala Leu Ser Lys Lys Ala Trp Pro Gly Val Trp Thr Asn
50 55 60

Ser Val Cys Gly His Pro Gln Leu Gly Glu Ser Asn Glu Asp Ala Val
65 70 75 80

Ile Arg Arg Cys Arg Tyr Glu Leu Gly Val Glu Ile Thr Pro Pro Glu
85 90 95

Ser Ile Tyr Pro Asp Phe Arg Tyr Arg Ala Thr Asp Pro Ser Gly Ile

100 105 110

Val Glu Asn Glu Val Cys Pro Val Phe Ala Ala Arg Thr Thr Ser Ala

115 120 125

Leu Gln Ile Asn Asp Asp Glu Val Met Asp Tyr Gln Trp Cys Asp Leu

130 135 140

Ala Asp Val Leu His Gly Ile Asp Ala Thr Pro Trp Ala Phe Ser Pro

145 150 155 160

Trp Met Val Met Gln Ala Thr Asn Arg Glu Ala Arg Lys Arg Leu Ser

165 170 175

Ala Phe Thr Gln Leu Lys

180

Fig. 1

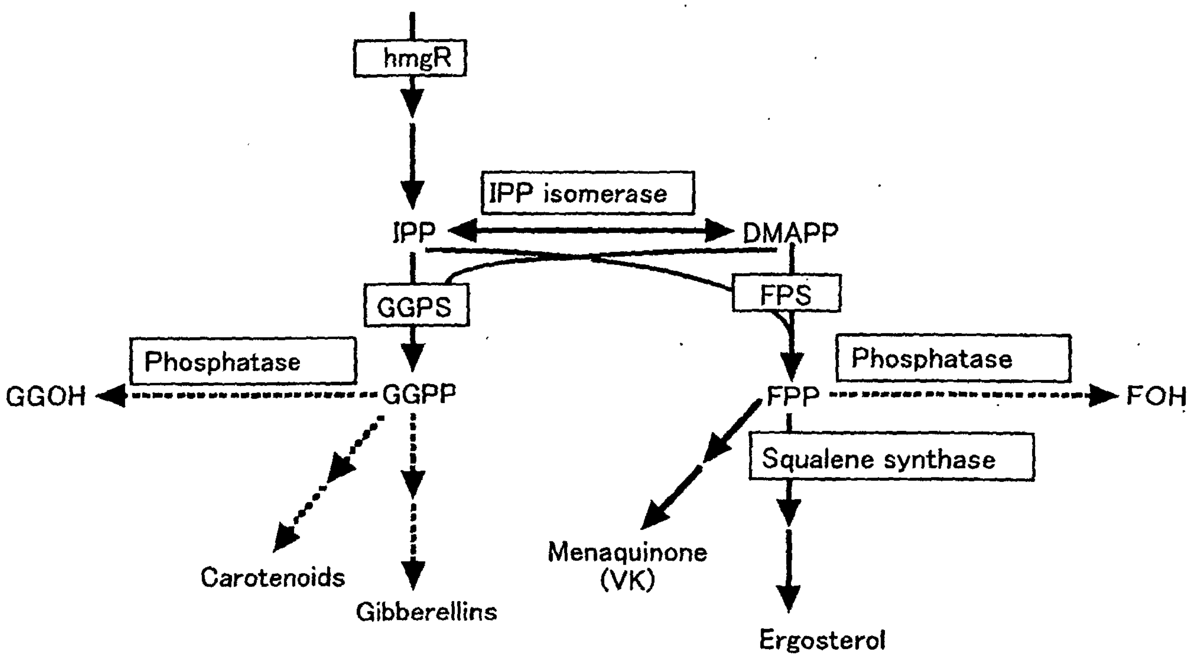
hmgR
IPP isomerase
IPP
DMAPP
GGPS
FPS
Phosphatase
GGOH
GGPP
Phosphatase
FPP
FOH
Squalene synthase
Carotenoids
Gibberellins
Menaquinone
(VK)
Ergosterol

Fig.2A

| Nucleotide | Amino Acid |
|---|---|
| C203T | S68F |
| T426C | |
| T1026C | |
| T1167G | |
| T1248C | |
| G1557A | |
| A1605G | |
| T1820C | L607S |
| A2451G | |
| A2726G | H909R |
| T2787C | |
| G2940A | |

Fig.2B

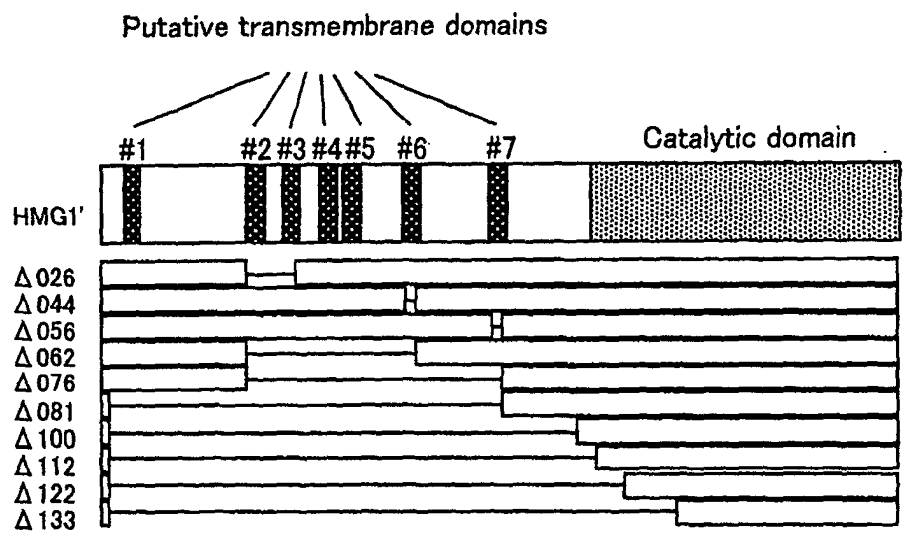

Fig.3

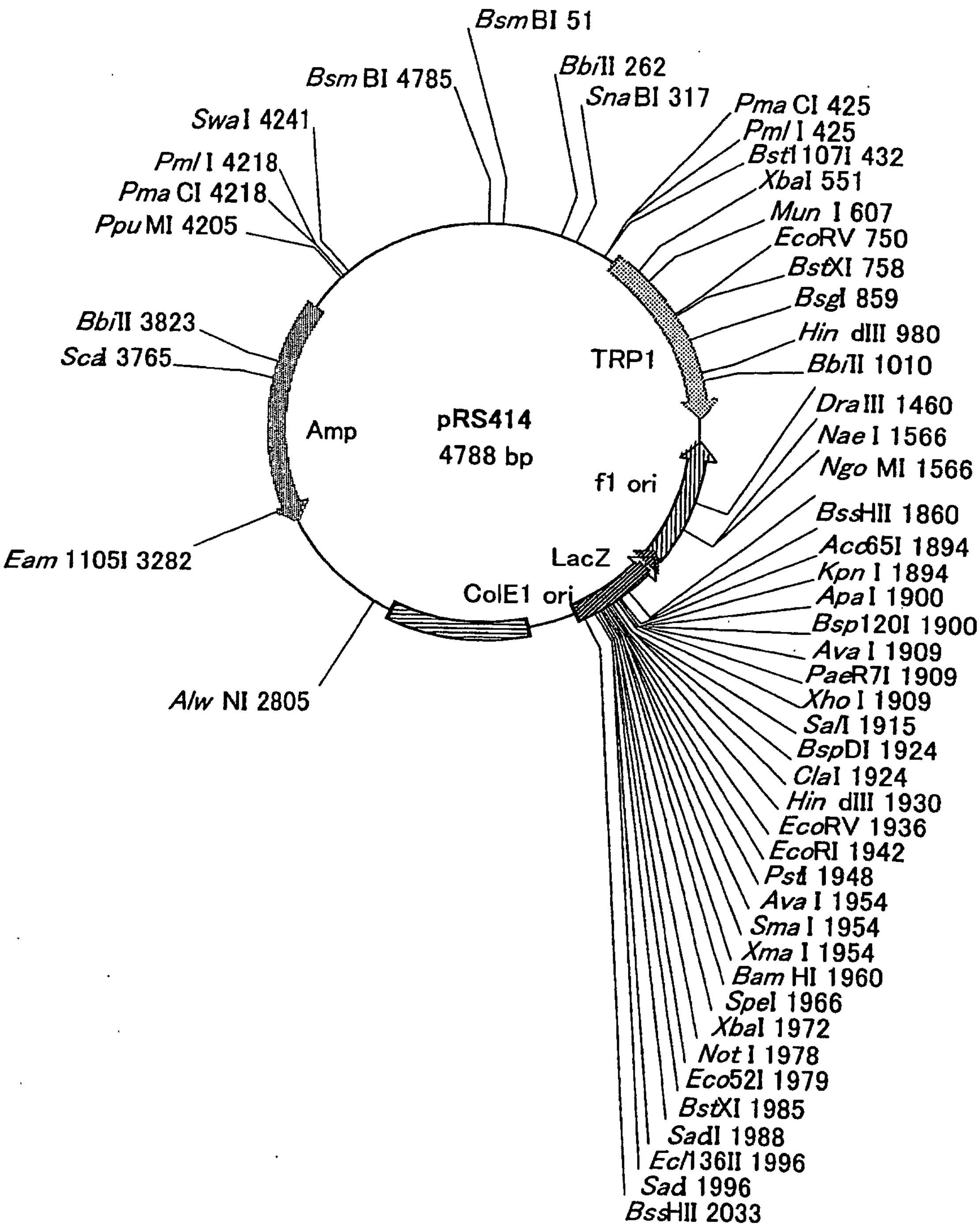
BsmBI 51
BbiII 262
SnaBI 317
PmaCI 425
PmlI 425
Bst1107I 432
XbaI 551
MunI 607
EcoRV 750
BstXI 758
BsgI 859
HindIII 980
BbiII 1010
DraIII 1460
NaeI 1566
NgoMI 1566
BssHII 1860
Acc65I 1894
KpnI 1894
ApaI 1900
Bsp120I 1900
AvaI 1909
PaeR7I 1909
XhoI 1909
SalI 1915
BspDI 1924
ClaI 1924
HindIII 1930
EcoRV 1936
EcoRI 1942
PstI 1948
AvaI 1954
SmaI 1954
XmaI 1954
BamHI 1960
SpeI 1966
XbaI 1972
NotI 1978
Eco52I 1979
BstXI 1985
SacII 1988
Ecl136II 1996
SacI 1996
BssHII 2033
AlwNI 2805
Eam1105I 3282
ScaI 3765
BbiII 3823
PpuMI 4205
PmaCI 4218
PmlI 4218
SwaI 4241
BsmBI 4785
TRP1
f1 ori
LacZ
ColE1 ori
Amp
pRS414
4788 bp

Fig.4

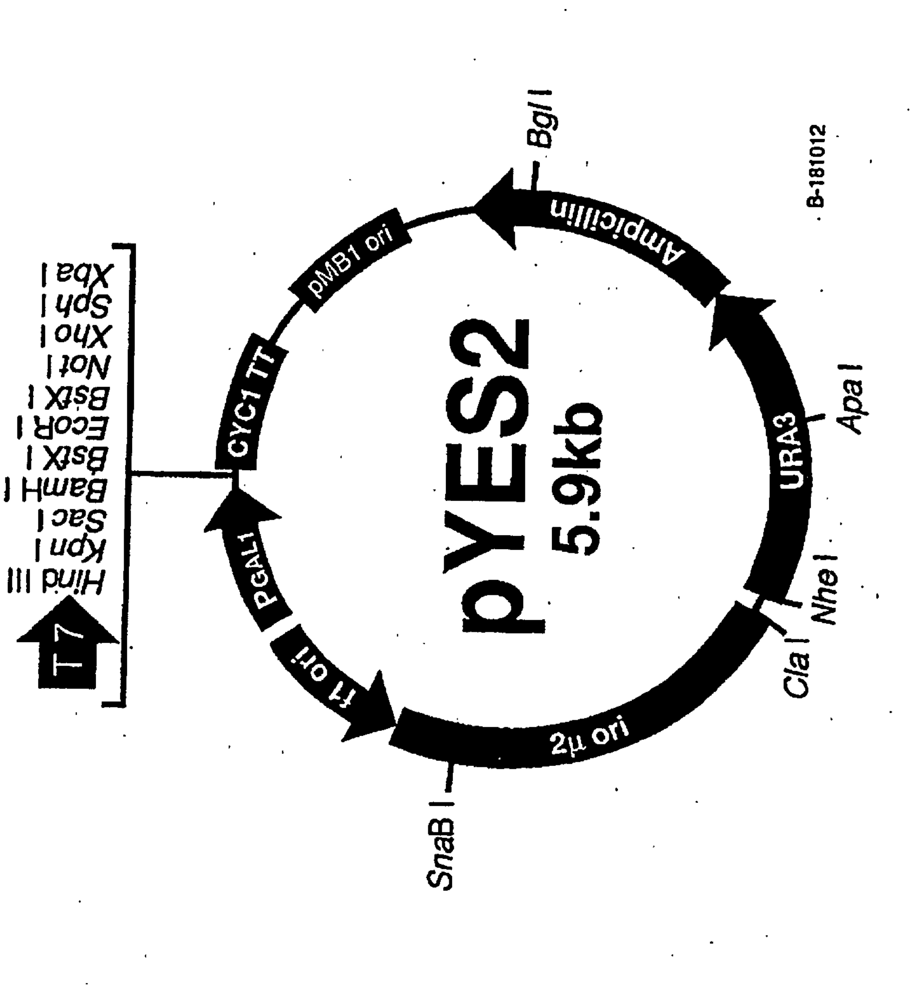
T7
Hind III
Kpn I
Sac I
BamH I
BstX I
EcoR I
BstX I
Not I
Xho I
Sph I
Xba I
pYES2
5.9kb
PGAL1
CYC1 TT
pMB1 ori
Ampicillin
Bgl I
URA3
Apa I
Nhe I
Cla I
2μ ori
f1 ori
SnaB I
B-181012

Fig.5

```
GGATCCTCTA GCTCCCTAAC ATGTAGGTGG CGGAGGGGAG ATATACAATA GAACAGATAC CAGACAAGAC
CCTAGGAGAT CGAGGGATTG TACATCCACC GCCTCCCCTC TATATGTTAT CTTGTCTATG GTCTGTTCTG
        10         20         30         40         50         60         70

ATAATGGGCT AAACAAGACT ACACCAATTA CACTGCCTCA TTGATGGTGG TACATAACGA ACTAATACTG
TATTACCCGA TTTGTTCTGA TGTGGTTAAT GTGACGGAGT AACTACCACC ATGTATTGCT TGATTATGAC
        80         90        100        110        120        130        140

TAGCCCTAGA CTTGATAGCC ATCATCATAT CGAAGTTTCA CTACCCTTTT TCCATTTGCC ATCTATTGAA
ATCGGGATCT GAACTATCGG TAGTAGTATA GCTTCAAAGT GATGGGAAAA AGGTAAACGG TAGATAACTT
       150        160        170        180        190        200        210

GTAATAATAG GCGCATGCAA CTTCTTTTCT TTTTTTTTCT TTTCTCTCTC CCCCGTTGTT GTCTCACCAT
CATTATTATC CGCGTACGTT GAAGAAAAGA AAAAAAAAGA AAAGAGAGAG GGGGCAACAA CAGAGTGGTA
       220        230        240        250        260        270        280

ATCCGCAATG ACAAAAAAAT GATGGAAGAC ACTAAAGGAA AAAATTAACG ACAAAGACAG CACCAACAGA
TAGGCGTTAC TGTTTTTTTA CTACCTTCTG TGATTTCCTT TTTTAATTGC TGTTTCTGTC GTGGTTGTCT
       290        300        310        320        330        340        350

TGTCGTTGTT CCAGAGCTGA TGAGGGGTAT CTCGAAGCAC ACGAAACTTT TTCCTTCCTT CATTCACGCA
ACAGCAACAA GGTCTCGACT ACTCCCCATA GAGCTTCGTG TGCTTTGAAA AAGGAAGGAA GTAAGTGCGT
       360        370        380        390        400        410        420

CACTACTCTC TAATGAGCAA CGGTATACGG CCTTCCTTCC AGTTACTTGA ATTTGAAATA AAAAAAGTTT
GTGATGAGAG ATTACTCGTT GCCATATGCC GGAAGGAAGG TCAATGAACT TAAACTTTAT TTTTTTCAAA
       430        440        450        460        470        480        490

GCTGTCTTGC TATCAAGTAT AAATAGACCT GCAATTATTA ATCTTTTGTT TCCTCGTCAT TGTTCTCGTT
CGACAGAACG ATAGTTCATA TTTATCTGGA CGTTAATAAT TAGAAAACAA AGGAGCAGTA ACAAGAGCAA
       500        510        520        530        540        550        560

CCCTTTCTTC CTTGTTTCTT TTTCTGCACA ATATTTCAAG CTATACCAAG CATACAATCA ACTGGTACCC
GGGAAAGAAG GAACAAAGAA AAAGACGTGT TATAAAGTTC GATATGGTTC GTATGTTAGT TGACCATGGG
       570        580        590        600        610        620        630

GGGTCGACTC GAGCTCTAGA GGTTAACTAA GCGAATTTCT TATGATTTAT GATTTTTATT ATTAAATAAG
CCCAGCTGAG CTCGAGATCT CCAATTGATT CGCTTAAAGA ATACTAAATA CTAAAAATAA TAATTTATTC
       640        650        660        670        680        690        700

TTATAAAAAA AATAAGTGTA TACAAATTTT AAAGTGACTC TTAGGTTTTA AAACGAAAAT TCTTATTCTT
AATATTTTTT TTATTCACAT ATGTTTAAAA TTTCACTGAG AATCCAAAAT TTTGCTTTTA AGAATAAGAA
       710        720        730        740        750        760        770

GAGTAACTCT TTCCTGTAGG TCAGGTTGCT TTCTCAGGTA TAGCATGAGG TCGCTCTTAT TGACCACATC
CTCATTGAGA AAGGACATCC AGTCCAACGA AAGAGTCCAT ATCGTACTCC AGCGAGAATA ACTGGTGTAG
       780        790        800        810        820        830        840

TCTACCGGCA TGCCGAGCAA ATGCCTGCAA ATCGCTCCCC ATTTCACCCA ATTGTAGATA TGCTAACTCC
AGATGGCCGT ACGGCTCGTT TACGGACGTT TAGCGAGGGG TAAAGTGGGT TAACATCTAT ACGATTGAGG
       850        860        870        880        890        900        910

AGCAATGAGT TGATGAATCT CGGTGTGTAT TTTATGTCCT CAGAGGACAA CACCTGTTGT AATCGTTCTT
TCGTTACTCA ACTACTTAGA GCCACACATA AAATACAGGA GTCTCCTGTT GTGGACAACA TTAGCAAGAA
       920        930        940        950        960        970        980

CCACACGGAT CC
GGTGTGCCTA GG
       990
```

Fig.6A

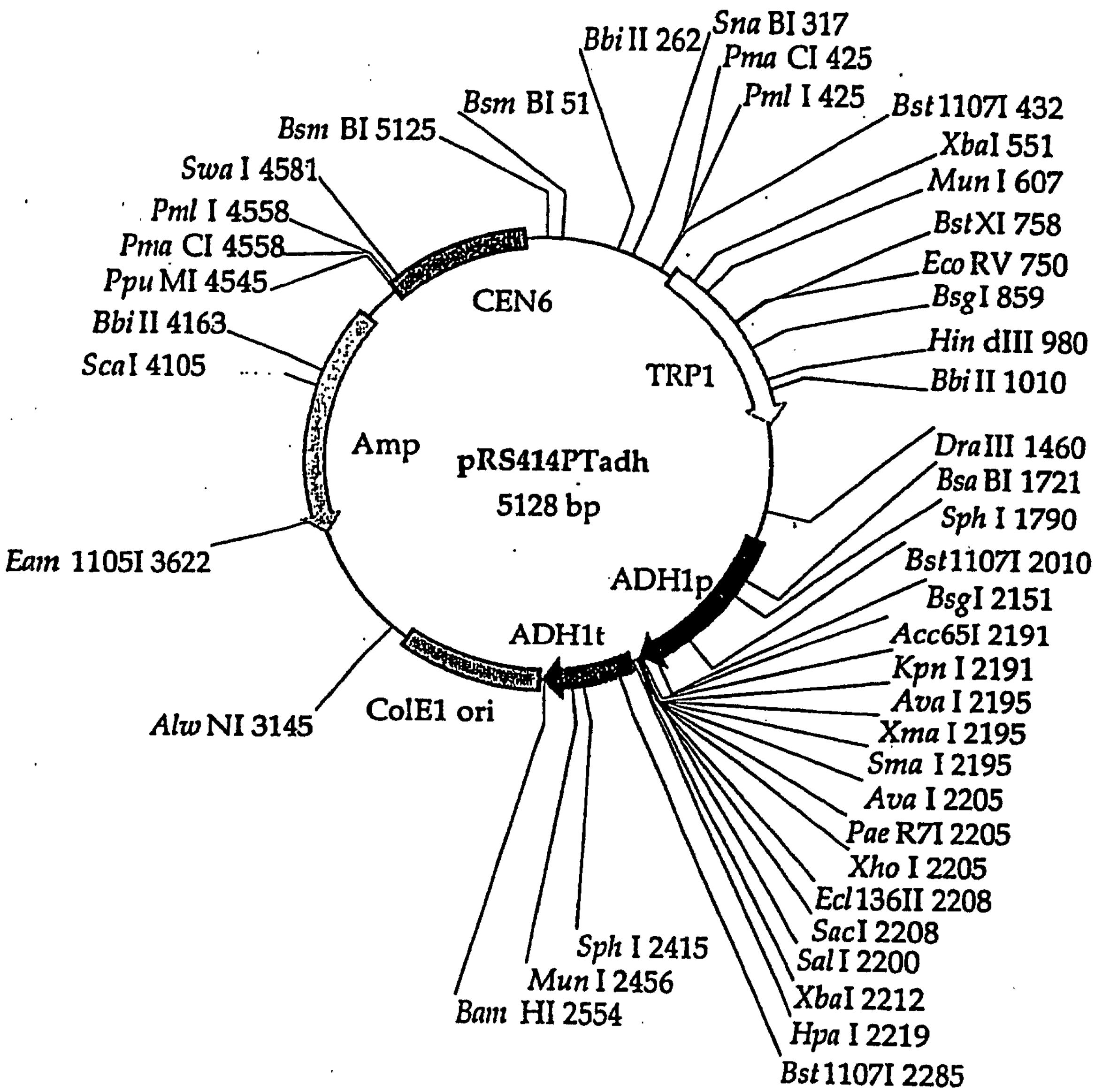
pRS414PTadh
5128 bp
CEN6
TRP1
Amp
ADH1p
ADH1t
ColE1 ori
Bsm BI 51
Bbi II 262
Sna BI 317
Pma CI 425
Pml I 425
Bst 1107I 432
Xba I 551
Mun I 607
Bst XI 758
Eco RV 750
Bsg I 859
Hin dIII 980
Bbi II 1010
Dra III 1460
Bsa BI 1721
Sph I 1790
Bst 1107I 2010
Bsg I 2151
Acc65I 2191
Kpn I 2191
Ava I 2195
Xma I 2195
Sma I 2195
Ava I 2205
Pae R7I 2205
Xho I 2205
Ecl 136II 2208
Sac I 2208
Sal I 2200
Xba I 2212
Hpa I 2219
Bst 1107I 2285
Sph I 2415
Mun I 2456
Bam HI 2554
Alw NI 3145
Eam 1105I 3622
Sca I 4105
Bbi II 4163
Ppu MI 4545
Pma CI 4558
Pml I 4558
Swa I 4581
Bsm BI 5125

Fig.6B

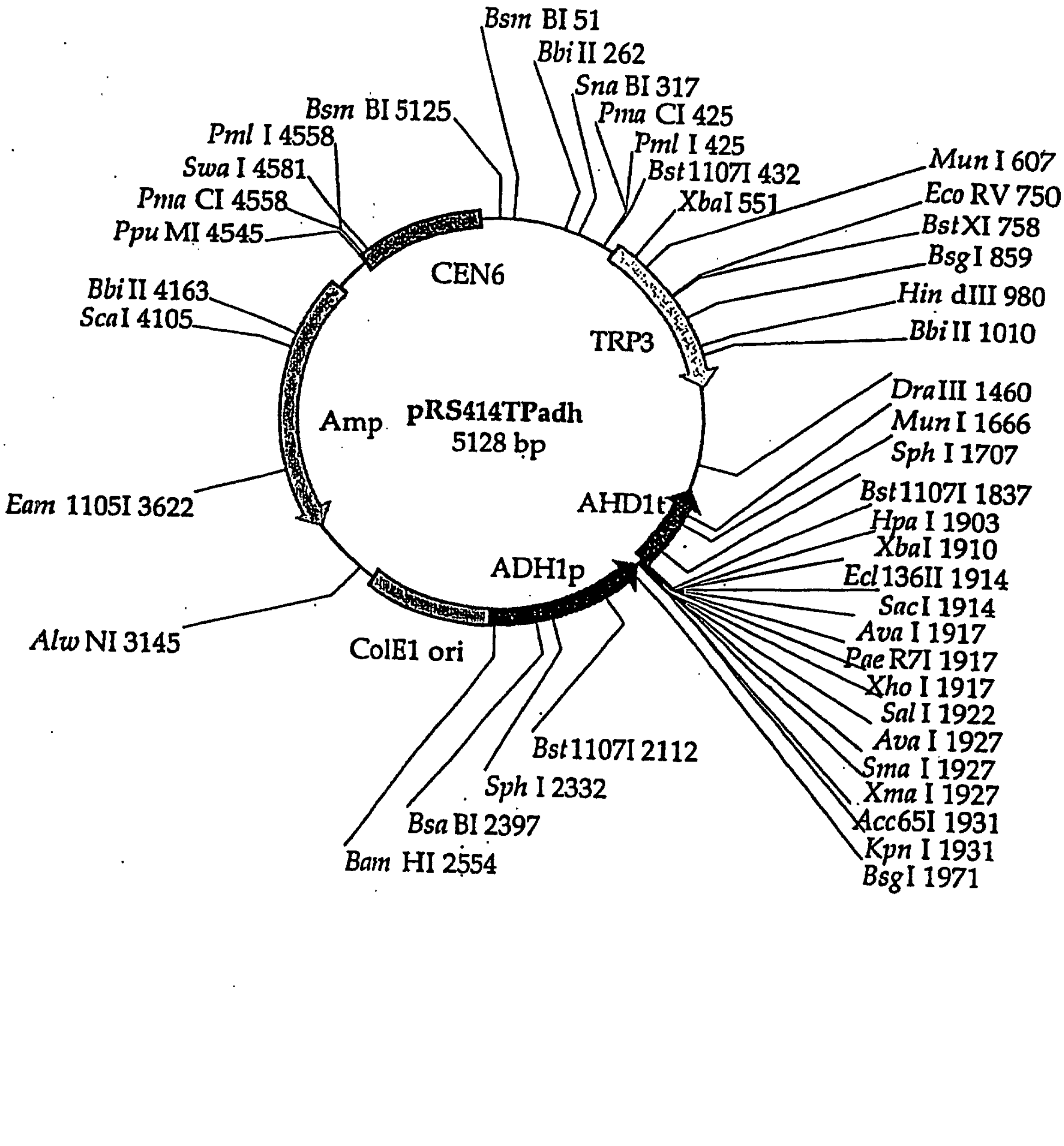
Bsm BI 51
Bbi II 262
Sna BI 317
Pma CI 425
Pml I 425
Bst 1107I 432
Xba I 551
Mun I 607
Eco RV 750
Bst XI 758
Bsg I 859
Hin dIII 980
Bbi II 1010
Dra III 1460
Mun I 1666
Sph I 1707
Bst 1107I 1837
Hpa I 1903
Xba I 1910
Ecl 136II 1914
Sac I 1914
Ava I 1917
Pae R7I 1917
Xho I 1917
Sal I 1922
Ava I 1927
Sma I 1927
Xma I 1927
Acc65I 1931
Kpn I 1931
Bsg I 1971
Bst 1107I 2112
Sph I 2332
Bsa BI 2397
Bam HI 2554
Alw NI 3145
Eam 1105I 3622
Sca I 4105
Bbi II 4163
Ppu MI 4545
Pma CI 4558
Swa I 4581
Pml I 4558
Bsm BI 5125
CEN6
TRP3
Amp
pRS414TPadh
5128 bp
AHD1t
ADH1p
ColE1 ori

Fig.7A-1

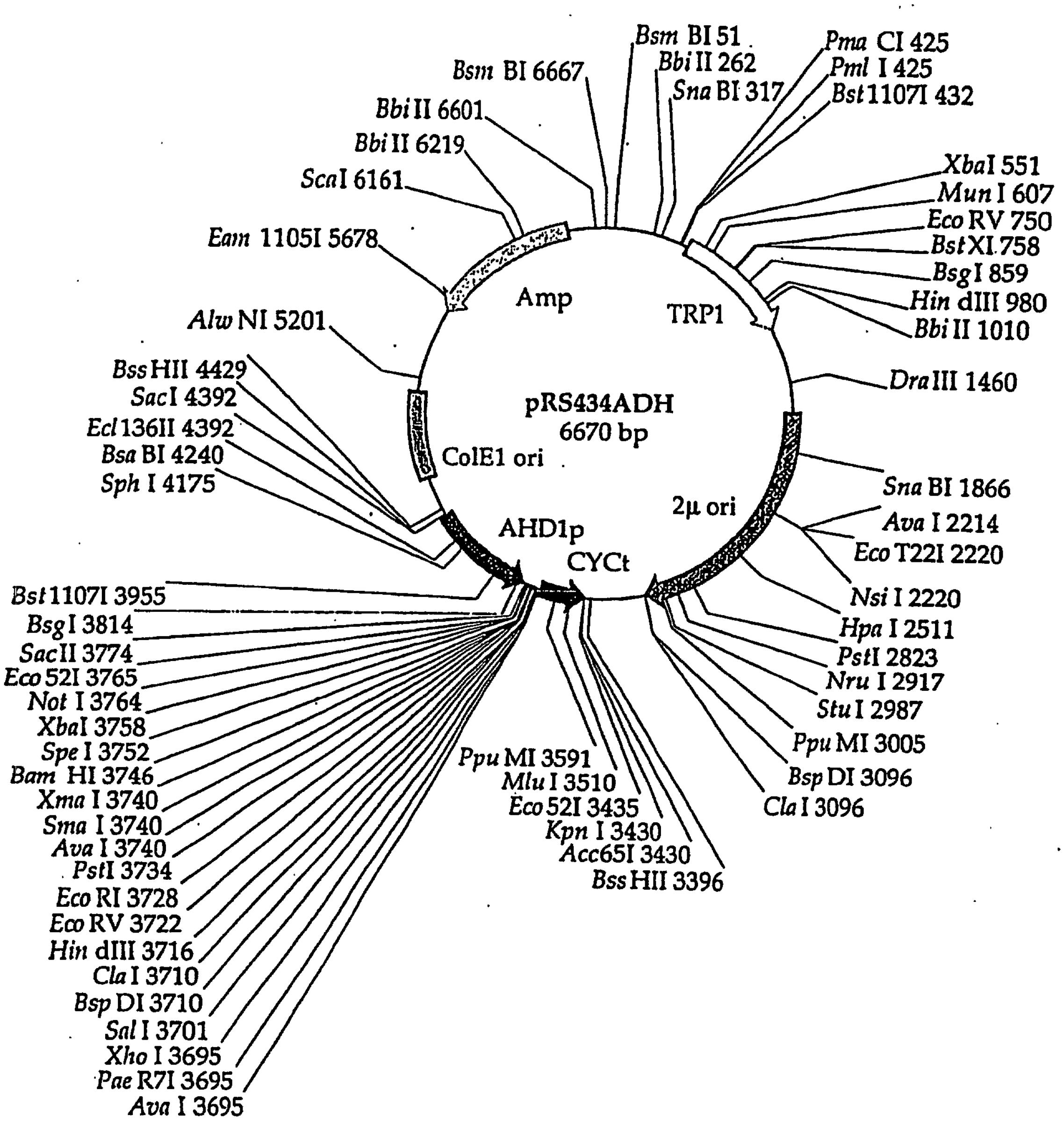
Bsm BI 51
Bbi II 262
Sna BI 317
Pma CI 425
Pml I 425
Bst1107I 432
Bsm BI 6667
Bbi II 6601
Bbi II 6219
Sca I 6161
Eam 1105I 5678
Xba I 551
Mun I 607
Eco RV 750
Bst XI 758
Bsg I 859
Hin dIII 980
Bbi II 1010
Amp
TRP1
Alw NI 5201
Dra III 1460
Bss HII 4429
Sac I 4392
Ecl 136II 4392
Bsa BI 4240
Sph I 4175
pRS434ADH
6670 bp
ColE1 ori
2μ ori
Sna BI 1866
AHD1p
CYCt
Ava I 2214
Eco T22I 2220
Nsi I 2220
Hpa I 2511
Pst I 2823
Nru I 2917
Stu I 2987
Ppu MI 3005
Bsp DI 3096
Cla I 3096
Bst1107I 3955
Bsg I 3814
Sac II 3774
Eco 52I 3765
Not I 3764
Xba I 3758
Spe I 3752
Bam HI 3746
Xma I 3740
Sma I 3740
Ava I 3740
Pst I 3734
Eco RI 3728
Eco RV 3722
Hin dIII 3716
Cla I 3710
Bsp DI 3710
Sal I 3701
Xho I 3695
Pae R7I 3695
Ava I 3695
Ppu MI 3591
Mlu I 3510
Eco 52I 3435
Kpn I 3430
Acc65I 3430
Bss HII 3396

Fig.7A-2

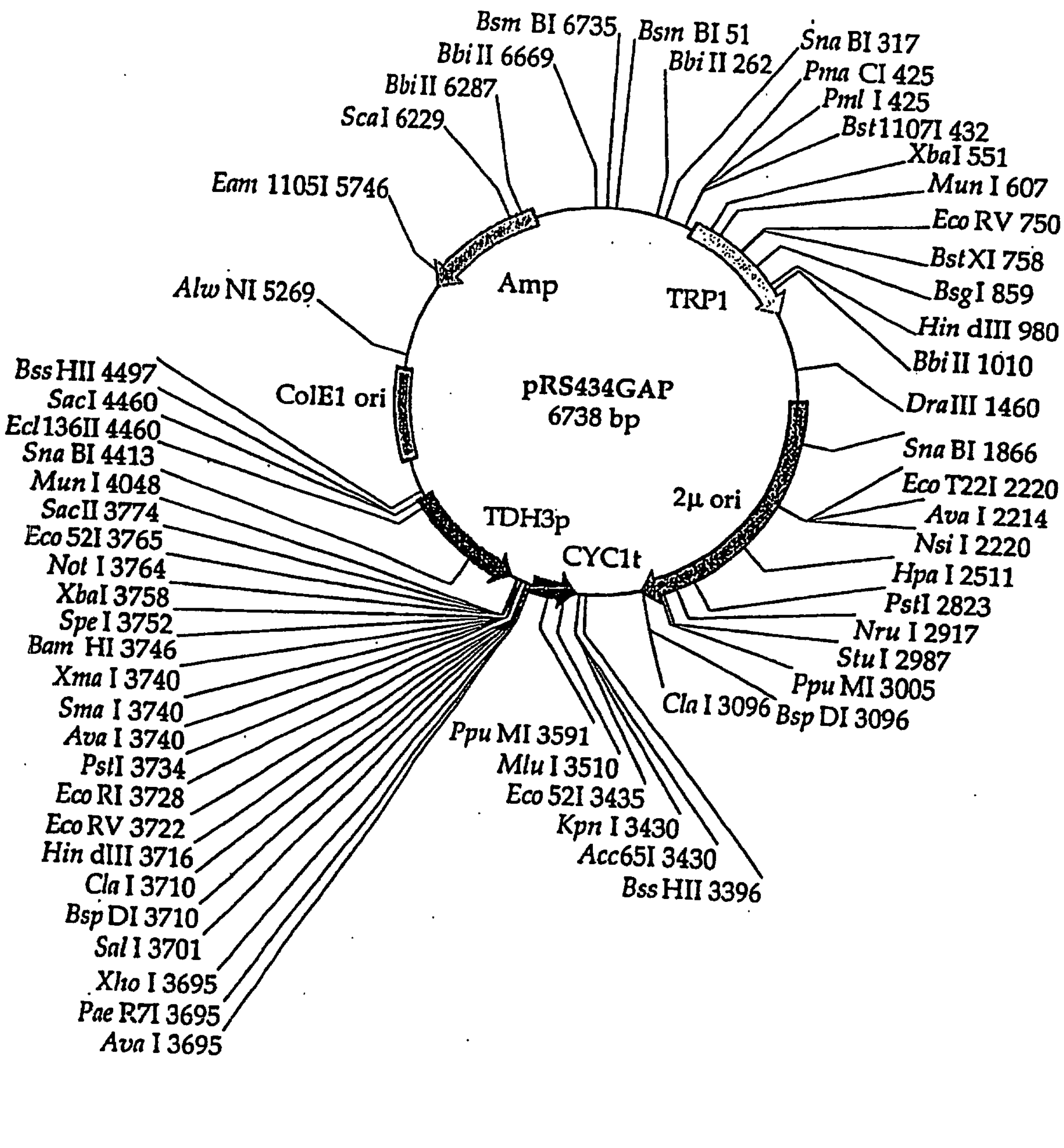
Bsm BI 6735
Bsm BI 51
Sna BI 317
Bbi II 6669
Bbi II 262
Pma CI 425
Bbi II 6287
Pml I 425
Sca I 6229
Bst 1107I 432
Xba I 551
Eam 1105I 5746
Mun I 607
Eco RV 750
Bst XI 758
Amp
TRP1
Alw NI 5269
Bsg I 859
Hin dIII 980
Bbi II 1010
Bss HII 4497
pRS434GAP
6738 bp
Sac I 4460
ColE1 ori
Dra III 1460
Ecl 136II 4460
Sna BI 4413
Sna BI 1866
Mun I 4048
2μ ori
Eco T22I 2220
Sac II 3774
Ava I 2214
TDH3p
Eco 52I 3765
Nsi I 2220
CYC1t
Not I 3764
Hpa I 2511
Xba I 3758
Pst I 2823
Spe I 3752
Nru I 2917
Bam HI 3746
Stu I 2987
Xma I 3740
Ppu MI 3005
Sma I 3740
Cla I 3096
Bsp DI 3096
Ava I 3740
Ppu MI 3591
Pst I 3734
Mlu I 3510
Eco RI 3728
Eco 52I 3435
Eco RV 3722
Kpn I 3430
Hin dIII 3716
Acc65I 3430
Cla I 3710
Bss HII 3396
Bsp DI 3710
Sal I 3701
Xho I 3695
Pae R7I 3695
Ava I 3695

Fig.7B-1

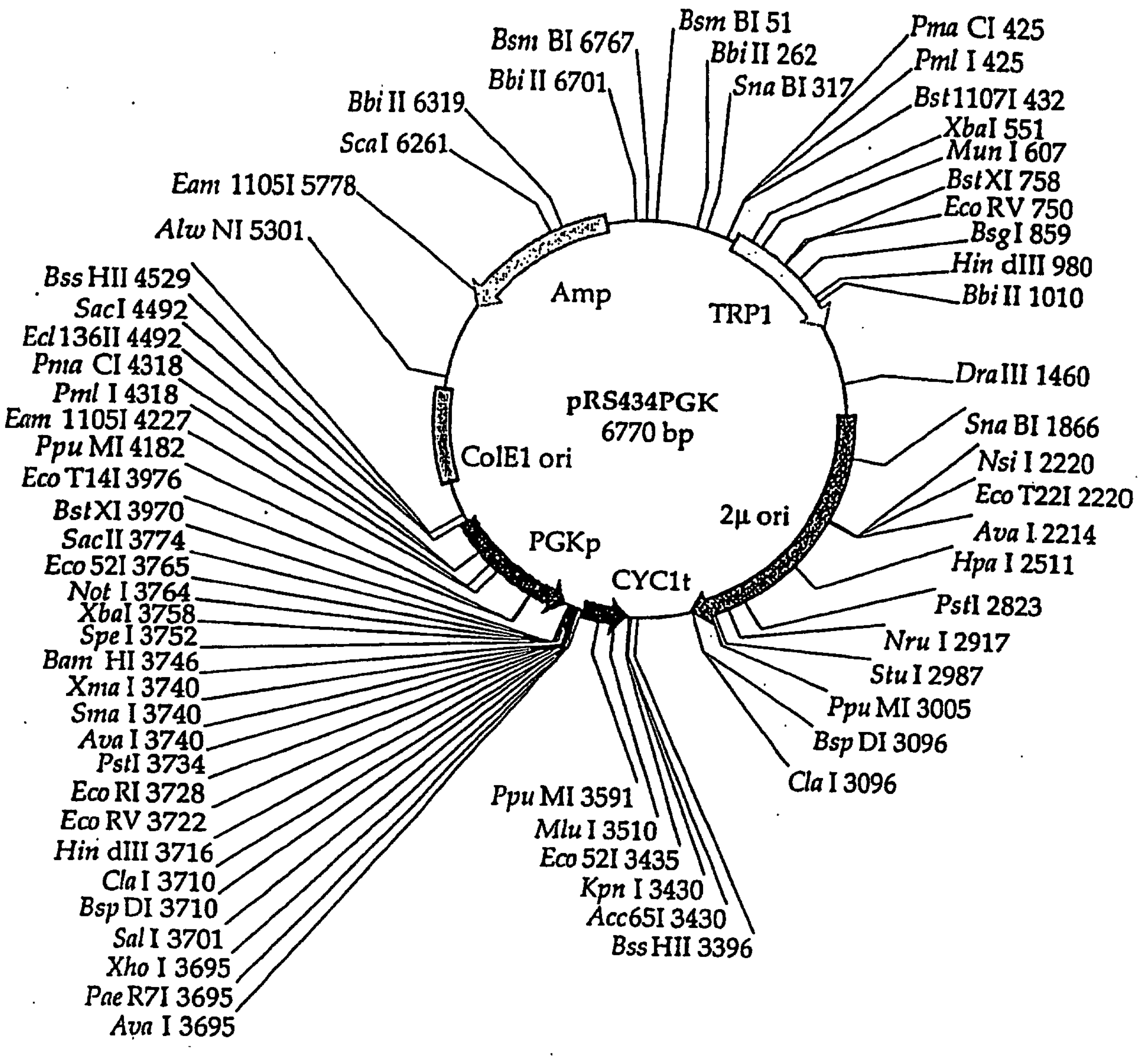
Bsm BI 6767
Bsm BI 51
Bbi II 262
Bbi II 6701
Sna BI 317
Bbi II 6319
Sca I 6261
Eam 1105I 5778
Alw NI 5301
Pma CI 425
Pml I 425
Bst 1107I 432
Xba I 551
Mun I 607
Bst XI 758
Eco RV 750
Bsg I 859
Hin dIII 980
Bbi II 1010
Dra III 1460
Sna BI 1866
Nsi I 2220
Eco T22I 2220
Ava I 2214
Hpa I 2511
Pst I 2823
Nru I 2917
Stu I 2987
Ppu MI 3005
Bsp DI 3096
Cla I 3096
Ppu MI 3591
Mlu I 3510
Eco 52I 3435
Kpn I 3430
Acc65I 3430
Bss HII 3396
Bss HII 4529
Sac I 4492
Ecl 136II 4492
Pma CI 4318
Pml I 4318
Eam 1105I 4227
Ppu MI 4182
Eco T14I 3976
Bst XI 3970
Sac II 3774
Eco 52I 3765
Not I 3764
Xba I 3758
Spe I 3752
Bam HI 3746
Xma I 3740
Sma I 3740
Ava I 3740
Pst I 3734
Eco RI 3728
Eco RV 3722
Hin dIII 3716
Cla I 3710
Bsp DI 3710
Sal I 3701
Xho I 3695
Pae R7I 3695
Ava I 3695
Amp
TRP1
pRS434PGK
6770 bp
ColE1 ori
2µ ori
PGKp
CYC1t

Fig.7B-2

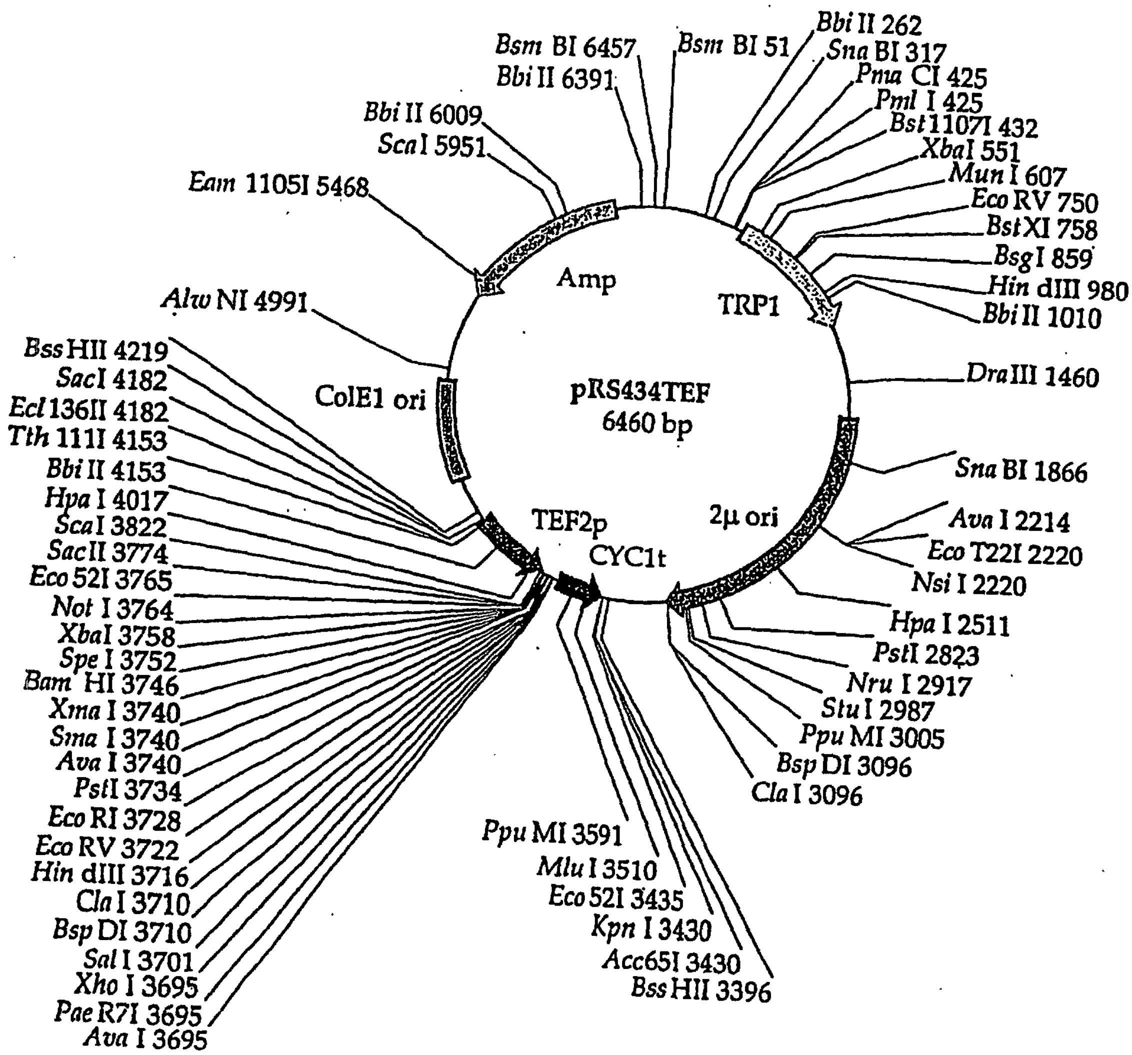
Bsm BI 6457
Bsm BI 51
Bbi II 262
Sna BI 317
Pma CI 425
Pml I 425
Bst 1107I 432
Xba I 551
Mun I 607
Eco RV 750
Bst XI 758
Bsg I 859
Hin dIII 980
Bbi II 1010
Dra III 1460
Sna BI 1866
Ava I 2214
Eco T22I 2220
Nsi I 2220
Hpa I 2511
Pst I 2823
Nru I 2917
Stu I 2987
Ppu MI 3005
Bsp DI 3096
Cla I 3096
Ppu MI 3591
Mlu I 3510
Eco 52I 3435
Kpn I 3430
Acc65I 3430
Bss HII 3396
Bbi II 6391
Bbi II 6009
Sca I 5951
Eam 1105I 5468
Alw NI 4991
Bss HII 4219
Sac I 4182
Ecl 136II 4182
Tth 111I 4153
Bbi II 4153
Hpa I 4017
Sca I 3822
Sac II 3774
Eco 52I 3765
Not I 3764
Xba I 3758
Spe I 3752
Bam HI 3746
Xma I 3740
Sma I 3740
Ava I 3740
Pst I 3734
Eco RI 3728
Eco RV 3722
Hin dIII 3716
Cla I 3710
Bsp DI 3710
Sal I 3701
Xho I 3695
Pae R7I 3695
Ava I 3695
Amp
TRP1
ColE1 ori
pRS434TEF
6460 bp
2μ ori
TEF2p
CYC1t

Fig.7C-1

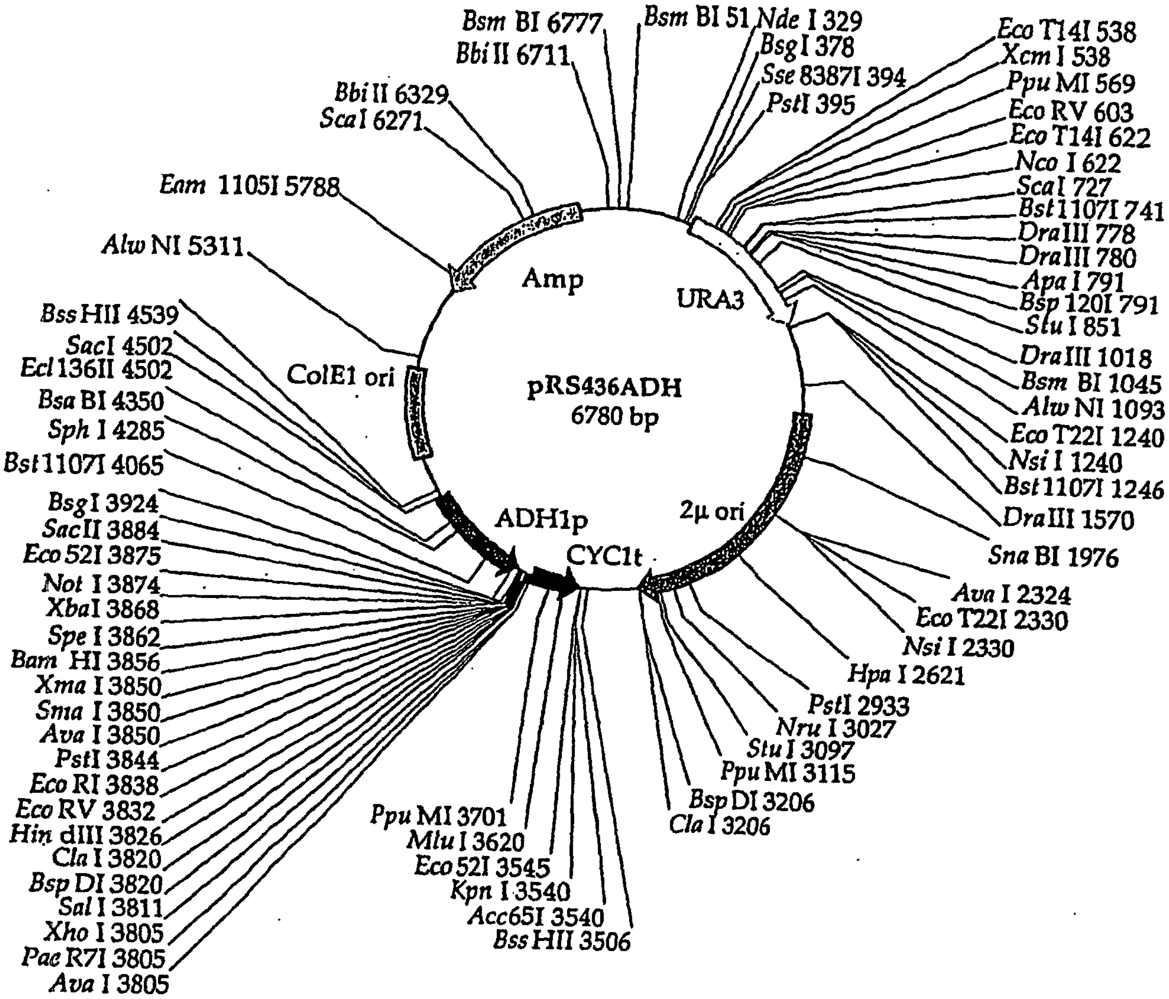
pRS436ADH
6780 bp
Amp
URA3
ColE1 ori
ADH1p
CYC1t
2μ ori
Bsm BI 6777
Bbi II 6711
Bbi II 6329
Sca I 6271
Eam 1105I 5788
Alw NI 5311
Bsm BI 51
Nde I 329
Bsg I 378
Sse 8387I 394
Pst I 395
Eco T14I 538
Xcm I 538
Ppu MI 569
Eco RV 603
Eco T14I 622
Nco I 622
Sca I 727
Bst 1107I 741
Dra III 778
Dra III 780
Apa I 791
Bsp 120I 791
Stu I 851
Dra III 1018
Bsm BI 1045
Alw NI 1093
Eco T22I 1240
Nsi I 1240
Bst 1107I 1246
Dra III 1570
Sna BI 1976
Ava I 2324
Eco T22I 2330
Nsi I 2330
Hpa I 2621
Pst I 2933
Nru I 3027
Stu I 3097
Ppu MI 3115
Bsp DI 3206
Cla I 3206
Ppu MI 3701
Mlu I 3620
Eco 52I 3545
Kpn I 3540
Acc65I 3540
Bss HII 3506
Bss HII 4539
Sac I 4502
Ecl 136II 4502
Bsa BI 4350
Sph I 4285
Bst 1107I 4065
Bsg I 3924
Sac II 3884
Eco 52I 3875
Not I 3874
Xba I 3868
Spe I 3862
Bam HI 3856
Xma I 3850
Sma I 3850
Ava I 3850
Pst I 3844
Eco RI 3838
Eco RV 3832
Hin dIII 3826
Cla I 3820
Bsp DI 3820
Sal I 3811
Xho I 3805
Pae R7I 3805
Ava I 3805

Fig.7C-2

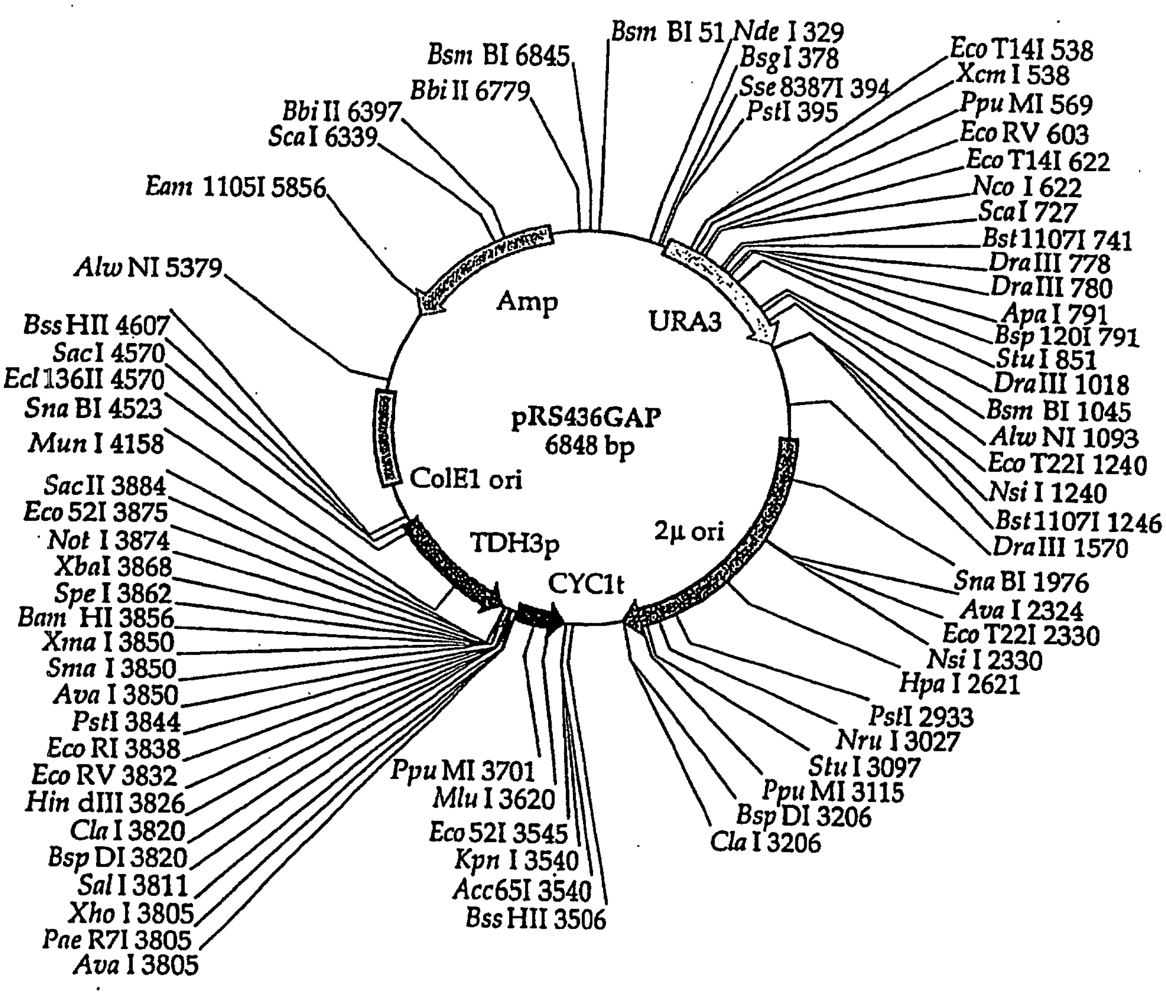
Bsm BI 51
Nde I 329
Bsg I 378
Sse 8387I 394
PstI 395
Eco T14I 538
Xcm I 538
Ppu MI 569
Eco RV 603
Eco T14I 622
Nco I 622
Sca I 727
Bst1107I 741
Dra III 778
Dra III 780
Apa I 791
Bsp 120I 791
Stu I 851
Dra III 1018
Bsm BI 1045
Alw NI 1093
Eco T22I 1240
Nsi I 1240
Bst1107I 1246
Dra III 1570
Sna BI 1976
Ava I 2324
Eco T22I 2330
Nsi I 2330
Hpa I 2621
PstI 2933
Nru I 3027
Stu I 3097
Ppu MI 3115
Bsp DI 3206
Cla I 3206
Ppu MI 3701
Mlu I 3620
Eco 52I 3545
Kpn I 3540
Acc65I 3540
Bss HII 3506
Sac II 3884
Eco 52I 3875
Not I 3874
XbaI 3868
Spe I 3862
Bam HI 3856
Xma I 3850
Sma I 3850
Ava I 3850
PstI 3844
Eco RI 3838
Eco RV 3832
Hin dIII 3826
Cla I 3820
Bsp DI 3820
Sal I 3811
Xho I 3805
Pae R7I 3805
Ava I 3805
Bss HII 4607
Sac I 4570
Ecl 136II 4570
Sna BI 4523
Mun I 4158
Alw NI 5379
Eam 1105I 5856
Sca I 6339
Bbi II 6397
Bbi II 6779
Bsm BI 6845
Amp
URA3
pRS436GAP
6848 bp
ColE1 ori
TDH3p
2μ ori
CYC1t

Fig.7D-1

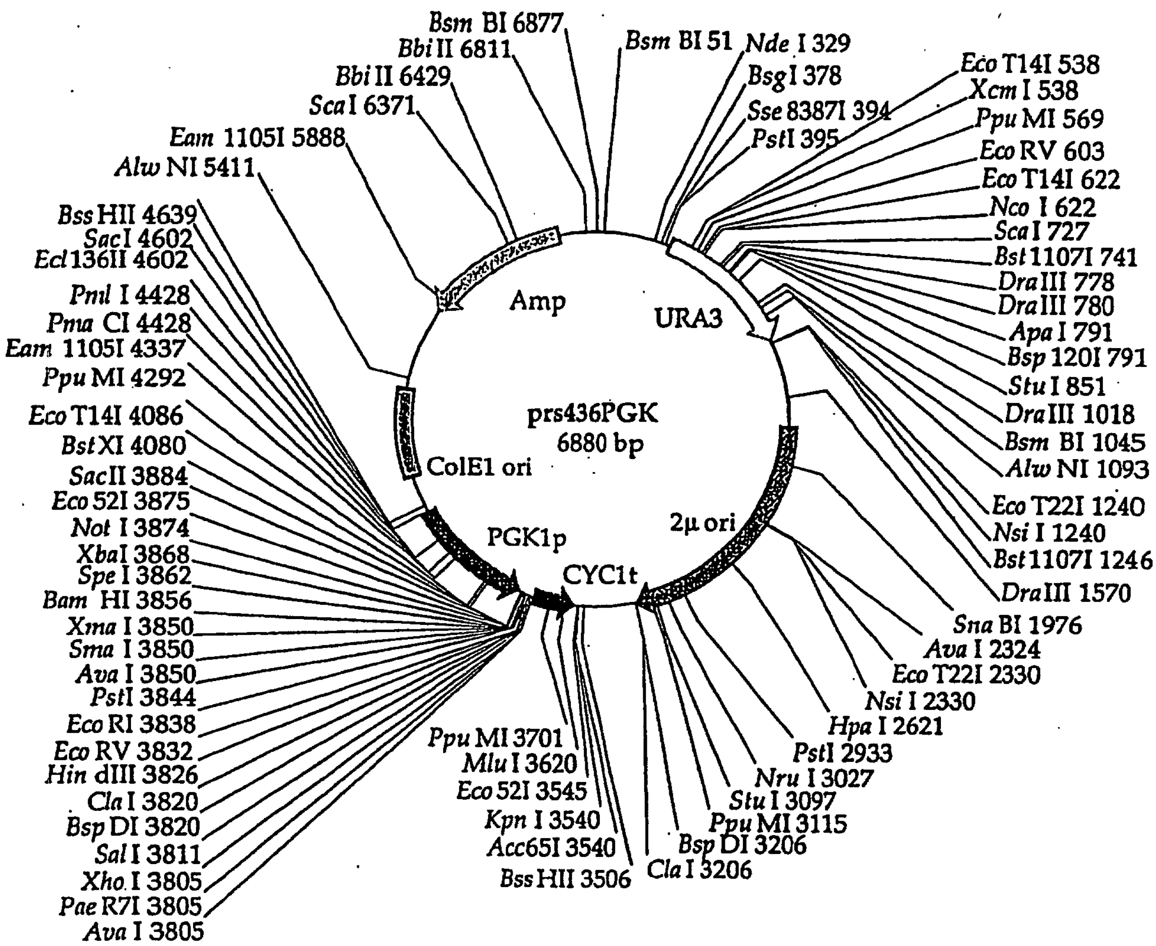

Bsm BI 6877
Bbi II 6811
Bbi II 6429
Sca I 6371
Eam 1105I 5888
Alw NI 5411
Bsm BI 51
Nde I 329
Bsg I 378
Sse 8387I 394
Pst I 395
Eco T14I 538
Xcm I 538
Ppu MI 569
Eco RV 603
Eco T14I 622
Nco I 622
Sca I 727
Bst 1107I 741
Dra III 778
Dra III 780
Apa I 791
Bsp 120I 791
Stu I 851
Dra III 1018
Bsm BI 1045
Alw NI 1093
Eco T22I 1240
Nsi I 1240
Bst 1107I 1246
Dra III 1570
Sna BI 1976
Ava I 2324
Eco T22I 2330
Nsi I 2330
Hpa I 2621
Pst I 2933
Nru I 3027
Stu I 3097
Ppu MI 3115
Bsp DI 3206
Cla I 3206
Bss HII 3506
Acc65I 3540
Kpn I 3540
Eco 52I 3545
Mlu I 3620
Ppu MI 3701
Bss HII 4639
Sac I 4602
Ecl 136II 4602
Pml I 4428
Pma CI 4428
Eam 1105I 4337
Ppu MI 4292
Eco T14I 4086
Bst XI 4080
Sac II 3884
Eco 52I 3875
Not I 3874
Xba I 3868
Spe I 3862
Bam HI 3856
Xma I 3850
Sma I 3850
Ava I 3850
Pst I 3844
Eco RI 3838
Eco RV 3832
Hin dIII 3826
Cla I 3820
Bsp DI 3820
Sal I 3811
Xho I 3805
Pae R7I 3805
Ava I 3805
Amp
URA3
prs436PGK
6880 bp
ColE1 ori
2μ ori
PGK1p
CYC1t

Fig.7D-2

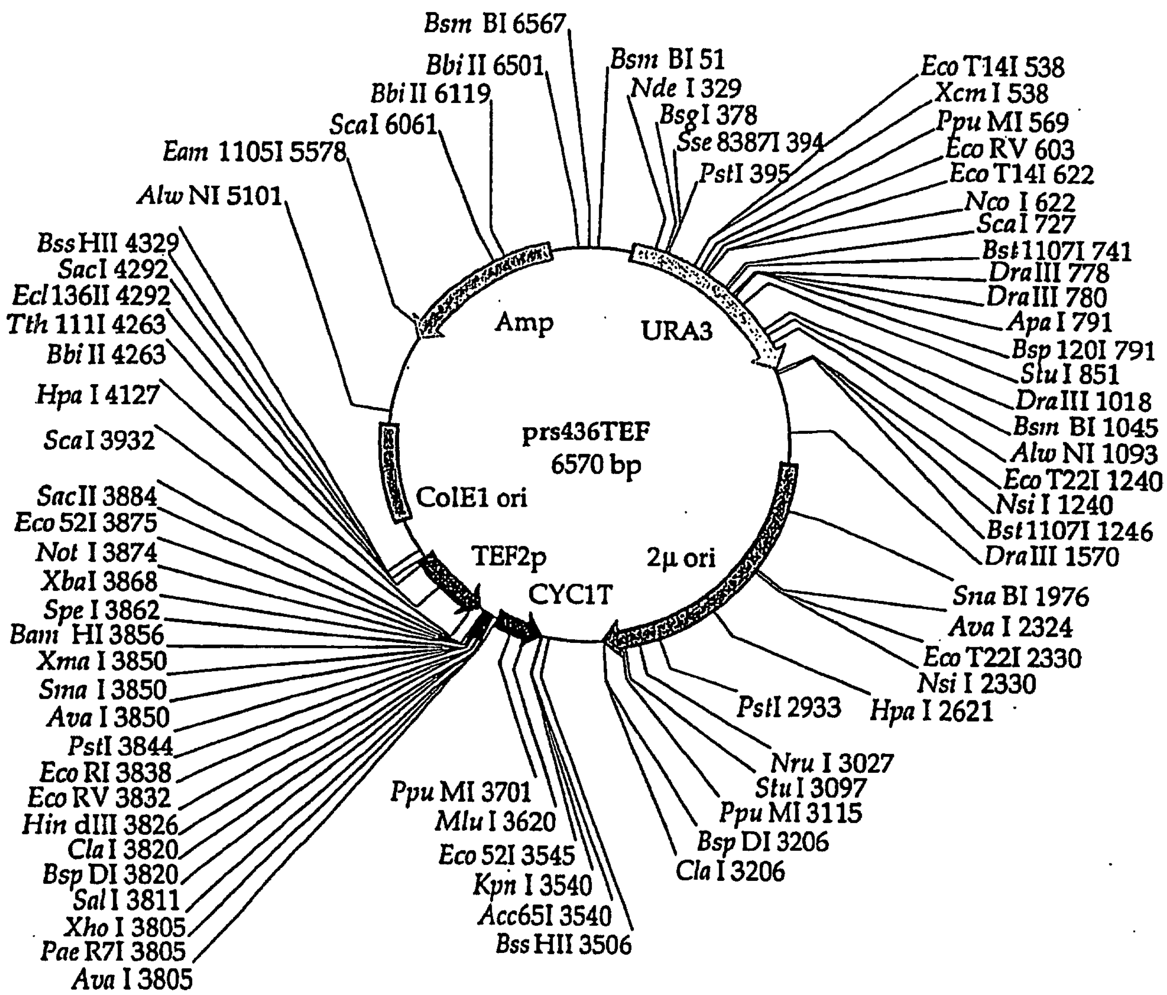
Bsm BI 6567
Bbi II 6501
Bsm BI 51
Bbi II 6119
Nde I 329
Sca I 6061
Bsg I 378
Eam 1105I 5578
Sse 8387I 394
Pst I 395
Alw NI 5101
Eco T14I 538
Xcm I 538
Ppu MI 569
Eco RV 603
Eco T14I 622
Nco I 622
Sca I 727
Bst 1107I 741
Dra III 778
Dra III 780
Apa I 791
Bsp 120I 791
Stu I 851
Dra III 1018
Bsm BI 1045
Alw NI 1093
Eco T22I 1240
Nsi I 1240
Bst 1107I 1246
Dra III 1570
Bss HII 4329
Sac I 4292
Ecl 136II 4292
Tth 111I 4263
Bbi II 4263
Hpa I 4127
Sca I 3932
Sac II 3884
Eco 52I 3875
Not I 3874
Xba I 3868
Spe I 3862
Bam HI 3856
Xma I 3850
Sma I 3850
Ava I 3850
Pst I 3844
Eco RI 3838
Eco RV 3832
Hin dIII 3826
Cla I 3820
Bsp DI 3820
Sal I 3811
Xho I 3805
Pae R7I 3805
Ava I 3805
Amp
URA3
prs436TEF
6570 bp
ColE1 ori
TEF2p
CYC1T
2μ ori
Sna BI 1976
Ava I 2324
Eco T22I 2330
Nsi I 2330
Pst I 2933
Hpa I 2621
Nru I 3027
Stu I 3097
Ppu MI 3115
Bsp DI 3206
Cla I 3206
Ppu MI 3701
Mlu I 3620
Eco 52I 3545
Kpn I 3540
Acc65I 3540
Bss HII 3506

Fig.7E-1

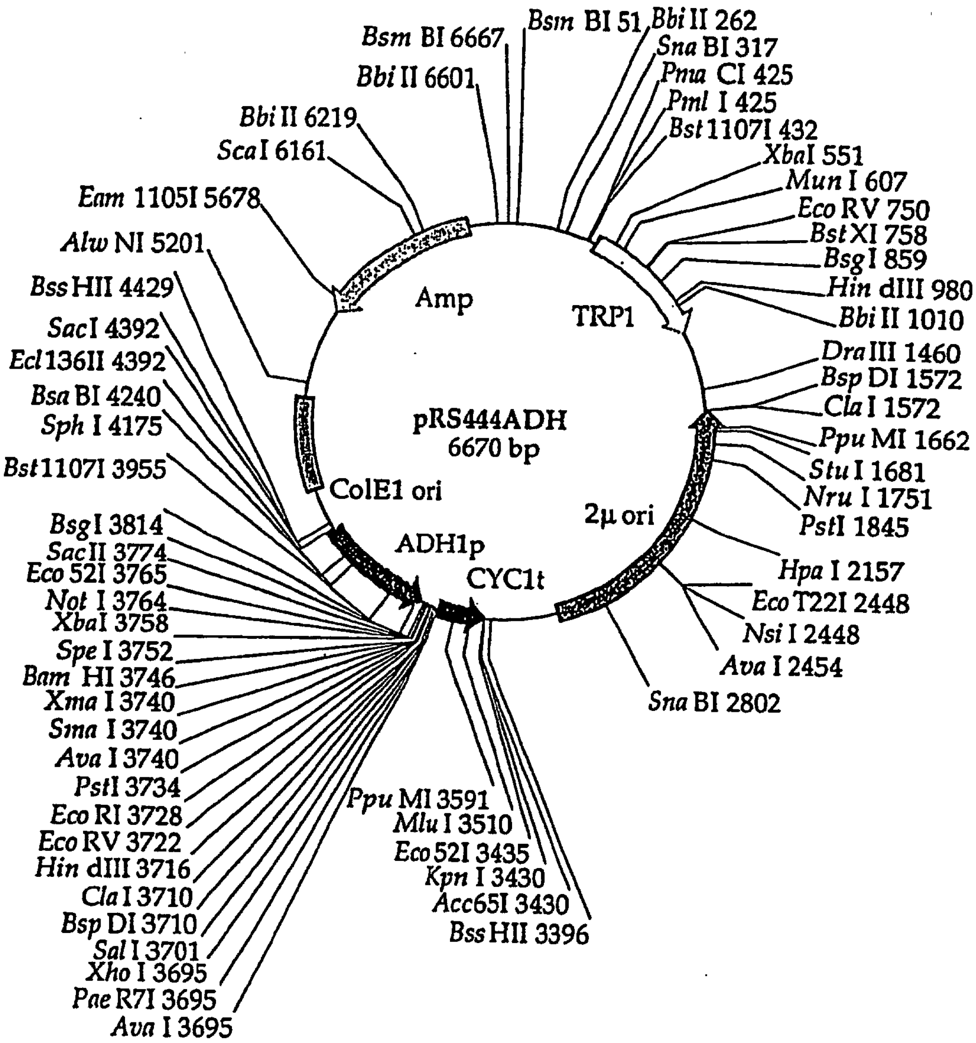
Bsm BI 6667
Bsm BI 51
Bbi II 262
Sna BI 317
Pma CI 425
Pml I 425
Bst1107I 432
Xba I 551
Mun I 607
Eco RV 750
Bst XI 758
Bsg I 859
Hin dIII 980
Bbi II 1010
Dra III 1460
Bsp DI 1572
Cla I 1572
Ppu MI 1662
Stu I 1681
Nru I 1751
Pst I 1845
Hpa I 2157
Eco T22I 2448
Nsi I 2448
Ava I 2454
Sna BI 2802
Bbi II 6601
Bbi II 6219
Sca I 6161
Eam 1105I 5678
Alw NI 5201
Bss HII 4429
Sac I 4392
Ecl136II 4392
Bsa BI 4240
Sph I 4175
Bst1107I 3955
Bsg I 3814
Sac II 3774
Eco 52I 3765
Not I 3764
Xba I 3758
Spe I 3752
Bam HI 3746
Xma I 3740
Sma I 3740
Ava I 3740
Pst I 3734
Eco RI 3728
Eco RV 3722
Hin dIII 3716
Cla I 3710
Bsp DI 3710
Sal I 3701
Xho I 3695
Pae R7I 3695
Ava I 3695
Ppu MI 3591
Mlu I 3510
Eco 52I 3435
Kpn I 3430
Acc65I 3430
Bss HII 3396
Amp
TRP1
pRS444ADH
6670 bp
ColE1 ori
ADH1p
CYC1t
2μ ori

Fig.7E-2

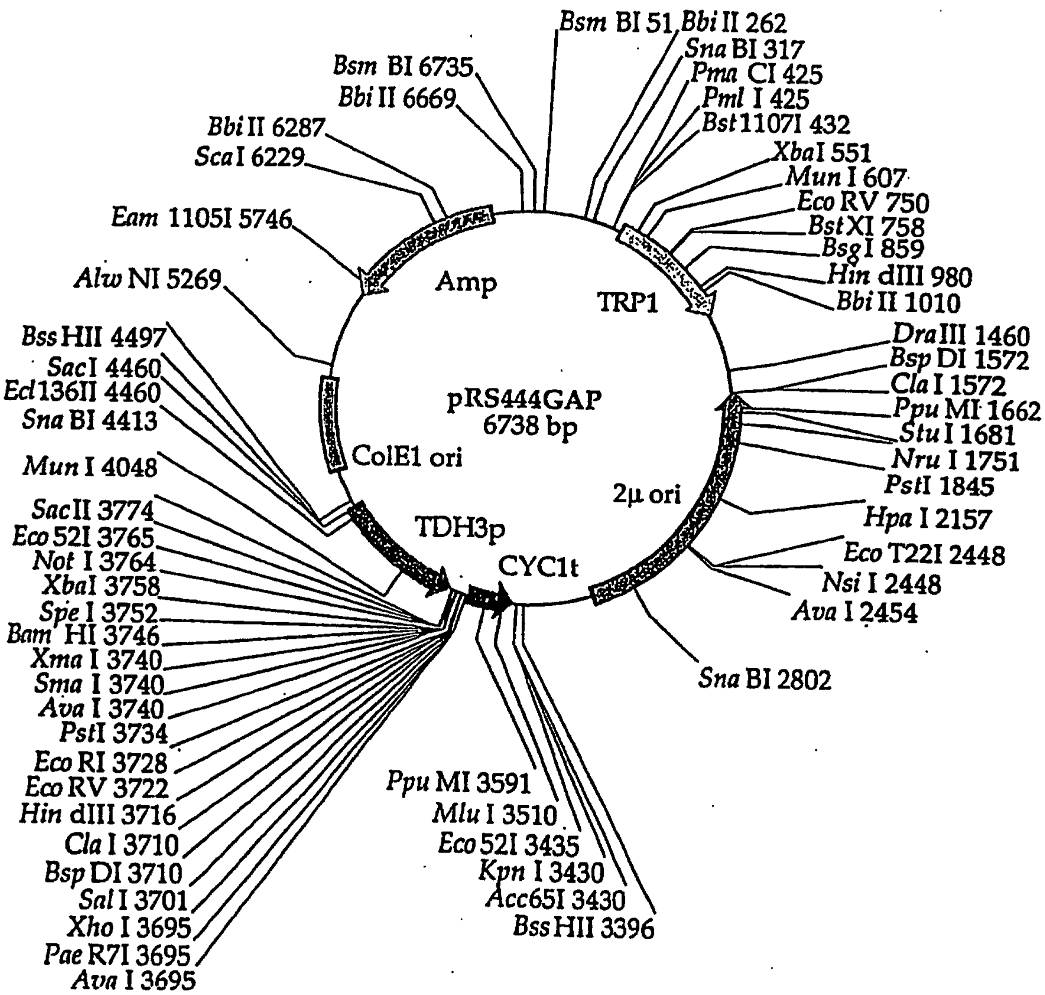
Bsm BI 51
Bbi II 262
Sna BI 317
Pma CI 425
Pml I 425
Bst1107I 432
Xba I 551
Mun I 607
Eco RV 750
Bst XI 758
Bsg I 859
Hin dIII 980
Bbi II 1010
Dra III 1460
Bsp DI 1572
Cla I 1572
Ppu MI 1662
Stu I 1681
Nru I 1751
Pst I 1845
Hpa I 2157
Eco T22I 2448
Nsi I 2448
Ava I 2454
Sna BI 2802
Ppu MI 3591
Mlu I 3510
Eco 52I 3435
Kpn I 3430
Acc65I 3430
Bss HII 3396
Sac II 3774
Eco 52I 3765
Not I 3764
Xba I 3758
Spe I 3752
Bam HI 3746
Xma I 3740
Sma I 3740
Ava I 3740
Pst I 3734
Eco RI 3728
Eco RV 3722
Hin dIII 3716
Cla I 3710
Bsp DI 3710
Sal I 3701
Xho I 3695
Pae R7I 3695
Ava I 3695
Mun I 4048
Bss HII 4497
Sac I 4460
Ecl136II 4460
Sna BI 4413
Alw NI 5269
Eam 1105I 5746
Sca I 6229
Bbi II 6287
Bbi II 6669
Bsm BI 6735
Amp
TRP1
pRS444GAP
6738 bp
ColE1 ori
2μ ori
TDH3p
CYC1t

Fig.7F-1

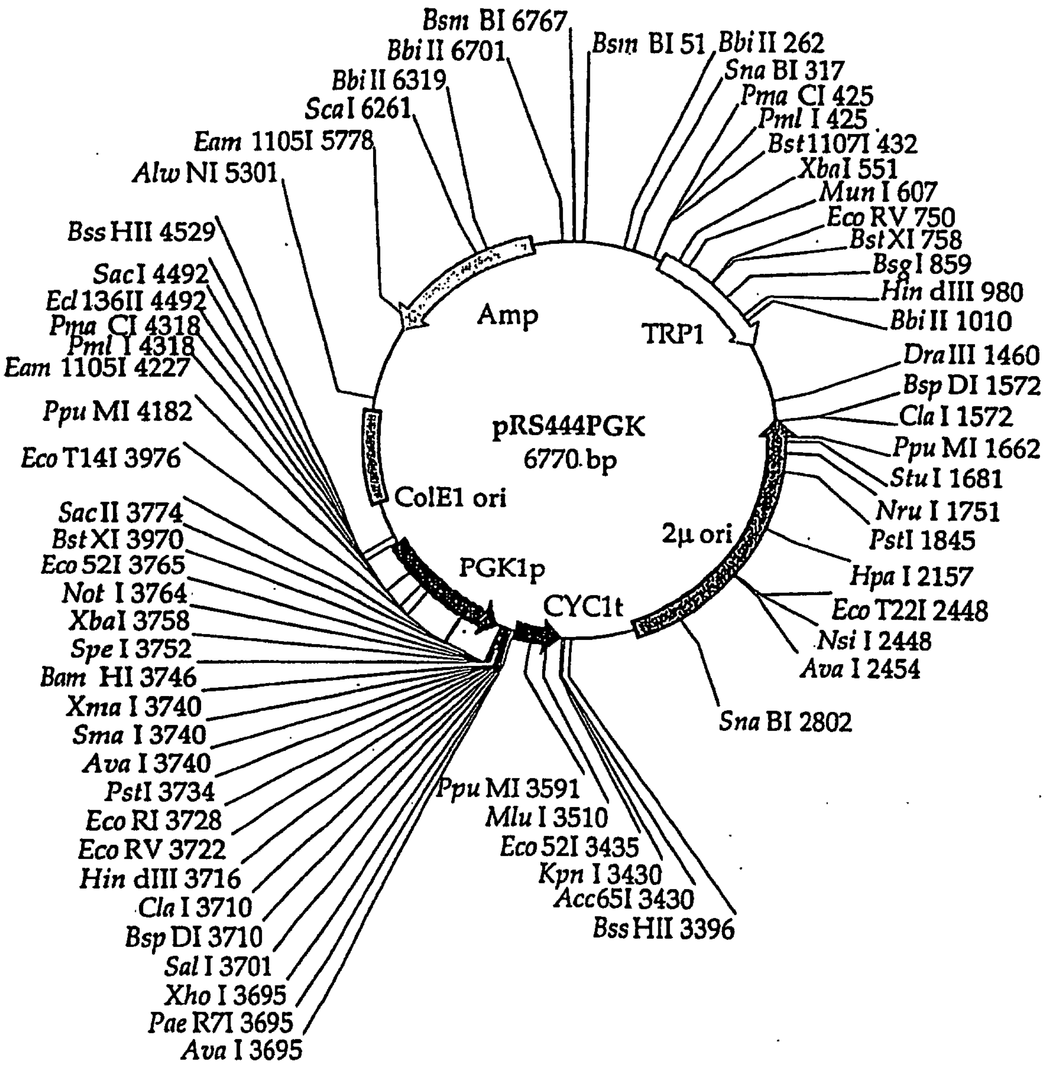
Bsm BI 6767
Bbi II 6701
Bbi II 6319
Sca I 6261
Eam 1105I 5778
Alw NI 5301
Bss HII 4529
Sac I 4492
Ecl 136II 4492
Pma CI 4318
Pml I 4318
Eam 1105I 4227
Ppu MI 4182
Eco T14I 3976
Sac II 3774
Bst XI 3970
Eco 52I 3765
Not I 3764
Xba I 3758
Spe I 3752
Bam HI 3746
Xma I 3740
Sma I 3740
Ava I 3740
Pst I 3734
Eco RI 3728
Eco RV 3722
Hin dIII 3716
Cla I 3710
Bsp DI 3710
Sal I 3701
Xho I 3695
Pae R7I 3695
Ava I 3695
Bsm BI 51
Bbi II 262
Sna BI 317
Pma CI 425
Pml I 425
Bst 1107I 432
Xba I 551
Mun I 607
Eco RV 750
Bst XI 758
Bsg I 859
Hin dIII 980
Bbi II 1010
Dra III 1460
Bsp DI 1572
Cla I 1572
Ppu MI 1662
Stu I 1681
Nru I 1751
Pst I 1845
Hpa I 2157
Eco T22I 2448
Nsi I 2448
Ava I 2454
Sna BI 2802
Ppu MI 3591
Mlu I 3510
Eco 52I 3435
Kpn I 3430
Acc65I 3430
Bss HII 3396
Amp
TRP1
pRS444PGK
6770 bp
ColE1 ori
2μ ori
PGK1p
CYC1t

Fig.7F-2

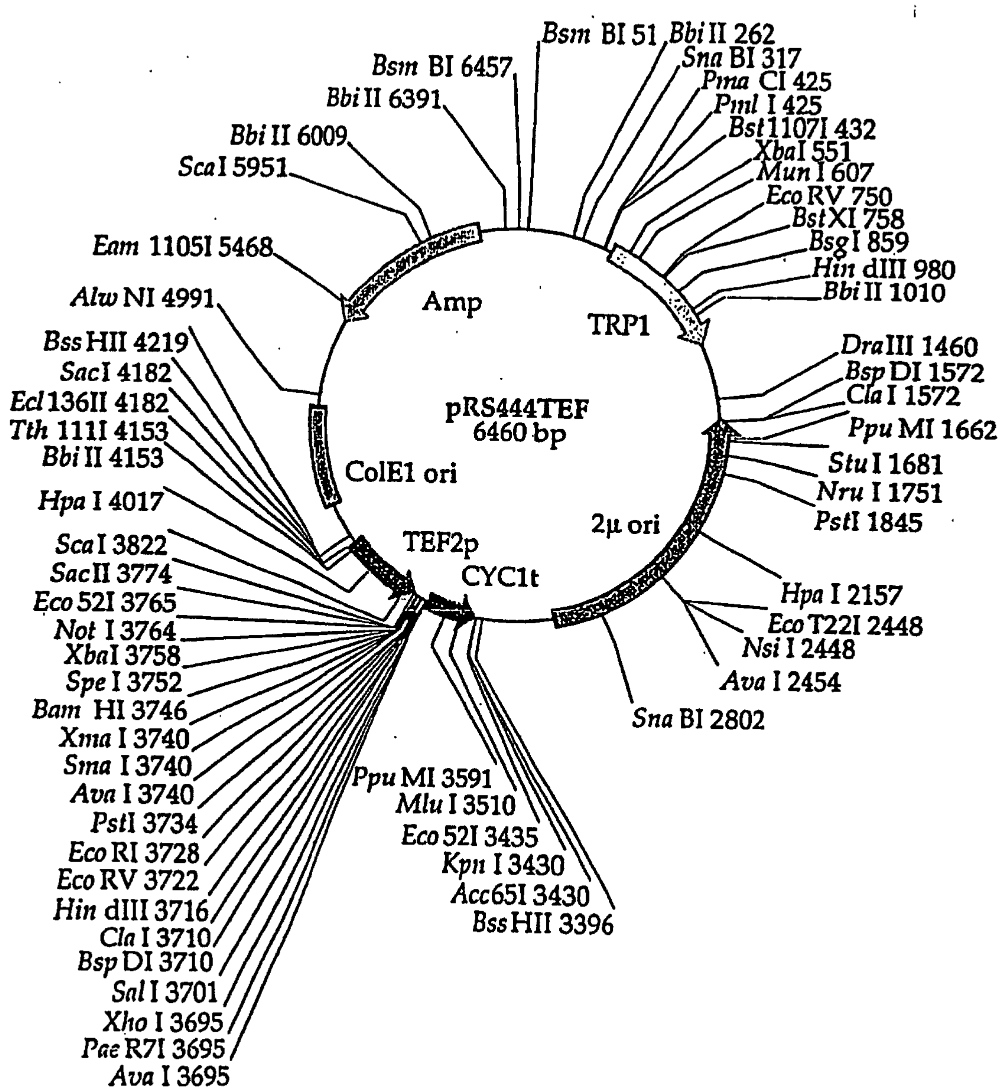

Bsm BI 51
Bbi II 262
Sna BI 317
Pma CI 425
Pml I 425
Bst1107I 432
XbaI 551
Mun I 607
Eco RV 750
BstXI 758
BsgI 859
Hin dIII 980
Bbi II 1010
DraIII 1460
Bsp DI 1572
Cla I 1572
Ppu MI 1662
Stu I 1681
Nru I 1751
PstI 1845
Hpa I 2157
Eco T22I 2448
Nsi I 2448
Ava I 2454
Sna BI 2802
Ppu MI 3591
Mlu I 3510
Eco 52I 3435
Kpn I 3430
Acc65I 3430
Bss HII 3396
Bsm BI 6457
Bbi II 6391
Bbi II 6009
Sca I 5951
Eam 1105I 5468
Alw NI 4991
Bss HII 4219
SacI 4182
Ecl136II 4182
Tth 111I 4153
Bbi II 4153
Hpa I 4017
Sca I 3822
SacII 3774
Eco 52I 3765
Not I 3764
XbaI 3758
Spe I 3752
Bam HI 3746
Xma I 3740
Sma I 3740
Ava I 3740
PstI 3734
Eco RI 3728
Eco RV 3722
Hin dIII 3716
Cla I 3710
Bsp DI 3710
Sal I 3701
Xho I 3695
Pae R7I 3695
Ava I 3695
Amp
TRP1
pRS444TEF
6460 bp
ColE1 ori
2μ ori
TEF2p
CYC1t

Fig.7G-1

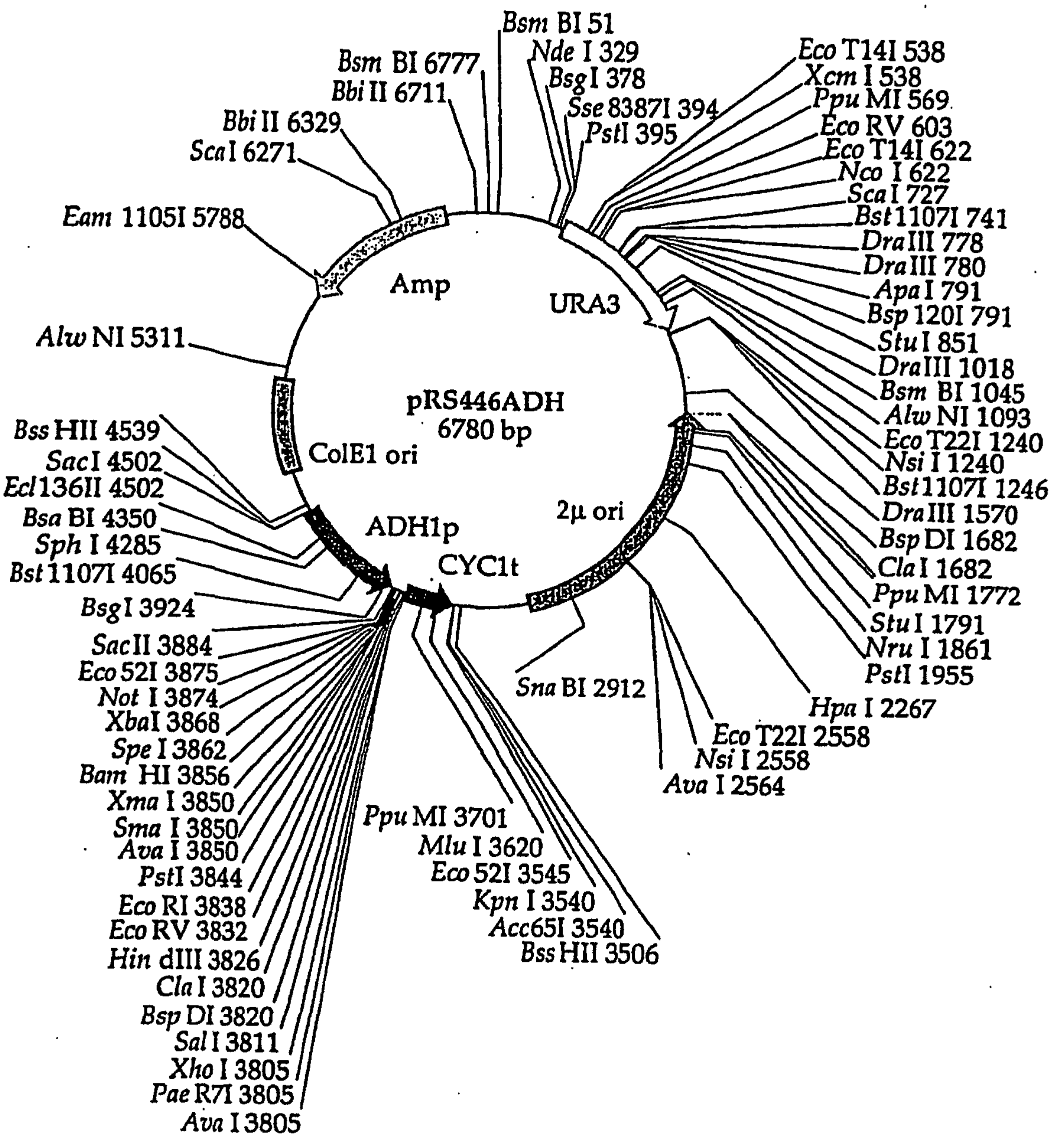
pRS446ADH
6780 bp
Amp
URA3
ColE1 ori
ADH1p
CYC1t
2μ ori
Bsm BI 51
Nde I 329
Bsg I 378
Sse 8387I 394
Pst I 395
Eco T14I 538
Xcm I 538
Ppu MI 569
Eco RV 603
Eco T14I 622
Nco I 622
Sca I 727
Bst 1107I 741
Dra III 778
Dra III 780
Apa I 791
Bsp 120I 791
Stu I 851
Dra III 1018
Bsm BI 1045
Alw NI 1093
Eco T22I 1240
Nsi I 1240
Bst 1107I 1246
Dra III 1570
Bsp DI 1682
Cla I 1682
Ppu MI 1772
Stu I 1791
Nru I 1861
Pst I 1955
Hpa I 2267
Eco T22I 2558
Nsi I 2558
Ava I 2564
Sna BI 2912
Ppu MI 3701
Mlu I 3620
Eco 52I 3545
Kpn I 3540
Acc65I 3540
Bss HII 3506
Bsm BI 6777
Bbi II 6711
Bbi II 6329
Sca I 6271
Eam 1105I 5788
Alw NI 5311
Bss HII 4539
Sac I 4502
Ecl136II 4502
Bsa BI 4350
Sph I 4285
Bst 1107I 4065
Bsg I 3924
Sac II 3884
Eco 52I 3875
Not I 3874
Xba I 3868
Spe I 3862
Bam HI 3856
Xma I 3850
Sma I 3850
Ava I 3850
Pst I 3844
Eco RI 3838
Eco RV 3832
Hin dIII 3826
Cla I 3820
Bsp DI 3820
Sal I 3811
Xho I 3805
Pae R7I 3805
Ava I 3805

Fig.7G-2

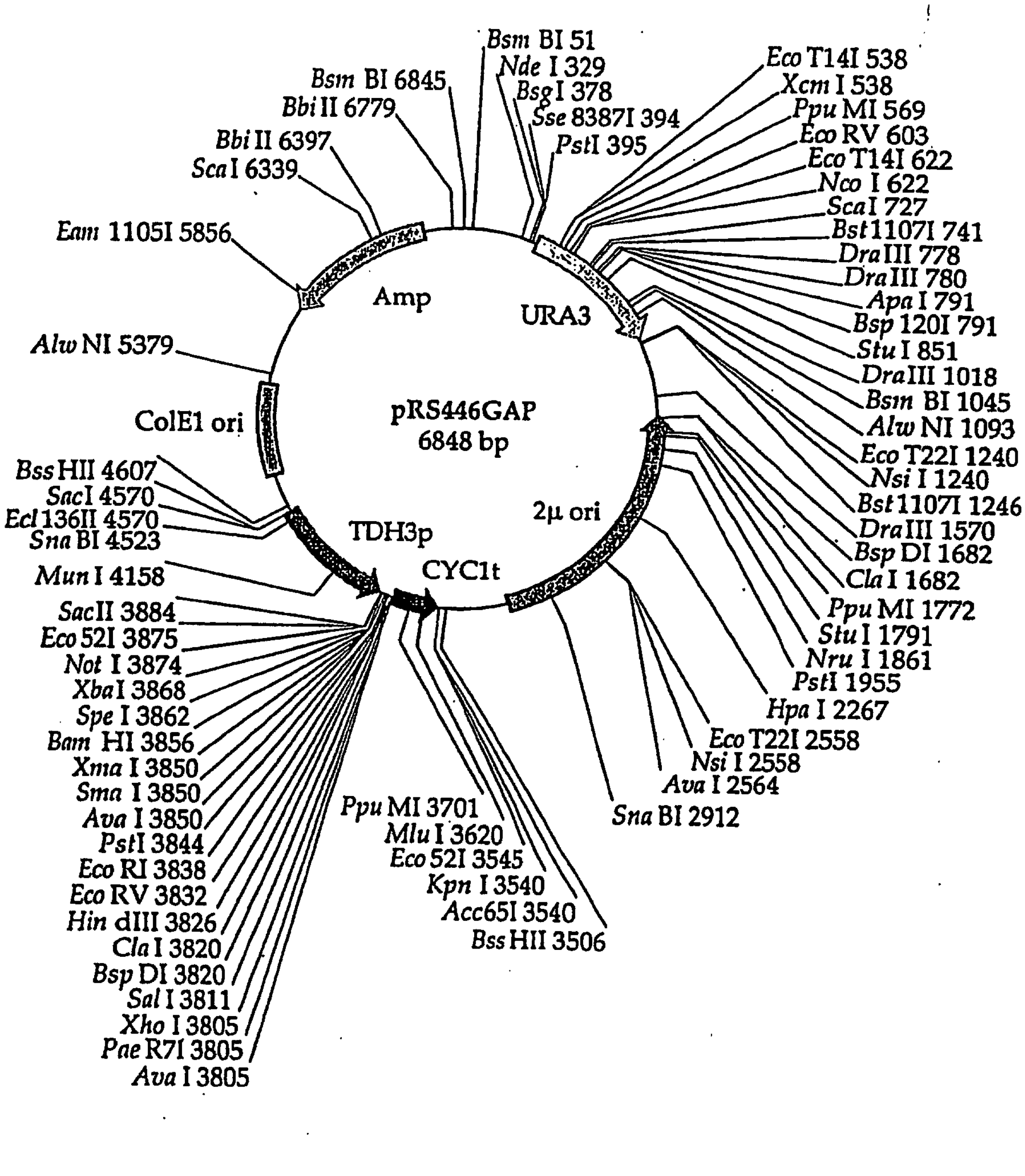
pRS446GAP
6848 bp
Amp
URA3
ColE1 ori
2μ ori
TDH3p
CYC1t
Bsm BI 51
Nde I 329
Bsg I 378
Sse 8387I 394
PstI 395
Eco T14I 538
Xcm I 538
Ppu MI 569
Eco RV 603
Eco T14I 622
Nco I 622
Sca I 727
Bst 1107I 741
Dra III 778
Dra III 780
Apa I 791
Bsp 120I 791
Stu I 851
Dra III 1018
Bsm BI 1045
Alw NI 1093
Eco T22I 1240
Nsi I 1240
Bst 1107I 1246
Dra III 1570
Bsp DI 1682
Cla I 1682
Ppu MI 1772
Stu I 1791
Nru I 1861
PstI 1955
Hpa I 2267
Eco T22I 2558
Nsi I 2558
Ava I 2564
Sna BI 2912
Ppu MI 3701
Mlu I 3620
Eco 52I 3545
Kpn I 3540
Acc65I 3540
Bss HII 3506
SacII 3884
Eco 52I 3875
Not I 3874
Xba I 3868
Spe I 3862
Bam HI 3856
Xma I 3850
Sma I 3850
Ava I 3850
PstI 3844
Eco RI 3838
Eco RV 3832
Hin dIII 3826
Cla I 3820
Bsp DI 3820
Sal I 3811
Xho I 3805
Pae R7I 3805
Ava I 3805
Mun I 4158
Bss HII 4607
SacI 4570
Ecl 136II 4570
Sna BI 4523
Alw NI 5379
Eam 1105I 5856
Sca I 6339
Bbi II 6397
Bbi II 6779
Bsm BI 6845

Fig.7H-1

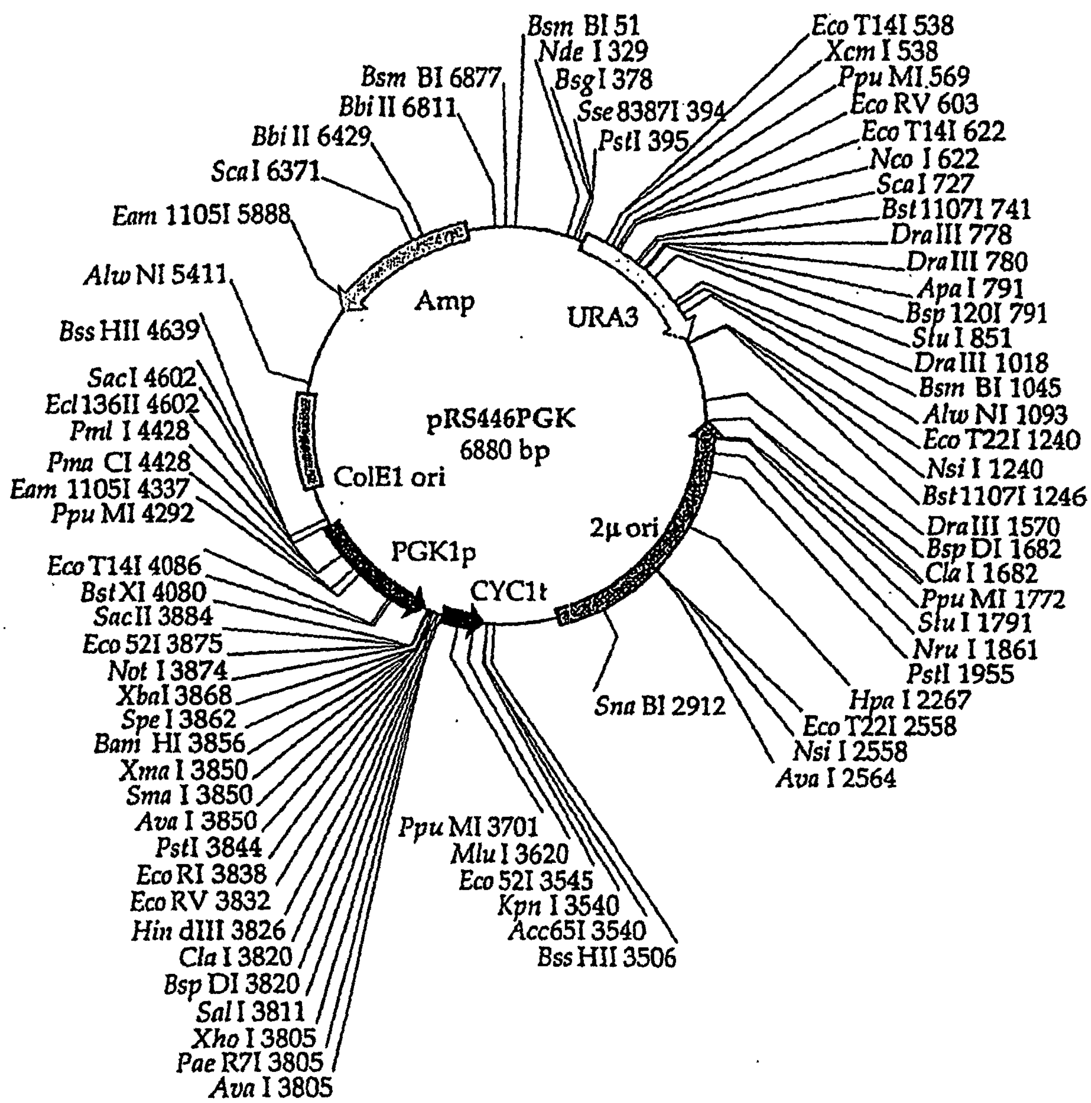
Bsm BI 51
Nde I 329
Bsg I 378
Sse 8387I 394
PstI 395
Eco T14I 538
Xcm I 538
Ppu MI 569
Eco RV 603
Eco T14I 622
Nco I 622
Sca I 727
Bst 1107I 741
Dra III 778
Dra III 780
Apa I 791
Bsp 120I 791
Stu I 851
Dra III 1018
Bsm BI 1045
Alw NI 1093
Eco T22I 1240
Nsi I 1240
Bst 1107I 1246
Dra III 1570
Bsp DI 1682
Cla I 1682
Ppu MI 1772
Stu I 1791
Nru I 1861
PstI 1955
Hpa I 2267
Eco T22I 2558
Nsi I 2558
Ava I 2564
Sna BI 2912
Bsm BI 6877
Bbi II 6811
Bbi II 6429
Sca I 6371
Eam 1105I 5888
Alw NI 5411
Bss HII 4639
Sac I 4602
Ecl 136II 4602
Pml I 4428
Pma CI 4428
Eam 1105I 4337
Ppu MI 4292
Eco T14I 4086
Bst XI 4080
Sac II 3884
Eco 52I 3875
Not I 3874
Xba I 3868
Spe I 3862
Bam HI 3856
Xma I 3850
Sma I 3850
Ava I 3850
PstI 3844
Eco RI 3838
Eco RV 3832
Hin dIII 3826
Cla I 3820
Bsp DI 3820
Sal I 3811
Xho I 3805
Pae R7I 3805
Ava I 3805
Ppu MI 3701
Mlu I 3620
Eco 52I 3545
Kpn I 3540
Acc65I 3540
Bss HII 3506
Amp
URA3
pRS446PGK
6880 bp
ColE1 ori
2μ ori
PGK1p
CYC1t

Fig.7H-2

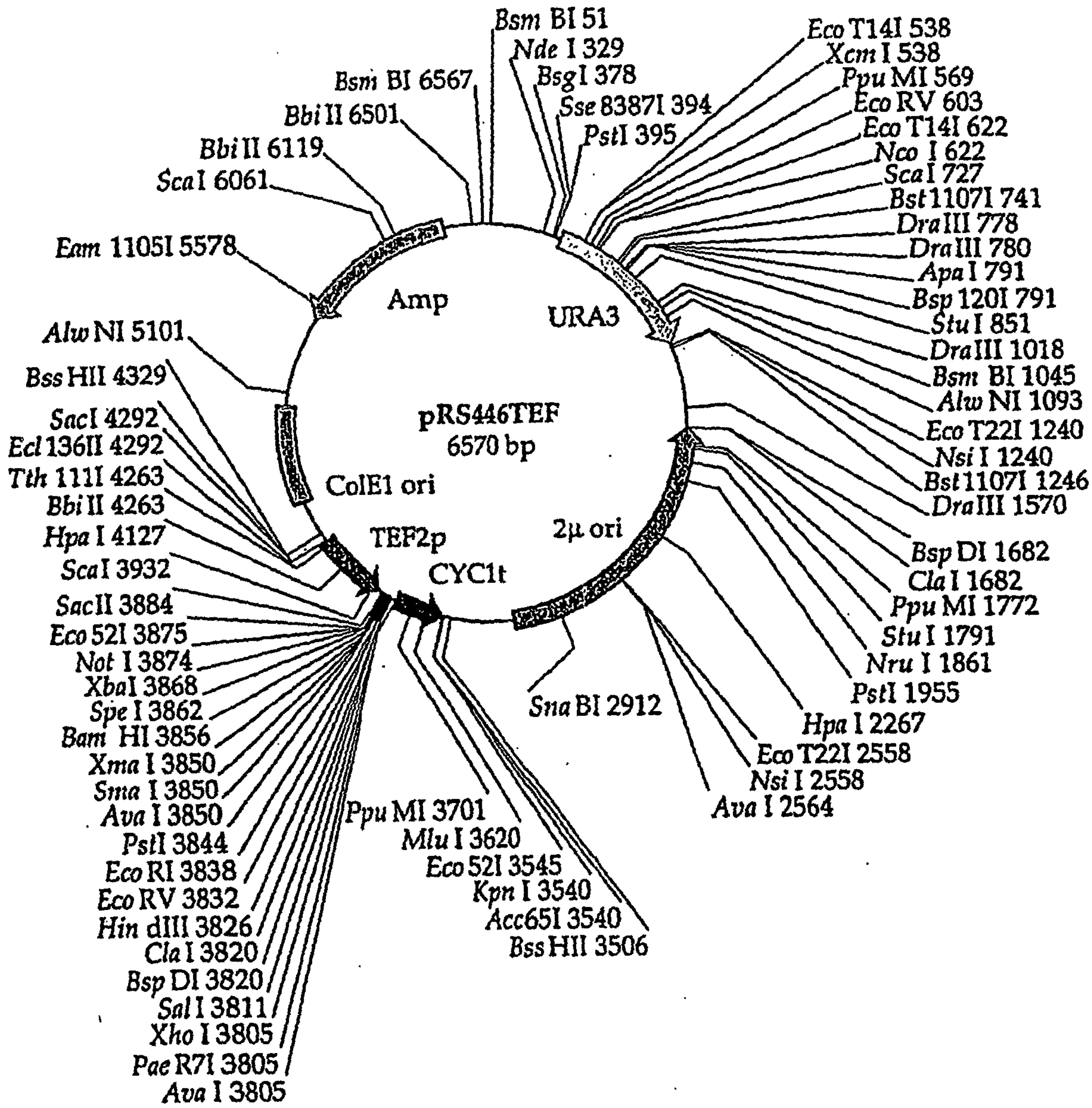

pRS446TEF
6570 bp
Amp
URA3
ColE1 ori
TEF2p
CYC1t
2μ ori
Bsm BI 51
Nde I 329
Bsg I 378
Sse 8387I 394
PstI 395
Eco T14I 538
Xcm I 538
Ppu MI 569
Eco RV 603
Eco T14I 622
Nco I 622
Sca I 727
Bst 1107I 741
Dra III 778
Dra III 780
Apa I 791
Bsp 120I 791
Stu I 851
Dra III 1018
Bsm BI 1045
Alw NI 1093
Eco T22I 1240
Nsi I 1240
Bst 1107I 1246
Dra III 1570
Bsp DI 1682
Cla I 1682
Ppu MI 1772
Stu I 1791
Nru I 1861
PstI 1955
Hpa I 2267
Eco T22I 2558
Nsi I 2558
Ava I 2564
Sna BI 2912
Ppu MI 3701
Mlu I 3620
Eco 52I 3545
Kpn I 3540
Acc65I 3540
Bss HII 3506
Bsm BI 6567
Bbi II 6501
Bbi II 6119
Sca I 6061
Eam 1105I 5578
Alw NI 5101
Bss HII 4329
SacI 4292
Ecl 136II 4292
Tth 111I 4263
Bbi II 4263
Hpa I 4127
Sca I 3932
SacII 3884
Eco 52I 3875
Not I 3874
XbaI 3868
Spe I 3862
Bam HI 3856
Xma I 3850
Sma I 3850
Ava I 3850
PstI 3844
Eco RI 3838
Eco RV 3832
Hin dIII 3826
Cla I 3820
Bsp DI 3820
Sal I 3811
Xho I 3805
Pae R7I 3805
Ava I 3805

Fig.8

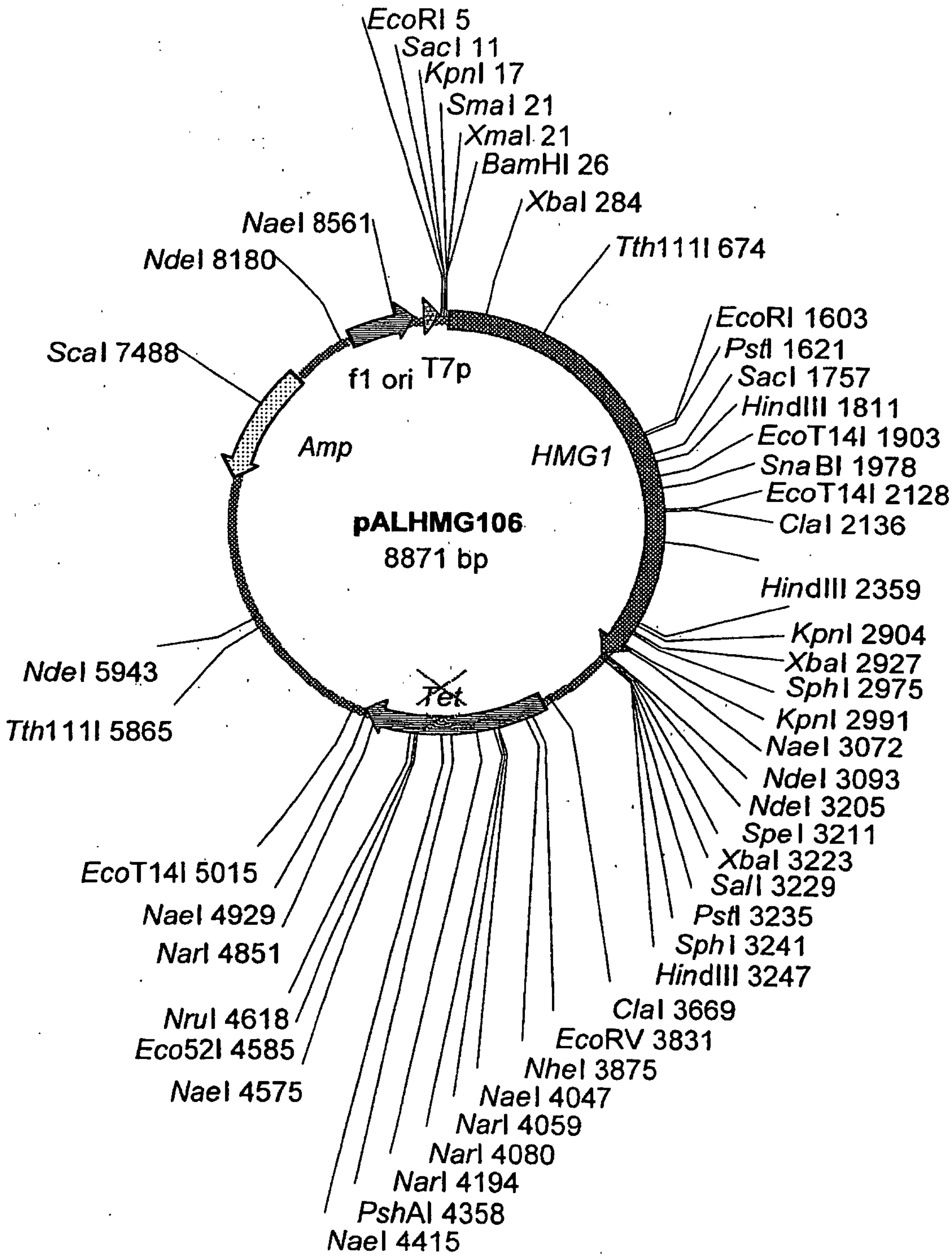
EcoRI 5
SacI 11
KpnI 17
SmaI 21
XmaI 21
BamHI 26
XbaI 284
Tth111I 674
NaeI 8561
NdeI 8180
ScaI 7488
f1 ori
T7p
Amp
HMG1
pALHMG106
8871 bp
EcoRI 1603
PstI 1621
SacI 1757
HindIII 1811
EcoT14I 1903
SnaBI 1978
EcoT14I 2128
ClaI 2136
HindIII 2359
KpnI 2904
XbaI 2927
SphI 2975
KpnI 2991
NaeI 3072
NdeI 3093
NdeI 3205
SpeI 3211
XbaI 3223
SalI 3229
PstI 3235
SphI 3241
HindIII 3247
ClaI 3669
EcoRV 3831
NheI 3875
NaeI 4047
NarI 4059
NarI 4080
NarI 4194
PshAI 4358
NaeI 4415
NaeI 4575
Eco52I 4585
NruI 4618
NarI 4851
NaeI 4929
EcoT14I 5015
Tth111I 5865
NdeI 5943
Tet

Fig.9

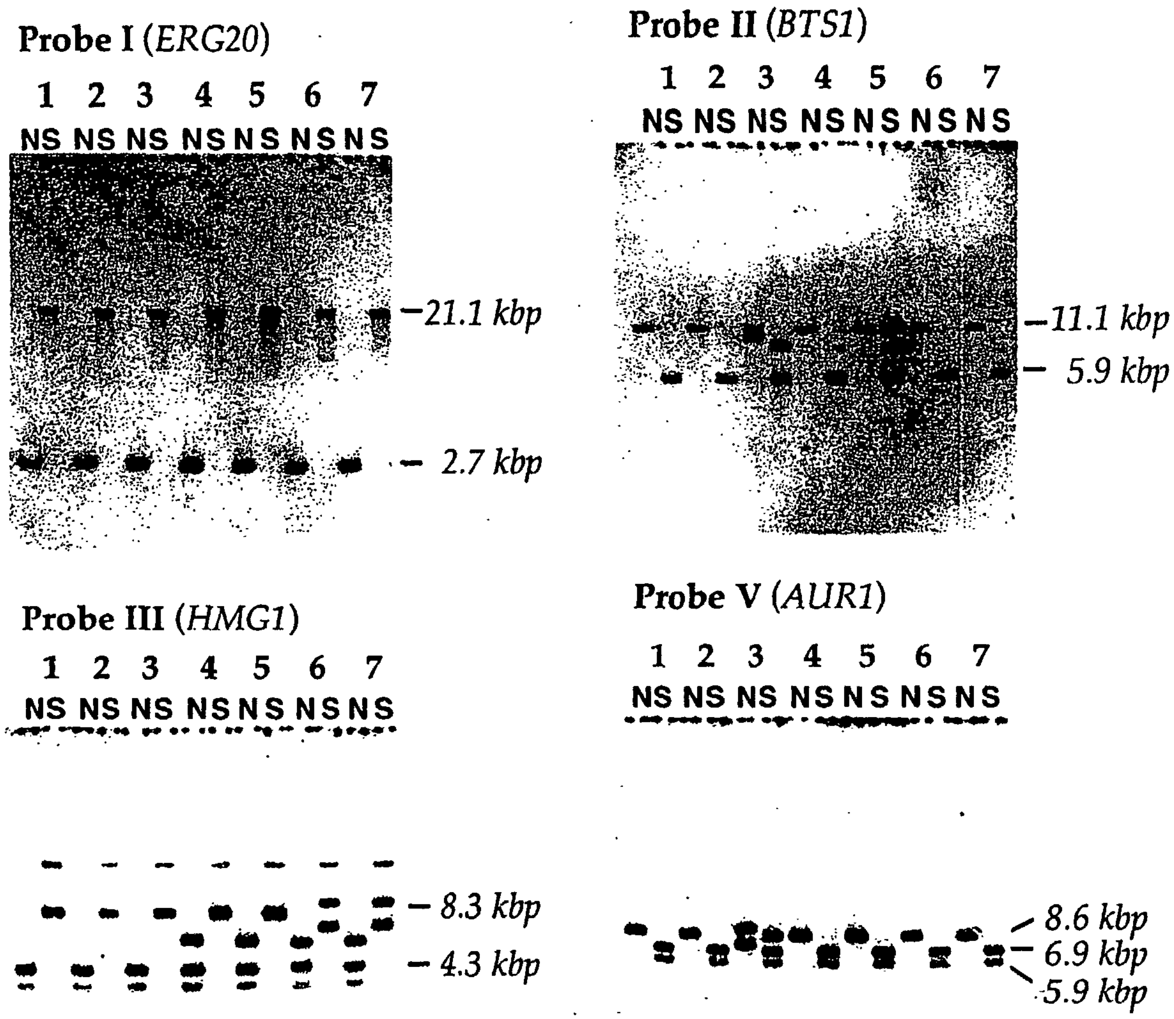
Probe I (ERG20)
1 2 3 4 5 6 7
NS NS NS NS NS NS NS
21.1 kbp
2.7 kbp
Probe II (BTS1)
1 2 3 4 5 6 7
NS NS NS NS NS NS NS
11.1 kbp
5.9 kbp
Probe III (HMG1)
1 2 3 4 5 6 7
NS NS NS NS NS NS NS
8.3 kbp
4.3 kbp
Probe V (AUR1)
1 2 3 4 5 6 7
NS NS NS NS NS NS NS
8.6 kbp
6.9 kbp
5.9 kbp

Fig.10

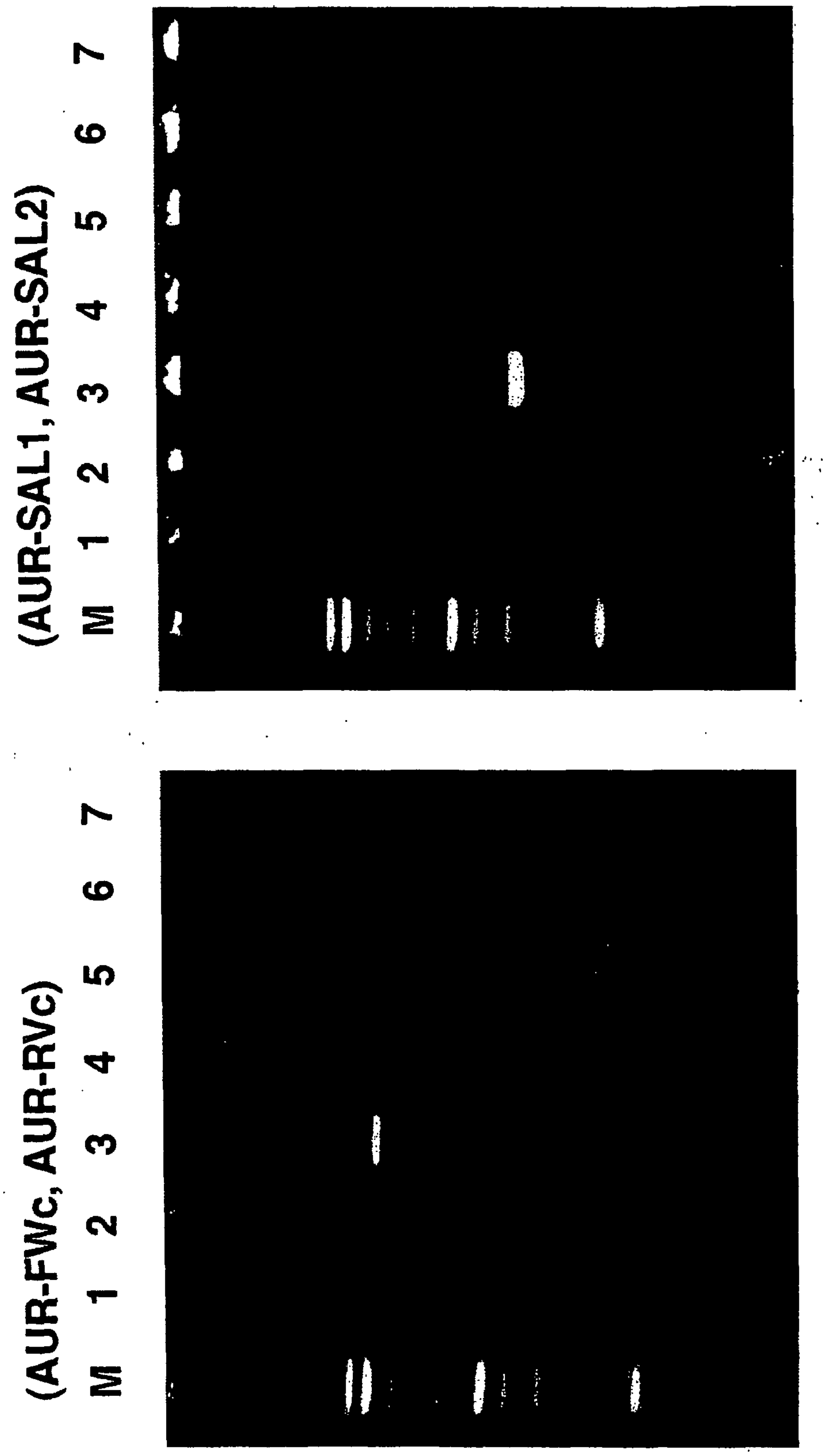

Fig.11

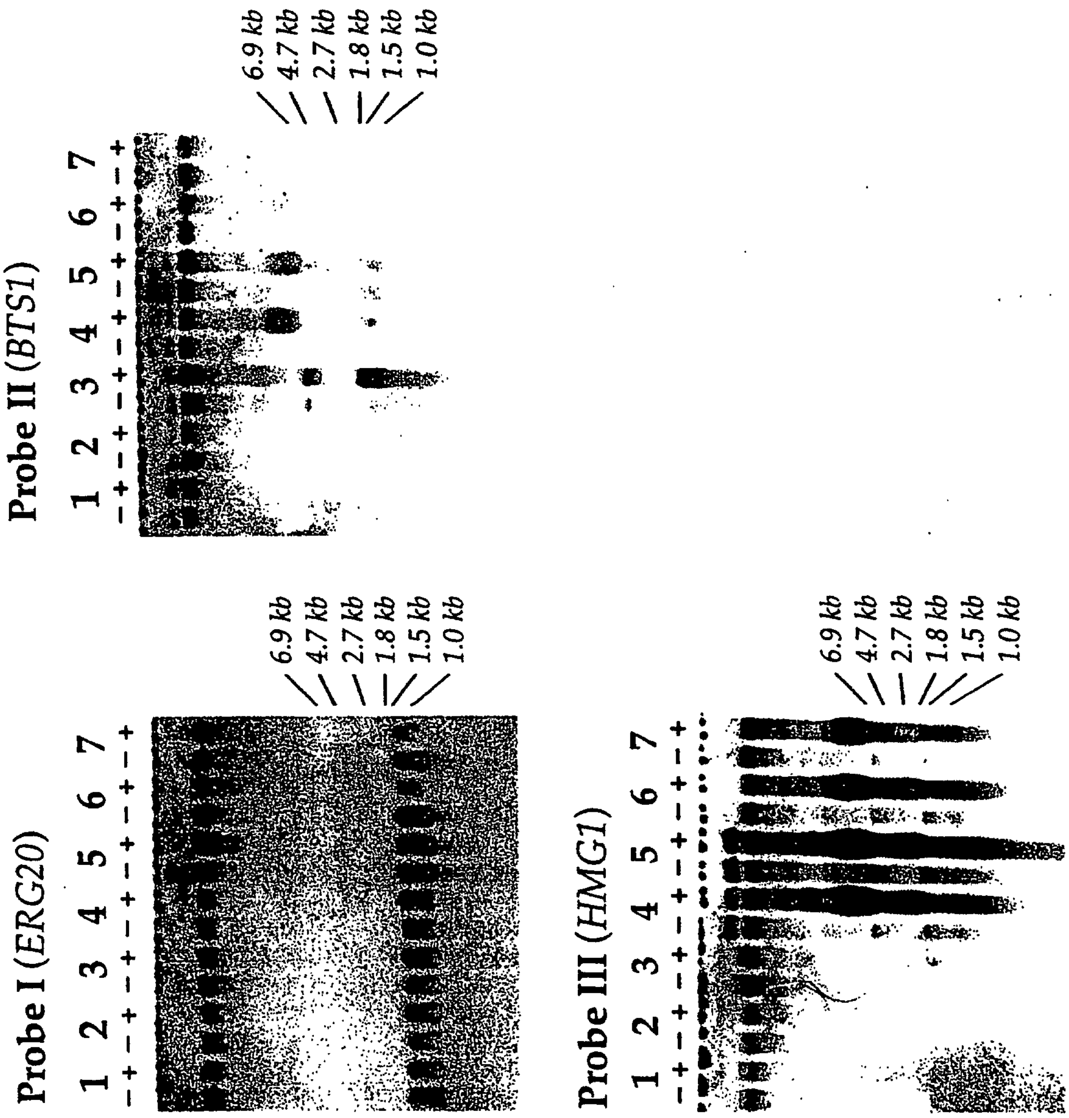
Probe I (ERG20)
Probe II (BTS1)
Probe III (HMG1)
1 2 3 4 5 6 7
6.9 kb
4.7 kb
2.7 kb
1.8 kb
1.5 kb
1.0 kb

Fig.12A

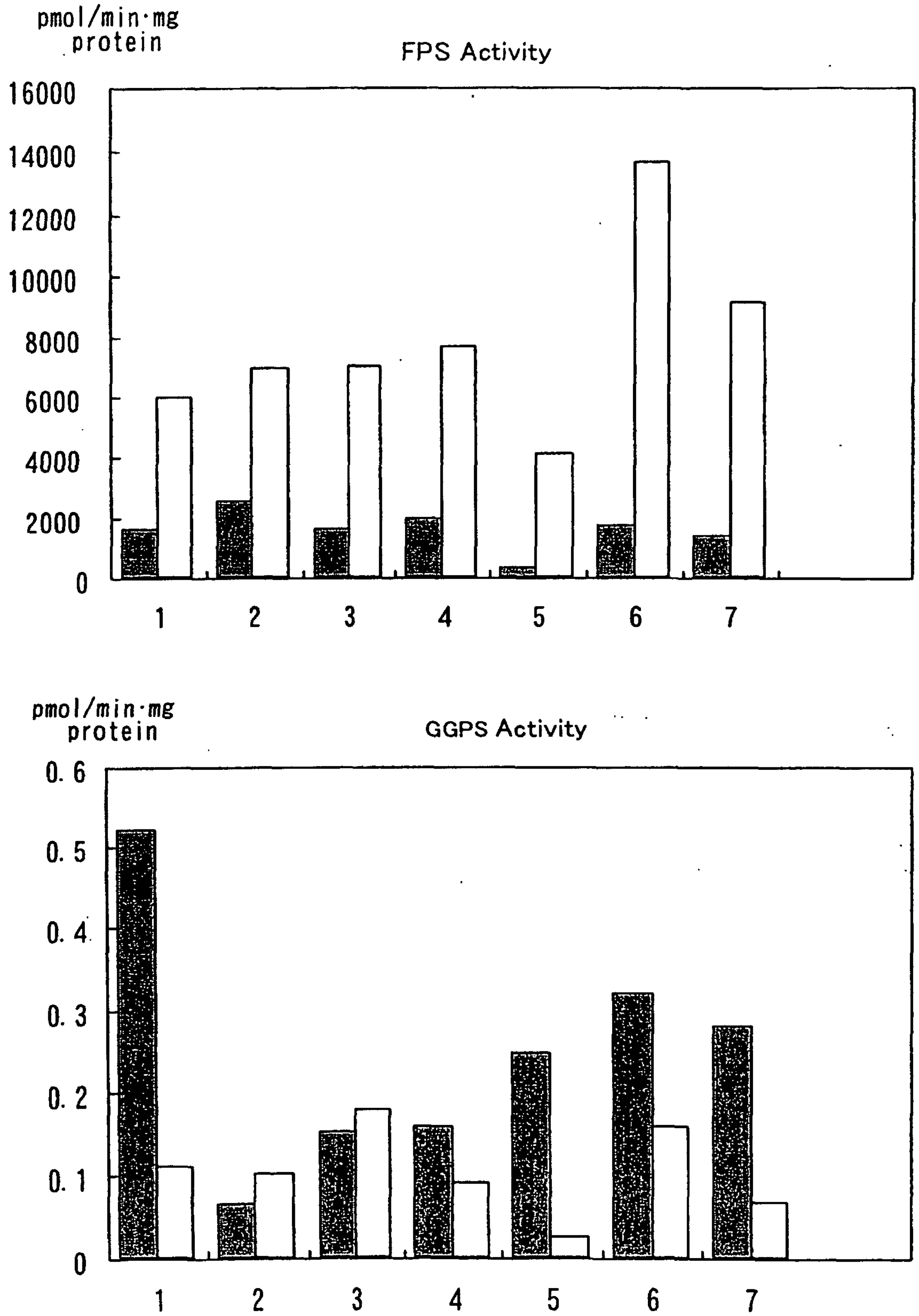
pmol/min·mg
protein
FPS Activity
16000
14000
12000
10000
8000
6000
4000
2000
0
1
2
3
4
5
6
7
pmol/min·mg
protein
GGPS Activity
0. 6
0. 5
0. 4
0. 3
0. 2
0. 1
0
1
2
3
4
5
6
7

# Fig.12B

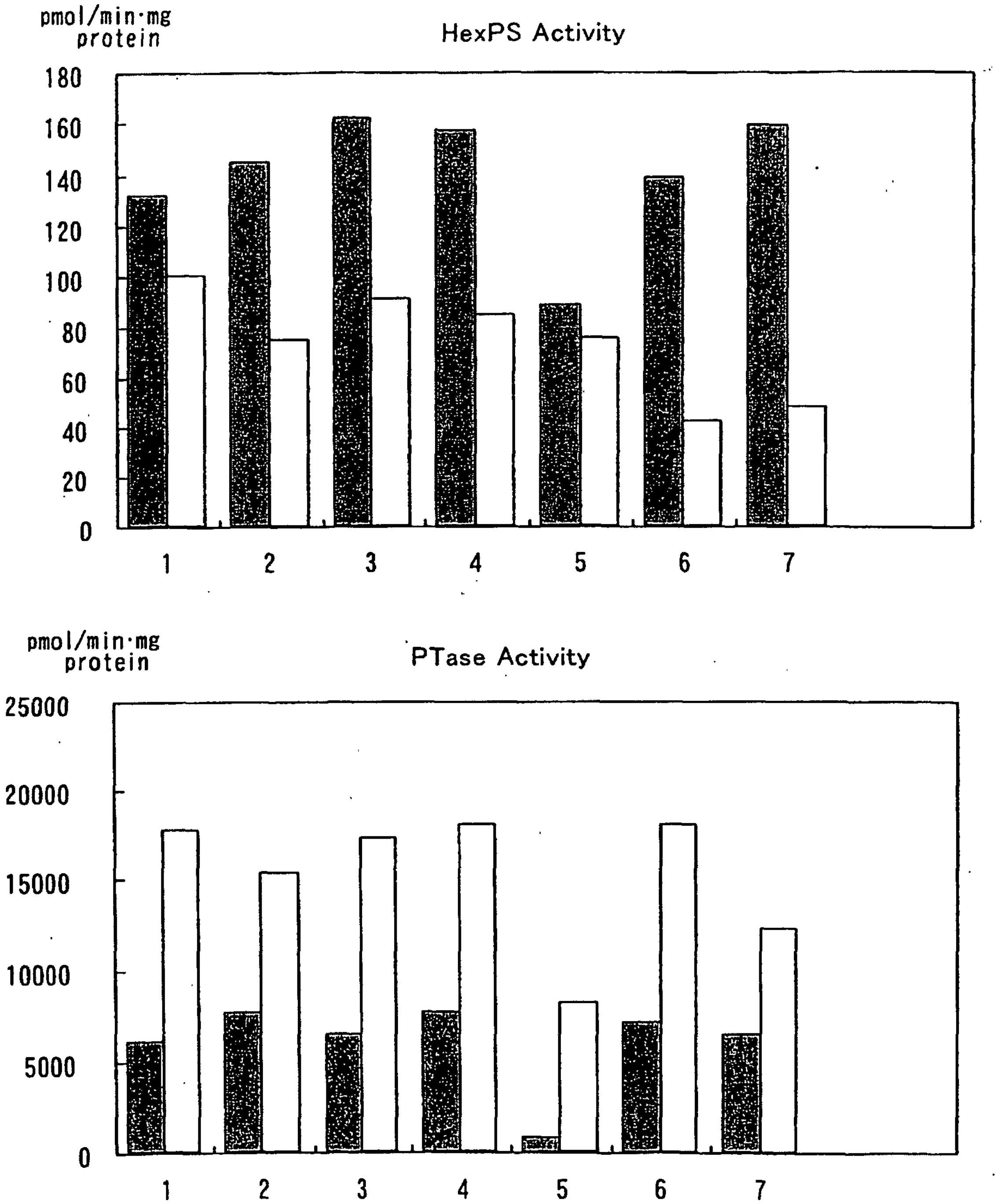

pmol/min·mg protein
HexPS Activity
180
160
140
120
100
80
60
40
20
0
1
2
3
4
5
6
7
pmol/min·mg protein
PTase Activity
25000
20000
15000
10000
5000
0
1
2
3
4
5
6
7

Fig.13

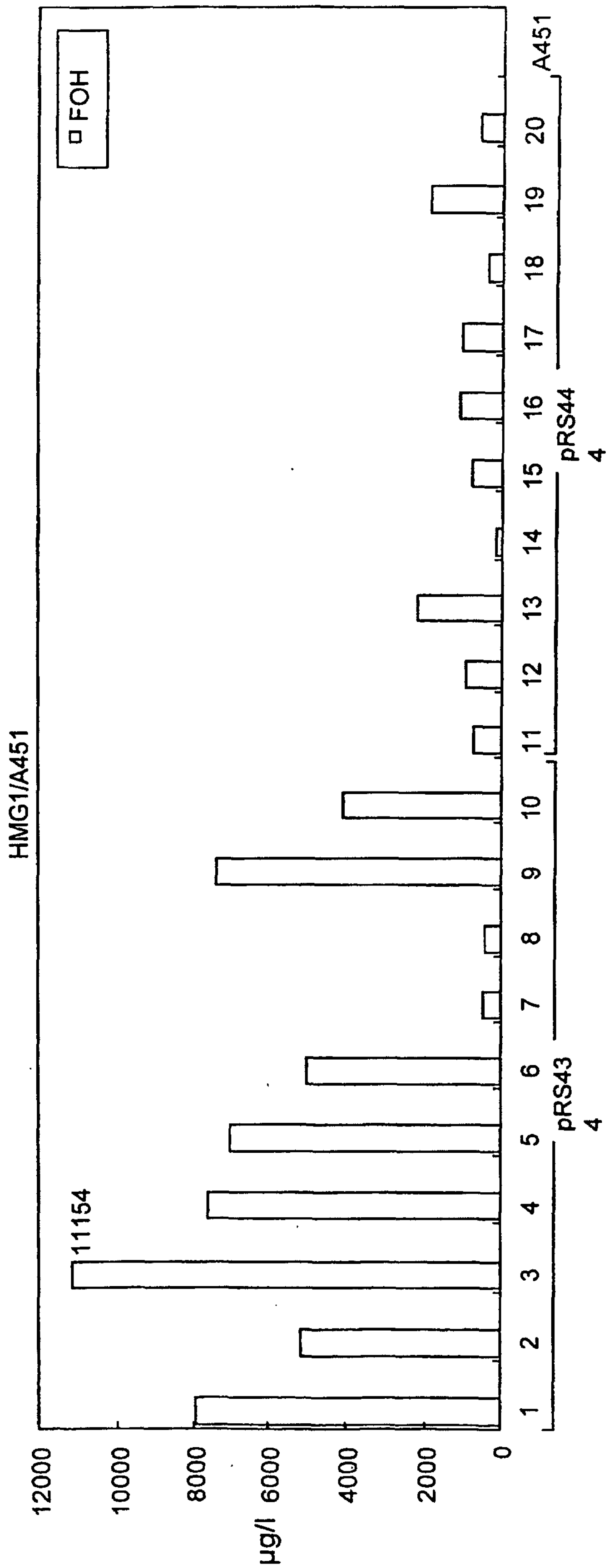
HMG1/A451
FOH
11154
μg/l
12000
10000
8000
6000
4000
2000
0
1
2
3
4
5
6
7
8
9
10
11
12
13
14
15
16
17
18
19
20
pRS43
4
pRS44
4
A451

Fig.14

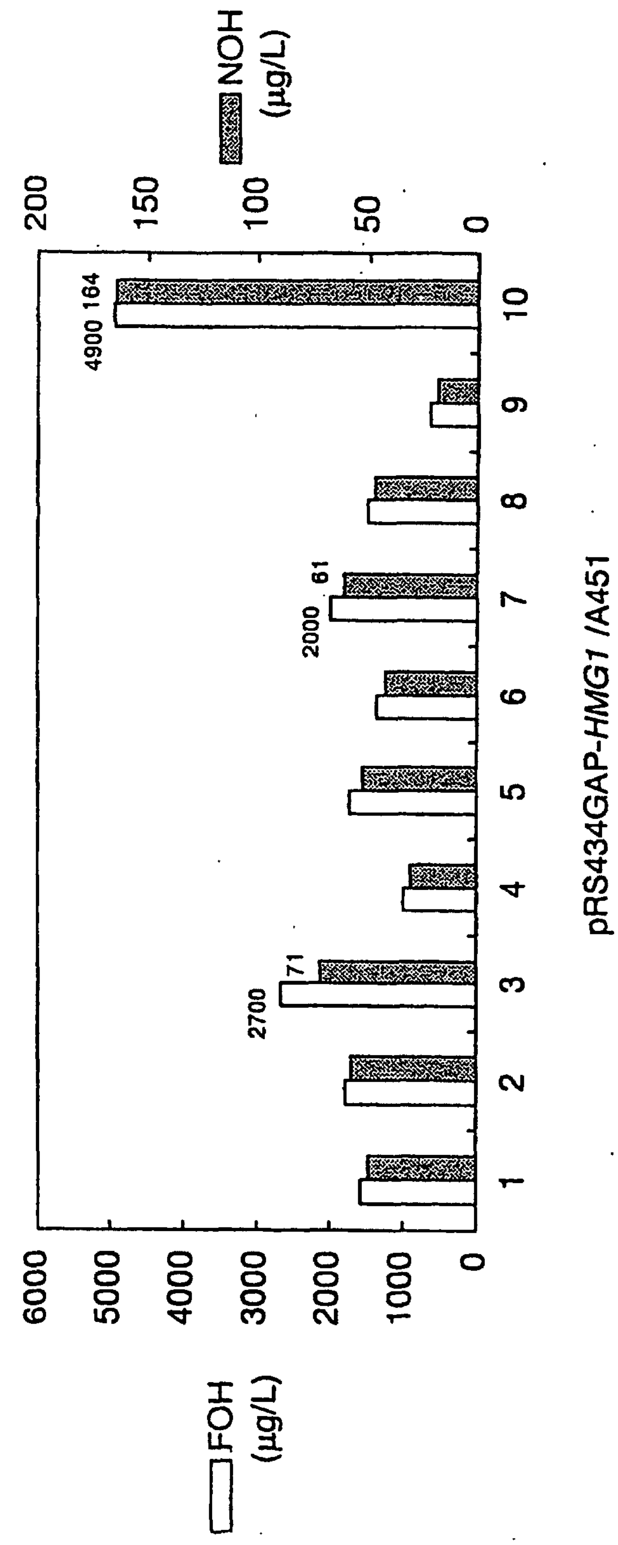
FOH
(μg/L)
NOH
(μg/L)
6000
5000
4000
3000
2000
1000
0
200
150
100
50
0
1
2
3
4
5
6
7
8
9
10
2700
71
2000
61
4900
164
pRS434GAP-*HMG1* /A451

Fig.15

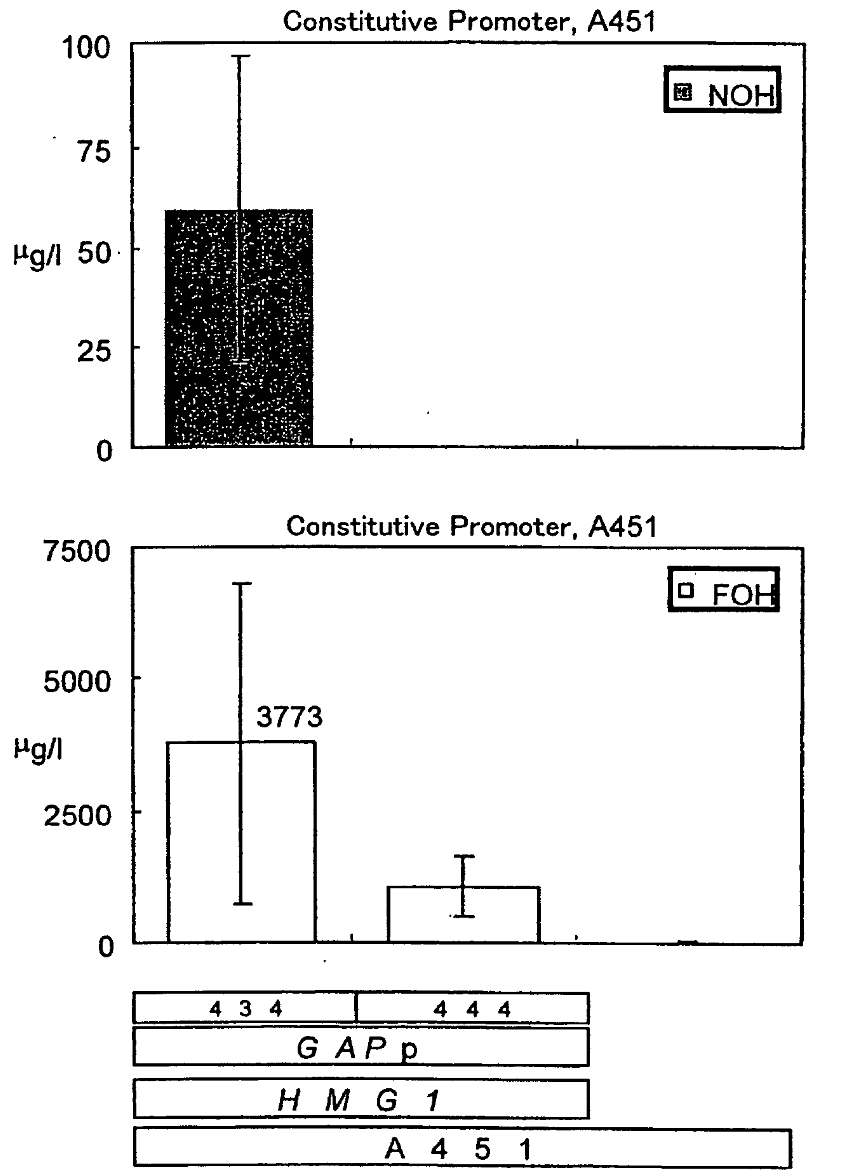
Constitutive Promoter, A451
100
75
μg/l 50
25
0
NOH
Constitutive Promoter, A451
7500
5000
μg/l
2500
0
FOH
3773
4 3 4
4 4 4
G A P p
H M G 1
A 4 5 1

Fig.16

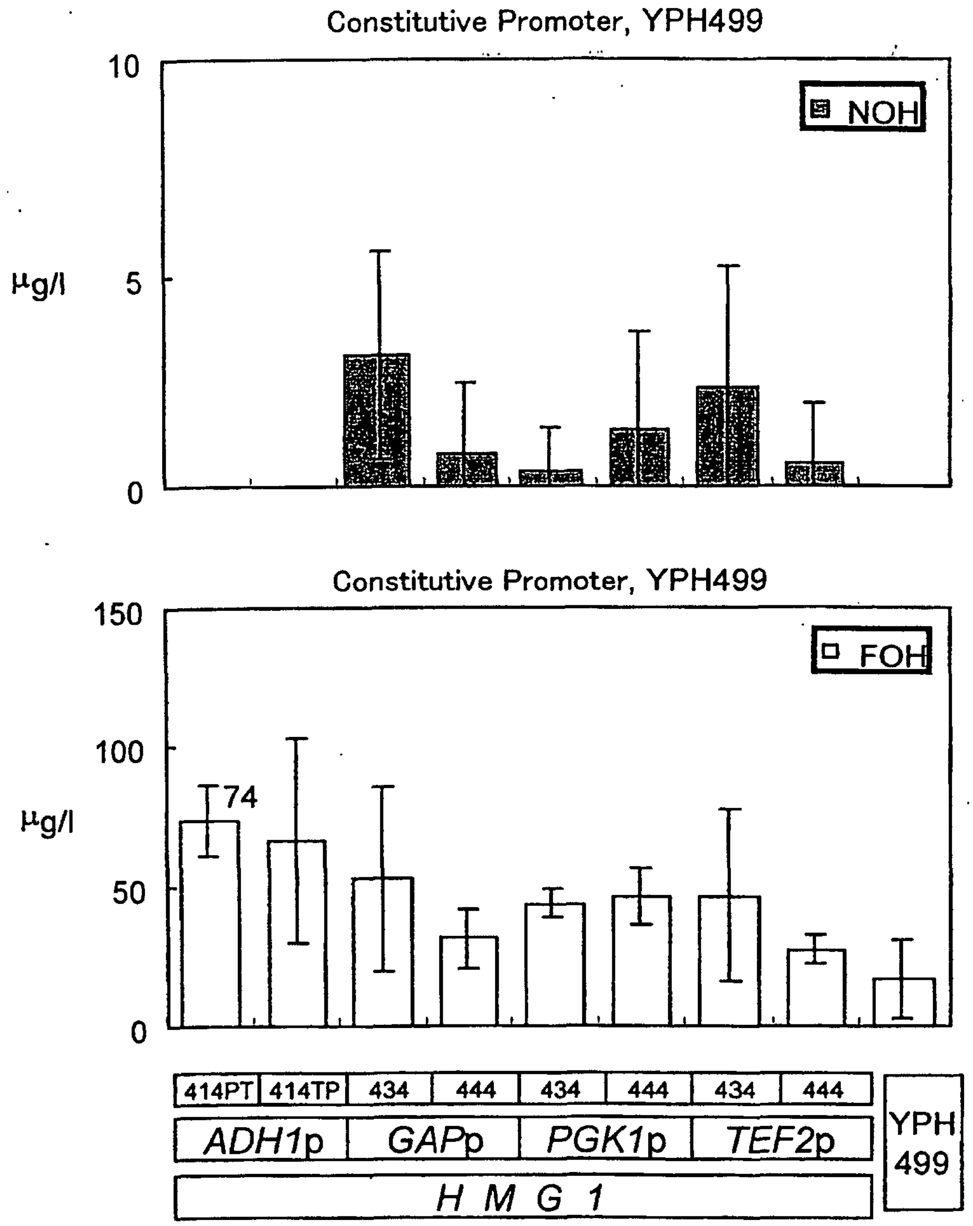

Constitutive Promoter, YPH499
NOH
μg/l
10
5
0
Constitutive Promoter, YPH499
FOH
μg/l
150
100
50
0
74
414PT
414TP
434
444
434
444
434
444
ADH1p
GAPp
PGK1p
TEF2p
HMG1
YPH 499

Fig.17

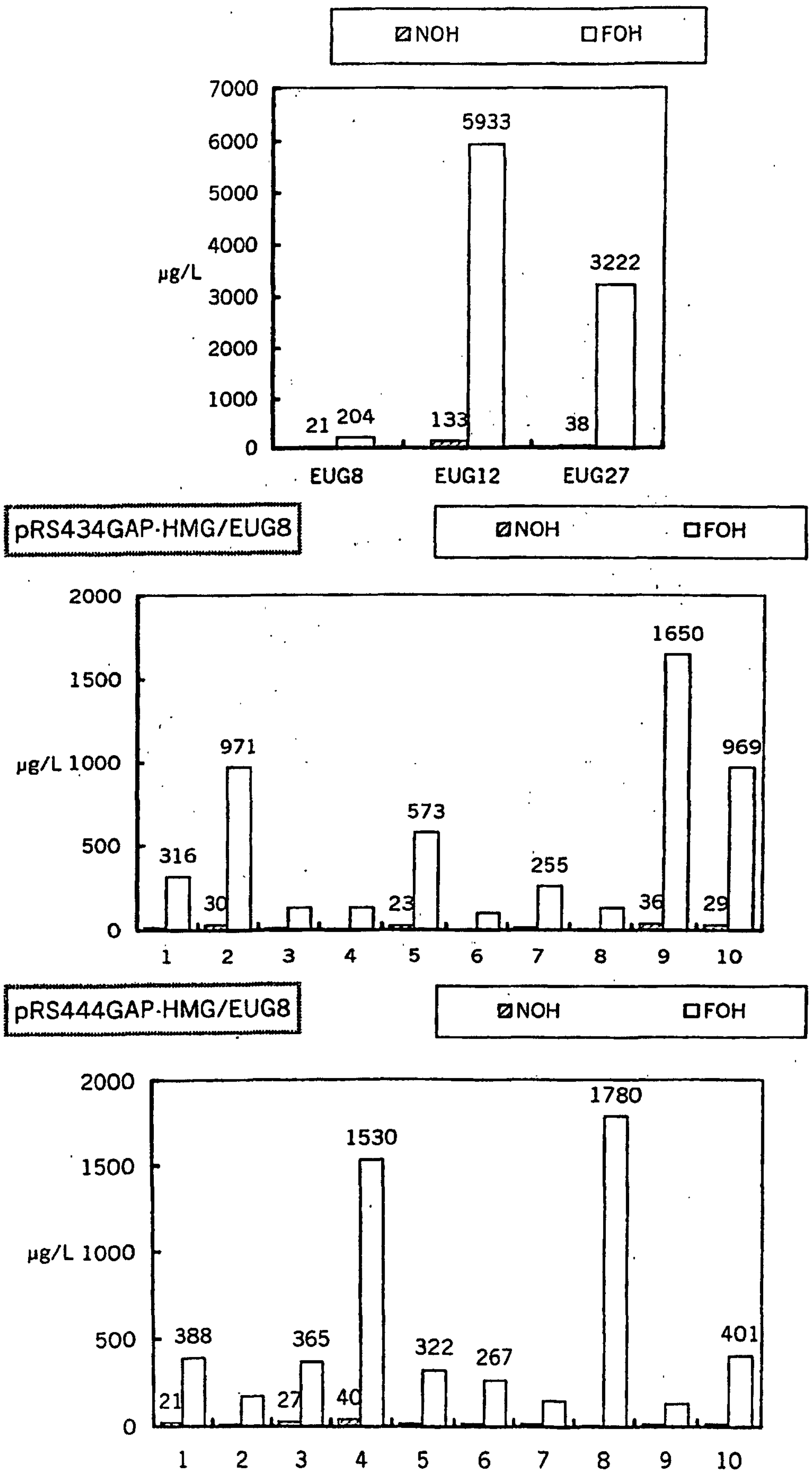
NOH
FOH
7000
6000
5000
4000
3000
2000
1000
0
μg/L
5933
3222
21
204
133
38
EUG8
EUG12
EUG27
pRS434GAP·HMG/EUG8
NOH
FOH
2000
1500
μg/L 1000
500
0
1650
971
969
573
316
255
30
23
36
29
1
2
3
4
5
6
7
8
9
10
pRS444GAP·HMG/EUG8
NOH
FOH
2000
1500
μg/L 1000
500
0
1780
1530
388
365
322
267
401
21
27
40
1
2
3
4
5
6
7
8
9
10

Fig.18

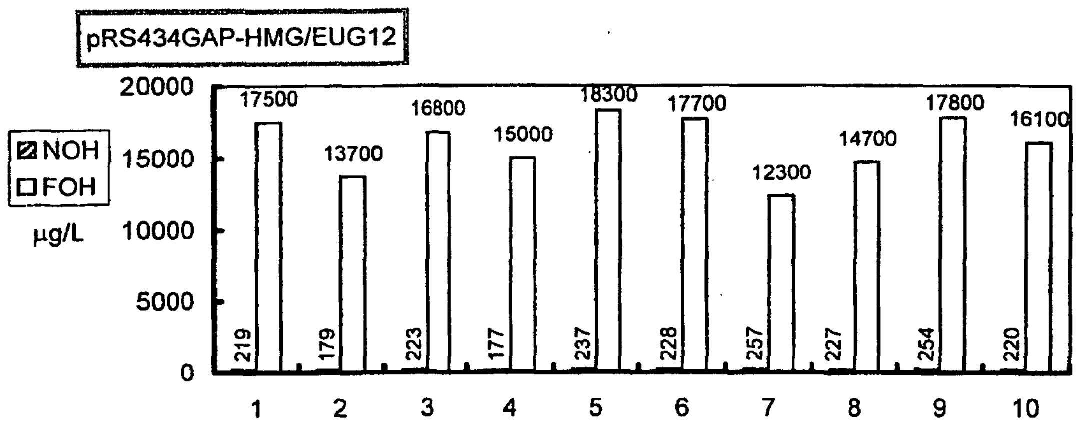
pRS434GAP-HMG/EUG12
NOH
FOH
μg/L
20000
15000
10000
5000
0
17500
13700
16800
15000
18300
17700
12300
14700
17800
16100
219
179
223
177
237
228
257
227
254
220
1
2
3
4
5
6
7
8
9
10

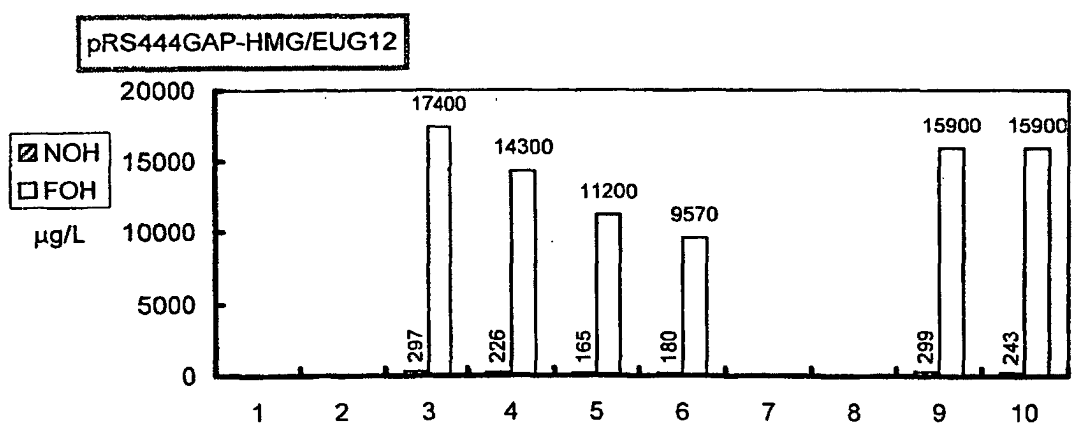
pRS444GAP-HMG/EUG12
NOH
FOH
μg/L
20000
15000
10000
5000
0
17400
14300
11200
9570
15900
15900
297
226
165
180
299
243
1
2
3
4
5
6
7
8
9
10

Fig.19

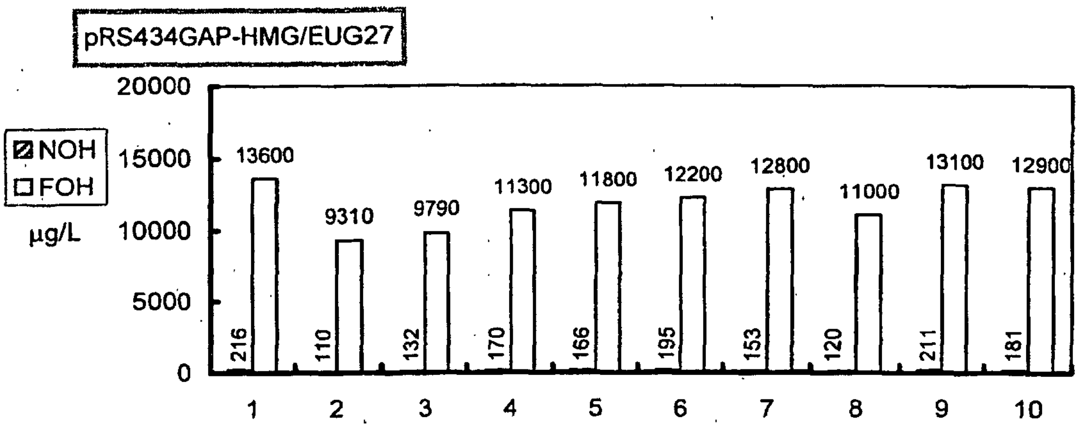
pRS434GAP-HMG/EUG27
NOH
FOH
µg/L
20000
15000
10000
5000
0
13600
9310
9790
11300
11800
12200
12800
11000
13100
12900
216
110
132
170
166
195
153
120
211
181
1
2
3
4
5
6
7
8
9
10

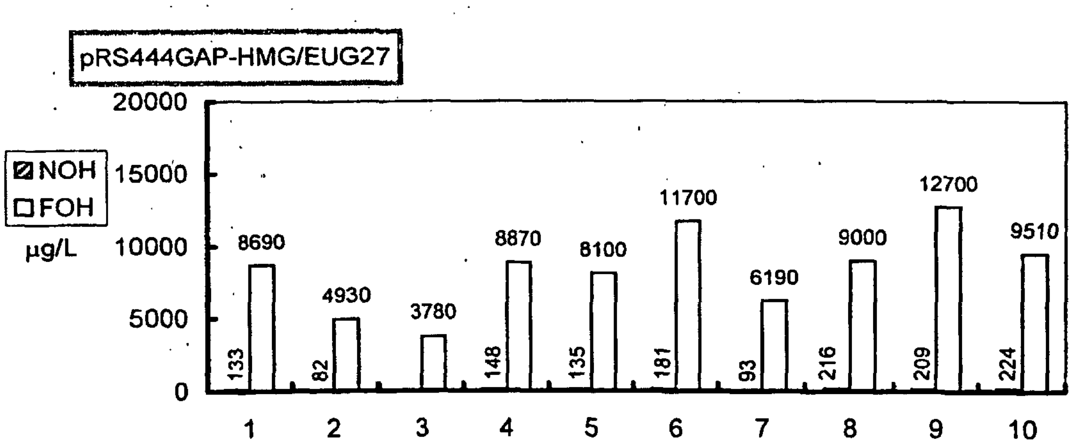
pRS444GAP-HMG/EUG27
NOH
FOH
µg/L
20000
15000
10000
5000
0
8690
4930
3780
8870
8100
11700
6190
9000
12700
9510
133
82
148
135
181
93
216
209
224
1
2
3
4
5
6
7
8
9
10

Fig.20A

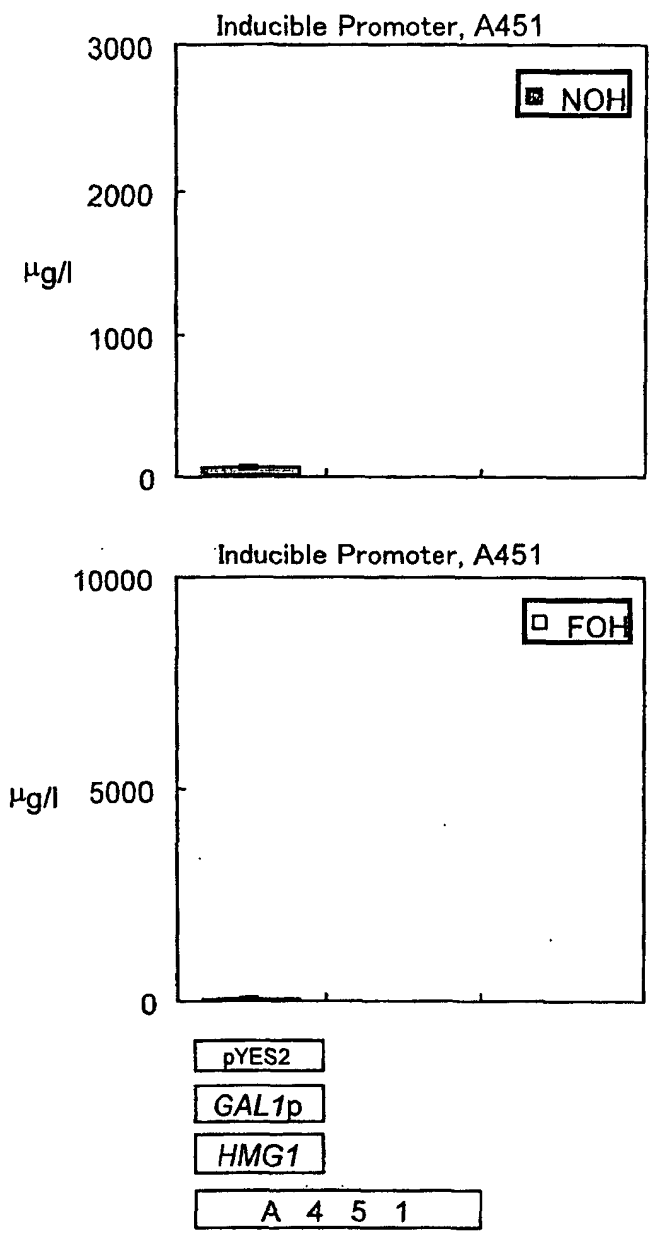
Inducible Promoter, A451
3000
2000
1000
0
μg/l
NOH
Inducible Promoter, A451
10000
5000
0
μg/l
FOH
pYES2
GAL1p
HMG1
A 4 5 1

Fig.20B

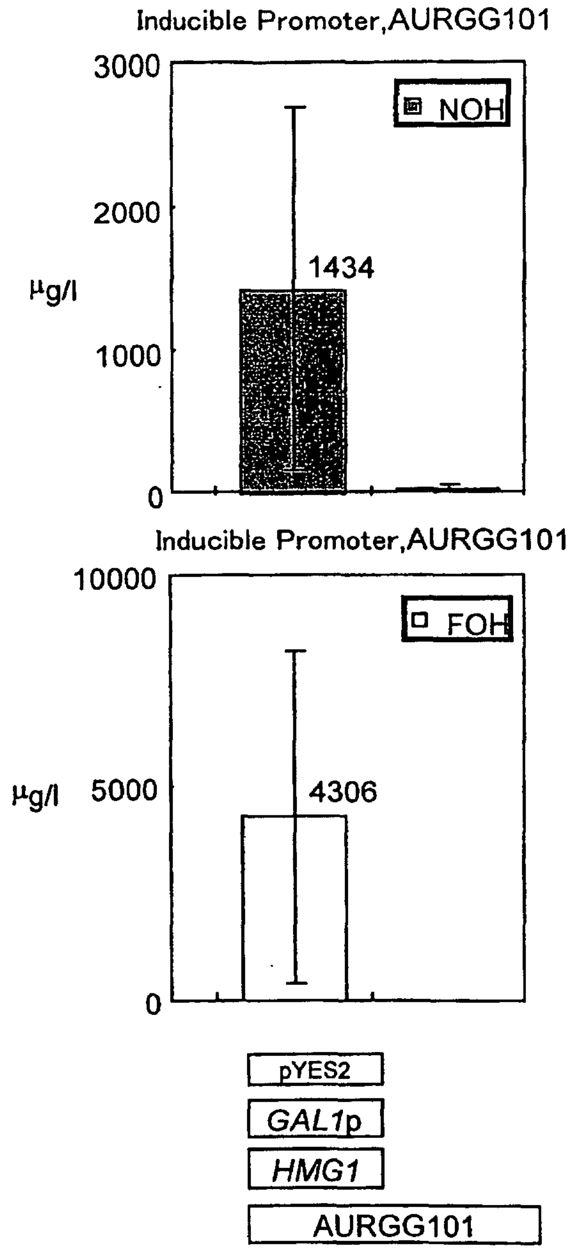
Inducible Promoter,AURGG101
3000
2000
1000
0
µg/l
NOH
1434
Inducible Promoter,AURGG101
10000
5000
0
µg/l
FOH
4306
pYES2
GAL1p
HMG1
AURGG101

Fig.21

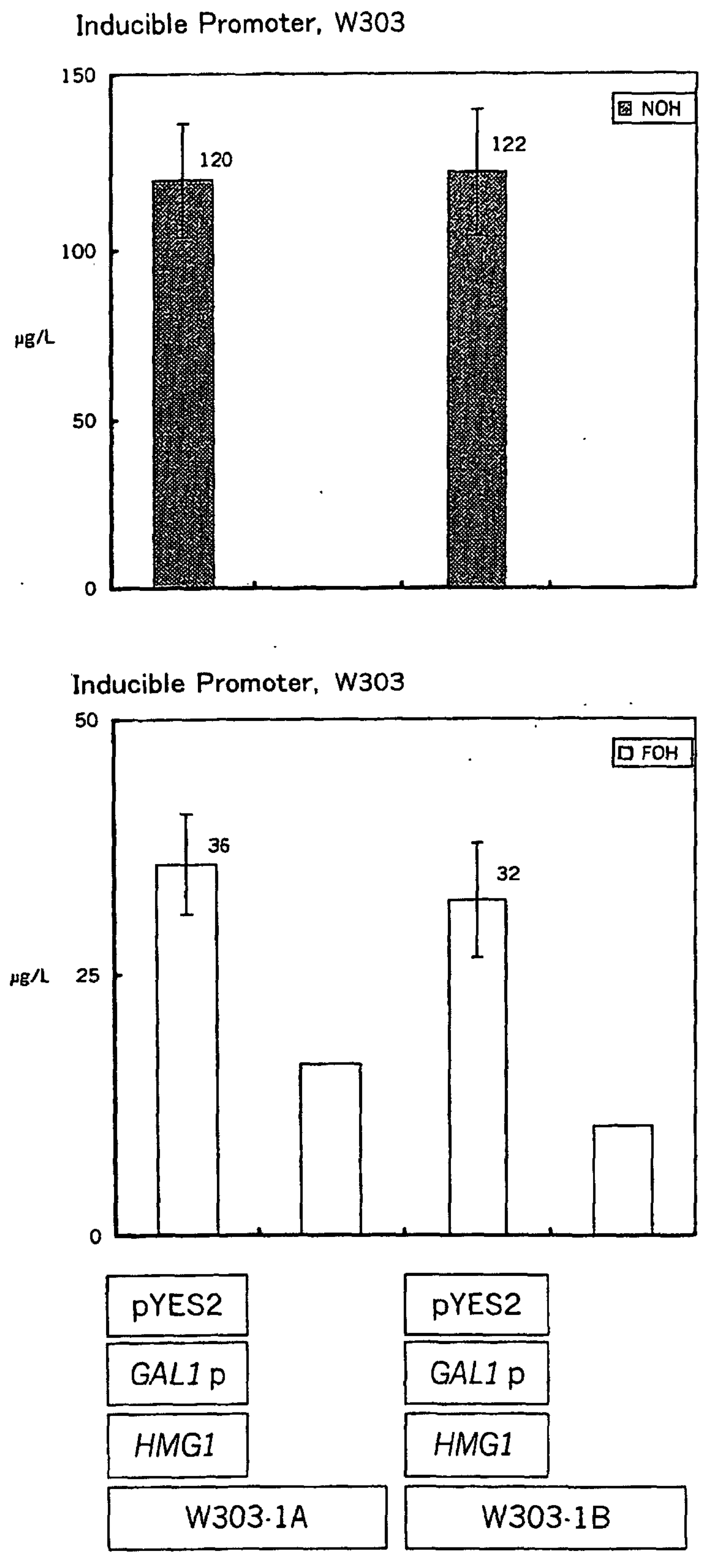
Inducible Promoter, W303
150
100
50
0
μg/L
NOH
120
122
Inducible Promoter, W303
50
25
0
μg/L
FOH
36
32
pYES2
GAL1 p
HMG1
W303-1A
pYES2
GAL1 p
HMG1
W303-1B

Fig.22

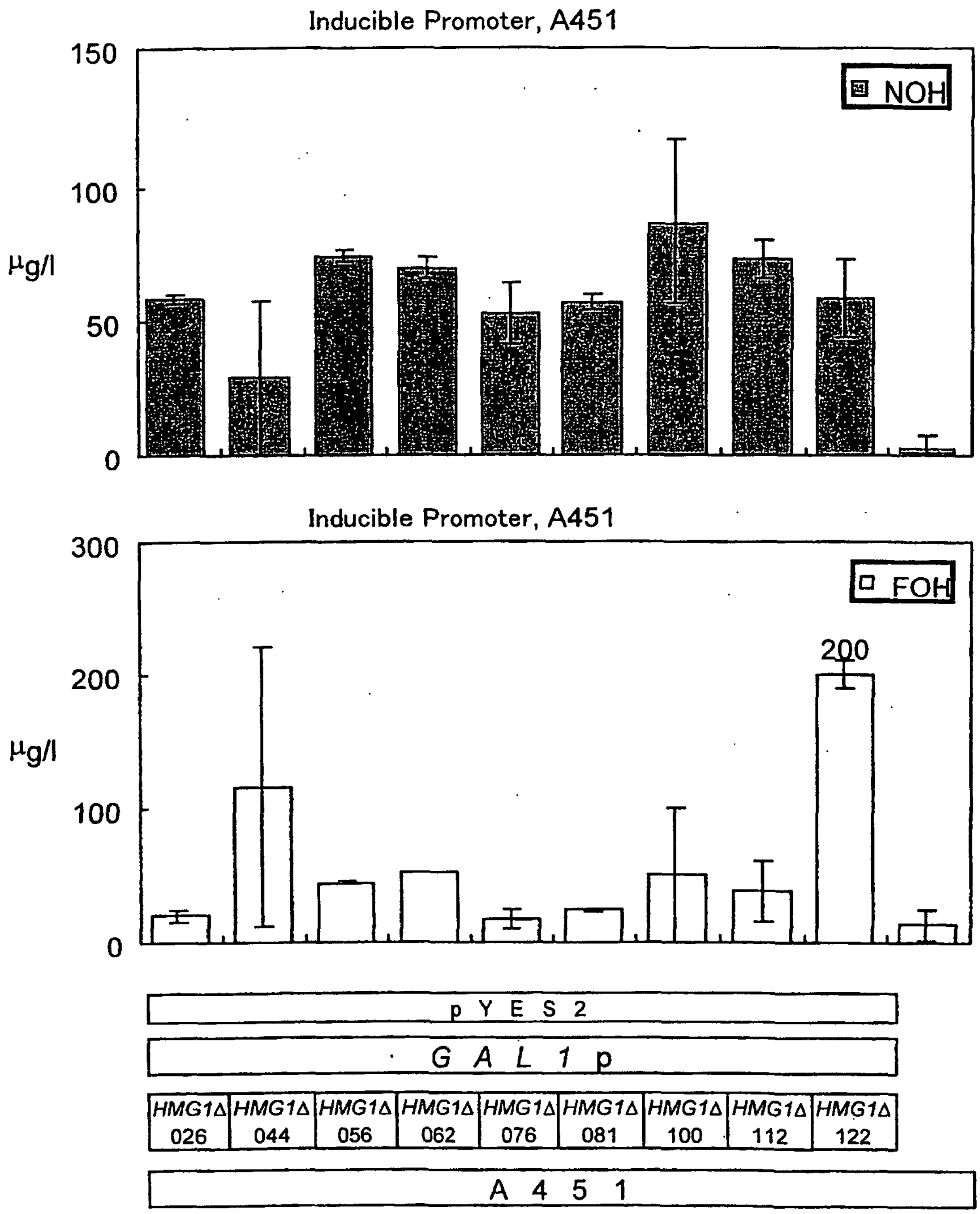
Inducible Promoter, A451
150
100
50
0
μg/l
NOH
Inducible Promoter, A451
300
200
100
0
μg/l
FOH
200
pYES2
GAL1p
HMG1Δ 026
HMG1Δ 044
HMG1Δ 056
HMG1Δ 062
HMG1Δ 076
HMG1Δ 081
HMG1Δ 100
HMG1Δ 112
HMG1Δ 122
A451

Fig.23

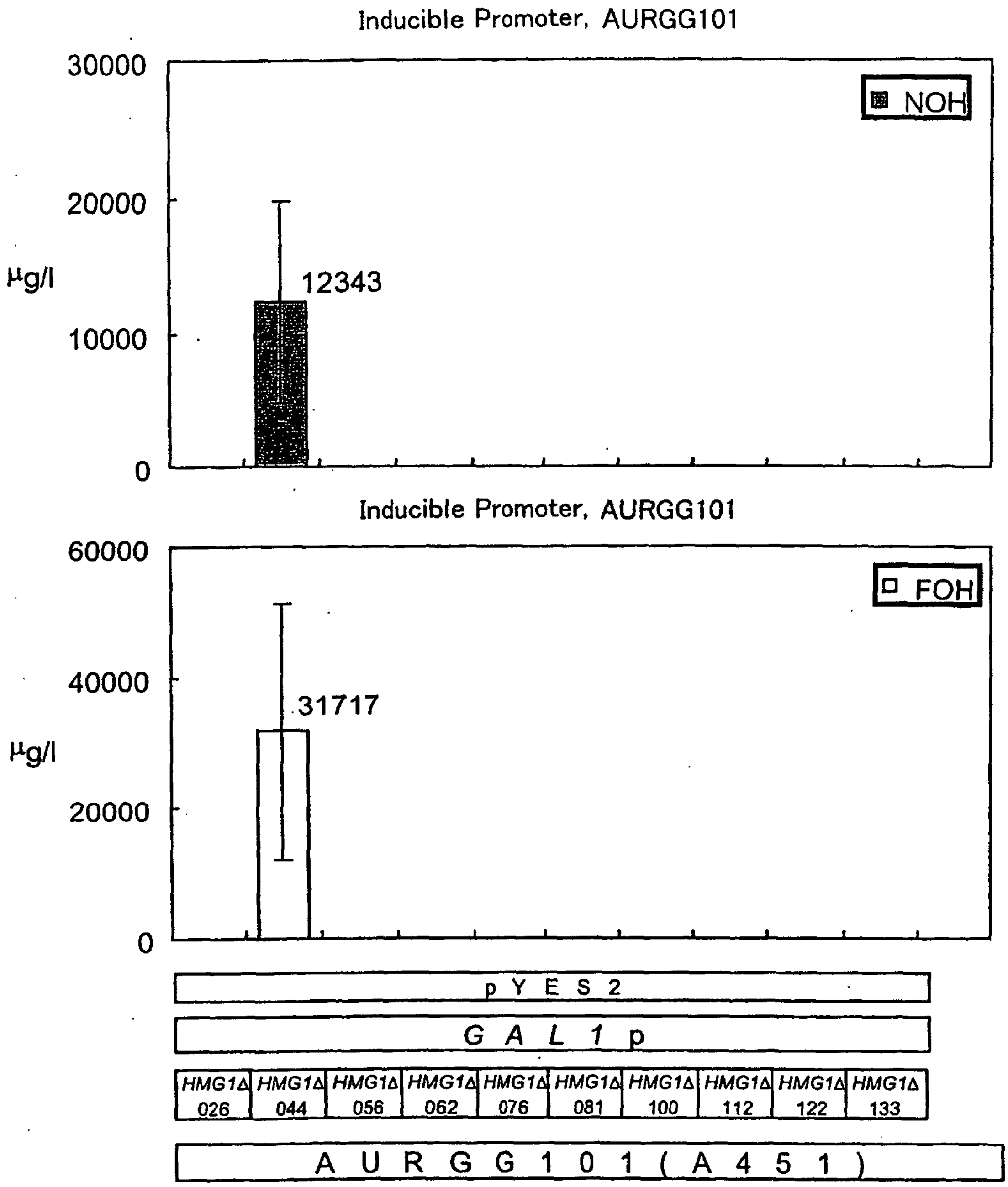
Inducible Promoter, AURGG101
30000
20000
10000
0
μg/l
12343
NOH
Inducible Promoter, AURGG101
60000
40000
20000
0
μg/l
31717
FOH
p Y E S 2
G A L 1 p
HMG1Δ 026
HMG1Δ 044
HMG1Δ 056
HMG1Δ 062
HMG1Δ 076
HMG1Δ 081
HMG1Δ 100
HMG1Δ 112
HMG1Δ 122
HMG1Δ 133
A U R G G 1 0 1 ( A 4 5 1 )

Fig.24

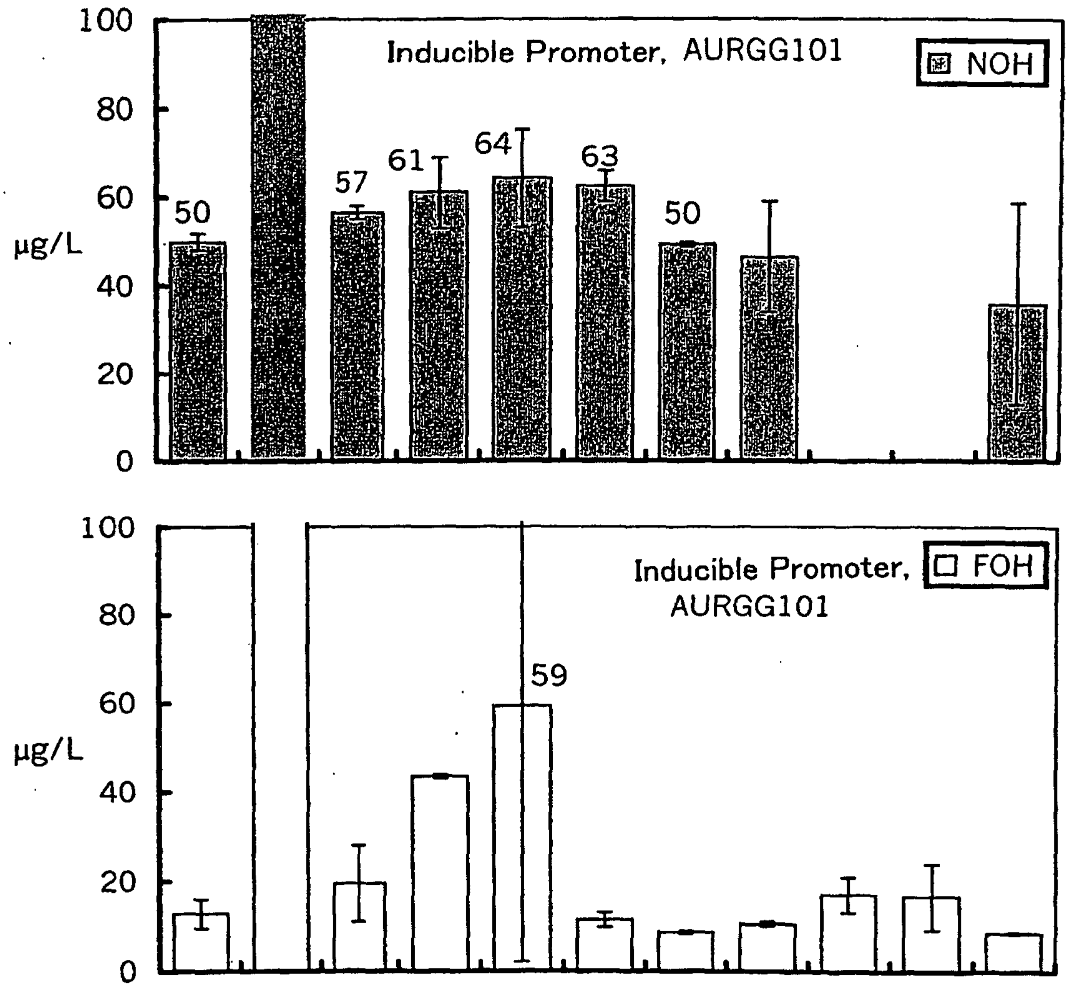
100
80
60
40
20
0
µg/L
Inducible Promoter, AURGG101
NOH
50
57
61
64
63
50
100
80
60
40
20
0
µg/L
Inducible Promoter,
AURGG101
FOH
59

Fig.25

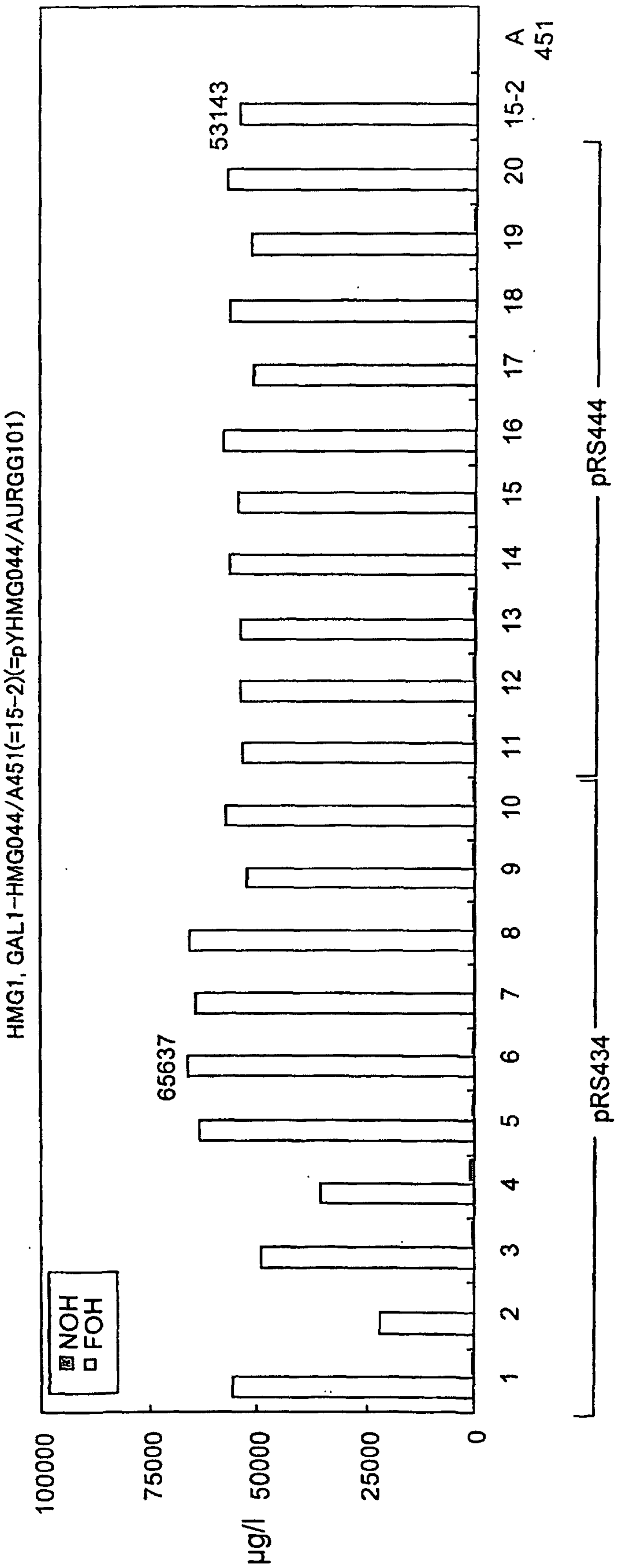
HMG1, GAL1-HMG044/A451(=15-2)(=pYHMG044/AURGG101)
NOH
FOH
µg/l
100000
75000
50000
25000
0
65637
53143
1
2
3
4
5
6
7
8
9
10
11
12
13
14
15
16
17
18
19
20
15-2
A
451
pRS434
pRS444

Fig.26

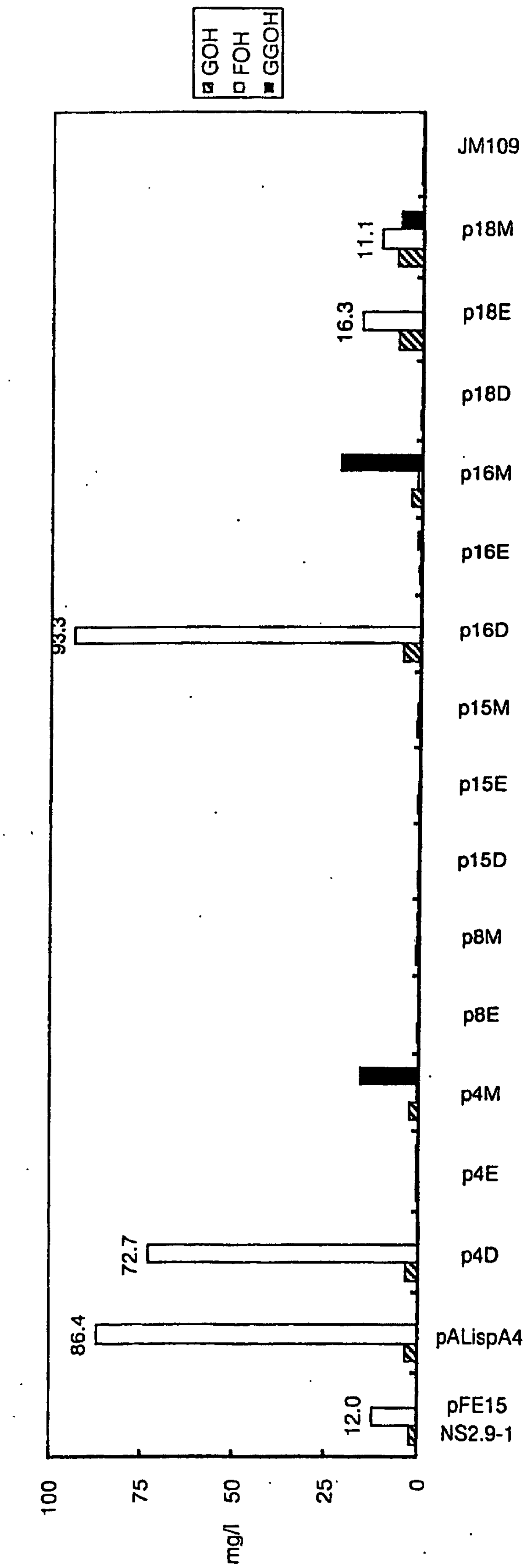
GOH
FOH
GGOH
JM109
p18M
11.1
p18E
16.3
p18D
p16M
p16E
p16D
93.3
p15M
p15E
p15D
p8M
p8E
p4M
p4E
p4D
72.7
pALispA4
86.4
pFE15
NS2.9-1
12.0
100
75
50
25
0
mg/l

Fig.27

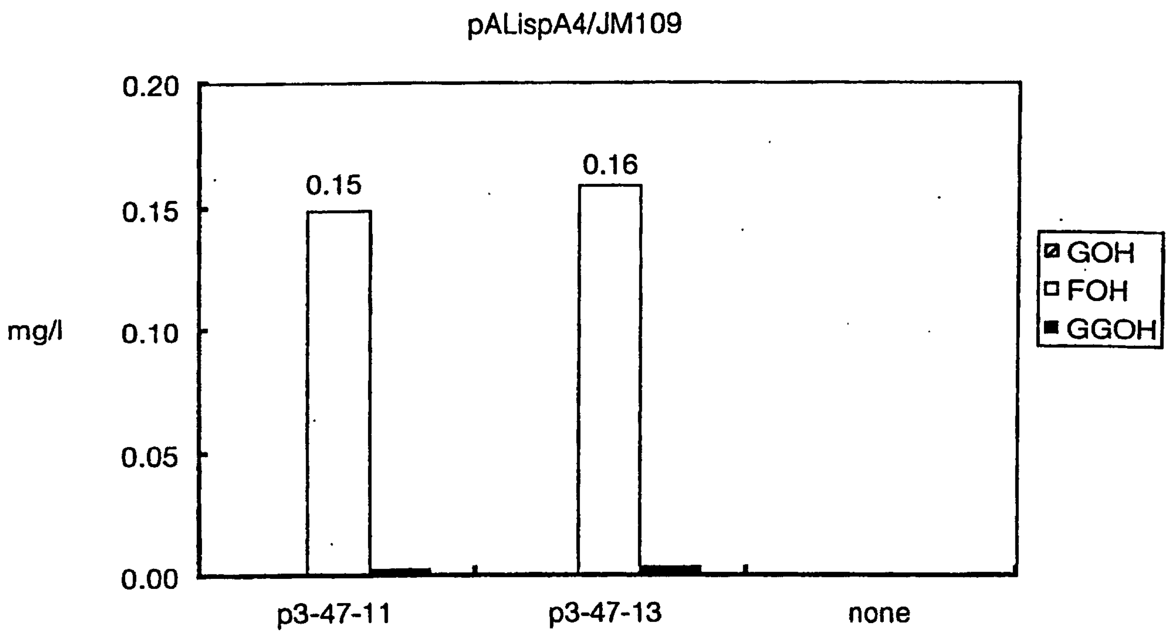
pALispA4/JM109
0.20
0.15
0.10
0.05
0.00
mg/l
0.15
0.16
p3-47-11
p3-47-13
none
GOH
FOH
GGOH

Fig.28

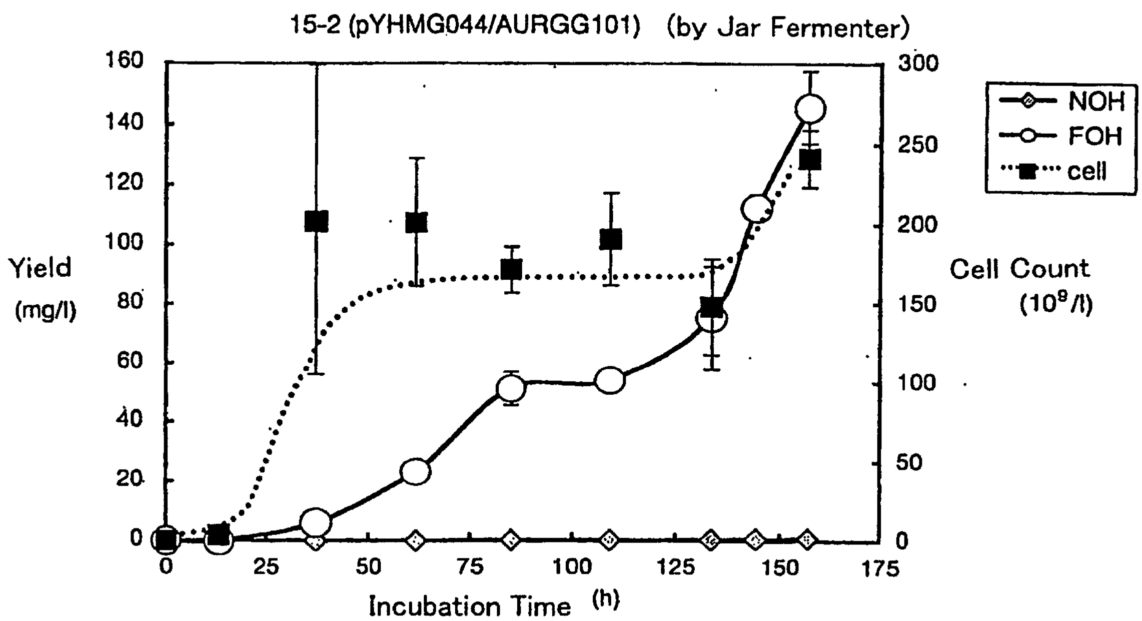
15-2 (pYHMG044/AURGG101) (by Jar Fermenter)
Yield (mg/l)
160
140
120
100
80
60
40
20
0
300
250
200
150
100
50
0
Cell Count (10⁹/l)
0
25
50
75
100
125
150
175
Incubation Time (h)
NOH
FOH
cell

## INTERNATIONAL SEARCH REPORT

International application No.
PCT/JP01/11213

**A. CLASSIFICATION OF SUBJECT MATTER**
Int.Cl[7] C12N15/52, C12P7/04, C12N1/19, C12N1/21

According to International Patent Classification (IPC) or to both national classification and IPC

**B. FIELDS SEARCHED**

Minimum documentation searched (classification system followed by classification symbols)
Int.Cl[7] C12N15/52, C12P7/04, C12N1/19, C12N1/21

Documentation searched other than minimum documentation to the extent that such documents are included in the fields searched

Electronic data base consulted during the international search (name of data base and, where practicable, search terms used)
WPIDS/BIOSIS/BIOTECHABS/CA(STN)

**C. DOCUMENTS CONSIDERED TO BE RELEVANT**

| Category* | Citation of document, with indication, where appropriate, of the relevant passages | Relevant to claim No. |
|---|---|---|
| A | Polakowski T. et al., Overexpression of a cytosolic hydroxymethyl-CoA-reductase leads to squalene accumulation in yeast; recombinant enzyme expression in Saccharomyces cerevisiae; metabolic engineering for use in ergosterol production, Appl. Microbiol. Biotechnol., 1998, Vol.49, No.1, pages 66 to 71 | 1-21 |
| A | WO, 00/01650, A1 (DCV, Inc. doing business as Bio-Technical Resouces), 30 January, 2000 (30.01.00), & EP 1095002 A1 & AU 9948630 A | 1-21 |
| A | Plochocka D. et al., The role of ERG20 gene (encoding yeast farnesyl diphosphate synthase) mutation in long dolichol formation. Molecular modeling of FPP synthase, Biochimie, 2000 Aug., Vol.82, No.8, pages 733 to 738 | 1-21 |

☒ Further documents are listed in the continuation of Box C. ☐ See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 05 April, 2002 (05.04.02) | 16 April, 2002 (16.04.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP01/11213

C (Continuation). DOCUMENTS CONSIDERED TO BE RELEVANT

| Category* | Citation of document, with indication, where appropriate, of the relevant passages | Relevant to claim No. |
|---|---|---|
| A | Blanchard L. et al., Characterization of a lysine-to-glutamic acid mutation in a conservative sequence of farnesyl diphosphate synthase from Saccharomyces cerevisiae, Gene, 1993, Vol.125, No.2, pages 185 to 189 | 1-21 |
| A | Chabon C. et al., Isolation and properties of yeast mutants affected in farnesyl diphosphate synthetase, Curr. Genet., 1990, Vol.18, pages 41 to 46 | 1-21 |

Form PCT/ISA/210 (continuation of second sheet) (July 1998)